# EUROPEAN PATENT APPLICATION

(11) **EP 2 116 616 A2**
(43) Date of publication of application: **11.11.2009**
(21) Application number: 09007584.7
(22) Date of filing: 10.09.2003
(51) Int. Cl.: C12Q 1/68

(54) **Genes differentially expressed in activated T cells and uses thereof**

(30) Priority: 11.09.2002 US 410174 P
(62) Divisional of application: 03749572.8
(71) Applicant: Genentech, Inc., South San Francisco CA 94080-4990 (US)
(72) Inventor: BODARY, Sarah C., Palo Alto, CA 94066 (US); CLARK, Hilary, San Francisco, CA 94134 (US); HUNTE, Brisdell, San Francisco, CA 94110 (US); JACKMAN, Janet K., Half Moon Bay, CA 94019 (US); SCHOENFELD, Jill R., Ashland, OR 97520 (US); WILLIAMS, P. Mickey, Half Moon Bay, CA 94019 (US); WOOD, William I., Hillsborough, CA 94010 (US); WU, Thomas D., San Francisco, CA 94110 (US)
(74) Representative: Walton, Seán Malcolm

(57) **Abstract**

The present invention relates to compositions containing novel proteins and methods of using those compositions for the diagnosis and treatment of immune related diseases.

## Description

### Field of the Invention

The present invention relates to compositions and methods useful for the diagnosis and treatment of immune related diseases.

### Background of the Invention

Immune related and inflammatory diseases are the manifestation or consequence of fairly complex, often multiple interconnected biological pathways which in normal physiology are critical to respond to insult or injury, initiate repair from insult or injury, and mount innate and acquired defense against foreign organisms. Disease or pathology occurs when these normal physiological pathways cause additional insult or injury either as directly related to the intensity of the response, as a consequence of abnormal regulation or excessive stimulation, as a reaction to self, or as a combination of these.

Though the genesis of these diseases often involves multistep pathways and often multiple different biological systems/pathways, intervention at critical points in one or more of these pathways can have an ameliorative or therapeutic effect. Therapeutic intervention can occur by either antagonism of a detrimental process/pathway or stimulation of a beneficial process/pathway.

Many immune related diseases are known and have been extensively studied. Such diseases include immune-mediated inflammatory diseases, non-immune-mediated inflammatory diseases, infectious diseases, immunodeficiency diseases, neoplasia, etc.

T lymphocytes (T cells) are an important component of a mammalian immune response. T cells recognize antigens which are associated with a self-molecule encoded by genes within the major histocompatibility complex (MHC). The antigen may be displayed together with MHC molecules on the surface of antigen presenting cells, virus infected cells, cancer cells, grafts, etc. The T cell system eliminates these altered cells which pose a health threat to the host mammal. T cells include helper T cells and cytotoxic T cells. Helper T cells proliferate extensively following recognition of an antigen -MHC complex on an antigen presenting cell. Helper T cells also secrete a variety of cytokines, *i*.*e*., lymphokines, which play a central role in the activation of B cells, cytotoxic T cells and a variety of other cells which participate in the immune response.

Immune related diseases could be treated by suppressing the immune response. Using neutralizing antibodies that inhibit molecules having immune stimulatory activity would be beneficial in the treatment of immune-mediated and inflammatory diseases. Molecules which inhibit the immune response can be utilized (proteins directly or via the use of antibody agonists) to inhibit the immune response and thus ameliorate immune related disease.

CD4+ T cells are known to be important regulators of inflammation. Herein, CD4+ T cells were activated and the profile of genes differentially expressed upon activation was analyzed. As such, the activation specific genes may be potential therapeutic targets. *In vivo* co-stimulation is necessary for a productive immune proliferative response. The list of costimulatory molecules is quite extensive and it is still unclear just which co-stimulatory molecules play critical roles in different types and stages of inflammation.

CD4+ T cells are known to be important regulators of inflammation. Herein, CD4+ T cells were activated and the profile of genes differentially expressed upon activation was analyzed. As such, the activation specific genes may be potential therapeutic targets. *In vivo* co-stimulation is necessary for a productive immune proliferative response. The list of costimulatory molecules is quite extensive and it is still unclear just which co-stimulatory molecules play critical roles in different types and stages of inflammation. In this application the focus is on genes which are specifically upregulated by stimulation with anti-CD3/ICAM, or anti-CD3/anti-CD28 and may be useful in targeting inflammatory processes which are associated with these different molecules.

Despite the above identified advances in T cell research, there is a great need for additional diagnostic and therapeutic agents capable of detecting the presence of a T cell mediated disorders in a mammal and for effectively reducing these disorders. Accordingly, it is an objective of the present invention to identify polypeptides that are overexpressed in activated T cells as compared to resting T cells, and to use those polypeptides, and their encoding nucleic acids, to produce compositions of matter useful in the therapeutic treatment and diagnostic detection of T cell mediated disorders in mammals.

### Summary of the Invention

### A. Embodiments

The present invention concerns compositions and methods useful for the diagnosis and treatment of immune related disease in mammals, including humans. The present invention is based on the identification of proteins (including agonist and antagonist antibodies) which are a result of stimulation of the immune response in mammals. Immune related diseases can be treated by suppressing or enhancing the immune response. Molecules that enhance the immune response stimulate or potentiate the immune response to an antigen. Molecules which stimulate the immune response can be used therapeutically where enhancement of the immune response would be beneficial. Alternatively, molecules that suppress the immune response attenuate or reduce the immune response to an antigen (*e*.*g*., neutralizing antibodies) can be used therapeutically where attenuation of the immune response would be beneficial (*e*.*g*., inflammation). Accordingly, the PRO polypeptides, agonists and antagonists thereof are also useful to prepare medicines and medicaments for the treatment of immune-related and inflammatory diseases. In a specific aspect, such medicines and medicaments comprise a therapeutically effective amount of a PRO polypeptide, agonist or antagonist thereof with a pharmaceutically acceptable carrier. Preferably, the admixture is sterile.

In a further embodiment, the invention concerns a method of identifying agonists or antagonists to a PRO polypeptide which comprises contacting the PRO polypeptide with a candidate molecule and monitoring a biological activity mediated by said PRO polypeptide. Preferably, the PRO polypeptide is a native sequence PRO polypeptide. In a specific aspect, the PRO agonist or antagonist is an anti-PRO antibody.

In another embodiment, the invention concerns a composition of matter comprising a PRO polypeptide or an agonist or antagonist antibody which binds the polypeptide in admixture with a carrier or excipient. In one aspect, the composition comprises a therapeutically effective amount of the polypeptide or antibody. In another aspect, when the composition comprises an immune stimulating molecule, the composition is useful for: (a) increasing infiltration of inflammatory cells into a tissue of a mammal in need thereof, (b) stimulating or enhancing an immune response in a mammal in need thereof, (c) increasing the proliferation of T-lymphocytes in a mammal in need thereof in response to an antigen, (d) stimulating the activity of T-lymphocytes or (e) increasing the vascular permeability. In a further aspect, when the composition comprises an immune inhibiting molecule, the composition is useful for: (a) decreasing infiltration of inflammatory cells into a tissue of a mammal in need thereof, (b) inhibiting or reducing an immune response in a mammal in need thereof, (c) decreasing the activity of T-lymphocytes or (d) decreasing the proliferation of T-lymphocytes in a mammal in need thereof in response to an antigen. In another aspect, the composition comprises a further active ingredient, which may, for example, be a further antibody or a cytotoxic or chemotherapeutic agent. Preferably, the composition is sterile.

In another embodiment, the invention concerns a method of treating an immune related disorder in a mammal in need thereof, comprising administering to the mammal an effective amount of a PRO polypeptide, an agonist thereof, or an antagonist thereto. In a preferred aspect, the immune related disorder is selected from the group consisting of: systemic lupus erythematosis, rheumatoid arthritis, osteoarthritis, juvenile chronic arthritis, spondyloarthropathies, systemic sclerosis, idiopathic inflammatory myopathies, Sjögren's syndrome, systemic vasculitis, sarcoidosis, autoimmune hemolytic anemia, autoimmune thrombocytopenia, thyroiditis, diabetes mellitus, immune-mediated renal disease, demyelinating diseases of the central and peripheral nervous systems such as multiple sclerosis, idiopathic demyelinating polyneuropathy or Guillain-Barré syndrome, and chronic inflammatory demyelinating polyneuropathy, hepatobiliary diseases such as infectious, autoimmune chronic active hepatitis, primary biliary cirrhosis, granulomatous hepatitis, and sclerosing cholangitis, inflammatory bowel disease, gluten-sensitive enteropathy, and Whipple's disease, autoimmune or immune-mediated skin diseases including bullous skin diseases, erythema multiforme and contact dermatitis, psoriasis, allergic diseases such as asthma, allergic rhinitis, atopic dermatitis, food hypersensitivity and urticaria, immunologic diseases of the lung such as eosinophilic pneumonias, idiopathic pulmonary fibrosis and hypersensitivity pneumonitis, transplantation associated diseases including graft rejection and graft -versus-host-disease.

In another embodiment, the invention provides an antibody which specifically binds to any of the above or below described polypeptides. Optionally, the antibody is a monoclonal antibody, humanized antibody, antibody fragment or single-chain antibody. In one aspect, the present invention concerns an isolated antibody which binds a PRO polypeptide. In another aspect, the antibody mimics the activity of a PRO polypeptide (an agonist antibody) or conversely the antibody inhibits or neutralizes the activity of a PRO polypeptide (an antagonist antibody). In another aspect, the antibody is a monoclonal antibody, which preferably has nonhuman complementarity determining region (CDR) residues and human framework region (FR) residues. The antibody may be labeled and may be immobilized on a solid support. In a further aspect, the antibody is an antibody fragment, a monoclonal antibody, a single-chain antibody, or an anti-idiotypic antibody.

In yet another embodiment, the present invention provides a composition comprising an anti-PRO antibody in admixture with a pharmaceutically acceptable carrier. In one aspect, the composition comprises a therapeutically effective amount of the antibody. Preferably, the composition is sterile. The composition may be administered in the form of a liquid pharmaceutical formulation, which may be preserved to achieve extended storage stability. Alternatively, the antibody is a monoclonal antibody, an antibody fragment, a humanized antibody, or a single-chain antibody.

In a further embodiment, the invention concerns an article of manufacture, comprising:
(a) a composition of matter comprising a PRO polypeptide or agonist or antagonist thereof;
(b) a container containing said composition; and
(c) a label affixed to said container, or a package insert included in said container referring to the use of said PRO polypeptide or agonist or antagonist thereof in the treatment of an immune related disease. The composition may comprise a therapeutically effective amount of the PRO polypeptide or the agonist or antagonist thereof.

In yet another embodiment, the present invention concerns a method of diagnosing an immune related disease in a mammal, comprising detecting the level of expression of a gene encoding a PRO polypeptide (a) in a test sample of tissue cells obtained from the mammal, and (b) in a control sample of known normal tissue cells of the same cell type, wherein a higher or lower expression level in the test sample as compared to the control sample indicates the presence of immune related disease in the mammal from which the test tissue cells were obtained.

In another embodiment, the present invention concerns a method of diagnosing an immune disease in a mammal, comprising (a) contacting an anti-PRO antibody with a test sample of tissue cells obtained from the mammal, and (b) detecting the formation of a complex between the antibody and a PRO polypeptide, in the test sample; wherein the formation of said complex is indicative of the presence or absence of said disease. The detection may be qualitative or quantitative, and may be performed in comparison with monitoring the complex formation in a control sample of known normal tissue cells of the same cell type. A larger quantity of complexes formed in the test sample indicates the presence or absence of an immune disease in the mammal from which the test tissue cells were obtained. The antibody preferably carries a detectable label. Complex formation can be monitored, for example, by light microscopy, flow cytometry, fluorimetry, or other techniques known in the art. The test sample is usually obtained from an individual suspected of having a deficiency or abnormality of the immune system.

In another embodiment, the invention provides a method for determining the presence of a PRO polypeptide in a sample comprising exposing a test sample of cells suspected of containing the PRO polypeptide to an anti-PRO antibody and determining the binding of said antibody to said cell sample. In a specific aspect, the sample comprises a cell suspected of containing the PRO polypeptide and the antibody binds to the cell. The antibody is preferably detectably labeled and/or bound to a solid support.

In another embodiment, the present invention concerns an immune-related disease diagnostic kit, comprising an anti-PRO antibody and a carrier in suitable packaging. The kit preferably contains instructions for using the antibody to detect the presence of the PRO polypeptide. Preferably the carrier is pharmaceutically acceptable.

In another embodiment, the present invention concerns a diagnostic kit, containing an anti-PRO antibody in suitable packaging. The kit preferably contains instructions for using the antibody to detect the PRO polypeptide.

In another embodiment, the invention provides a method of diagnosing an immune-related disease in a mammal which comprises detecting the presence or absence or a PRO polypeptide in a test sample of tissue cells obtained from said mammal, wherein the presence or absence of the PRO polypeptide in said test sample is indicative of the presence of an immune-related disease in said mammal.

In another embodiment, the present invention concerns a method for identifying an agonist of a PRO polypeptide comprising:
(a) contacting cells and a test compound to be screened under conditions suitable for the induction of a cellular response normally induced by a PRO polypeptide; and
(b) determining the induction of said cellular response to determine if the test compound is an effective agonist, wherein the induction of said cellular response is indicative of said test compound being an effective agonist.

In another embodiment, the invention concerns a method for identifying a compound capable of inhibiting the activity of a PRO polypeptide comprising contacting a candidate compound with a PRO polypeptide under conditions and for a time sufficient to allow these two components to interact and determining whether the activity of the PRO polypeptide is inhibited. In a specific aspect, either the candidate compound or the PRO polypeptide is immobilized on a solid support. In another aspect, the non-immobilized component carries a detectable label. In a preferred aspect, this method comprises the steps of:
(a) contacting cells and a test compound to be screened in the presence of a PRO polypeptide under conditions suitable for the induction of a cellular response normally induced by a PRO polypeptide; and
(b) determining the induction of said cellular response to determine if the test compound is an effective antagonist.

In another embodiment, the invention provides a method for identifying a compound that inhibits the expression of a PRO polypeptide in cells that normally express the polypeptide, wherein the method comprises contacting the cells with a test compound and determining whether the expression of the PRO polypeptide is inhibited. In a preferred aspect, this method comprises the steps of:
(a) contacting cells and a test compound to be screened under conditions suitable for allowing expression of the PRO polypeptide; and
(b) determining the inhibition of expression of said polypeptide.

In yet another embodiment, the present invention concerns a method for treating an immune-related disorder in a mammal that suffers therefrom comprising administering to the mammal a nucleic acid molecule that codes for either (a) a PRO polypeptide, (b) an agonist of a PRO polypeptide or (c) an antagonist of a PRO polypeptide, wherein said agonist or antagonist may be an anti-PRO antibody. In a preferred embodiment, the mammal is human. In another preferred embodiment, the nucleic acid is administered via *ex vivo* gene therapy. In a further preferred embodiment, the nucleic acid is comprised within a vector, more preferably an adenoviral, adeno-associated viral, lentiviral or retroviral vector.

In yet another aspect, the invention provides a recombinant viral particle comprising a viral vector consisting essentially of a promoter, nucleic acid encoding (a) a PRO polypeptide, (b) an agonist polypeptide of a PRO polypeptide, or (c) an antagonist polypeptide of a PRO polypeptide, and a signal sequence for cellular secretion of the polypeptide, wherein the viral vector is in association with viral structural proteins. Preferably, the signal sequence is from a mammal, such as from a native PRO polypeptide.

In a still further embodiment, the invention concerns an *ex vivo* producer cell comprising a nucleic acid construct that expresses retroviral structural proteins and also comprises a retroviral vector consisting essentially of a promoter, nucleic acid encoding (a) a PRO polypeptide, (b) an agonist polypeptide of a PRO polypeptide or (c) an antagonist polypeptide of a PRO polypeptide, and a signal sequence for cellular secretion of the polypeptide, wherein said producer cell packages the retroviral vector in association with the structural proteins to produce recombinant retroviral particles.

In a still further embodiment, the invention provides a method of increasing the activity of T-lymphocytes in a mammal comprising administering to said mammal (a) a PRO polypeptide, (b) an agonist of a PRO polypeptide, or (c) an antagonist of a PRO polypeptide, wherein the activity of T-lymphocytes in the mammal is increased.

In a still further embodiment, the invention provides a method of decreasing the activity of T-lymphocytes in a mammal comprising administering to said mammal (a) a PRO polypeptide, (b) an agonist of a PRO polypeptide, or (c) an antagonist of a PRO polypeptide, wherein the activity of T-lymphocytes in the mammal is decreased.

In a still further embodiment, the invention provides a method of increasing the proliferation of T-lymphocytes in a mammal comprising administering to said mammal (a) a PRO polypeptide, (b) an agonist of a PRO polypeptide, or (c) an antagonist of a PRO polypeptide, wherein the proliferation of T-lymphocytes in the mammal is increased.

In a still further embodiment, the invention provides a method of decreasing the proliferation of T-lymphocytes in a mammal comprising administering to said mammal (a) a PRO polypeptide, (b) an agonist of a PRO polypeptide, or (c) an antagonist of a PRO polypeptide, wherein the proliferation of T-lymphocytes in the mammal is decreased.

### B. Additional Embodiments

In other embodiments of the present invention, the invention provides vectors comprising DNA encoding any of the herein described polypeptides. Host cell comprising any such vector are also provided. By way of example, the host cells may be CHO cells, *E. coli*, or yeast. A process for producing any of the herein described polypeptides is further provided and comprises culturing host cells under conditions suitable for expression of the desired polypeptide and recovering the desired polypeptide from the cell culture.

In other embodiments, the invention provides chimeric molecules comprising any of the herein described polypeptides fused to a heterologous polypeptide or amino acid sequence. Example of such chimeric molecules comprise any of the herein described polypeptides fused to an epitope tag sequence or a Fc region of an immunoglobulin.

In another embodiment, the invention provides an antibody which specifically binds to any of the above or below described polypeptides. Optionally, the antibody is a monoclonal antibody, humanized antibody, antibody fragment or single-chain antibody.

In yet other embodiments, the invention provides oligonucleotide probes useful for isolating genomic and cDNA nucleotide sequences or as antisense probes, wherein those probes may be derived from any of the above or below described nucleotide sequences.

In other embodiments, the invention provides an isolated nucleic acid molecule comprising a nucleotide sequence that encodes a PRO polypeptide.

In one aspect, the isolated nucleic acid molecule comprises a nucleotide sequence having at least about 80% nucleic acid sequence identity, alternatively at least about 81% nucleic acid sequence identity, alternatively at least about 82% nucleic acid sequence identity, alternatively at least about 83% nucleic acid sequence identity, alternatively at least about 84% nucleic acid sequence identity, alternatively at least about 85% nucleic acid sequence identity, alternatively at least about 86% nucleic acid sequence identity, alternatively at least about 87% nucleic acid sequence identity, alternatively at least about 88% nucleic acid sequence identity, alternatively at least about 89% nucleic acid sequence identity, alternatively at least about 90% nucleic acid sequence identity, alternatively at least about 91 % nucleic acid sequence identity, alternatively at least about 92% nucleic acid sequence identity, alternatively at least about 93% nucleic acid sequence identity, alternatively at least about 94% nucleic acid sequence identity, alternatively at least about 95% nucleic acid sequence identity, alternatively at least about 96% nucleic acid sequence identity, alternatively at least about 97% nucleic acid sequence identity, alternatively at least about 98% nucleic acid sequence identity and alternatively at least about 99% nucleic acid sequence identity to (a) a DNA molecule encoding a PRO polypeptide having a full-length amino acid sequence as disclosed herein, an amino acid sequence lacking the signal peptide as disclosed herein, an extracellular domain of a transmembrane protein, with or without the signal peptide, as disclosed herein or any other specifically defined fragment of the full-length amino acid sequence as disclosed herein, or (b) the complement of the DNA molecule of (a).

In other aspects, the isolated nucleic acid molecule comprises a nucleotide sequence having at least about 80% nucleic acid sequence identity, alternatively at least about 81% nucleic acid sequence identity, alternatively at least about 82% nucleic acid sequence identity, alternatively at least about 83% nucleic acid sequence identity, alternatively at least about 84% nucleic acid sequence identity, alternatively at least about 85% nucleic acid sequence identity, alternatively at least about 86% nucleic acid sequence identity, alternatively at least about 87% nucleic acid sequence identity, alternatively at least about 88% nucleic acid sequence identity, alternatively at least about 89% nucleic acid sequence identity, alternatively at least about 90% nucleic acid sequence identity, alternatively at least about 91 % nucleic acid sequence identity, alternatively at least about 92% nucleic acid sequence identity, alternatively at least about 93% nucleic acid sequence identity, alternatively at least about 94% nucleic acid sequence identity, alternatively at least about 95% nucleic acid sequence identity, alternatively at least about 96% nucleic acid sequence identity, alternatively at least about 97% nucleic acid sequence identity, alternatively at least about 98% nucleic acid sequence identity and alternatively at least about 99% nucleic acid sequence identity to (a) a DNA molecule comprising the coding sequence of a full-length PRO polypeptide cDNA as disclosed herein, the coding sequence of a PRO polypeptide lacking the signal peptide as disclosed herein, the coding sequence of an extracellular domain of a transmembrane PRO polypeptide, with or without the signal peptide, as disclosed herein or the coding sequence of any other specifically defined fragment of the full-length amino acid sequence as disclosed herein, or (b) the complement of the DNA molecule of (a).

In a further aspect, the invention concerns an isolated nucleic acid molecule comprising a nucleotide sequence having at least about 80% nucleic acid sequence identity, alternatively at least about 81% nucleic acid sequence identity, alternatively at least about 82% nucleic acid sequence identity, alternatively at least about 83% nucleic acid sequence identity, alternatively at least about 84% nucleic acid sequence identity, alternatively at least about 85% nucleic acid sequence identity, alternatively at least about 86% nucleic acid sequence identity, alternatively at least about 87% nucleic acid sequence identity, alternatively at least about 88% nucleic acid sequence identity, alternatively at least about 89% nucleic acid sequence identity, alternatively at least about 90% nucleic acid sequence identity, alternatively at least about 91% nucleic acid sequence identity, alternatively at least about 92% nucleic acid sequence identity, alternatively at least about 93% nucleic acid sequence identity, alternatively at least about 94% nucleic acid sequence identity, alternatively at least about 95% nucleic acid sequence identity, alternatively at least about 96% nucleic acid sequence identity, alternatively at least about 97% nucleic acid sequence identity, alternatively at least about 98% nucleic acid sequence identity and alternatively at least about 99% nucleic acid sequence identity to (a) a DNA molecule that encodes the same mature polypeptide encoded by any of the human protein cDNAs as disclosed herein, or (b) the complement of the DNA molecule of (a).

Another aspect the invention provides an isolated nucleic acid molecule comprising a nucleotide sequence encoding a PRO polypeptide which is either transmembrane domain-deleted or transmembrane domain-inactivated, or is complementary to such encoding nucleotide sequence, wherein the transmembrane domain(s) of such polypeptide are disclosed herein. Therefore, soluble extracellular domains of the herein described PRO polypeptides are contemplated.

Another embodiment is directed to fragments of a PRO polypeptide coding sequence, or the complement thereof, that may find use as, for example, hybridization probes, for encoding fragments of a PRO polypeptide that may optionally encode a polypeptide comprising a binding site for an anti-PRO antibody or as antisense oligonucleotide probes. Such nucleic acid fragments are usually at least about 20 nucleotides in length, alternatively at least about 30 nucleotides in length, alternatively at least about 40 nucleotides in length, alternatively at least about 50 nucleotides in length, alternatively at least about 60 nucleotides in length, alternatively at least about 70 nucleotides in length, alternatively at least about 80 nucleotides in length, alternatively at least about 90 nucleotides in length, alternatively at least about 100 nucleotides in length, alternatively at least about 110 nucleotides in length, alternatively at least about 120 nucleotides in length, alternatively at least about 130 nucleotides in length, alternatively at least about 140 nucleotides in length, alternatively at least about 150 nucleotides in length, alternatively at least about 160 nucleotides in length, alternatively at least about 170 nucleotides in length, alternatively at least about 180 nucleotides in length, alternatively at least about 190 nucleotides in length, alternatively at least about 200 nucleotides in length, alternatively at least about 250 nucleotides in length, alternatively at least about 300 nucleotides in length, alternatively at least about 350 nucleotides in length, alternatively at least about 400 nucleotides in length, alternatively at least about 450 nucleotides in length, alternatively at least about 500 nucleotides in length, alternatively at least about 600 nucleotides in length, alternatively at least about 700 nucleotides in length, alternatively at least about 800 nucleotides in length, alternatively at least about 900 nucleotides in length and alternatively at least about 1000 nucleotides in length, wherein in this context the term "about" means the referenced nucleotide sequence length plus or minus 10% of that referenced length. It is noted that novel fragments of a PRO polypeptide-encoding nucleotide sequence may be determined in a routine manner by aligning the PRO polypeptide-encoding nucleotide sequence with other known nucleotide sequences using any of a number of well known sequence alignment programs and determining which PRO polypeptide-encoding nucleotide sequence fragment(s) are novel. All of such PRO polypeptide-encoding nucleotide sequences are contemplated herein. Also contemplated are the PRO polypeptide fragments encoded by these nucleotide molecule fragments, preferably those PRO polypeptide fragments that comprise a binding site for an anti-PRO antibody.

In another embodiment, the invention provides isolated PRO polypeptide encoded by any of the isolated nucleic acid sequences herein above identified.

In a certain aspect, the invention concerns an isolated PRO polypeptide, comprising an amino acid sequence having at least about 80% amino acid sequence identity, alternatively at least about 81% amino acid sequence identity, alternatively at least about 82% amino acid sequence identity, alternatively at least about 83% amino acid sequence identity, alternatively at least about 84% amino acid sequence identity, alternatively at least about 85% amino acid sequence identity, alternatively at least about 86% amino acid sequence identity, alternatively at least about 87% amino acid sequence identity, alternatively at least about 88% amino acid sequence identity, alternatively at least about 89% amino acid sequence identity, alternatively at least about 90% amino acid sequence identity, alternatively at least about 91% amino acid sequence identity, alternatively at least about 92% amino acid sequence identity, alternatively at least about 93% amino acid sequence identity, alternatively at least about 94% amino acid sequence identity, alternatively at least about 95% amino acid sequence identity, alternatively at least about 96% amino acid sequence identity, alternatively at least about 97% amino acid sequence identity, alternatively at least about 98% amino acid sequence identity and alternatively at least about 99% amino acid sequence identity to a PRO polypeptide having a full-length amino acid sequence as disclosed herein, an amino acid sequence lacking the signal peptide as disclosed herein, an extracellular domain of a transmembrane protein, with or without the signal peptide, as disclosed herein or any other specifically defined fragment of the full-length amino acid sequence as disclosed herein.

In a further aspect, the invention concerns an isolated PRO polypeptide comprising an amino acid sequence having at least about 80% amino acid sequence identity, alternatively at least about 81 % amino acid sequence identity, alternatively at least about 82% amino acid sequence identity, alternatively at least about 83% amino acid sequence identity, alternatively at least about 84% amino acid sequence identity, alternatively at least about 85% amino acid sequence identity, alternatively at least about 86% amino acid sequence identity, alternatively at least about 87% amino acid sequence identity, alternatively at least about 88% amino acid sequence identity, alternatively at least about 89% amino acid sequence identity, alternatively at least about 90% amino acid sequence identity, alternatively at least about 91% amino acid sequence identity, alternatively at least about 92% amino acid sequence identity, alternatively at least about 93% amino acid sequence identity, alternatively at least about 94% amino acid sequence identity, alternatively at least about 95% amino acid sequence identity, alternatively at least about 96% amino acid sequence identity, alternatively at least about 97% amino acid sequence identity, alternatively at least about 98% amino acid sequence identity and alternatively at least about 99% amino acid sequence identity to an amino acid sequence encoded by any of the human protein cDNAs as disclosed herein.

In a specific aspect, the invention provides an isolated PRO polypeptide without the N-terminal signal sequence and/or the initiating methionine and is encoded by a nucleotide sequence that encodes such an amino acid sequence as herein before described. Processes for producing the same are also herein described, wherein those processes comprise culturing a host cell comprising a vector which comprises the appropriate encoding nucleic acid molecule under conditions suitable for expression of the PRO polypeptide and recovering the PRO polypeptide from the cell culture.

Another aspect the invention provides an isolated PRO polypeptide which is either transmembrane domain-deleted or transmembrane domain-inactivated. Processes for producing the same are also herein described, wherein those processes comprise culturing a host cell comprising a vector which comprises the appropriate encoding nucleic acid molecule under conditions suitable for expression of the PRO polypeptide and recovering the PRO polypeptide from the cell culture.

In yet another embodiment, the invention concerns agonists and antagonists of a native PRO polypeptide as defined herein. In a particular embodiment, the agonist or antagonist is an anti-PRO antibody or a small molecule.

In a further embodiment, the invention concerns a method of identifying agonists or antagonists to a PRO polypeptide which comprise contacting the PRO polypeptide with a candidate molecule and monitoring a biological activity mediated by said PRO polypeptide. Preferably, the PRO polypeptide is a native PRO polypeptide.

In a still further embodiment, the invention concerns a composition of matter comprising a PRO polypeptide, or an agonist or antagonist of a PRO polypeptide as herein described, or an anti-PRO antibody, in combination with a carrier. Optionally, the carrier is a pharmaceutically acceptable carrier.

Another embodiment of the present invention is directed to the use of a PRO polypeptide, or an agonist or antagonist thereof as herein before described, or an anti-PRO antibody, for the preparation of a medicament useful in the treatment of a condition which is responsive to the PRO polypeptide, an agonist or antagonist thereof or an anti-PRO antibody.

### BRIEF DESCRIPTION OF THE DRAWINGS

Figure 1 shows a nucleotide sequence (SEQ ID NO:1) of a native sequence PRO83478 cDNA, wherein SEQ ID NO:1 is a clone designated herein as "DNA327205".
Figure 2 shows the amino acid sequence (SEQ ID NO:2) derived from the coding sequence of SEQ ID NO:1 shown in Figure 1.
Figure 3 shows a nucleotide sequence (SEQ ID NO:3) of a native sequence PR069889 cDNA, wherein SEQ ID NO:3 is a clone designated herein as "DNA304780".
Figure 4 shows the amino acid sequence (SEQ ID NO:4) derived from the coding sequence of SEQ ID NO:3 shown in Figure 3.
Figure 5 shows a nucleotide sequence (SEQ ID NO:5) of a native sequence PR037073 cDNA, wherein SEQ ID NO:5 is a clone designated herein as "DNA304459".
Figure 6 shows the amino acid sequence (SEQ ID NO:6) derived from the coding sequence of SEQ ID NO:5 shown in Figure 5.
Figure 7 shows a nucleotide sequence (SEQ ID NO:7) of a native sequence PRO4984 cDNA, wherein SEQ ID NO:7 is a clone designated herein as "DNA304460".
Figure 8 shows the amino acid sequence (SEQ ID NO:8) derived from the coding sequence of SEQ ID NO:7 shown in Figure 7.
Figure 9 shows a nucleotide sequence (SEQ ID NO:9) of a native sequence PR071039 cDNA, wherein SEQ ID NO:9 is a clone designated herein as "DNA304661".
Figure 10 shows the amino acid sequence (SEQ ID NO:10) derived from the coding sequence of SEQ ID NO:9 shown in Figure 9.
Figure 11 shows a nucleotide sequence (SEQ ID NO:11) of a native sequence PR071191 cDNA, wherein SEQ ID NO:11 is a clone designated herein as "DNA304781".
Figure 12 shows the amino acid sequence (SEQ ID NO:12) derived from the coding sequence of SEQ ID NO:11 shown in Figure 11.
Figure 13 shows a nucleotide sequence (SEQ ID NO:13) of a native sequence PRO271 cDNA, wherein SEQ ID NO:13 is a clone designated herein as "DNA327206".
Figure 14 shows the amino acid sequence (SEQ ID NO:14) derived from the coding sequence of SEQ ID NO:14 shown in Figure 14.
Figure 15A-B shows a nucleotide sequence (SEQ ID NO:15) of a native sequence PRO71042 cDNA, wherein SEQ ID NO:15 is a clone designated herein as "DNA304464".
Figure 16 shows the amino acid sequence (SEQ ID NO:16) derived from the coding sequence of SEQ ID NO:15 shown in Figure 15A-B.
Figure 17 shows a nucleotide sequence (SEQ ID NO:17) of a native sequence PR071242 cDNA, wherein SEQ ID NO:17 is a clone designated herein as "DNA304835".
Figure 18 shows the amino acid sequence (SEQ ID NO:18) derived from the coding sequence of SEQ ID NO:17 shown in Figure 17.
Figure 19 shows a nucleotide sequence (SEQ ID NO:19) of a native sequence PR06015 cDNA, wherein SEQ ID NO:19 is a clone designated herein as "DNA96866".
Figure 20 shows the amino acid sequence (SEQ ID NO:20) derived from the coding sequence of SEQ ID NO:19 shown in Figure 19.
Figure 21 shows a nucleotide sequence (SEQ ID NO:21) of a native sequence PR034336 cDNA, wherein SEQ ID NO:21 is a clone designated herein as "DNA304466".
Figure 22 shows the amino acid sequence (SEQ ID NO:22) derived from the coding sequence of SEQ ID NO:21 shown in Figure 21.
Figure 23 shows a nucleotide sequence (SEQ ID NO:23) of a native sequence PRO71043 cDNA, wherein SEQ ID NO:23 is a clone designated herein as "DNA304467".
Figure 24 shows the amino acid sequence (SEQ ID NO:24) derived from the coding sequence of SEQ ID NO:23 shown in Figure 23.
Figure 25 shows a nucleotide sequence (SEQ ID NO:25) of a native sequence PR071044 cDNA, wherein SEQ ID NO:25 is a clone designated herein as "DNA304468".
Figure 26 shows the amino acid sequence (SEQ ID NO:26) derived from the coding sequence of SEQ ID NO:25 shown in Figure 25.
Figure 27 shows a nucleotide sequence (SEQ ID NO:27) of a native sequence PR071045 cDNA, wherein SEQ ID NO:27 is a clone designated herein as "DNA304469".
Figure 28 shows the amino acid sequence (SEQ ID NO:28) derived from the coding sequence of SEQ ID NO:27 shown in Figure 27.
Figure 29 shows a nucleotide sequence (SEQ ID NO:29) of a native sequence PRO71046 cDNA, wherein SEQ ID NO:29 is a clone designated herein as "DNA304470".
Figure 30 shows the amino acid sequence (SEQ ID NO:30) derived from the coding sequence of SEQ ID NO:29 shown in Figure 29.
Figure 31 shows a nucleotide sequence (SEQ ID NO:31) of a native sequence PRO83479 cDNA, wherein SEQ ID NO:31 is a clone designated herein as "DNA327207".
Figure 32 shows the amino acid sequence (SEQ ID NO:32) derived from the coding sequence of SEQ ID NO:31 shown in Figure 31.
Figure 33 shows a nucleotide sequence (SEQ ID NO:33) of a native sequence PRO535 cDNA, wherein SEQ ID NO:33 is a clone designated herein as "DNA304472".
Figure 34 shows the amino acid sequence (SEQ ID NO:34) derived from the coding sequence of SEQ ID NO:33 shown in Figure 33.
Figure 35 shows a nucleotide sequence (SEQ ID NO:35) of a native sequence PR04426 cDNA, wherein SEQ ID NO:35 is a clone designated herein as "DNA304783".
Figure 36 shows the amino acid sequence (SEQ ID NO:36) derived from the coding sequence of SEQ ID NO:35 shown in Figure 35.
Figure 37 shows a nucleotide sequence (SEQ ID NO:37) of a native sequence PRO34447 cDNA, wherein SEQ ID NO:37 is a clone designated herein as "DNA218651 ".
Figure 38 shows the amino acid sequence (SEQ ID NO:38) derived from the coding sequence of SEQ ID NO:37 shown in Figure 37.
Figure 39 shows a nucleotide sequence (SEQ ID NO:39) of a native sequence PR02023 cDNA, wherein SEQ ID NO:39 is a clone designated herein as "DNA304473".
Figure 40 shows the amino acid sequence (SEQ ID NO:40) derived from the coding sequence of SEQ ID NO:39 shown in Figure 39.
Figure 41 shows a nucleotide sequence (SEQ ID NO:41) of a native sequence PR025349 cDNA, wherein SEQ ID NO:41 is a clone designated herein as "DNA189412".
Figure 42 shows the amino acid sequence (SEQ ID NO:42) derived from the coding sequence of SEQ ID NO:41 shown in Figure 41
Figure 43 shows a nucleotide sequence (SEQ ID NO:43) of a native sequence PRO1265 cDNA, wherein SEQ ID NO:43 is a clone designated herein as "DNA304827".
Figure 44 shows the amino acid sequence (SEQ ID NO:44) derived from the coding sequence of SEQ ID NO:43 shown in Figure 43.
Figure 45 shows a nucleotide sequence (SEQ ID NO:45) of a native sequence PR034298 cDNA, wherein SEQ ID NO:45 is a clone designated herein as "DNA217256".
Figure 46 shows the amino acid sequence (SEQ ID NO:46) derived from the coding sequence of SEQ ID NO:45 shown in Figure 45.
Figure 47 shows a nucleotide sequence (SEQ ID NO:47) of a native sequence PRO738 cDNA, wherein SEQ ID NO:47 is a clone designated herein as "DNA304784".
Figure 48 shows the amino acid sequence (SEQ ID NO:48) derived from the coding sequence of SEQ ID NO:47 shown in Figure 47.
Figure 49 shows a nucleotide sequence (SEQ ID NO:49) of a native sequence PR071049 cDNA, wherein SEQ ID NO:49 is a clone designated herein as "DNA304475".
Figure 50 shows the amino acid sequence (SEQ ID NO:50) derived from the coding sequence of SEQ ID NO:49 shown in Figure 49.
Figure 51 shows a nucleotide sequence (SEQ ID NO:51) of a native sequence PR021341 cDNA, wherein SEQ ID NO:51 is a clone designated herein as "DNA287180".
Figure 52 shows the amino acid sequence (SEQ ID NO:52) derived from the coding sequence of SEQ ID NO:51 shown in Figure 51.
Figure 53 shows a nucleotide sequence (SEQ ID NO:53) of a native sequence PRO1125 cDNA, wherein SEQ ID NO:53 is a clone designated herein as "DNA304476".
Figure 54 shows the amino acid sequence (SEQ ID NO:54) derived from the coding sequence of SEQ ID NO:53 shown in Figure 53.
Figure 55 shows a nucleotide sequence (SEQ ID NO:55) of a native sequence PR04369 cDNA, wherein SEQ ID NO:55 is a clone designated herein as "DNA327208".
Figure 56 shows the amino acid sequence (SEQ ID NO:56) derived from the coding sequence of SEQ ID NO:55 shown in Figure 55.
Figure 57 shows a nucleotide sequence (SEQ ID NO:57) of a native sequence PRO177 cDNA, wherein SEQ ID NO:57 is a clone designated herein as "DNA327209".
Figure 58 shows the amino acid sequence (SEQ ID NO:58) derived from the coding sequence of SEQ ID NO:57 shown in Figure 57.
Figure 59 shows a nucleotide sequence (SEQ ID NO:59) of a native sequence PRO83480 cDNA, wherein SEQ ID NO:59 is a clone designated herein as "DNA327210".
Figure 60 shows the amino acid sequence (SEQ ID NO:60) derived from the coding sequence of SEQ ID NO:59 shown in Figure 59.
Figure 61 shows a nucleotide sequence (SEQ ID NO:61) of a native sequence PRO71052 cDNA, wherein SEQ ID NO:61 is a clone designated herein as "DNA327211".
Figure 62 shows the amino acid sequence (SEQ ID NO:62) derived from the coding sequence of SEQ ID NO:61 shown in Figure 61.
Figure 63 shows a nucleotide sequence (SEQ ID NO:63) of a native sequence PRO37125 cDNA, wherein SEQ ID NO:63 is a clone designated herein as "DNA226662".
Figure 64 shows the amino acid sequence (SEQ ID NO:64) derived from the coding sequence of SEQ ID NO:63 shown in Figure 63.
Figure 65 shows a nucleotide sequence (SEQ ID NO:65) of a native sequence PRO83481 cDNA, wherein SEQ ID NO:65 is a clone designated herein as "DNA327212".
Figure 66 shows the amino acid sequence (SEQ ID NO:66) derived from the coding sequence of SEQ ID NO:65 shown in Figure 65.
Figure 67 shows a nucleotide sequence (SEQ ID NO:67) of a native sequence PR071054 cDNA, wherein SEQ ID NO:67 is a clone designated herein as "DNA304482".
Figure 68 shows the amino acid sequence (SEQ ID NO:68) derived from the coding sequence of SEQ ID NO:67 shown in Figure 67.
Figure 69 shows a nucleotide sequence (SEQ ID NO:69) of a native sequence PR069462 cDNA, wherein SEQ ID NO:69 is a clone designated herein as "DNA287171".
Figure 70 shows the amino acid sequence (SEQ ID NO:70) derived from the coding sequence of SEQ ID NO:69 shown in Figure 69.
Figure 71 shows a nucleotide sequence (SEQ ID NO:71) of a native sequence PRO268 cDNA, wherein SEQ ID NO:71 is a clone designated herein as "DNA304484".
Figure 72 shows the amino acid sequence (SEQ ID NO:72) derived from the coding sequence of SEQ ID NO:71 shown in Figure 71.
Figure 73 shows a nucleotide sequence (SEQ ID NO:73) of a native sequence PRO615 cDNA, wherein SEQ ID NO:73 is a clone designated herein as "DNA304485".
Figure 74 shows the amino acid sequence (SEQ ID NO:74) derived from the coding sequence of SEQ ID NO:73 shown in Figure 73.
Figure 75 shows a nucleotide sequence (SEQ ID NO:75) of a native sequence PRO25402 cDNA, wherein SEQ ID NO:75 is a clone designated herein as "DNA189504".
Figure 76 shows the amino acid sequence (SEQ ID NO:76) derived from the coding sequence of SEQ ID NO:75 shown in Figure 75.
Figure 77 shows a nucleotide sequence (SEQ ID NO:77) of a native sequence PRO71055 cDNA, wherein SEQ ID NO:77 is a clone designated herein as "DNA304486".
Figure 78 shows the amino acid sequence (SEQ ID NO:78) derived from the coding sequence of SEQ ID NO:77 shown in Figure 77.
Figure 79 shows a nucleotide sequence (SEQ ID NO:79) of a native sequence PRO71056 cDNA, wherein SEQ ID NO:79 is a clone designated herein as "DNA304487".
Figure 80 shows the amino acid sequence (SEQ ID NO:80) derived from the coding sequence of SEQ ID NO:79 shown in Figure 79.
Figure 81 shows a nucleotide sequence (SEQ ID NO:81) of a native sequence PR034332 cDNA, wherein SEQ ID NO:81 is a clone designated herein as "DNA218280".
Figure 82 shows the amino acid sequence (SEQ ID NO:82) derived from the coding sequence of SEQ ID NO:81 shown in Figure 81.
Figure 83 shows a nucleotide sequence (SEQ ID NO:83) of a native sequence PR071057 cDNA, wherein SEQ ID NO:83 is a clone designated herein as "DNA304488".
Figure 84 shows the amino acid sequence (SEQ ID NO:84) derived from the coding sequence of SEQ ID NO:83 shown in Figure 83.
Figure 85 shows a nucleotide sequence (SEQ ID NO:85) of a native sequence PR071058 cDNA, wherein SEQ ID NO:85 is a clone designated herein as "DNA304489".
Figure 86 shows the amino acid sequence (SEQ ID NO:86) derived from the coding sequence of SEQ ID NO:85 shown in Figure 85.
Figure 87 shows a nucleotide sequence (SEQ ID NO:87) of a native sequence PRO83482 cDNA, wherein SEQ ID NO:87 is a clone designated herein as "DNA327213".
Figure 88 shows the amino acid sequence (SEQ ID NO:88) derived from the coding sequence of SEQ ID NO:87 shown in Figure 87.
Figure 89 shows a nucleotide sequence (SEQ ID NO:89) of a native sequence PR034454 cDNA, wherein SEQ ID NO:89 is a clone designated herein as "DNA218676".
Figure 90 shows the amino acid sequence (SEQ ID NO:90) derived from the coding sequence of SEQ ID NO:89 shown in Figure 89.
Figure 91 shows a nucleotide sequence (SEQ ID NO:91) of a native sequence PR06243 cDNA, wherein SEQ ID NO:91 is a clone designated herein as "DNA304491".
Figure 92 shows the amino acid sequence (SEQ ID NO:92) derived from the coding sequence of SEQ ID NO:91 shown in Figure 91.
Figure 93 shows a nucleotide sequence (SEQ ID NO:93) of a native sequence PRO1864 cDNA, wherein SEQ ID NO:93 is a clone designated herein as "DNA304492".
Figure 94 shows the amino acid sequence (SEQ ID NO:94) derived from the coding sequence of SEQ ID NO:93 shown in Figure 93.
Figure 95 shows a nucleotide sequence (SEQ ID NO:95) of a native sequence PRO71060 cDNA, wherein SEQ ID NO:95 is a clone designated herein as "DNA304493".
Figure 96 shows the amino acid sequence (SEQ ID NO:96) derived from the coding sequence of SEQ ID NO:95 shown in Figure 95.
Figure 97 shows a nucleotide sequence (SEQ ID NO:97) of a native sequence PR071061 cDNA, wherein SEQ ID NO:97 is a clone designated herein as "DNA304494".
Figure 98 shows the amino acid sequence (SEQ ID NO:98) derived from the coding sequence of SEQ ID NO:97 shown in Figure 97.
Figure 99 shows a nucleotide sequence (SEQ ID NO:99) of a native sequence PRO793 cDNA, wherein SEQ ID NO:99 is a clone designated herein as "DNA304495".
Figure 100 shows the amino acid sequence (SEQ ID NO:100) derived from the coding sequence of SEQ ID NO:99 shown in Figure 99.
Figure 101 shows a nucleotide sequence (SEQ ID NO:101) of a native sequence PRO83483 cDNA, wherein SEQ ID NO:101 is a clone designated herein as "DNA327214".
Figure 102 shows the amino acid sequence (SEQ ID NO:102) derived from the coding sequence of SEQ ID NO:101 shown in Figure 101.
Figure 103 shows a nucleotide sequence (SEQ ID NO:103) of a native sequence PRO60929 cDNA, wherein SEQ ID NO:103 is a clone designated herein as "DNA272832".
Figure 104 shows the amino acid sequence (SEQ ID NO:104) derived from the coding sequence of SEQ ID NO:103 shown in Figure 103.

### DETAILED DESCRIPTION OF THE PREFERRED EMBODIMENTS

### I. Definitions

The terms "PRO polypeptide" and "PRO" as used herein and when immediately followed by a numerical designation refer to various polypeptides, wherein the complete designation (i.e., PRO/number) refers to specific polypeptide sequences as described herein. The terms "PRO/number polypeptide" and "PRO/number" wherein the term "number" is provided as an actual numerical designation as used herein encompass native sequence polypeptides and polypeptide variants (which are further defined herein). The PRO polypeptides described herein may be isolated from a variety of sources, such as from human tissue types or from another source, or prepared by recombinant or synthetic methods. The term "PRO polypeptide" refers to each individual PRO/number polypeptide disclosed herein. All disclosures in this specification which refer to the "PRO polypeptide" refer to each of the polypeptides individually as well as jointly. For example, descriptions of the preparation of, purification of, derivation of, formation of antibodies to or against, administration of, compositions containing, treatment of a disease with, etc., pertain to each polypeptide of the invention individually. The term "PRO polypeptide" also includes variants of the PRO/number polypeptides disclosed herein.

A "native sequence PRO polypeptide" comprises a polypeptide having the same amino acid sequence as the corresponding PRO polypeptide derived from nature. Such native sequence PRO polypeptides can be isolated from nature or can be produced by recombinant or synthetic means. The term "native sequence PRO polypeptide" specifically encompasses naturally-occurring truncated or secreted forms of the specific PRO polypeptide (*e*.*g*., an extracellular domain sequence), naturally-occurring variant forms (*e*.*g*., alternatively spliced forms) and naturally-occurring allelic variants of the polypeptide. In various embodiments of the invention, the native sequence PRO polypeptides disclosed herein are mature or full-length native sequence polypeptides comprising the full-length amino acids sequences shown in the accompanying figures. Start and stop codons are shown in bold font and underlined in the figures. However, while the PRO polypeptide disclosed in the accompanying figures are shown to begin with methionine residues designated herein as amino acid position 1 in the figures, it is conceivable and possible that other methionine residues located either upstream or downstream from the amino acid position 1 in the figures may be employed as the starting amino acid residue for the PRO polypeptides.

The PRO polypeptide "extracellular domain" or "ECD" refers to a form of the PRO polypeptide which is essentially free of the transmembrane and cytoplasmic domains. Ordinarily, a PRO polypeptide ECD will have less than 1% of such transmembrane and/or cytoplasmic domains and preferably, will have less than 0.5% of such domains. It will be understood that any transmembrane domains identified for the PRO polypeptides of the present invention are identified pursuant to criteria routinely employed in the art for identifying that type of hydrophobic domain. The exact boundaries of a transmembrane domain may vary but most likely by no more than about 5 amino acids at either end of the domain as initially identified herein. Optionally, therefore, an extracellular domain of a PRO polypeptide may contain from about 5 or fewer amino acids on either side of the transmembrane domain/extracellular domain boundary as identified in the Examples or specification and such polypeptides, with or without the associated signal peptide, and nucleic acid encoding them, are contemplated by the present invention.

The approximate location of the "signal peptides" of the various PRO polypeptides disclosed herein are shown in the present specification and/or the accompanying figures. It is noted, however, that the C-terminal boundary of a signal peptide may vary, but most likely by no more than about 5 amino acids on either side of the signal peptide C-terminal boundary as initially identified herein, wherein the C-terminal boundary of the signal peptide may be identified pursuant to criteria routinely employed in the art for identifying that type of amino acid sequence element (e.g., Nielsen et al., Prot. Eng. 10:1-6 (1997) and von Heinje et al., Nucl. Acids. Res. 14:4683-4690 (1986)). Moreover, it is also recognized that, in some cases, cleavage of a signal sequence from a secreted polypeptide is not entirely uniform, resulting in more than one secreted species. These mature polypeptides, where the signal peptide is cleaved within no more than about 5 amino acids on either side of the C-terminal boundary of the signal peptide as identified herein, and the polynucleotides encoding them, are contemplated by the present invention.

"PRO polypeptide variant" means an active PRO polypeptide as defined above or below having at least about 80% amino acid sequence identity with a full-length native sequence PRO polypeptide sequence as disclosed herein, a PRO polypeptide sequence lacking the signal peptide as disclosed herein, an extracellular domain of a PRO polypeptide, with or without the signal peptide, as disclosed herein or any other fragment of a full-length PRO polypeptide sequence as disclosed herein. Such PRO polypeptide variants include, for instance, PRO polypeptides wherein one or more amino acid residues are added, or deleted, at the N- or C-terminus of the full-length native amino acid sequence. Ordinarily, a PRO polypeptide variant will have at least about 80% amino acid sequence identity, alternatively at least about 81% amino acid sequence identity, alternatively at least about 82% amino acid sequence identity, alternatively at least about 83% amino acid sequence identity, alternatively at least about 84% amino acid sequence identity, alternatively at least about 85% amino acid sequence identity, alternatively at least about 86% amino acid sequence identity, alternatively at least about 87% amino acid sequence identity, alternatively at least about 88% amino acid sequence identity, alternatively at least about 89% amino acid sequence identity, alternatively at least about 90% amino acid sequence identity, alternatively at least about 91 % amino acid sequence identity, alternatively at least about 92% amino acid sequence identity, alternatively at least about 93% amino acid sequence identity, alternatively at least about 94% amino acid sequence identity, alternatively at least about 95% amino acid sequence identity, alternatively at least about 96% amino acid sequence identity, alternatively at least about 97% amino acid sequence identity, alternatively at least about 98% amino acid sequence identity and alternatively at least about 99% amino acid sequence identity to a full-length native sequence PRO polypeptide sequence as disclosed herein, a PRO polypeptide sequence lacking the signal peptide as disclosed herein, an extracellular domain of a PRO polypeptide, with or without the signal peptide, as disclosed herein or any other specifically defined fragment of a full-length PRO polypeptide sequence as disclosed herein. Ordinarily, PRO variant polypeptides are at least about 10 amino acids in length, alternatively at least about 20 amino acids in length, alternatively at least about 30 amino acids in length, alternatively at least about 40 amino acids in length, alternatively at least about 50 amino acids in length, alternatively at least about 60 amino acids in length, alternatively at least about 70 amino acids in length, alternatively at least about 80 amino acids in length, alternatively at least about 90 amino acids in length, alternatively at least about 100 amino acids in length, alternatively at least about 150 amino acids in length, alternatively at least about 200 amino acids in length, alternatively at least about 300 amino acids in length, or more.

"Percent (%) amino acid sequence identity" with respect to the PRO polypeptide sequences identified herein is defined as the percentage of amino acid residues in a candidate sequence that are identical with the amino acid residues in the specific PRO polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for measuring alignment, including any algorithms needed to achieve maximal alignment over the full length of the sequences being compared. For purposes herein, however, % amino acid sequence identity values are generated using the sequence comparison computer program ALIGN-2, wherein the complete source code for the ALIGN-2 program is provided in Table 1 below. The ALIGN-2 sequence comparison computer program was authored by Genentech, Inc. and the source code shown in Table 1 below has been filed with user documentation in the U.S. Copyright Office, Washington D.C., 20559, where it is registered under U.S. Copyright Registration No. TXU510087. The ALIGN-2 program is publicly available through Genentech, Inc., South San Francisco, California or may be compiled from the source code provided in Table 1 below. The ALIGN-2 program should be compiled for use on a UNIX operating system, preferably digital UNIX V4.0D. All sequence comparison parameters are set by the ALIGN-2 program and do not vary.

In situations where ALIGN-2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:
100 times the fraction X/Y
where X is the number of amino acid residues scored as identical matches by the sequence alignment program ALIGN-2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A. As examples of % amino acid sequence identity calculations using this method, Tables 2 and 3 demonstrate how to calculate the % amino acid sequence identity of the amino acid sequence designated "Comparison Protein" to the amino acid sequence designated "PRO", wherein "PRO" represents the amino acid sequence of a hypothetical PRO polypeptide of interest, "Comparison Protein" represents the amino acid sequence of a polypeptide against which the "PRO" polypeptide of interest is being compared, and "X, "Y" and "Z" each represent different hypothetical amino acid residues.

Unless specifically stated otherwise, all % amino acid sequence identity values used herein are obtained as described in the immediately preceding paragraph using the ALIGN-2 computer program. However, % amino acid sequence identity values may also be obtained as described below by using the WU-BLAST-2 computer program (Altschul et al., Methods in Enzymology 266:460-480 (1996)). Most of the WU-BLAST-2 search parameters are set to the default values. Those not set to default values, i.e., the adjustable parameters, are set with the following values: overlap span = 1, overlap fraction = 0.125, word threshold (T) = 11, and scoring matrix = BLOSUM62. When WU-BLAST-2 is employed, a % amino acid sequence identity value is determined by dividing (a) the number of matching identical amino acid residues between the amino acid sequence of the PRO polypeptide of interest having a sequence derived from the native PRO polypeptide and the comparison amino acid sequence of interest (i.e., the sequence against which the PRO polypeptide of interest is being compared which may be a PRO variant polypeptide) as determined by WU-BLAST-2 by (b) the total number of amino acid residues of the PRO polypeptide of interest. For example, in the statement "a polypeptide comprising an the amino acid sequence A which has or having at least 80% amino acid sequence identity to the amino acid sequence B", the amino acid sequence A is the comparison amino acid sequence of interest and the amino acid sequence B is the amino acid sequence of the PRO polypeptide of interest.

Percent amino acid sequence identity may also be determined using the sequence comparison program NCBI-BLAST2 (Altschul et al., Nucleic Acids Res. 25:3389-3402 (1997)). The NCBI-BLAST2 sequence comparison program may be downloaded from http://www.ncbi.nlm.nih.gov or otherwise obtained from the National Institute of Health, Bethesda, MD. NCBI-BLAST2 uses several search parameters, wherein all of those search parameters are set to default values including, for example, unmask = yes, strand = all, expected occurrences = 10, minimum low complexity length = 15/5, multi-pass e-value = 0.01, constant for multi-pass = 25, dropoff for final gapped alignment = 25 and scoring matrix = BLOSUM62.

In situations where NCBI-BLAST2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:
100 times the fraction X/Y
where X is the number of amino acid residues scored as identical matches by the sequence alignment program NCBI-BLAST2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A.

"PRO variant polynucleotide" or "PRO variant nucleic acid sequence" means a nucleic acid molecule which encodes an active PRO polypeptide as defined below and which has at least about 80% nucleic acid sequence identity with a nucleotide acid sequence encoding a full-length native sequence PRO polypeptide sequence as disclosed herein, a full-length native sequence PRO polypeptide sequence lacking the signal peptide as disclosed herein, an extracellular domain of a PRO polypeptide, with or without the signal peptide, as disclosed herein or any other fragment of a full-length PRO polypeptide sequence as disclosed herein. Ordinarily, a PRO variant polynucleotide will have at least about 80% nucleic acid sequence identity, alternatively at least about 81% nucleic acid sequence identity, alternatively at least about 82% nucleic acid sequence identity, alternatively at least about 83% nucleic acid sequence identity, alternatively at least about 84% nucleic acid sequence identity, alternatively at least about 85% nucleic acid sequence identity, alternatively at least about 86% nucleic acid sequence identity, alternatively at least about 87% nucleic acid sequence identity, alternatively at least about 88% nucleic acid sequence identity, alternatively at least about 89% nucleic acid sequence identity, alternatively at least about 90% nucleic acid sequence identity, alternatively at least about 91 % nucleic acid sequence identity, alternatively at least about 92% nucleic acid sequence identity, alternatively at least about 93% nucleic acid sequence identity, alternatively at least about 94% nucleic acid sequence identity, alternatively at least about 95% nucleic acid sequence identity, alternatively at least about 96% nucleic acid sequence identity, alternatively at least about 97% nucleic acid sequence identity, alternatively at least about 98% nucleic acid sequence identity and alternatively at least about 99% nucleic acid sequence identity with a nucleic acid sequence encoding a full-length native sequence PRO polypeptide sequence as disclosed herein, a full-length native sequence PRO polypeptide sequence lacking the signal peptide as disclosed herein, an extracellular domain of a PRO polypeptide, with or without the signal sequence, as disclosed herein or any other fragment of a full-length PRO polypeptide sequence as disclosed herein. Variants do not encompass the native nucleotide sequence.

Ordinarily, PRO variant polynucleotides are at least about 30 nucleotides in length, alternatively at least about 60 nucleotides in length, alternatively at least about 90 nucleotides in length, alternatively at least about 120 nucleotides in length, alternatively at least about 150 nucleotides in length, alternatively at least about 180 nucleotides in length, alternatively at least about 210 nucleotides in length, alternatively at least about 240 nucleotides in length, alternatively at least about 270 nucleotides in length, alternatively at least about 300 nucleotides in length, alternatively at least about 450 nucleotides in length, alternatively at least about 600 nucleotides in length, alternatively at least about 900 nucleotides in length, or more.

"Percent (%) nucleic acid sequence identity" with respect to PRO-encoding nucleic acid sequences identified herein is defined as the percentage of nucleotides in a candidate sequence that are identical with the nucleotides in the PRO nucleic acid sequence of interest, after aligning the sequences and introducing gaps, if necessary, to achieve the maximum percent sequence identity. Alignment for purposes of determining percent nucleic acid sequence identity can be achieved in various ways that are within the skill in the art, for instance, using publicly available computer software such as BLAST, BLAST-2, ALIGN or Megalign (DNASTAR) software. For purposes herein, however, % nucleic acid sequence identity values are generated using the sequence comparison computer program ALIGN-2, wherein the complete source code for the ALIGN-2 program is provided in Table 1 below. The ALIGN-2 sequence comparison computer program was authored by Genentech, Inc. and the source code shown in Table 1 below has been filed with user documentation in the U.S. Copyright Office, Washington D.C., 20559, where it is registered under U.S. Copyright Registration No. TXU510087. The ALIGN-2 program is publicly available through Genentech, Inc., South San Francisco, California or may be compiled from the source code provided in Table 1 below. The ALIGN-2 program should be compiled for use on a UNIX operating system, preferably digital UNIX V4.0D. All sequence comparison parameters are set by the ALIGN-2 program and do not vary.

In situations where ALIGN-2 is employed for nucleic acid sequence comparisons, the % nucleic acid sequence identity of a given nucleic acid sequence C to, with, or against a given nucleic acid sequence D (which can alternatively be phrased as a given nucleic acid sequence C that has or comprises a certain % nucleic acid sequence identity to, with, or against a given nucleic acid sequence D) is calculated as follows:
100 times the fraction W/Z
where W is the number of nucleotides scored as identical matches by the sequence alignment program ALIGN-2 in that program's alignment of C and D, and where Z is the total number of nucleotides in D. It will be appreciated that where the length of nucleic acid sequence C is not equal to the length of nucleic acid sequence D, the % nucleic acid sequence identity of C to D will not equal the % nucleic acid sequence identity of D to C. As examples of % nucleic acid sequence identity calculations, Tables 4 and 5, demonstrate how to calculate the % nucleic acid sequence identity of the nucleic acid sequence designated "Comparison DNA" to the nucleic acid sequence designated "PRO-DNA", wherein "PRO-DNA" represents a hypothetical PRO-encoding nucleic acid sequence of interest, "Comparison DNA" represents the nucleotide sequence of a nucleic acid molecule against which the "PRO-DNA" nucleic acid molecule of interest is being compared, and "N", "L" and "V" each represent different hypothetical nucleotides.

Unless specifically stated otherwise, all % nucleic acid sequence identity values used herein are obtained as described in the immediately preceding paragraph using the ALIGN-2 computer program. However, % nucleic acid sequence identity values may also be obtained as described below by using the WU-BLAST-2 computer program (Altschul et al., Methods in Enzymology 266:460-480 (1996)). Most of the WU-BLAST-2 search parameters are set to the default values. Those not set to default values, i.e., the adjustable parameters, are set with the following values: overlap span = 1, overlap fraction = 0.125, word threshold (T) = 11, and scoring matrix = BLOSUM62. When WU-BLAST-2 is employed, a % nucleic acid sequence identity value is determined by dividing (a) the number of matching identical nucleotides between the nucleic acid sequence of the PRO polypeptide-encoding nucleic acid molecule of interest having a sequence derived from the native sequence PRO polypeptide-encoding nucleic acid and the comparison nucleic acid molecule of interest (i.e., the sequence against which the PRO polypeptide-encoding nucleic acid molecule of interest is being compared which may be a variant PRO polynucleotide) as determined by WU-BLAST-2 by (b) the total number of nucleotides of the PRO polypeptide-encoding nucleic acid molecule of interest. For example, in the statement "an isolated nucleic acid molecule comprising a nucleic acid sequence A which has or having at least 80% nucleic acid sequence identity to the nucleic acid sequence B", the nucleic acid sequence A is the comparison nucleic acid molecule of interest and the nucleic acid sequence B is the nucleic acid sequence of the PRO polypeptide-encoding nucleic acid molecule of interest.

Percent nucleic acid sequence identity may also be determined using the sequence comparison program NCBI-BLAST2 (Altschul et al., Nucleic Acids Res. 25:3389-3402 (1997)). The NCBI-BLAST2 sequence comparison program may be downloaded from http://www.ncbi.nlm.nih.gov or otherwise obtained from the National Institute of Health, Bethesda, MD. NCBI-BLAST2 uses several search parameters, wherein all of those search parameters are set to default values including, for example, unmask = yes, strand = all, expected occurrences = 10, minimum low complexity length = 15/5, multi-pass e-value = 0.01, constant for multi-pass = 25, dropoff for final gapped alignment = 25 and scoring matrix = BLOSUM62.

In situations where NCBI-BLAST2 is employed for sequence comparisons, the % nucleic acid sequence identity of a given nucleic acid sequence C to, with, or against a given nucleic acid sequence D (which can alternatively be phrased as a given nucleic acid sequence C that has or comprises a certain % nucleic acid sequence identity to, with, or against a given nucleic acid sequence D) is calculated as follows:
100 times the fraction W/Z
where W is the number of nucleotides scored as identical matches by the sequence alignment program NCBI-BLAST2 in that program's alignment of C and D, and where Z is the total number of nucleotides in D. It will be appreciated that where the length of nucleic acid sequence C is not equal to the length of nucleic acid sequence D, the % nucleic acid sequence identity of C to D will not equal the % nucleic acid sequence identity of D to C.

In other embodiments, PRO variant polynucleotides are nucleic acid molecules that encode an active PRO polypeptide and which are capable of hybridizing, preferably under stringent hybridization and wash conditions, to nucleotide sequences encoding a full-length PRO polypeptide as disclosed herein. PRO variant polypeptides may be those that are encoded by a PRO variant polynucleotide.

"Isolated," when used to describe the various polypeptides disclosed herein, means polypeptide that has been identified and separated and/or recovered from a component of its natural environment. Contaminant components of its natural environment are materials that would typically interfere with diagnostic or therapeutic uses for the polypeptide, and may include enzymes, hormones, and other proteinaceous or non-proteinaceous solutes. In preferred embodiments, the polypeptide will be purified (1) to a degree sufficient to obtain at least 15 residues of N-terminal or internal amino acid sequence by use of a spinning cup sequenator, or (2) to homogeneity by SDS-PAGE under non-reducing or reducing conditions using Coomassie blue or, preferably, silver stain. Isolated polypeptide includes polypeptide *in situ* within recombinant cells, since at least one component of the PRO polypeptide natural environment will not be present. Ordinarily, however, isolated polypeptide will be prepared by at least one purification step.

An "isolated" PRO polypeptide-encoding nucleic acid or other polypeptide-encoding nucleic acid is a nucleic acid molecule that is identified and separated from at least one contaminant nucleic acid molecule with which it is ordinarily associated in the natural source of the polypeptide-encoding nucleic acid. An isolated polypeptide-encoding nucleic acid molecule is other than in the form or setting in which it is found in nature. Isolated polypeptide-encoding nucleic acid molecules therefore are distinguished from the specific polypeptide-encoding nucleic acid molecule as it exists in natural cells. However, an isolated polypeptide-encoding nucleic acid molecule includes polypeptide-encoding nucleic acid molecules contained in cells that ordinarily express the polypeptide where, for example, the nucleic acid molecule is in a chromosomal location different from that of natural cells.

The term "control sequences" refers to DNA sequences necessary for the expression of an operably linked coding sequence in a particular host organism. The control sequences that are suitable for prokaryotes, for example, include a promoter, optionally an operator sequence, and a ribosome binding site. Eukaryotic cells are known to utilize promoters, polyadenylation signals, and enhancers.

Nucleic acid is "operably linked" when it is placed into a functional relationship with another nucleic acid sequence. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous, and, in the case of a secretory leader, contiguous and in reading phase. However, enhancers do not have to be contiguous. Linking is accomplished by ligation at convenient restriction sites. If such sites do not exist, the synthetic oligonucleotide adaptors or linkers are used in accordance with conventional practice.

The term "antibody" is used in the broadest sense and specifically covers, for example, single anti-PRO monoclonal antibodies (including agonist, antagonist, and neutralizing antibodies), anti-PRO antibody compositions with polyepitopic specificity, single chain anti-PRO antibodies, and fragments of anti-PRO antibodies (see below). The term "monoclonal antibody" as used herein refers to an antibody obtained from a population of substantially homogeneous antibodies, i.e., the individual antibodies comprising the population are identical except for possible naturally-occurring mutations that may be present in minor amounts.

"Stringency" of hybridization reactions is readily determinable by one of ordinary skill in the art, and generally is an empirical calculation dependent upon probe length, washing temperature, and salt concentration. In general, longer probes require higher temperatures for proper annealing, while shorter probes need lower temperatures. Hybridization generally depends on the ability of denatured DNA to reanneal when complementary strands are present in an environment below their melting temperature. The higher the degree of desired homology between the probe and hybridizable sequence, the higher the relative temperature which can be used. As a result, it follows that higher relative temperatures would tend to make the reaction conditions more stringent, while lower temperatures less so. For additional details and explanation of stringency of hybridization reactions, see Ausubel et al., Current Protocols in Molecular Biology, Wiley Interscience Publishers, (1995).

"Stringent conditions" or "high stringency conditions", as defined herein, may be identified by those that: (1) employ low ionic strength and high temperature for washing, for example 0.015 M sodium chloride/0.0015 M sodium citrate/0.1% sodium dodecyl sulfate at 50°C; (2) employ during hybridization a denaturing agent, such as formamide, for example, 50% (v/v) formamide with 0.1% bovine serum albumin/0.1% Ficoll/0.1% polyvinylpyrrolidone/50mM sodium phosphate buffer at pH 6.5 with 750 mM sodium chloride, 75 mM sodium citrate at 42°C; or (3) employ 50% formamide, 5 x SSC (0.75 M NaCl, 0.075 M sodium citrate), 50 mM sodium phosphate (pH 6.8), 0.1% sodium pyrophosphate, 5 x Denhardt's solution, sonicated.salmon sperm DNA (50 µg/ml), 0.1% SDS, and 10% dextran sulfate at 42°C, with washes at 42°C in 0.2 x SSC (sodium chloride/sodium citrate) and 50% formamide at 55°C, followed by a high-stringency wash consisting of 0.1 x SSC containing EDTA at 55°C.

"Moderately stringent conditions" may be identified as described by Sambrook et al., Molecular Cloning: A Laboratory Manual, New York: Cold Spring Harbor Press, 1989, and include the use of washing solution and hybridization conditions (e.g., temperature, ionic strength and %SDS) less stringent that those described above. An example of moderately stringent conditions is overnight incubation at 37°C in a solution comprising: 20% formamide, 5 x SSC (150 mM NaCl, 15 mM trisodium citrate), 50 mM sodium phosphate (pH 7.6), 5 x Denhardt's solution, 10% dextran sulfate, and 20 mg/ml denatured sheared salmon sperm DNA, followed by washing the filters in 1 x SSC at about 37-50°C. The skilled artisan will recognize how to adjust the temperature, ionic strength, etc. as necessary to accommodate factors such as probe length and the like.

The term "epitope tagged" when used herein refers to a chimeric polypeptide comprising a PRO polypeptide fused to a "tag polypeptide". The tag polypeptide has enough residues to provide an epitope against which an antibody can be made, yet is short enough such that it does not interfere with activity of the polypeptide to which it is fused. The tag polypeptide preferably also is fairly unique so that the antibody does not substantially cross-react with other epitopes. Suitable tag polypeptides generally have at least six amino acid residues and usually between about 8 and 50 amino acid residues (preferably, between about 10 and 20 amino acid residues).

As used herein, the term "immunoadhesin" designates antibody-like molecules which combine the binding specificity of a heterologous protein (an "adhesin") with the effector functions of immunoglobulin constant domains. Structurally, the immunoadhesins comprise a fusion of an amino acid sequence with the desired binding specificity which is other than the antigen recognition and binding site of an antibody (i.e., is "heterologous"), and an immunoglobulin constant domain sequence. The adhesin part of an immunoadhesin molecule typically is a contiguous amino acid sequence comprising at least the binding site of a receptor or a ligand. The immunoglobulin constant domain sequence in the immunoadhesin may be obtained from any immunoglobulin, such as IgG-1, IgG-2, IgG-3, or IgG-4 subtypes, IgA (including IgA-1 and IgA-2), IgE, IgD or IgM.

"Active" or "activity" for the purposes herein refers to form(s) of a PRO polypeptide which retain a biological and/or an immunological activity of native or naturally-occurring PRO, wherein "biological" activity refers to a biological function (either inhibitory or stimulatory) caused by a native or naturally-occurring PRO other than the ability to induce the production of an antibody against an antigenic epitope possessed by a native or naturally-occurring PRO and an "immunological" activity refers to the ability to induce the production of an antibody against an antigenic epitope possessed by a native or naturally-occurring PRO.

The term "antagonist" is used in the broadest sense, and includes any molecule that partially or fully blocks, inhibits, or neutralizes a biological activity of a native PRO polypeptide disclosed herein. In a similar manner, the term "agonist" is used in the broadest sense and includes any molecule that mimics a biological activity of a native PRO polypeptide disclosed herein. Suitable agonist or antagonist molecules specifically include agonist or antagonist antibodies or antibody fragments, fragments or amino acid sequence variants of native PRO polypeptides, peptides, antisense oligonucleotides, small organic molecules, etc. Methods for identifying agonists or antagonists of a PRO polypeptide may comprise contacting a PRO polypeptide with a candidate agonist or antagonist molecule and measuring a detectable change in one or more biological activities normally associated with the PRO polypeptide.

"Treatment" refers to both therapeutic treatment and prophylactic or preventative measures, wherein the object is to prevent or slow down (lessen) the targeted pathologic condition or disorder. Those in need of treatment include those already with the disorder as well as those prone to have the disorder or those in whom the disorder is to be prevented.

"Chronic" administration refers to administration of the agent(s) in a continuous mode as opposed to an acute mode, so as to maintain the initial therapeutic effect (activity) for an extended period of time. "Intermittent" administration is treatment that is not consecutively done without interruption, but rather is cyclic in nature.

"Mammal" for purposes of treatment refers to any animal classified as a mammal, including humans, domestic and farm animals, and zoo, sports, or pet animals, such as dogs, cats, cattle, horses, sheep, pigs, goats, rabbits, etc. Preferably, the mammal is human.

Administration "in combination with" one or more further therapeutic agents includes simultaneous (concurrent) and consecutive administration in any order.

"Carriers" as used herein include pharmaceutically acceptable carriers, excipients, or stabilizers which are nontoxic to the cell or mammal being exposed thereto at the dosages and concentrations employed. Often the physiologically acceptable carrier is an aqueous pH buffered solution. Examples of physiologically acceptable carriers include buffers such as phosphate, citrate, and other organic acids; antioxidants including ascorbic acid; low molecular weight (less than about 10 residues) polypeptide; proteins, such as serum albumin, gelatin, or immunoglobulins; hydrophilic polymers such as polyvinylpyrrolidone; amino acids such as glycine, glutamine, asparagine, arginine or lysine; monosaccharides, disaccharides, and other carbohydrates including glucose, mannose, or dextrins; chelating agents such as EDTA; sugar alcohols such as mannitol or sorbitol; salt-forming counterions such as sodium; and/or nonionic surfactants such as TWEEN™, polyethylene glycol (PEG), and PLURONICS™.

"Antibody fragments" comprise a portion of an intact antibody, preferably the antigen binding or variable region of the intact antibody. Examples of antibody fragments include Fab, Fab', F(ab')₂, and Fv fragments; diabodies; linear antibodies (Zapata et al., Protein Eng. 8(10): 1057-1062 [1995]); single-chain antibody molecules; and multispecific antibodies formed from antibody fragments.

Papain digestion of antibodies produces two identical antigen-binding fragments, called "Fab" fragments, each with a single antigen-binding site, and a residual "Fc" fragment, a designation reflecting the ability to crystallize readily. Pepsin treatment yields an F(ab')₂ fragment that has two antigen-combining sites and is still capable of cross-linking antigen.

"Fv" is the minimum antibody fragment which contains a complete antigen-recognition and - binding site. This region consists of a dimer of one heavy- and one light-chain variable domain in tight, non-covalent association. It is in this configuration that the three CDRs of each variable domain interact to define an antigen-binding site on the surface of the V_{H}-V_{L} dimer. Collectively, the six CDRs confer antigen-binding specificity to the antibody. However, even a single variable domain (or half of an Fv comprising only three CDRs specific for an antigen) has the ability to recognize and bind antigen, although at a lower affinity than the entire binding site.

The Fab fragment also contains the constant domain of the light chain and the first constant domain (CH1) of the heavy chain. Fab fragments differ from Fab' fragments by the addition of a few residues at the carboxy terminus of the heavy chain CH1 domain including one or more cysteines from the antibody hinge region. Fab'-SH is the designation herein for Fab' in which the cysteine residue(s) of the constant domains bear a free thiol group. F(ab')₂ antibody fragments originally were produced as pairs of Fab' fragments which have hinge cysteines between them. Other chemical couplings of antibody fragments are also known.

The "light chains" of antibodies (immunoglobulins) from any vertebrate species can be assigned to one of two clearly distinct types, called kappa and lambda, based on the amino acid sequences of their constant domains.

Depending on the amino acid sequence of the constant domain of their heavy chains, immunoglobulins can be assigned to different classes. There are five major classes of immunoglobulins: IgA, IgD, IgE, IgG, and IgM, and several of these may be further divided into subclasses (isotypes), e.g., IgG1, IgG2, IgG3, IgG4, IgA, and IgA2.

"Single-chain Fv" or "sFv" antibody fragments comprise the V_{H} and V_{L} domains of antibody, wherein these domains are present in a single polypeptide chain. Preferably, the Fv polypeptide further comprises a polypeptide linker between the V_{H} and V_{L} domains which enables the sFv to form the desired structure for antigen binding. For a review of sFv, see Pluckthun in The Pharmacology of Monoclonal Antibodies, vol. 113, Rosenburg and Moore eds., Springer-Verlag, New York, pp. 269-315 (1994).

The term "diabodies" refers to small antibody fragments with two antigen-binding sites, which fragments comprise a heavy-chain variable domain (V_{H}) connected to a light-chain variable domain (V_{L}) in the same polypeptide chain (V_{H}-V_{L}). By using a linker that is too short to allow pairing between the two domains on the same chain, the domains are forced to pair with the complementary domains of another chain and create two antigen-binding sites. Diabodies are described more fully in, for example, EP 404,097; WO 93/11161; and Hollinger et al., Proc. Natl. Acad. Sci. USA, 90:6444-6448 (1993).

An "isolated" antibody is one which has been identified and separated and/or recovered from a component of its natural environment. Contaminant components of its natural environment are materials which would interfere with diagnostic or therapeutic uses for the antibody, and may include enzymes, hormones, and other proteinaceous or nonproteinaceous solutes. In preferred embodiments, the antibody will be purified (1) to greater than 95% by weight of antibody as determined by the Lowry method, and most preferably more than 99% by weight, (2) to a degree sufficient to obtain at least 15 residues of N-terminal or internal amino acid sequence by use of a spinning cup sequenator, or (3) to homogeneity by SDS-PAGE under reducing or nonreducing conditions using Coomassie blue or, preferably, silver stain. Isolated antibody includes the antibody in situ within recombinant cells since at least one component of the antibody's natural environment will not be present. Ordinarily, however, isolated antibody will be prepared by at least one purification step.

An antibody that "specifically binds to" or is "specific for" a particular polypeptide or an epitope on a particular polypeptide is one that binds to that particular polypeptide or epitope on a particular polypeptide without substantially binding to any other polypeptide or polypeptide epitope.

The word "label" when used herein refers to a detectable compound or composition which is conjugated directly or indirectly to the antibody so as to generate a "labeled" antibody. The label may be detectable by itself (e.g. radioisotope labels or fluorescent labels) or, in the case of an enzymatic label, may catalyze chemical alteration of a substrate compound or composition which is detectable.

By "solid phase" is meant a non-aqueous matrix to which the antibody of the present invention can adhere. Examples of solid phases encompassed herein include those formed partially or entirely of glass (e.g., controlled pore glass), polysaccharides (e.g., agarose), polyacrylamides, polystyrene, polyvinyl alcohol and silicones. In certain embodiments, depending on the context, the solid phase can comprise the well of an assay plate; in others it is a purification column (e.g., an affinity chromatography column). This term also includes a discontinuous solid phase of discrete particles, such as those described in U.S. Patent No. 4,275,149.

A "liposome" is a small vesicle composed of various types of lipids, phospholipids and/or surfactant which is useful for delivery of a drug (such as a PRO polypeptide or antibody thereto) to a mammal. The components of the liposome are commonly arranged in a bilayer formation, similar to the lipid arrangement of biological membranes.

A "small molecule" is defined herein to have a molecular weight below about 500 Daltons.

The term "immune related disease" means a disease in which a component of the immune system of a mammal causes, mediates or otherwise contributes to a morbidity in the mammal. Also included are diseases in which stimulation or intervention of the immune response has an ameliorative effect on progression of the disease. Included within this term are immune-mediated inflammatory diseases, non-immune-mediated inflammatory diseases, infectious diseases, immunodeficiency diseases, neoplasia, *etc.*

The term "T cell mediated disease" means a disease in which T cells directly or indirectly mediate or otherwise contribute to a morbidity in a mammal. The T cell mediated disease may be associated with cell mediated effects, lymphokine mediated effects, *etc*., and even effects associated with B cells if the B cells are stimulated, for example, by the lymphokines secreted by T cells.

Examples of immune-related and inflammatory diseases, some of which are immune or T cell mediated, which can be treated according to the invention include systemic lupus erythematosis, rheumatoid arthritis, juvenile chronic arthritis, spondyloarthropathies, systemic sclerosis (scleroderma), idiopathic inflammatory myopathies (dermatomyositis, polymyositis), Sjögren's syndrome, systemic vasculitis, sarcoidosis, autoimmune hemolytic anemia (immune pancytopenia, paroxysmal nocturnal hemoglobinuria), autoimmune thrombocytopenia (idiopathic thrombocytopenic purpura, immune-mediated thrombocytopenia), thyroiditis (Grave's disease, Hashimoto's thyroiditis, juvenile lymphocytic thyroiditis, atrophic thyroiditis), diabetes mellitus, immune-mediated renal disease (glomerulonephritis, tubulointerstitial nephritis), demyelinating diseases of the central and peripheral nervous systems such as multiple sclerosis, idiopathic demyelinating polyneuropathy or Guillain-Barré syndrome, and chronic inflammatory demyelinating polyneuropathy, hepatobiliary diseases such as infectious hepatitis (hepatitis A, B, C, D, E and other non-hepatotropic viruses), autoimmune chronic active hepatitis, primary biliary cirrhosis, granulomatous hepatitis, and sclerosing cholangitis, inflammatory bowel disease (ulcerative colitis: Crohn's disease), gluten-sensitive enteropathy, and Whipple's disease, autoimmune or immune-mediated skin diseases including bullous skin diseases, erythema multiforme and contact dermatitis, psoriasis, allergic diseases such as asthma, allergic rhinitis, atopic dermatitis, food hypersensitivity and urticaria, immunologic diseases of the lung such as eosinophilic pneumonias, idiopathic pulmonary fibrosis and hypersensitivity pneumonitis, transplantation associated diseases including graft rejection and graft -versus-host-disease. Infectious diseases including viral diseases such as AIDS (HIV infection), hepatitis A, B, C, D, and E, herpes, etc., bacterial infections, fungal infections, protozoal infections and parasitic infections.

The term "effective amount" is a concentration or amount of a PRO polypeptide and/or agonist/antagonist which results in achieving a particular stated purpose. An "effective amount" of a PRO polypeptide or agonist or antagonist thereof may be determined empirically. Furthermore, a "therapeutically effective amount" is a concentration or amount of a PRO polypeptide and/or agonist/antagonist which is effective for achieving a stated therapeutic effect. This amount may also be determined empirically.

The term "cytotoxic agent" as used herein refers to a substance that inhibits or prevents the function of cells and/or causes destruction of cells. The term is intended to include radioactive isotopes (*e*.*g*., I¹³¹, I¹²⁵, Y⁹⁰ and Re¹⁸⁶), chemotherapeutic agents, and toxins such as enzymatically active toxins of bacterial, fungal, plant or animal origin, or fragments thereof.

A "chemotherapeutic agent" is a chemical compound useful in the treatment of cancer. Examples of chemotherapeutic agents include adriamycin, doxorubicin, epirubicin, 5-fluorouracil, cytosine arabinoside ("Ara-C"), cyclophosphamide, thiotepa, busulfan, cytoxin, taxoids, *e*.*g*., paclitaxel (Taxol, Bristol-Myers Squibb Oncology, Princeton, NJ), and doxetaxel (Taxotere, Rhône-Poulenc Rorer, Antony, France), toxotere, methotrexate, cisplatin, melphalan, vinblastine, bleomycin, etoposide, ifosfamide, mitomycin C, mitoxantrone, vincristine, vinorelbine, carboplatin, teniposide, daunomycin, carminomycin, aminopterin, dactinomycin, mitomycins, esperamicins (see U.S. Pat. No. 4,675,187), melphalan and other related nitrogen mustards. Also included in this definition are hormonal agents that act to regulate or inhibit hormone action on tumors such as tamoxifen and onapristone.

A "growth inhibitory agent" when used herein refers to a compound or composition which inhibits growth of a cell, especially cancer cell overexpressing any of the genes identified herein, either *in vitro* or *in vivo*. Thus, the growth inhibitory agent is one which significantly reduces the percentage of cells overexpressing such genes in S phase. Examples of growth inhibitory agents include agents that block cell cycle progression (at a place other than S phase), such as agents that induce G1 arrest and M-phase arrest. Classical M-phase blockers include the vincas (vincristine and vinblastine), taxol, and topo II inhibitors such as doxorubicin, epirubicin, daunorubicin, etoposide, and bleomycin. Those agents that arrest G1 also spill over into S-phase arrest, for example, DNA alkylating agents such as tamoxifen, prednisone, dacarbazine, mechlorethamine, cisplatin, methotrexate, 5-fluorouracil, and ara-C. Further information can be found in The Molecular Basis of Cancer, Mendelsohn and Israel, eds., Chapter 1, entitled "Cell cycle regulation, oncogens, and antineoplastic drugs" by Murakami et al. (WB Saunders: Philadelphia, 1995), especially p. 13.

The term "cytokine" is a generic term for proteins released by one cell population which act on another cell as intercellular mediators. Examples of such cytokines are lymphokines, monokines, and traditional polypeptide hormones. Included among the cytokines are growth hormone such as human growth hormone, N-methionyl human growth hormone, and bovine growth hormone; parathyroid hormone; thyroxine; insulin; proinsulin; relaxin; prorelaxin; glycoprotein hormones such as follicle stimulating hormone (FSH), thyroid stimulating hormone (TSH), and luteinizing hormone (LH); hepatic growth factor; fibroblast growth factor; prolactin; placental lactogen; tumor necrosis factor-α and -β; mullerian-inhibiting substance; mouse gonadotropin-associated peptide; inhibin; activin; vascular endothelial growth factor; integrin; thrombopoietin (TPO); nerve growth factors such as NGF-β; platelet-growth factor; transforming growth factors (TGFs) such as TGF-α and TGF-β; insulin-like growth factor-I and -II; erythropoietin (EPO); osteoinductive factors; interferons such as interferon-α, -β, and -γ; colony stimulating factors (CSFs) such as macrophage-CSF (M-CSF); granulocyte-macrophage-CSF (GM-CSF); and granulocyte-CSF (G-CSF); interleukins (ILs) such as IL-1, IL-1α, IL-2, IL-3, IL-4, IL-5, IL-6, IL-7, IL-8, IL-9, IL-11, IL-12; a tumor necrosis factor such as TNF-α or TNF-β; and other polypeptide factors including LIF and kit ligand (KL). As used herein, the term cytokine includes proteins from natural sources or from recombinant cell culture and biologically active equivalents of the native sequence cytokines.

As used herein, the term "immunoadhesin" designates antibody-like molecules which combine the binding specificity of a heterologous protein (an "adhesin") with the effector functions of immunoglobulin constant domains. Structurally, the immunoadhesins comprise a fusion of an amino acid sequence with the desired binding specificity which is other than the antigen recognition and binding site of an antibody (*i*.*e*., is "heterologous"), and an immunoglobulin constant domain sequence. The adhesin part of an immunoadhesin molecule typically is a contiguous amino acid sequence comprising at least the binding site of a receptor or a ligand. The immunoglobulin constant domain sequence in the immunoadhesin may be obtained from any immunoglobulin, such as IgG-1, IgG-2, IgG-3, or IgG-4 subtypes, IgA (including IgA-1 and IgA-2), IgE, IgD or IgM.

As used herein, the term "inflammatory cells" designates cells that enhance the inflammatory response such as mononuclear cells, eosinophils, macrophages, and polymorphonuclear neutrophils (PMN).

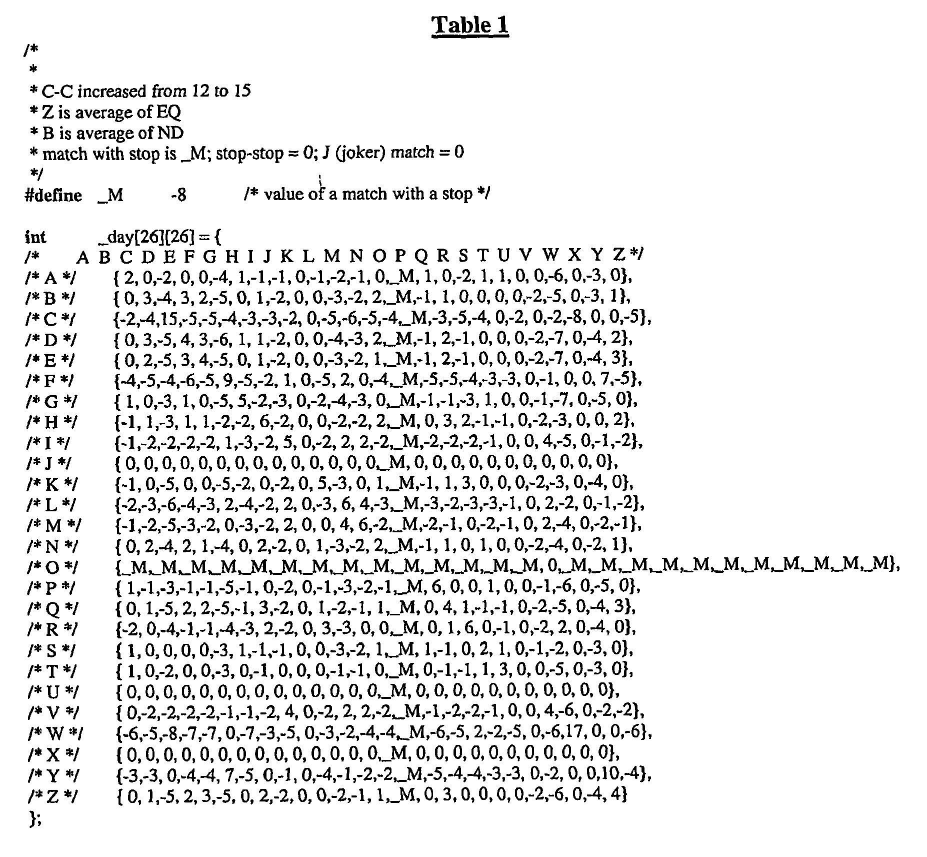

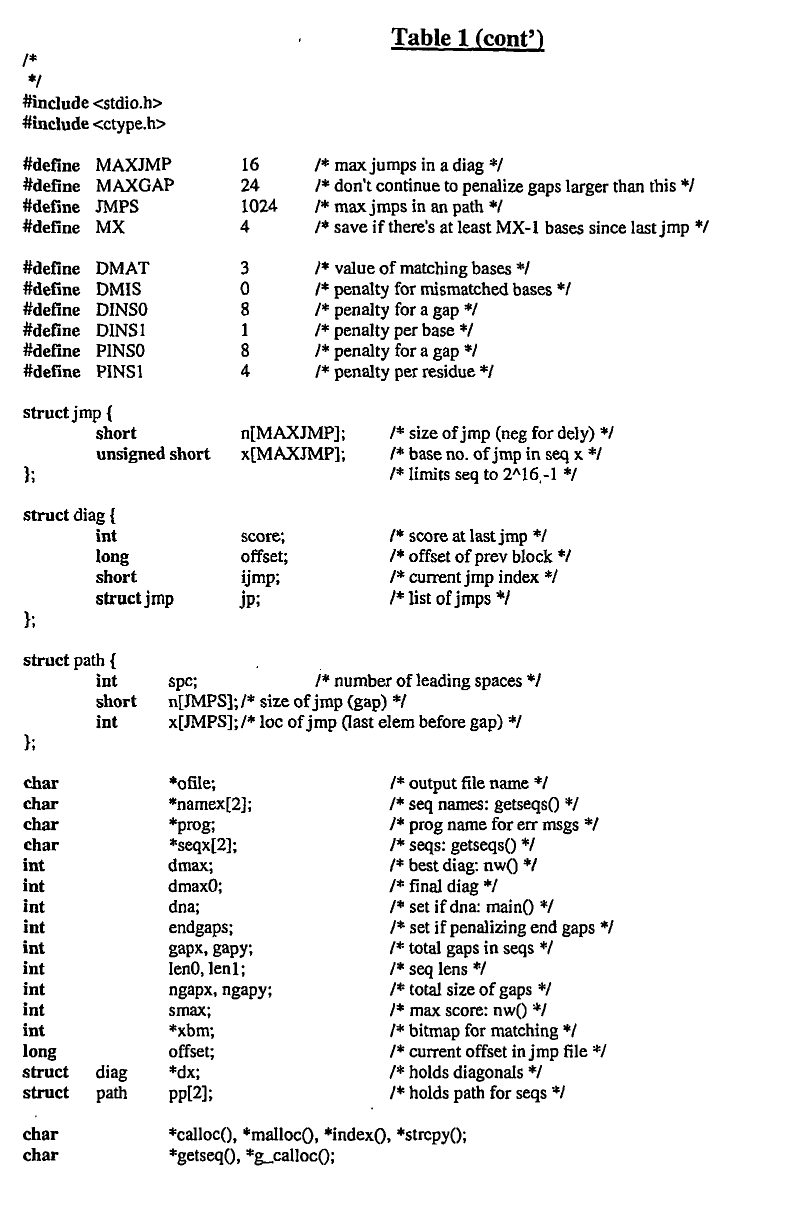

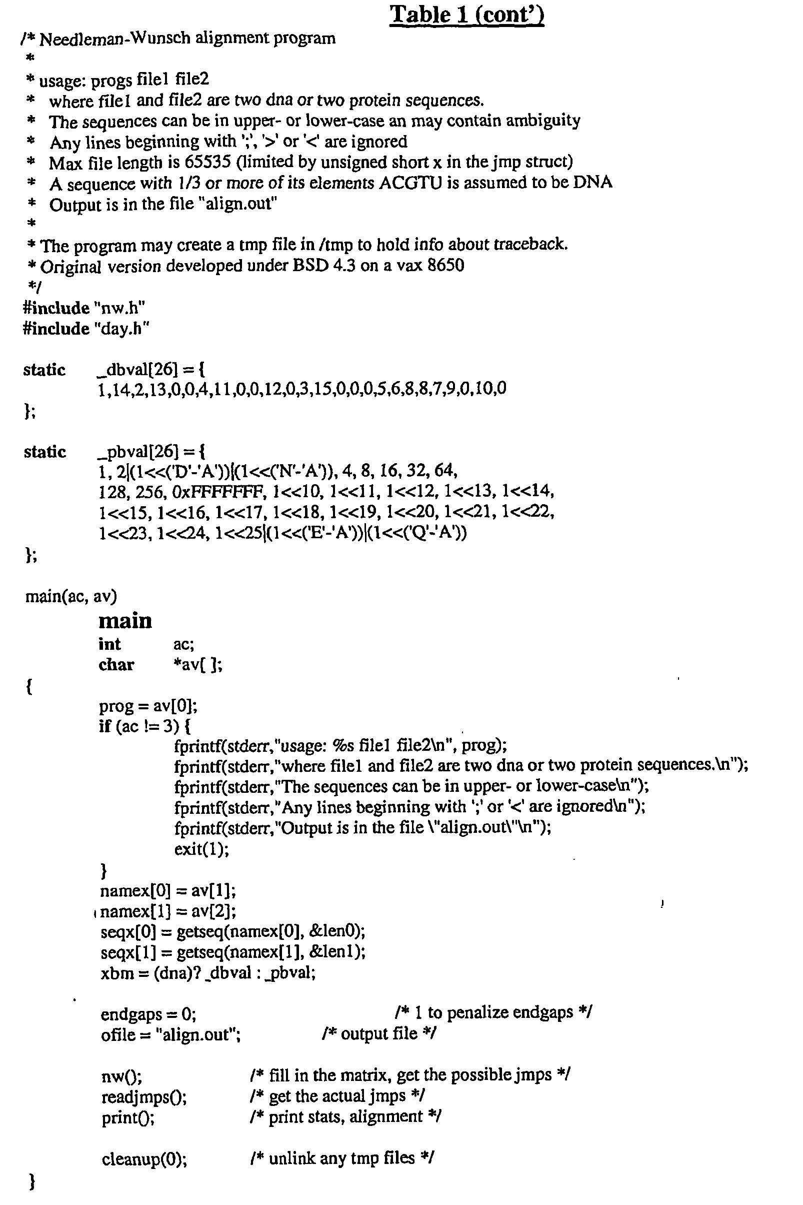

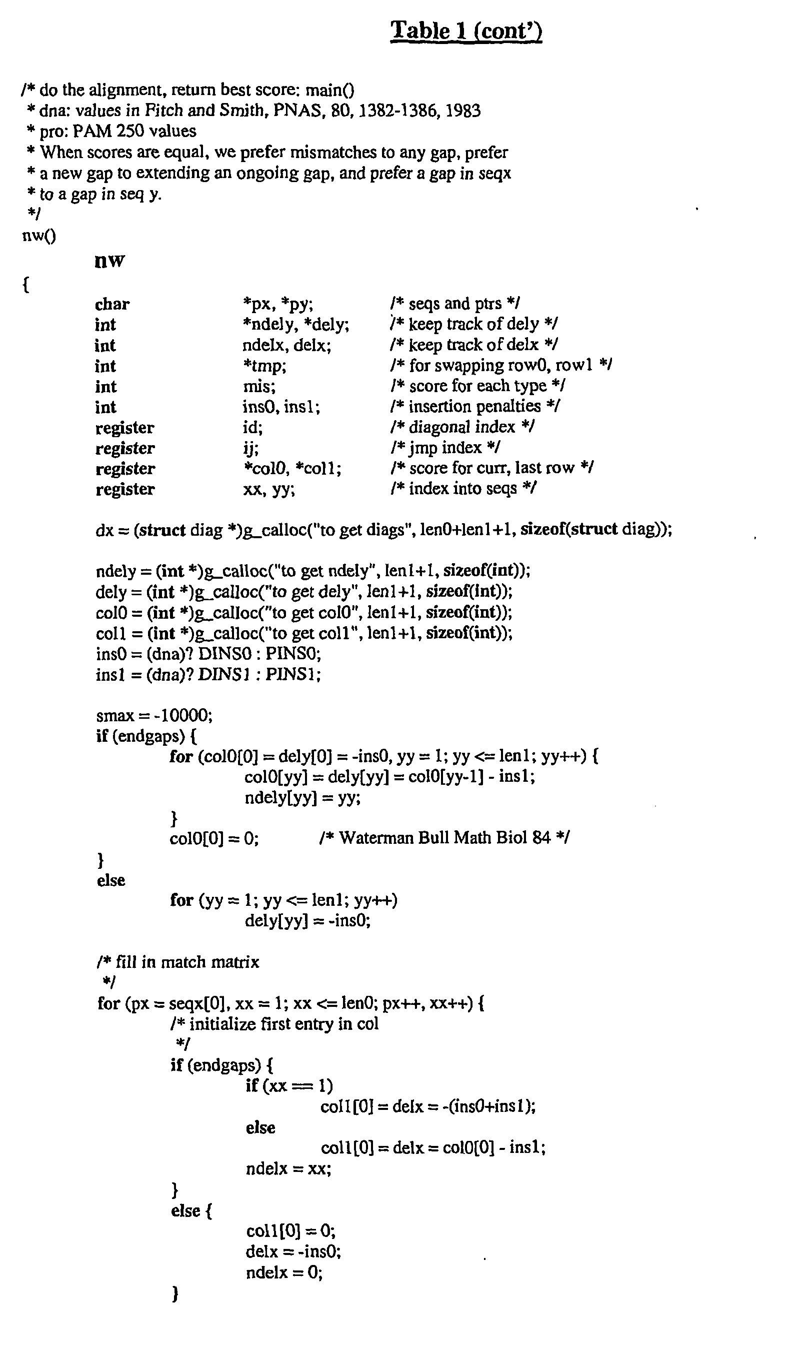

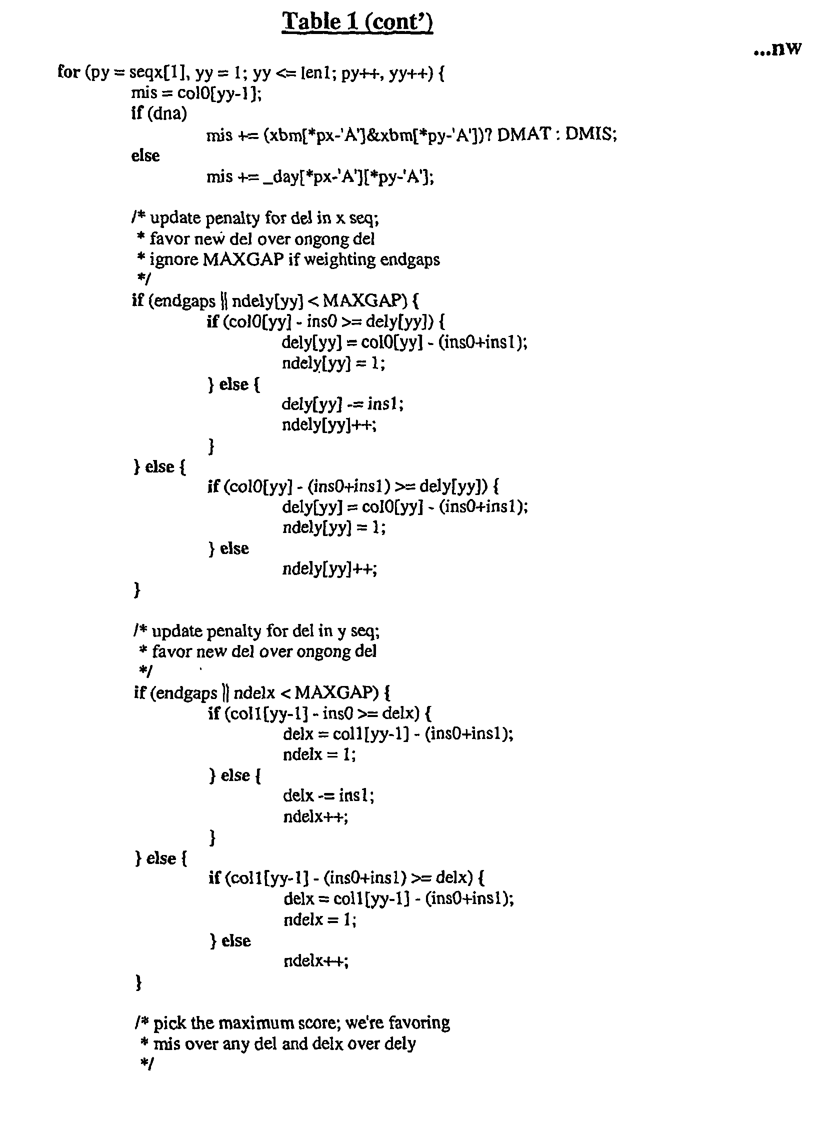

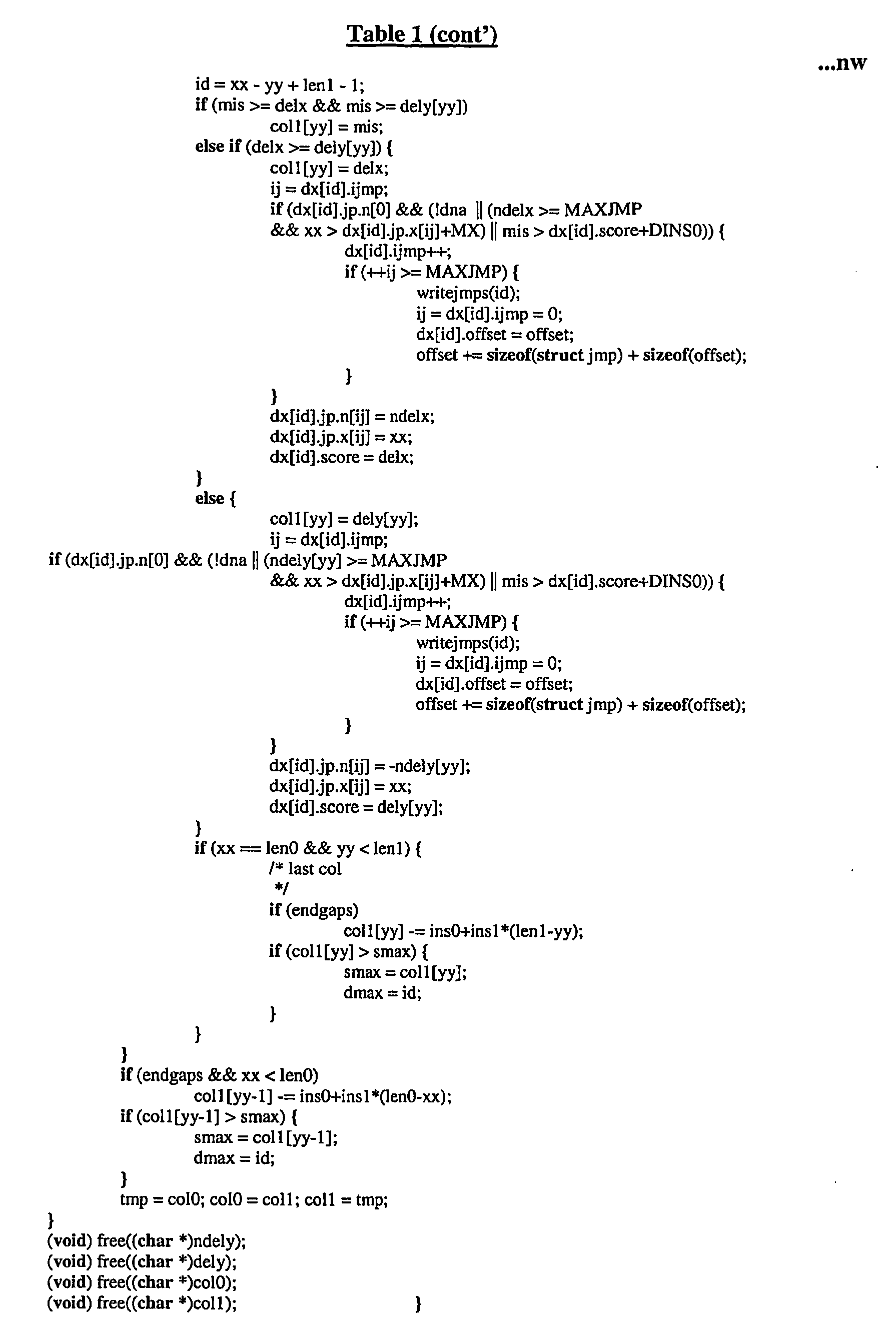

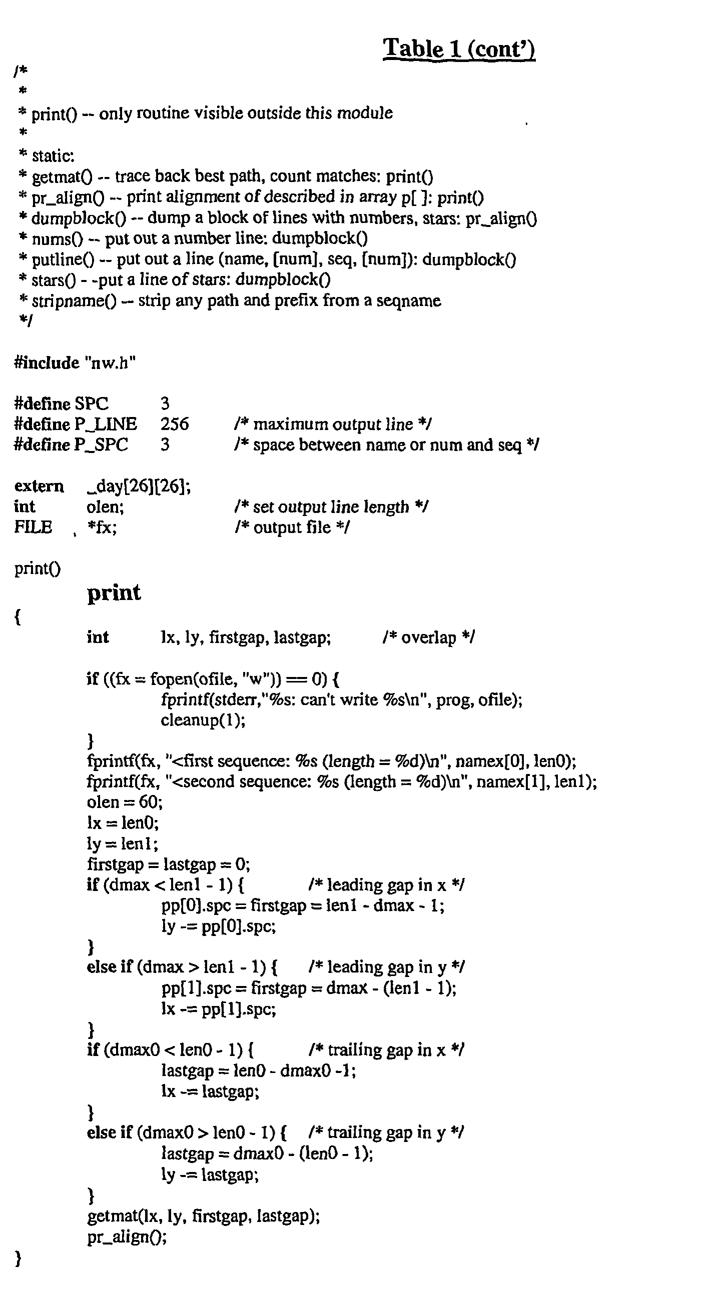

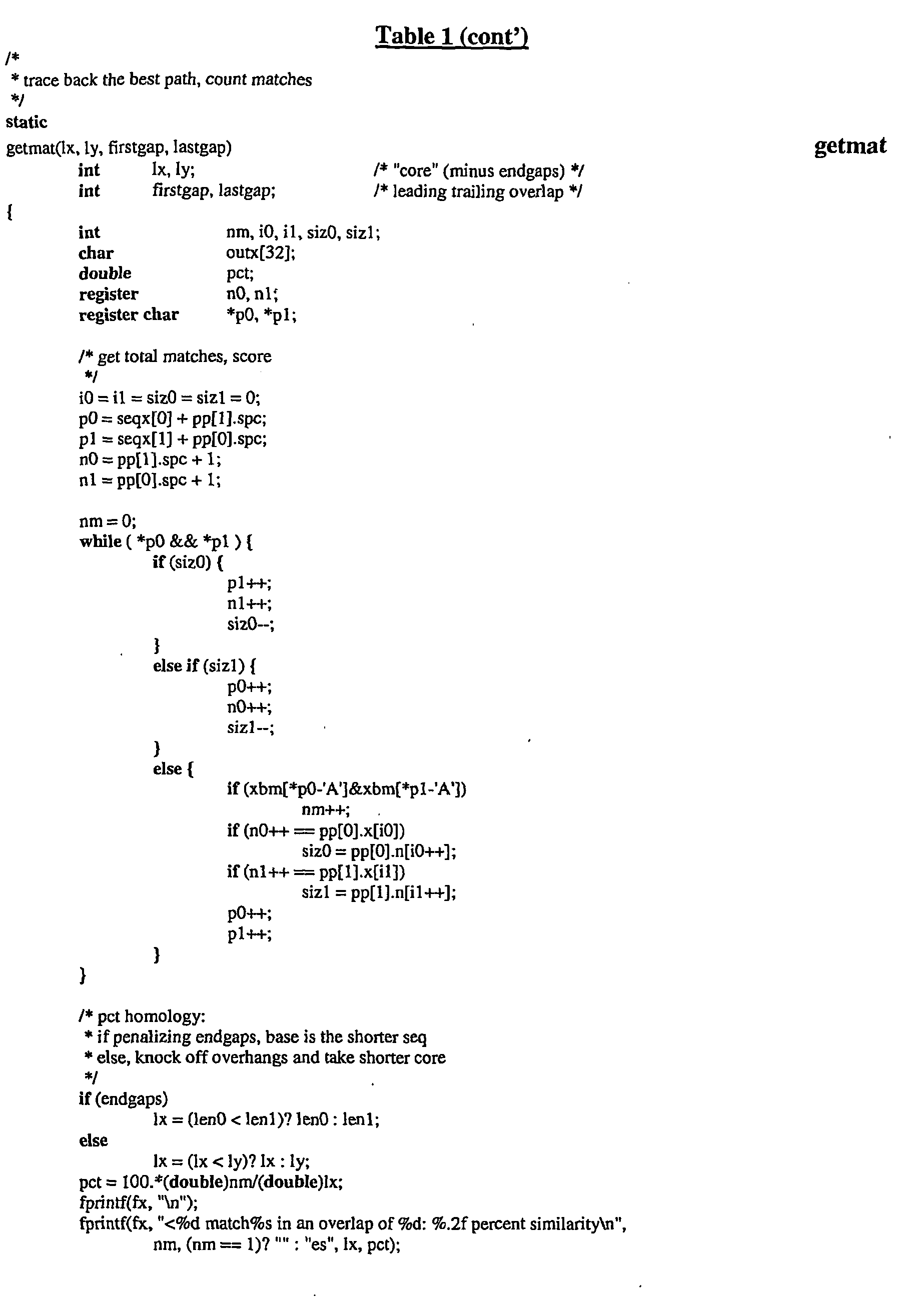

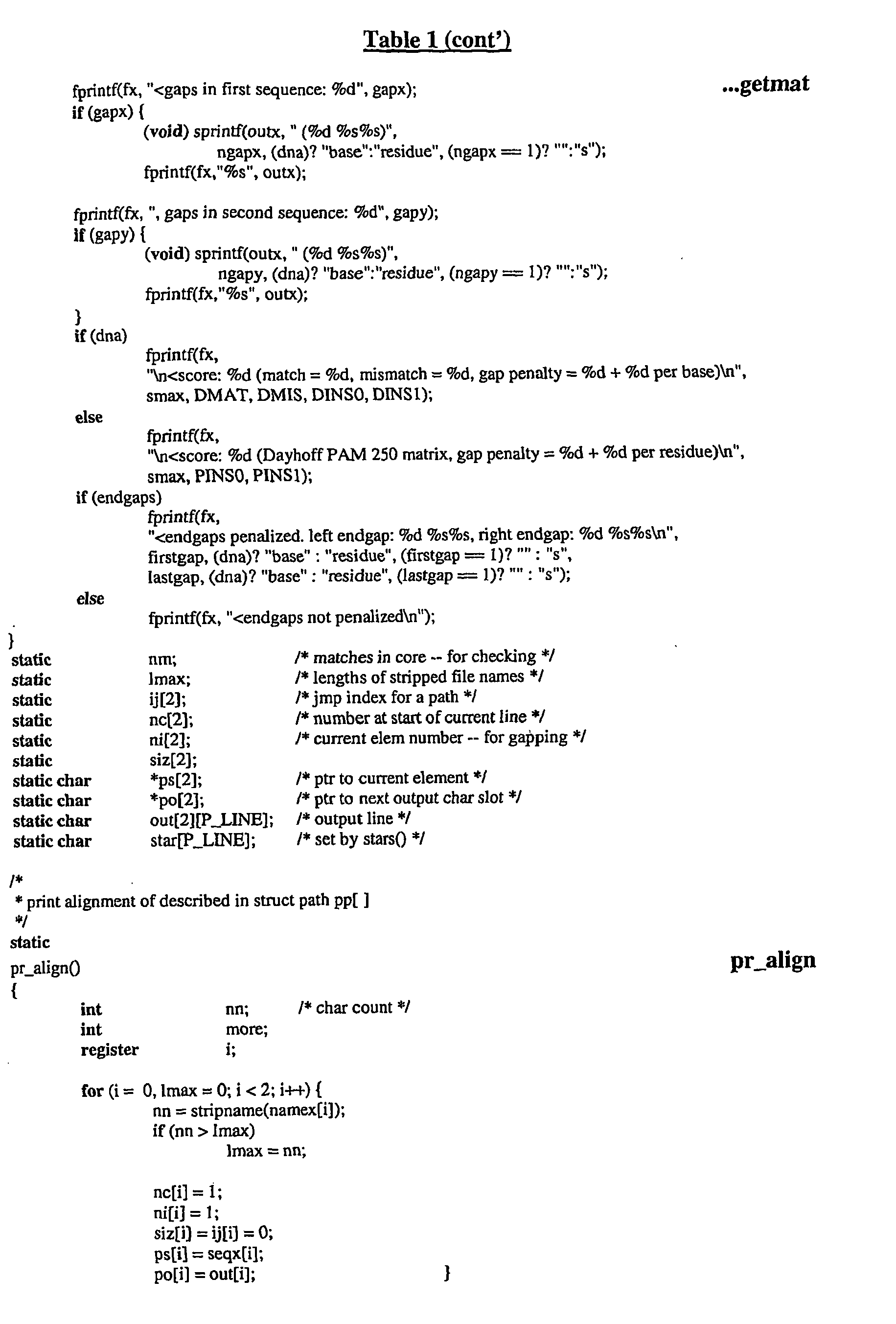

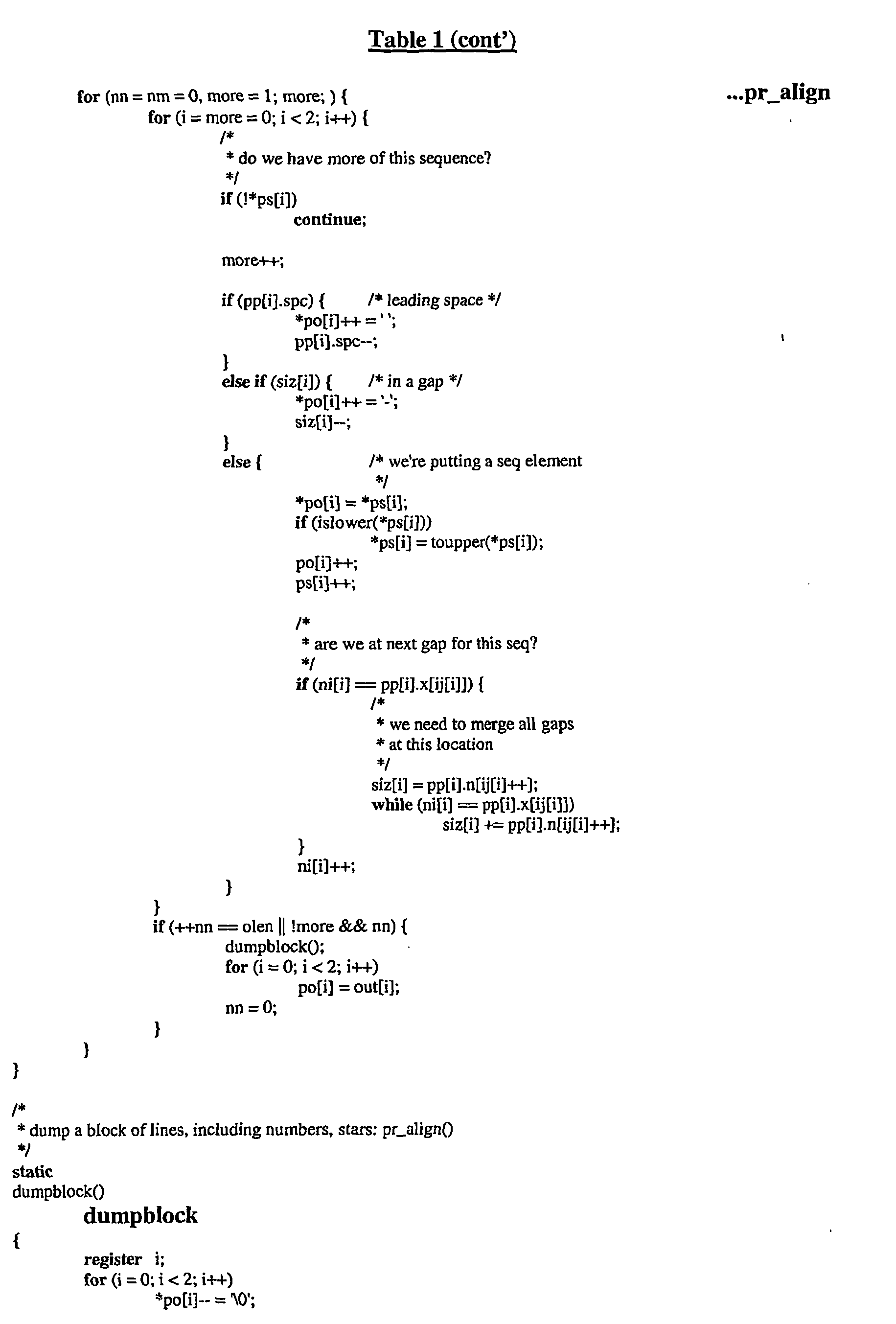

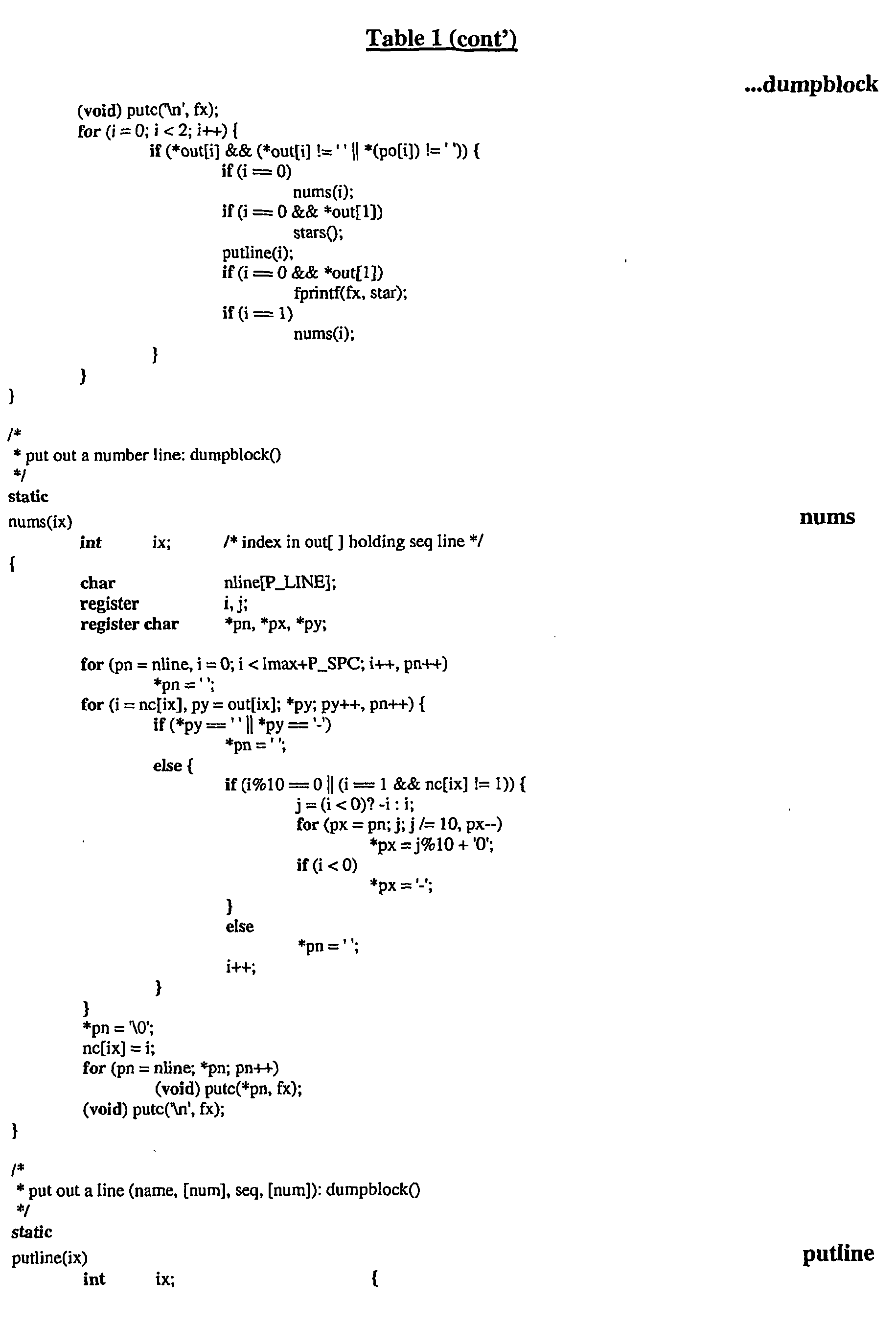

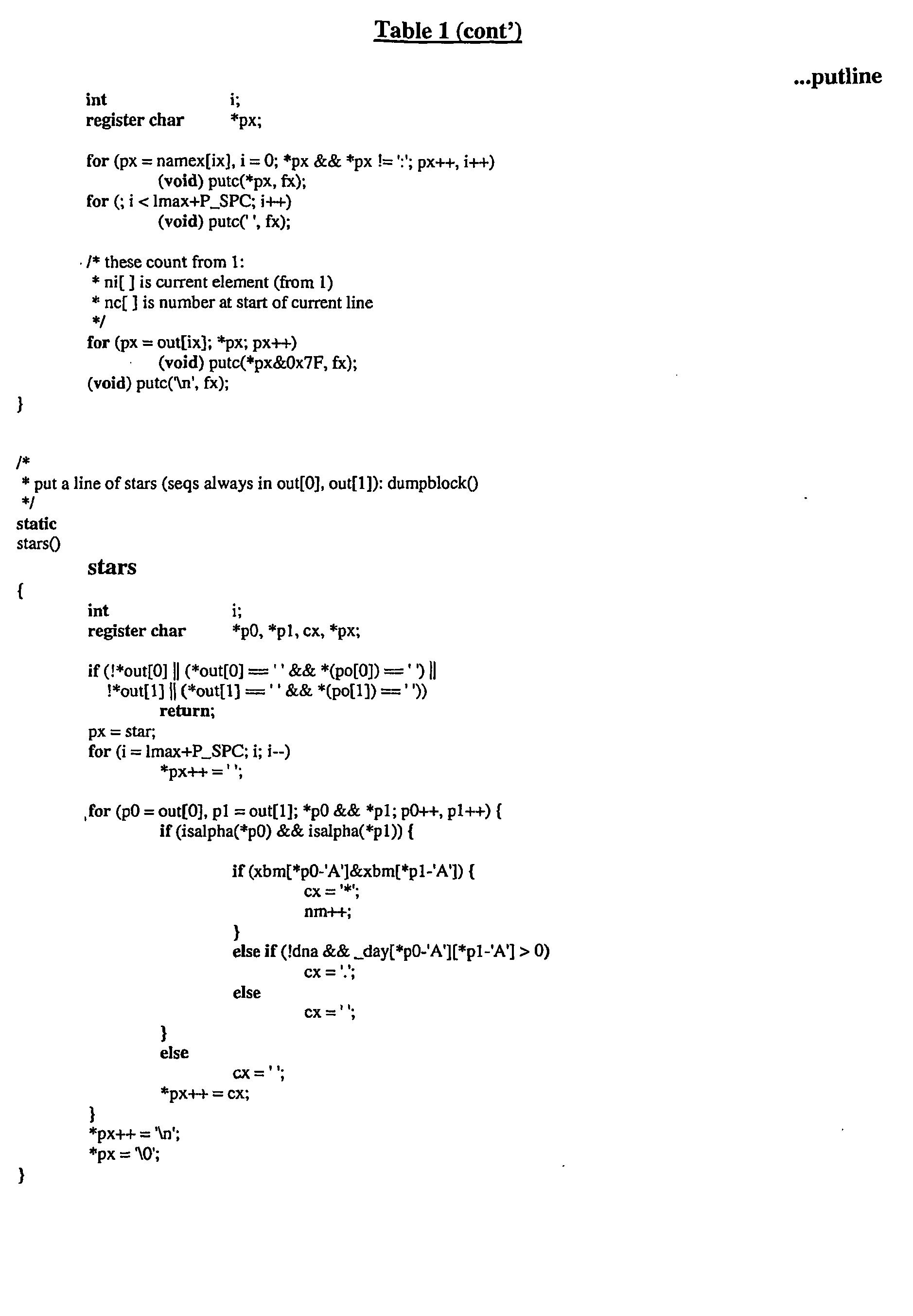

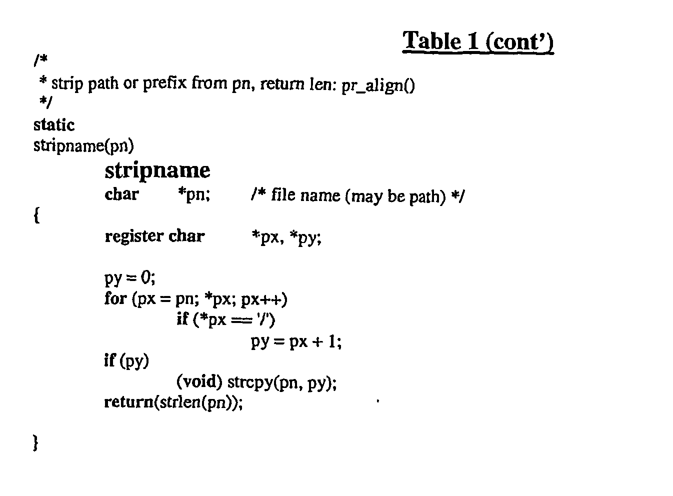

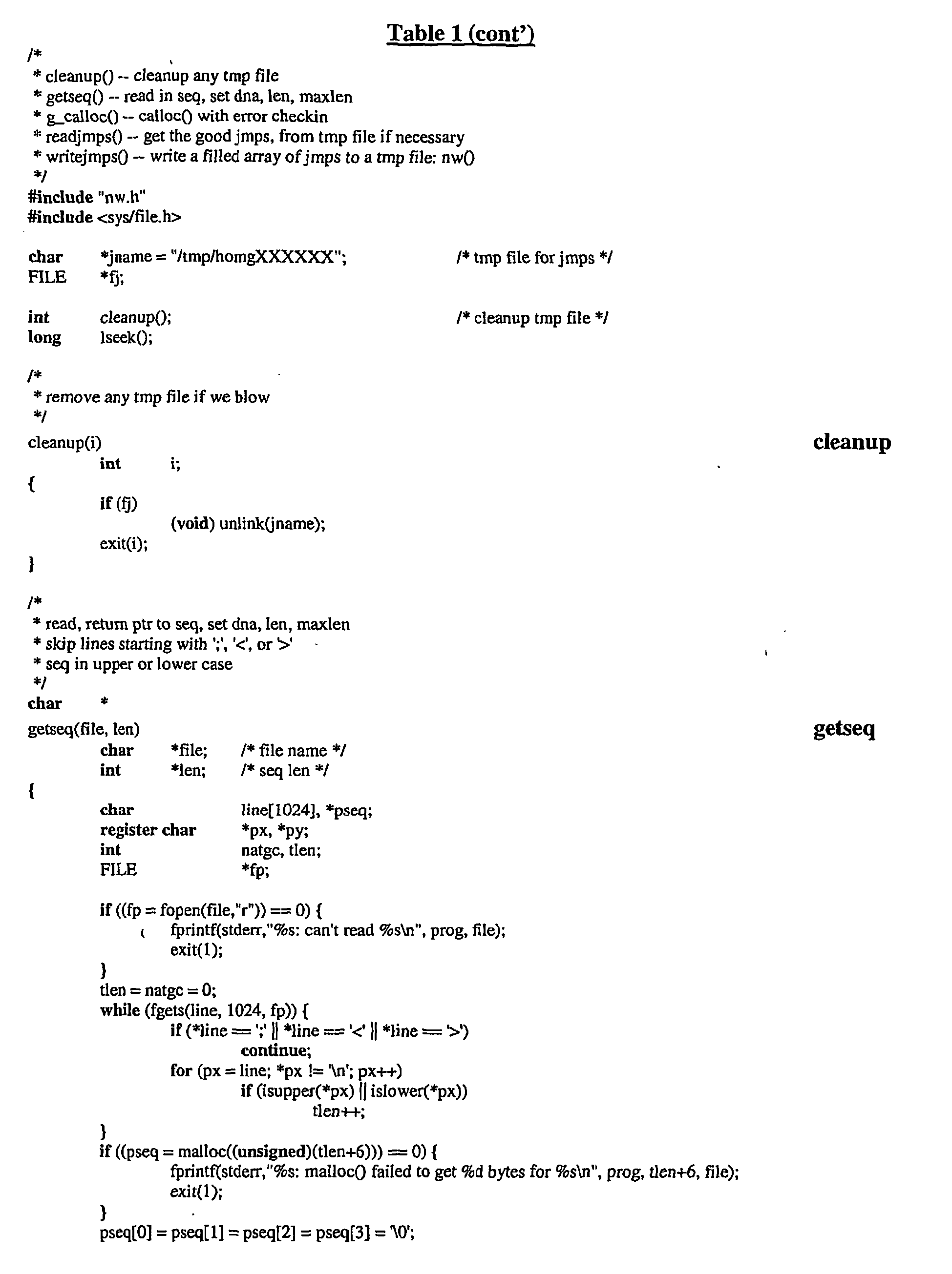

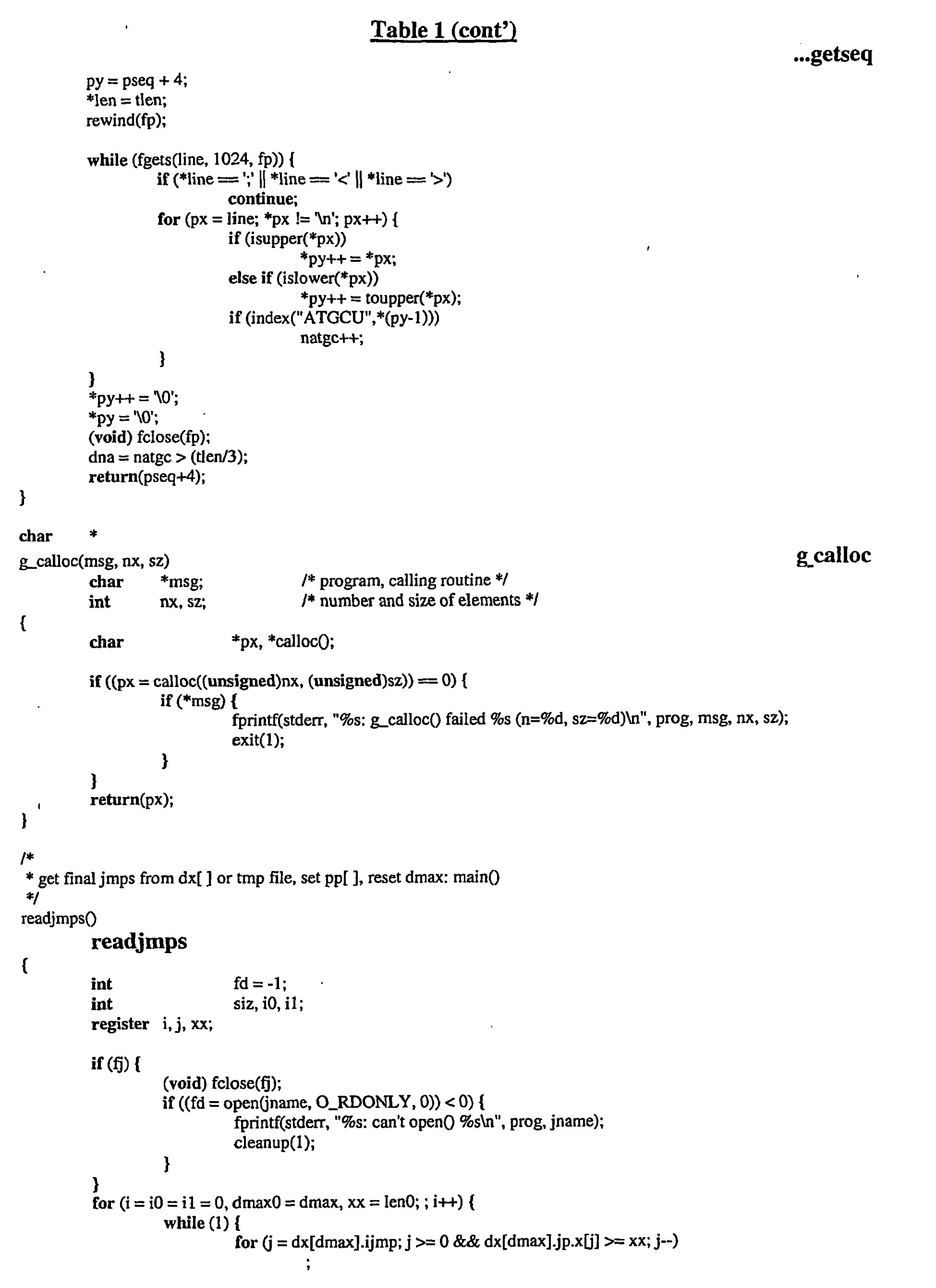

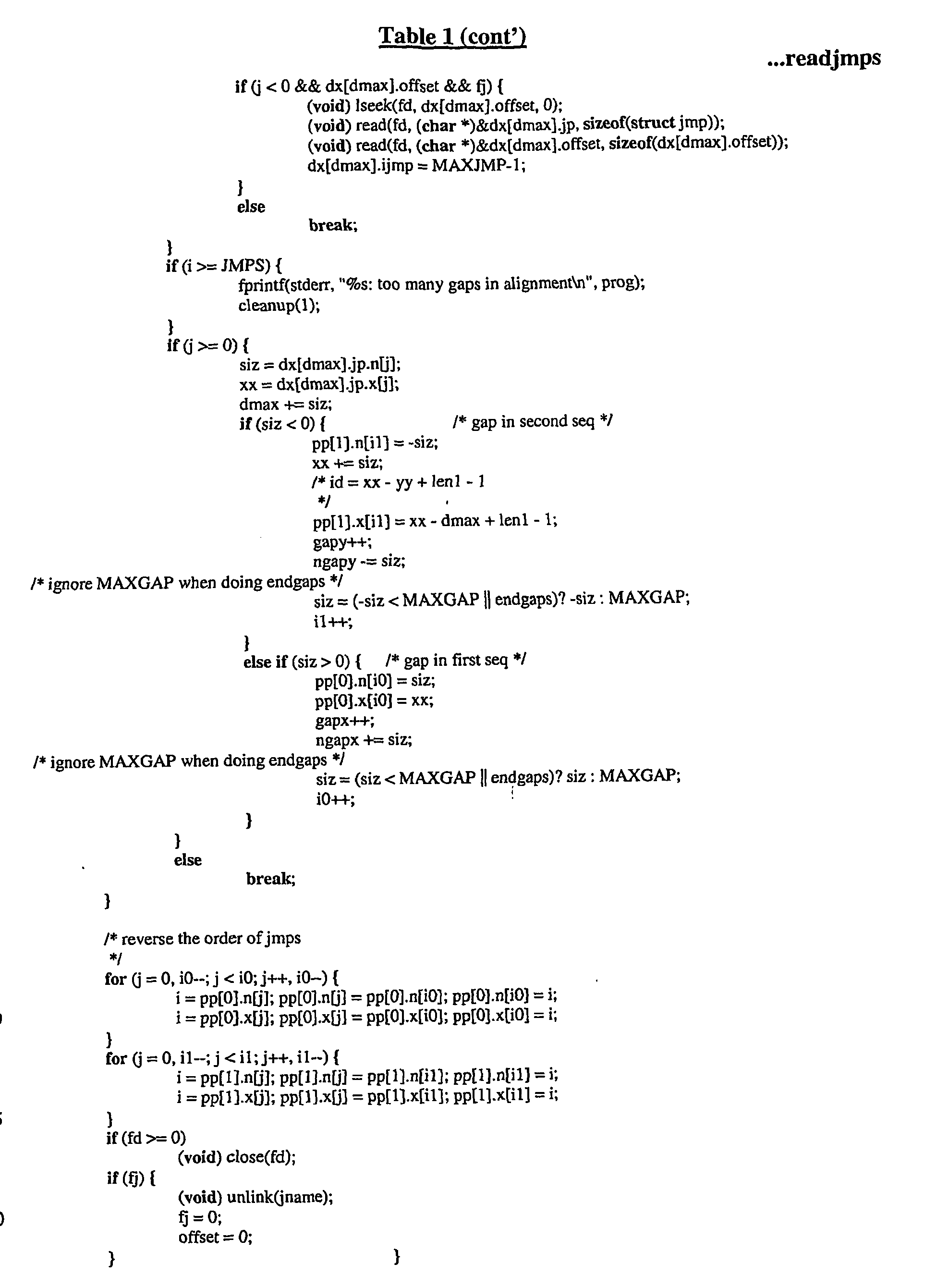

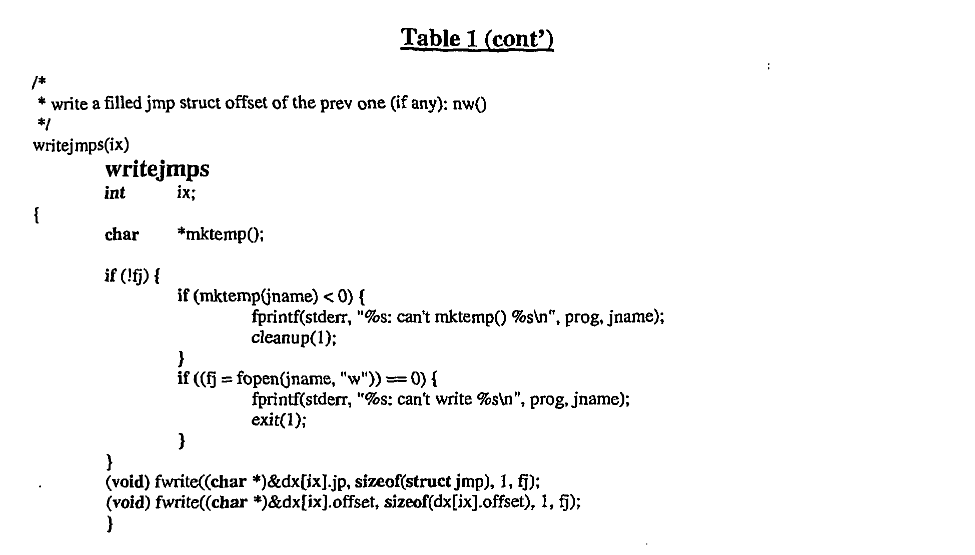

**Table 2**

| | | |
|---|---|---|
| PRO | XXXXXXXXXXXXXXX | (Length = 15 amino acids) |
| Comparison Protein | XXXXXYYYYYYY | (Length = 12 amino acids) |
| % amino acid sequence identity = | | |

(the number of identically matching amino acid residues between the two polypeptide sequences as determined by ALIGN-2) divided by (the total number of amino acid residues of the PRO polypeptide) = 5 divided by 15 = 33.3%

**Table 3**

| | | |
|---|---|---|
| PRO | XXXXXXXXXX | (Length = 10 amino acids) |
| Comparison Protein | XXXXXYYYYYYZZYZ | (Length = 15 amino acids) |
| % amino acid sequence identity = | | |

(the number of identically matching amino acid residues between the two polypeptide sequences as determined by ALIGN-2) divided by (the total number of amino acid residues of the PRO polypeptide) = 5 divided by 10 = 50%

**Table 4**

| | | |
|---|---|---|
| PRO-DNA | NNNNNNNNNNNNNN | (Length = 14 nucleotides) |
| Comparison DNA | NNNNNNLLLLLLLLLL | (Length = 16 nucleotides) |

% nucleic acid sequence identity =
(the number of identically matching nucleotides between the two nucleic acid sequences as determined by ALIGN-2) divided by (the total number of nucleotides of the PRO-DNA nucleic acid sequence) = 6 divided by 14 = 42.9%

**Table 5**

| | | |
|---|---|---|
| PRO-DNA | NNNNNNNNNNNN | (Length = 12 nucleotides) |
| Comparison DNA | NNNNLLLVV | (Length = 9 nucleotides) |

% nucleic acid sequence identity =
(the number of identically matching nucleotides between the two nucleic acid sequences as determined by ALIGN-2) divided by (the total number of nucleotides of the PRO-DNA nucleic acid sequence) = 4 divided by 12 = 33.3%

### II. Compositions and Methods of the Invention

### A. Full-Length PRO Polypeptides

The present invention provides newly identified and isolated nucleotide sequences encoding polypeptides referred to in the present application as PRO polypeptides. In particular, cDNAs encoding various PRO polypeptides have been identified and isolated, as disclosed in further detail in the Examples below. However, for sake of simplicity, in the present specification the protein encoded by the full length native nucleic acid molecules disclosed herein as well as all further native homologues and variants included in the foregoing definition of PRO, will be referred to as "PRO/number", regardless of their origin or mode of preparation.

As disclosed in the Examples below, various cDNA clones have been disclosed. The predicted amino acid sequence can be determined from the nucleotide sequence using routine skill. For the PRO polypeptides and encoding nucleic acids described herein, Applicants have identified what is believed to be the reading frame best identifiable with the sequence information available at the time.

### B. PRO Polypeptide Variants

In addition to the full-length native sequence PRO polypeptides described herein, it is contemplated that PRO variants can be prepared. PRO variants can be prepared by introducing appropriate nucleotide changes into the PRO DNA, and/or by synthesis of the desired PRO polypeptide. Those skilled in the art will appreciate that amino acid changes may alter post-translational processes of the PRO, such as changing the number or position of glycosylation sites or altering the membrane anchoring characteristics.

Variations in the native full-length sequence PRO or in various domains of the PRO described herein, can be made, for example, using any of the techniques and guidelines for conservative and non-conservative mutations set forth, for instance, in U.S. Patent No. 5,364,934. Variations may be a substitution, deletion or insertion of one or more codons encoding the PRO that results in a change in the amino acid sequence of the PRO as compared with the native sequence PRO. Optionally, the variation is by substitution of at least one amino acid with any other amino acid in one or more of the domains of the PRO. Guidance in determining which amino acid residue may be inserted, substituted or deleted without adversely affecting the desired activity may be found by comparing the sequence of the PRO with that of homologous known protein molecules and minimizing the number of amino acid sequence changes made in regions of high homology. Amino acid substitutions can be the result of replacing one amino acid with another amino acid having similar structural and/or chemical properties, such as the replacement of a leucine with a serine, i.e., conservative amino acid replacements. Insertions or deletions may optionally be in the range of about 1 to 5 amino acids. The variation allowed may be determined by systematically making insertions, deletions or substitutions of amino acids in the sequence and testing the resulting variants for activity exhibited by the full-length or mature native sequence.

PRO polypeptide fragments are provided herein. Such fragments may be truncated at the N-terminus or C-terminus, or may lack internal residues, for example, when compared with a full length native protein. Certain fragments lack amino acid residues that are not essential for a desired biological activity of the PRO polypeptide.

PRO fragments may be prepared by any of a number of conventional techniques. Desired peptide fragments may be chemically synthesized. An alternative approach involves generating PRO fragments by enzymatic digestion, e.g., by treating the protein with an enzyme known to cleave proteins at sites defined by particular amino acid residues, or by digesting the DNA with suitable restriction enzymes and isolating the desired fragment. Yet another suitable technique involves isolating and amplifying a DNA fragment encoding a desired polypeptide fragment, by polymerase chain reaction (PCR). Oligonucleotides that define the desired termini of the DNA fragment are employed at the 5' and 3' primers in the PCR. Preferably, PRO polypeptide fragments share at least one biological and/or immunological activity with the native PRO polypeptide disclosed herein.

In particular embodiments, conservative substitutions of interest are shown in Table 6 under the heading of preferred substitutions. If such substitutions result in a change in biological activity, then more substantial changes, denominated exemplary substitutions in Table 6, or as further described below in reference to amino acid classes, are introduced and the products screened.

**Table 6**

| Original Residue | Exemplary Substitutions | Preferred Substitutions |
|---|---|---|
| Ala (A) | val; leu; ile | val |
| Arg (R) | lys; gln; asn | lys |
| Asn (N) | gln; his; lys; arg | gln |
| Asp (D) | glu | glu |
| Cys (C) | ser | ser |
| Gln (Q) | asn | asn |
| Glu (E) | asp | asp |
| Gly (G) | pro; ala | ala |
| His (H) | asn; gln; lys; arg | arg |
| Ile (I) | leu; val; met; ala; phe; | |
| | norleucine | leu |
| Leu (L) | norleucine; ile; val; | |
| | met; ala; phe | ile |
| Lys (K) | arg; gln; asn | arg |
| Met (M) | leu; phe; ile | leu |
| Phe (F) | leu; val; ile; ala; tyr | leu |
| Pro (P) | ala | ala |
| Ser (S) | thr | thr |
| Thr (T) | ser | ser |
| Trp (W) | tyr; phe | tyr |
| Tyr (Y) | trp; phe; thr; ser | phe |
| Val (V) | ile; leu; met; phe; | |
| | ala; norleucine | leu |

Substantial modifications in function or immunological identity of the PRO polypeptide are accomplished by selecting substitutions that differ significantly in their effect on maintaining (a) the structure of the polypeptide backbone in the area of the substitution, for example, as a sheet or helical conformation, (b) the charge or hydrophobicity of the molecule at the target site, or (c) the bulk of the side chain. Naturally occurring residues are divided into groups based on common side-chain properties:
(1) hydrophobic: norleucine, met, ala, val, leu, ile;
(2) neutral hydrophilic: cys, ser, thr;
(3) acidic: asp, glu;
(4) basic: asn, gln, his, lys, arg;
(5) residues that influence chain orientation: gly, pro; and
(6) aromatic: trp, tyr, phe.

Non-conservative substitutions will entail exchanging a member of one of these classes for another class. Such substituted residues also may be introduced into the conservative substitution sites or, more preferably, into the remaining (non-conserved) sites.

The variations can be made using methods known in the art such as oligonucleotide-mediated (site-directed) mutagenesis, alanine scanning, and PCR mutagenesis. Site-directed mutagenesis [Carter et al., Nucl. Acids Res., 13:4331 (1986); Zoller et al., Nucl. Acids Res., 10:6487 (1987)], cassette mutagenesis [Wells et al., Gene, 34:315 (1985)], restriction selection mutagenesis [Wells et al., Philos. Trans. R. Soc. London SerA, 317:415 (1986)] or other known techniques can be performed on the cloned DNA to produce the PRO variant DNA.

Scanning amino acid analysis can also be employed to identify one or more amino acids along a contiguous sequence. Among the preferred scanning amino acids are relatively small, neutral amino acids. Such amino acids include alanine, glycine, serine, and cysteine. Alanine is typically a preferred scanning amino acid among this group because it eliminates the side-chain beyond the beta-carbon and is less likely to alter the main-chain conformation of the variant [Cunningham and Wells, Science, 244: 1081-1085 (1989)]. Alanine is also typically preferred because it is the most common amino acid. Further, it is frequently found in both buried and exposed positions [Creighton, The Proteins, (W.H. Freeman & Co., N.Y.); Chothia, J. Mol. Biol., 150:1 (1976)]. If alanine substitution does not yield adequate amounts of variant, an isoteric amino acid can be used.

### C. Modifications of PRO

Covalent modifications of PRO are included within the scope of this invention. One type of covalent modification includes reacting targeted amino acid residues of a PRO polypeptide with an organic derivatizing agent that is capable of reacting with selected side chains or the N- or C- terminal residues of the PRO. Derivatization with bifunctional agents is useful, for instance, for crosslinking PRO to a water-insoluble support matrix or surface for use in the method for purifying anti-PRO antibodies, and vice-versa. Commonly used crosslinking agents include, e.g., 1,1-bis(diazoacetyl)-2-phenylethane, glutaraldehyde, N-hydroxysuccinimide esters, for example, esters with 4-azidosalicylic acid, homobifunctional imidoesters, including disuccinimidyl esters such as 3,3'-dithiobis(succinimidylpropionate), bifunctional maleimides such as bis-N-maleimido-1,8-octane and agents such as methyl-3-[(p-azidophenyl)dithio]propioimidate.

Other modifications include deamidation of glutaminyl and asparaginyl residues to the corresponding glutamyl and aspartyl residues, respectively, hydroxylation of proline and lysine, phosphorylation of hydroxyl groups of seryl or threonyl residues, methylation of the α-amino groups of lysine, arginine, and histidine side chains [T.E. Creighton, Proteins: Structure and Molecular Properties, W.H. Freeman & Co., San Francisco, pp. 79-86 (1983)], acetylation of the N-terminal amine, and amidation of any C-terminal carboxyl group.

Another type of covalent modification of the PRO polypeptide included within the scope of this invention comprises altering the native glycosylation pattern of the polypeptide. "Altering the native glycosylation pattern" is intended for purposes herein to mean deleting one or more carbohydrate moieties found in native sequence PRO (either by removing the underlying glycosylation site or by deleting the glycosylation by chemical and/or enzymatic means), and/or adding one or more glycosylation sites that are not present in the native sequence PRO. In addition, the phrase includes qualitative changes in the glycosylation of the native proteins, involving a change in the nature and proportions of the various carbohydrate moieties present.

Addition of glycosylation sites to the PRO polypeptide may be accomplished by altering the amino acid sequence. The alteration may be made, for example, by the addition of, or substitution by, one or more serine or threonine residues to the native sequence PRO (for O-linked glycosylation sites). The PRO amino acid sequence may optionally be altered through changes at the DNA level, particularly by mutating the DNA encoding the PRO polypeptide at preselected bases such that codons are generated that will translate into the desired amino acids.

Another means of increasing the number of carbohydrate moieties on the PRO polypeptide is by chemical or enzymatic coupling of glycosides to the polypeptide. Such methods are described in the art, e.g., in WO 87/05330 published 11 September 1987, and in Aplin and Wriston, CRC Crit. Rev. Biochem., pp. 259-306 (1981).

Removal of carbohydrate moieties present on the PRO polypeptide may be accomplished chemically or enzymatically or by mutational substitution of codons encoding for amino acid residues that serve as targets for glycosylation. Chemical deglycosylation techniques are known in the art and described, for instance, by Hakimuddin, et al., Arch. Biochem. Biophys., 259:52 (1987) and by Edge et al., Anal. Biochem., 118:131 (1981). Enzymatic cleavage of carbohydrate moieties on polypeptides can be achieved by the use of a variety of endo- and exo-glycosidases as described by Thotakura et al., Meth. Enzymol., 138:350 (1987).

Another type of covalent modification of PRO comprises linking the PRO polypeptide to one of a variety of nonproteinaceous polymers, e.g., polyethylene glycol (PEG), polypropylene glycol, or polyoxyalkylenes, in the manner set forth in U.S. Patent Nos. 4,640,835; 4,496,689; 4,301,144; 4,670,417; 4,791,192 or 4,179,337.

The PRO of the present invention may also be modified in a way to form a chimeric molecule comprising PRO fused to another, heterologous polypeptide or amino acid sequence.

In one embodiment, such a chimeric molecule comprises a fusion of the PRO with a tag polypeptide which provides an epitope to which an anti-tag antibody can selectively bind. The epitope tag is generally placed at the amino- or carboxyl- terminus of the PRO. The presence of such epitope-tagged forms of the PRO can be detected using an antibody against the tag polypeptide. Also, provision of the epitope tag enables the PRO to be readily purified by affinity purification using an anti-tag antibody or another type of affinity matrix that binds to the epitope tag. Various tag polypeptides and their respective antibodies are well known in the art. Examples include poly-histidine (poly-his) or poly-histidine-glycine (poly-his-gly) tags; the flu HA tag polypeptide and its antibody 12CA5 [Field et al., Mol. Cell. Biol., 8:2159-2165 (1988)]; the c-myc tag and the 8F9, 3C7, 6E10, G4, B7 and 9E10 antibodies thereto [Evan et al., Molecular and Cellular Biology, 5:3610-3616 (1985)]; and the Herpes Simplex virus glycoprotein D (gD) tag and its antibody [Paborsky et al., Protein Engineering, 3(6):547-553 (1990)]. Other tag polypeptides include the Flag-peptide [Hopp et al., BioTechnology, 6:1204-1210 (1988)]; the KT3 epitope peptide [Martin et al., Science, 255:192-194 (1992)]; an alpha-tubulin epitope peptide [Skinner et al., J. Biol. Chem., 266:15163-15166 (1991)]; and the T7 gene 10 protein peptide tag [Lutz-Freyermuth et al., Proc. Natl. Acad. Sci. USA, 87:6393-6397 (1990)].

In an alternative embodiment, the chimeric molecule may comprise a fusion of the PRO with an immunoglobulin or a particular region of an immunoglobulin. For a bivalent form of the chimeric molecule (also referred to as an "immunoadhesin"), such a fusion could be to the Fc region of an IgG molecule. The Ig fusions preferably include the substitution of a soluble (transmembrane domain deleted or inactivated) form of a PRO polypeptide in place of at least one variable region within an Ig molecule. In a particularly preferred embodiment, the immunoglobulin fusion includes the hinge, CH2 and CH3, or the hinge, CH1, CH2 and CH3 regions of an IgG1 molecule. For the production of immunoglobulin fusions see also US Patent No. 5,428,130 issued June 27, 1995.

### D. Preparation of PRO

The description below relates primarily to production of PRO by culturing cells transformed or transfected with a vector containing PRO nucleic acid. It is, of course, contemplated that alternative methods, which are well known in the art, may be employed to prepare PRO. For instance, the PRO sequence, or portions thereof, may be produced by direct peptide synthesis using solid-phase techniques [see, e.g., Stewart et al., Solid-Phase Peptide Synthesis, W.H. Freeman Co., San Francisco, CA (1969); Merrifield, J. Am. Chem. Soc., 85:2149-2154 (1963)]. *In vitro* protein synthesis may be performed using manual techniques or by automation. Automated synthesis may be accomplished, for instance, using an Applied Biosystems Peptide Synthesizer (Foster City, CA) using manufacturer's instructions. Various portions of the PRO may be chemically synthesized separately and combined using chemical or enzymatic methods to produce the full-length PRO.

### 1. Isolation of DNA Encoring PRO

DNA encoding PRO may be obtained from a cDNA library prepared from tissue believed to possess the PRO mRNA and to express it at a detectable level. Accordingly, human PRO DNA can be conveniently obtained from a cDNA library prepared from human tissue, such as described in the Examples. The PRO-encoding gene may also be obtained from a genomic library or by known synthetic procedures (e.g., automated nucleic acid synthesis).

Libraries can be screened with probes (such as antibodies to the PRO or oligonucleotides of at least about 20-80 bases) designed to identify the gene of interest or the protein encoded by it. Screening the cDNA or genomic library with the selected probe may be conducted using standard procedures, such as described in Sambrook et al., Molecular Cloning: A Laboratory Manual (New York: Cold Spring Harbor Laboratory Press, 1989). An alternative means to isolate the gene encoding PRO is to use PCR methodology [Sambrook et al., supra; Dieffenbach et al., PCR Primer: A Laboratory Manual (Cold Spring Harbor Laboratory Press, 1995)].

The Examples below describe techniques for screening a cDNA library. The oligonucleotide sequences selected as probes should be of sufficient length and sufficiently unambiguous that false positives are minimized. The oligonucleotide is preferably labeled such that it can be detected upon hybridization to DNA in the library being screened. Methods of labeling are well known in the art, and include the use of radiolabels like ³²P-labeled ATP, biotinylation or enzyme labeling. Hybridization conditions, including moderate stringency and high stringency, are provided in Sambrook et al., supra.

Sequences identified in such library screening methods can be compared and aligned to other known sequences deposited and available in public databases such as GenBank or other private sequence databases. Sequence identity (at either the amino acid or nucleotide level) within defined regions of the molecule or across the full-length sequence can be determined using methods known in the art and as described herein.

Nucleic acid having protein coding sequence may be obtained by screening selected cDNA or genomic libraries using the deduced amino acid sequence disclosed herein for the first time, and, if necessary, using conventional primer extension procedures as described in Sambrook et al., supra, to detect precursors and processing intermediates of mRNA that may not have been reverse-transcribed into cDNA.

### 2. Selection and Transformation of Host Cells

Host cells are transfected or transformed with expression or cloning vectors described herein for PRO production and cultured in conventional nutrient media modified as appropriate for inducing promoters, selecting transformants, or amplifying the genes encoding the desired sequences. The culture conditions, such as media, temperature, pH and the like, can be selected by the skilled artisan without undue experimentation. In general, principles, protocols, and practical techniques for maximizing the productivity of cell cultures can be found in Mammalian Cell Biotechnology: a Practical Approach, M. Butler, ed. (IRL Press, 1991) and Sambrook et al., supra.

Methods of eukaryotic cell transfection and prokaryotic cell transformation are known to the ordinarily skilled artisan, for example, CaCl₂, CaPO₄, liposome-mediated and electroporation. Depending on the host cell used, transformation is performed using standard techniques appropriate to such cells. The calcium treatment employing calcium chloride, as described in Sambrook et al., supra, or electroporation is generally used for prokaryotes. Infection with *Agrobacterium tumefaciens* is used for transformation of certain plant cells, as described by Shaw et al., Gene, 23:315 (1983) and WO 89/05859 published 29 June 1989. For mammalian cells without such cell walls, the calcium phosphate precipitation method of Graham and van der Eb, Virology, 52:456-457 (1978) can be employed. General aspects of mammalian cell host system transfections have been described in U.S. Patent No. 4,399,216. Transformations into yeast are typically carried out according to the method of Van Solingen et al., J. Bact., 130:946 (1977) and Hsiao et al., Proc. Natl. Acad. Sci. (USA), 76:3829 (1979). However, other methods for introducing DNA into cells, such as by nuclear microinjection, electroporation, bacterial protoplast fusion with intact cells, or polycations, e.g., polybrene, polyornithine, may also be used. For various techniques for transforming mammalian cells, see Keown et al., Methods in Enzymology, 185:527-537 (1990) and Mansour et al., Nature, 336:348-352 (1988).

Suitable host cells for cloning or expressing the DNA in the vectors herein include prokaryote, yeast, or higher eukaryote cells. Suitable prokaryotes include but are not limited to eubacteria, such as Gram-negative or Gram-positive organisms, for example, Enterobacteriaceae such as *E. coli.* Various *E. coli* strains are publicly available, such as *E. coli* K12 strain MM294 (ATCC 31,446); *E. coli* X1776 (ATCC 31,537); *E. coli* strain W3110 (ATCC 27,325) and K5 772 (ATCC 53,635). Other suitable prokaryotic host cells include Enterobacteriaceae such as *Escherichia,* e.g., *E. coli, Enterobacter, Erwinia, Klebsiella, Proteus, Salmonella*, e.g., *Salmonella typhimurium, Serratia*, e.g., *Serratia marcescans*, and *Shigella*, as well as *Bacilli* such as *B. subtilis* and *B. licheniformis* (e.g., *B. licheniformis* 41P disclosed in DD 266,710 published 12 April 1989), *Pseudomonas* such as *P. aeruginosa*, and *Streptomyces*. These examples are illustrative rather than limiting. Strain W3110 is one particularly preferred host or parent host because it is a common host strain for recombinant DNA product fermentations. Preferably, the host cell secretes minimal amounts of proteolytic enzymes. For example, strain W3110 may be modified to effect a genetic mutation in the genes encoding proteins endogenous to the host, with examples of such hosts including *E. coli* W3110 strain 1A2, which has the complete genotype *tonA* ; *E. coli* W3110 strain 9E4, which has the complete genotype *tonA ptr3*; *E. coli* W3110 strain 27C7 (ATCC 55,244), which has the complete genotype *tonA ptr3 phoA E15 (argF-lac)169 degP ompT kan^{r}*; *E. coli* W3110 strain 37D6, which has the complete genotype *tonA ptr3 phoA E15 (argF-lac)169 degP ompT rbs7 ilvG kan^{r}*; *E. coli* W3110 strain 40B4, which is strain 37D6 with a non-kanamycin resistant *degP* deletion mutation; and an *E. coli* strain having mutant periplasmic protease disclosed in U.S. Patent No. 4,946,783 issued 7 August 1990. Alternatively, *in vitro* methods of cloning, e.g., PCR or other nucleic acid polymerase reactions, are suitable.

In addition to prokaryotes, eukaryotic microbes such as filamentous fungi or yeast are suitable cloning or expression hosts for PRO-encoding vectors. *Saccharomyces cerevisiae* is a commonly used lower eukaryotic host microorganism. Others include *Schizosaccharomyces pombe* (Beach and Nurse, Nature, 290: 140 [1981]; EP 139,383 published 2 May 1985); *Kluyveromyces* hosts (U.S. Patent No. 4,943,529; Fleer et al., Bio/Technology, 9:968-975 (1991)) such as, e.g., *K. lactis* (MW98-8C, CBS683, CBS4574; Louvencourt et al., J. Bacteriol., 154(2):737-742 [1983]), *K. fragilis* (ATCC 12,424), *K. bulgaricus* (ATCC 16,045), *K. wickeramii* (ATCC 24,178), *K. waltii* (ATCC 56,500), *K. drosophilarum* (ATCC 36,906; Van den Berg et al., Bio/Technology, 8:135 (1990)), *K. thermotolerans*, and *K. marxianus; yarrowia* (EP 402,226); *Pichia pastoris* (EP 183,070; Sreekrishna et al., J. Basic Microbiol., 28:265-278 [1988]); *Candida; Trichoderma reesia* (EP 244,234); *Neurospora crassa* (Case et al., Proc. Natl. Acad. Sci. USA, 76:5259-5263 [1979]); *Schwanniomyces* such as *Schwanniomyces occidentalis* (EP 394,538 published 31 October 1990); and filamentous fungi such as, e.g., *Neurospora, Penicillium, Tolypocladium* (WO 91/00357 published 10 January 1991), and *Aspergillus* hosts such as *A. nidulans* (Ballance et al., Biochem. Biophys. Res. Commun., 112:284-289 [1983]; Tilburn et al., Gene, 26:205-221 [1983]; Yelton et al., Proc. Natl. Acad. Sci. USA, 81: 1470-1474 [1984]) and *A. niger* (Kelly and Hynes, EMBO J., 4:475-479 [1985]). Methylotropic yeasts are suitable herein and include, but are not limited to, yeast capable of growth on methanol selected from the genera consisting of *Hansenula, Candida, Kloeckera, Pichia, Saccharomyces, Torulopsis*, and *Rhodotorula*. A list of specific species that are exemplary of this class of yeasts may be found in C. Anthony, The Biochemistry of Methylotrophs, 269 (1982).

Suitable host cells for the expression of glycosylated PRO are derived from multicellular organisms. Examples of invertebrate cells include insect cells such as Drosophila S2 and Spodoptera Sf9, as well as plant cells. Examples of useful mammalian host cell lines include Chinese hamster ovary (CHO) and COS cells. More specific examples include monkey kidney CV1 line transformed by SV40 (COS-7, ATCC CRL 1651); human embryonic kidney line (293 or 293 cells subcloned for growth in suspension culture, Graham et al., J. Gen Virol., 36:59 (1977)); Chinese hamster ovary cells/-DHFR (CHO, Urlaub and Chasin, Proc. Natl. Acad. Sci. USA, 77:4216 (1980)); mouse sertoli cells (TM4, Mather, Biol. Reprod., 23:243-251 (1980)); human lung cells (W138, ATCC CCL 75); human liver cells (Hep G2, HB 8065); and mouse mammary tumor (MMT 060562, ATCC CCL51). The selection of the appropriate host cell is deemed to be within the skill in the art.

### 3. Selection and Use of a Replicable Vector

The nucleic acid (e.g., cDNA or genomic DNA) encoding PRO may be inserted into a replicable vector for cloning (amplification of the DNA) or for expression. Various vectors are publicly available. The vector may, for example, be in the form of a plasmid, cosmid, viral particle, or phage. The appropriate nucleic acid sequence may be inserted into the vector by a variety of procedures. In general, DNA is inserted into an appropriate restriction endonuclease site(s) using techniques known in the art. Vector components generally include, but are not limited to, one or more of a signal sequence, an origin of replication, one or more marker genes, an enhancer element, a promoter, and a transcription termination sequence. Construction of suitable vectors containing one or more of these components employs standard ligation techniques which are known to the skilled artisan.

The PRO may be produced recombinantly not only directly, but also as a fusion polypeptide with a heterologous polypeptide, which may be a signal sequence or other polypeptide having a specific cleavage site at the N-terminus of the mature protein or polypeptide. In general, the signal sequence may be a component of the vector, or it may be a part of the PRO-encoding DNA that is inserted into the vector. The signal sequence may be a prokaryotic signal sequence selected, for example, from the group of the alkaline phosphatase, penicillinase, lpp, or heat-stable enterotoxin II leaders. For yeast secretion the signal sequence may be, e.g., the yeast invertase leader, alpha factor leader (including *Saccharomyces* and *Kluyveromyces* α-factor leaders, the latter described in U.S. Patent No. 5,010,182), or acid phosphatase leader, the *C. albicans* glucoamylase leader (EP 362,179 published 4 April 1990), or the signal described in WO 90/13646 published 15 November 1990. In mammalian cell expression, mammalian signal sequences may be used to direct secretion of the protein, such as signal sequences from secreted polypeptides of the same or related species, as well as viral secretory leaders.

Both expression and cloning vectors contain a nucleic acid sequence that enables the vector to replicate in one or more selected host cells. Such sequences are well known for a variety of bacteria, yeast, and viruses. The origin of replication from the plasmid pBR322 is suitable for most Gram-negative bacteria, the 2µ plasmid origin is suitable for yeast, and various viral origins (SV40, polyoma, adenovirus, VSV or BPV) are useful for cloning vectors in mammalian cells.

Expression and cloning vectors will typically contain a selection gene, also termed a selectable marker. Typical selection genes encode proteins that (a) confer resistance to antibiotics or other toxins, e.g., ampicillin, neomycin, methotrexate, or tetracycline, (b) complement auxotrophic deficiencies, or (c) supply critical nutrients not available from complex media, e.g., the gene encoding D-alanine racemase for *Bacilli*.

An example of suitable selectable markers for mammalian cells are those that enable the identification of cells competent to take up the PRO-encoding nucleic acid, such as DHFR or thymidine kinase. An appropriate host cell when wild-type DHFR is employed is the CHO cell line deficient in DHFR activity, prepared and propagated as described by Urlaub et al., Proc. Natl. Acad. Sci. USA, 77:4216 (1980). A suitable selection gene for use in yeast is the *trp*1 gene present in the yeast plasmid YRp7 [Stinchcomb et al., Nature, 282:39 (1979); Kingsman et al., Gene, 7:141 (1979); Tschemper et al., Gene, 10:157 (1980)]. The *trp*1 gene provides a selection marker for a mutant strain of yeast lacking the ability to grow in tryptophan, for example, ATCC No. 44076 or PEP4-1 [Jones, Genetics, 85:12 (1977)].

Expression and cloning vectors usually contain a promoter operably linked to the PRO-encoding nucleic acid sequence to direct mRNA synthesis. Promoters recognized by a variety of potential host cells are well known. Promoters suitable for use with prokaryotic hosts include the β-lactamase and lactose promoter systems [Chang et al., Nature, 275:615 (1978); Goeddel et al., Nature, 281:544 (1979)], alkaline phosphatase, a tryptophan (trp) promoter system [Goeddel, Nucleic Acids Res., 8:4057 (1980); EP 36,776], and hybrid promoters such as the tac promoter [deBoer et al., Proc. Natl. Acad. Sci. USA, 80:21-25 (1983)]. Promoters for use in bacterial systems also will contain a Shine-Dalgarno (S.D.) sequence operably linked to the DNA encoding PRO.

Examples of suitable promoting sequences for use with yeast hosts include the promoters for 3-phosphoglycerate kinase [Hitzeman et al., J. Biol. Chem., 255:2073 (1980)] or other glycolytic enzymes [Hess et al., J. Adv. Enzyme Reg., 7:149 (1968); Holland, Biochemistry, 17:4900 (1978)], such as enolase, glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase.

Other yeast promoters, which are inducible promoters having the additional advantage of transcription controlled by growth conditions, are the promoter regions for alcohol dehydrogenase 2, isocytochrome C, acid phosphatase, degradative enzymes associated with nitrogen metabolism, metallothionein, glyceraldehyde-3-phosphate dehydrogenase, and enzymes responsible for maltose and galactose utilization. Suitable vectors and promoters for use in yeast expression are further described in EP 73,657.

PRO transcription from vectors in mammalian host cells is controlled, for example, by promoters obtained from the genomes of viruses such as polyoma virus, fowlpox virus (UK 2,211,504 published 5 July 1989), adenovirus (such as Adenovirus 2), bovine papilloma virus, avian sarcoma virus, cytomegalovirus, a retrovirus, hepatitis-B virus and Simian Virus 40 (SV40), from heterologous mammalian promoters, e.g., the actin promoter or an immunoglobulin promoter, and from heat-shock promoters, provided such promoters are compatible with the host cell systems.

Transcription of a DNA encoding the PRO by higher eukaryotes may be increased by inserting an enhancer sequence into the vector. Enhancers are cis-acting elements of DNA, usually about from 10 to 300 bp, that act on a promoter to increase its transcription. Many enhancer sequences are now known from mammalian genes (globin, elastase, albumin, α-fetoprotein, and insulin). Typically, however, one will use an enhancer from a eukaryotic cell virus. Examples include the SV40 enhancer on the late side of the replication origin (bp 100-270), the cytomegalovirus early promoter enhancer, the polyoma enhancer on the late side of the replication origin, and adenovirus enhancers. The enhancer may be spliced into the vector at a position 5' or 3' to the PRO coding sequence, but is preferably located at a site 5' from the promoter.

Expression vectors used in eukaryotic host cells (yeast, fungi, insect, plant, animal, human, or nucleated cells from other multicellular organisms) will also contain sequences necessary for the termination of transcription and for stabilizing the mRNA. Such sequences are commonly available from the 5' and, occasionally 3', untranslated regions of eukaryotic or viral DNAs or cDNAs. These regions contain nucleotide segments transcribed as polyadenylated fragments in the untranslated portion of the mRNA encoding PRO.

Still other methods, vectors, and host cells suitable for adaptation to the synthesis of PRO in recombinant vertebrate cell culture are described in Gething et al., Nature, 293:620-625 (1981); Mantei et al., Nature, 281:40-46 (1979); EP 117,060; and EP 117,058.

### 4. Detecting Gene Amplification/Expression

Gene amplification and/or expression may be measured in a sample directly, for example, by conventional Southern blotting, Northern blotting to quantitate the transcription of mRNA [Thomas, Proc. Natl. Acad. Sci. USA, 77:5201-5205 (1980)], dot blotting (DNA analysis), or *in situ* hybridization, using an appropriately labeled probe, based on the sequences provided herein. Alternatively, antibodies may be employed that can recognize specific duplexes, including DNA duplexes, RNA duplexes, and DNA-RNA hybrid duplexes or DNA-protein duplexes. The antibodies in turn may be labeled and the assay may be carried out where the duplex is bound to a surface, so that upon the formation of duplex on the surface, the presence of antibody bound to the duplex can be detected.

Gene expression, alternatively, may be measured by immunological methods, such as immunohistochemical staining of cells or tissue sections and assay of cell culture or body fluids, to quantitate directly the expression of gene product. Antibodies useful for immunohistochemical staining and/or assay of sample fluids may be either monoclonal or polyclonal, and may be prepared in any mammal. Conveniently, the antibodies may be prepared against a native sequence PRO polypeptide or against a synthetic peptide based on the DNA sequences provided herein or against exogenous sequence fused to PRO DNA and encoding a specific antibody epitope.

### 5. Purification of Polypeptide

Forms of PRO may be recovered from culture medium or from host cell lysates. If membrane-bound, it can be released from the membrane using a suitable detergent solution (e.g. Triton-X 100) or by enzymatic cleavage. Cells employed in expression of PRO can be disrupted by various physical or chemical means, such as freeze-thaw cycling, sonication, mechanical disruption, or cell lysing agents.

It may be desired to purify PRO from recombinant cell proteins or polypeptides. The following procedures are exemplary of suitable purification procedures: by fractionation on an ion-exchange column; ethanol precipitation; reverse phase HPLC; chromatography on silica or on a cation-exchange resin such as DEAE; chromatofocusing; SDS-PAGE; ammonium sulfate precipitation; gel filtration using, for example, Sephadex G-75; protein A Sepharose columns to remove contaminants such as IgG; and metal chelating columns to bind epitope-tagged forms of the PRO. Various methods of protein purification may be employed and such methods are known in the art and described for example in Deutscher, Methods in Enzymology, 182 (1990); Scopes, Protein Purification: Principles and Practice, Springer-Verlag, New York (1982). The purification step(s) selected will depend, for example, on the nature of the production process used and the particular PRO produced.

### E. Tissue Distribution

The location of tissues expressing the PRO can be identified by determining mRNA expression in various human tissues. The location of such genes provides information about which tissues are most likely to be affected by the stimulating and inhibiting activities of the PRO polypeptides. The location of a gene in a specific tissue also provides sample tissue for the activity blocking assays discussed below.

As noted before, gene expression in various tissues may be measured by conventional Southern blotting, Northern blotting to quantitate the transcription of mRNA (Thomas, Proc. Natl. Acad. Sci. USA, 77:5201-5205 [1980]), dot blotting (DNA analysis), or *in situ* hybridization, using an appropriately labeled probe, based on the sequences provided herein. Alternatively, antibodies may be employed that can recognize .specific duplexes, including DNA duplexes, RNA duplexes, and DNA-RNA hybrid duplexes or DNA-protein duplexes.

Gene expression in various tissues, alternatively, may be measured by immunological methods, such as immunohistochemical staining of tissue sections and assay of cell culture or body fluids, to quantitate directly the expression of gene product. Antibodies useful for immunohistochemical staining and/or assay of sample fluids may be either monoclonal or polyclonal, and may be prepared in any mammal. Conveniently, the antibodies may be prepared against a native sequence of a PRO polypeptide or against a synthetic peptide based on the DNA sequences encoding the PRO polypeptide or against an exogenous sequence fused to a DNA encoding a PRO polypeptide and encoding a specific antibody epitope. General techniques for generating antibodies, and special protocols for Northern blotting and *in situ* hybridization are provided below.

### F. Antibody Binding Studies

The activity of the PRO polypeptides can be further verified by antibody binding studies, in which the ability of anti-PRO antibodies to inhibit the effect of the PRO polypeptides, respectively, on tissue cells is tested. Exemplary antibodies include polyclonal, monoclonal, humanized, bispecific, and heteroconjugate antibodies, the preparation of which will be described hereinbelow.

Antibody binding studies may be carried out in any known assay method, such as competitive binding assays, direct and indirect sandwich assays, and immunoprecipitation assays. Zola, Monoclonal Antibodies: A Manual of Techniques, pp.147-158 (CRC Press, Inc., 1987).

Competitive binding assays rely on the ability of a labeled standard to compete with the test sample analyte for binding with a limited amount of antibody. The amount of target protein in the test sample is inversely proportional to the amount of standard that becomes bound to the antibodies. To facilitate determining the amount of standard that becomes bound, the antibodies preferably are insolubilized before or after the competition, so that the standard and analyte that are bound to the antibodies may conveniently be separated from the standard and analyte which remain unbound.

Sandwich assays involve the use of two antibodies, each capable of binding to a different immunogenic portion, or epitope, of the protein to be detected. In a sandwich assay, the test sample analyte is bound by a first antibody which is immobilized on a solid support, and thereafter a second antibody binds to the analyte, thus forming an insoluble three-part complex. See, *e.g.,* US Pat No. 4,376,110. The second antibody may itself be labeled with a detectable moiety (direct sandwich assays) or may be measured using an anti-immunoglobulin antibody that is labeled with a detectable moiety (indirect sandwich assay). For example, one type of sandwich assay is an ELISA assay, in which case the detectable moiety is an enzyme.

For immunohistochemistry, the tissue sample may be fresh or frozen or may be embedded in paraffin and fixed with a preservative such as formalin, for example.

### G. Cell-Based Assays

Cell-based assays and animal models for immune related diseases can be used to further understand the relationship between the genes and polypeptides identified herein and the development and pathogenesis of immune related disease.

In a different approach, cells of a cell type known to be involved in a particular immune related disease are transfected with the cDNAs described herein, and the ability of these cDNAs to stimulate or inhibit immune function is analyzed. Suitable cells can be transfected with the desired gene, and monitored for immune function activity. Such transfected cell lines can then be used to test the ability of poly- or monoclonal antibodies or antibody compositions to inhibit or stimulate immune function, for example to modulate T-cell proliferation or inflammatory cell infiltration. Cells transfected with the coding sequences of the genes identified herein can further be used to identify drug candidates for the treatment of immune related diseases.

In addition, primary cultures derived from transgenic animals (as described below) can be used in the cell-based assays herein, although stable cell lines are preferred. Techniques to derive continuous cell lines from transgenic animals are well known in the art (see, *e.g.,* Small et al., Mol. Cell. Biol. 5: 642-648 [1985]).

One suitable cell based assay is the mixed lymphocyte reaction (MLR). Current Protocols in Immunology, unit 3.12; edited by J E Coligan, A M Kruisbeek, D H Marglies, E M Shevach, W Strober, National Institutes of Health, Published by John Wiley & Sons, Inc. In this assay, the ability of a test compound to stimulate or inhibit the proliferation of activated T cells is assayed. A suspension of responder T cells is cultured with allogeneic stimulator cells and the proliferation of T cells is measured by uptake of tritiated thymidine. This assay is a general measure of T cell reactivity. Since the majority of T cells respond to and produce IL-2 upon activation, differences in responsiveness in this assay in part reflect differences in IL-2 production by the responding cells. The MLR results can be verified by a standard lymphokine (IL-2) detection assay. *Current Protocols in Immunology*, above, 3.15, 6.3.

A proliferative T cell response in an MLR assay may be due to direct mitogenic properties of an assayed molecule or to external antigen induced activation. Additional verification of the T cell stimulatory activity of the PRO polypeptides can be obtained by a costimulation assay. T cell activation requires an antigen specific signal mediated through the T-cell receptor (TCR) and a costimulatory signal mediated through a second ligand binding interaction, for example, the B7 (CD80, CD86)/CD28 binding interaction. CD28 crosslinking increases lymphokine secretion by activated T cells. T cell activation has both negative and positive controls through the binding of ligands which have a negative or positive effect. CD28 and CTLA-4 are related glycoproteins in the Ig superfamily which bind to B7. CD28 binding to B7 has a positive costimulation effect of T cell activation; conversely, CTLA-4 binding to B7 has a T cell deactivating effect. Chambers, C. A. and Allison, J. P., Curr. Opin. Immunol. (1997) 9:396. Schwartz, R. H., Cell (1992) 71:1065; Linsey, P. S. and Ledbetter, J. A., Annu. Rev. Immunol. (1993) 11:191; June, C. H. et al, Immunol. Today (1994) 15:321; Jenkins, M. K., Immunity (1994) 1:405. In a costimulation assay, the PRO polypeptides are assayed for T cell costimulatory or inhibitory activity.

Direct use of a stimulating compound as in the invention has been validated in experiments with 4-1BB glycoprotein, a member of the tumor necrosis factor receptor family, which binds to a ligand (4-1BBL) expressed on primed T cells and signals T cell activation and growth. Alderson, M. E. et al., J. Immunol. (1994) 24:2219.

The use of an agonist stimulating compound has also been validated experimentally. Activation of 4-1BB by treatment with an agonist anti-4-1BB antibody enhances eradication of tumors. Hellstrom, I. and Hellstrom, K. E., Crit. Rev. Immunol. (1998) 18:1. Immunoadjuvant therapy for treatment of tumors, described in more detail below, is another example of the use of the stimulating compounds of the invention.

Alternatively, an immune stimulating or enhancing effect can also be achieved by administration of a PRO which has vascular permeability enhancing properties. Enhanced vascular permeability would be beneficial to disorders which can be attenuated by local infiltration of immune cells (*e*.*g*., monocytes, eosinophils, PMNs) and inflammation.

On the other hand, PRO polypeptides, as well as other compounds of the invention, which are direct inhibitors of T cell proliferation/activation, lymphokine secretion, and/or vascular permeability can be directly used to suppress the immune response. These compounds are useful to reduce the degree of the immune response and to treat immune related diseases characterized by a hyperactive, superoptimal, or autoimmune response. This use of the compounds of the invention has been validated by the experiments described above in which CTLA-4 binding to receptor B7 deactivates T cells. The direct inhibitory compounds of the invention function in an analogous manner. The use of compound which suppress vascular permeability would be expected to reduce inflammation. Such uses would be beneficial in treating conditions associated with excessive inflammation.

Alternatively, compounds, *e.g.,* antibodies, which bind to stimulating PRO polypeptides and block the stimulating effect of these molecules produce a net inhibitory effect and can be used to suppress the T cell mediated immune response by inhibiting T cell proliferation/activation and/or lymphokine secretion. Blocking the stimulating effect of the polypeptides suppresses the immune response of the mammal. This use has been validated in experiments using an anti-IL2 antibody. In these experiments, the antibody binds to IL2 and blocks binding of IL2 to its receptor thereby achieving a T cell inhibitory effect.

### H. Animal Models

The results of the cell based in vitro assays can be further verified using *in vivo* animal models and assays for T-cell function. A variety of well known animal models can be used to further understand the role of the genes identified herein in the development and pathogenesis of immune related disease, and to test the efficacy of candidate therapeutic agents, including antibodies, and other antagonists of the native polypeptides, including small molecule antagonists. The *in vivo* nature of such models makes them predictive of responses in human patients. Animal models of immune related diseases include both non-recombinant and recombinant (transgenic) animals. Non-recombinant animal models include, for example, rodent, *e*.*g*., murine models. Such models can be generated by introducing cells into syngeneic mice using standard techniques, *e*.*g*., subcutaneous injection, tail vein injection, spleen implantation, intraperitoneal implantation, implantation under the renal capsule, *etc.*

Graft-versus-host disease occurs when immunocompetent cells are transplanted into immunosuppressed or tolerant patients. The donor cells recognize and respond to host antigens. The response can vary from life threatening severe inflammation to mild cases of diarrhea and weight loss. Graft-versus-host disease models provide a means of assessing T cell reactivity against MHC antigens and minor transplant antigens. A suitable procedure is described in detail in Current Protocols in Immunology, above, unit 4.3.

An animal model for skin allograft rejection is a means of testing the ability of T cells to mediate *in vivo* tissue destruction and a measure of their role in transplant rejection. The most common and accepted models use murine tail-skin grafts. Repeated experiments have shown that skin allograft rejection is mediated by T cells, helper T cells and killer-effector T cells, and not antibodies. Auchincloss, H. Jr. and Sachs, D. H., Fundamental Immunology, 2nd ed., W. E. Paul ed., Raven Press, NY, 1989, 889-992. A suitable procedure is described in detail in *Current Protocols in Immunology,* above, unit 4.4. Other transplant rejection models which can be used to test the compounds of the invention are the allogeneic heart transplant models described by Tanabe, M. et al, Transplantation (1994) 58:23 and Tinubu, S. A. et al, J. Immunol. (1994) 4330-4338.

Animal models for delayed type hypersensitivity provides an assay of cell mediated immune function as well. Delayed type hypersensitivity reactions are a T cell mediated *in vivo* immune response characterized by inflammation which does not reach a peak until after a period of time has elapsed after challenge with an antigen. These reactions also occur in tissue specific autoimmune diseases such as multiple sclerosis (MS) and experimental autoimmune encephalomyelitis (EAE, a model for MS). A suitable procedure is described in detail in *Current Protocols in Immunology,* above, unit 4.5.

EAE is a T cell mediated autoimmune disease characterized by T cell and mononuclear cell inflammation and subsequent demyelination of axons in the central nervous system. EAE is generally considered to be a relevant animal model for MS in humans. Bolton, C., Multiple Sclerosis (1995) 1:143. Both acute and relapsing-remitting models have been developed. The compounds of the invention can be tested for T cell stimulatory or inhibitory activity against immune mediated demyelinating disease using the protocol described in *Current Protocols in Immunology,* above, units 15.1 and 15.2. See also the models for myelin disease in which oligodendrocytes or Schwann cells are grafted into the central nervous system as described in Duncan, I. D. et al, Molec. Med. Today (1997) 554-561.

Contact hypersensitivity is a simple delayed type hypersensitivity *in vivo* assay of cell mediated immune function. In this procedure, cutaneous exposure to exogenous haptens which gives rise to a delayed type hypersensitivity reaction which is measured and quantitated. Contact sensitivity involves an initial sensitizing phase followed by an elicitation phase. The elicitation phase occurs when the T lymphocytes encounter an antigen to which they have had previous contact. Swelling and inflammation occur, making this an excellent model of human allergic contact dermatitis. A suitable procedure is described in detail in Current Protocols in Immunology, Eds. J. E. Cologan, A. M. Kruisbeek, D. H. Margulies, E. M. Shevach and W. Strober, John Wiley & Sons, Inc., 1994, unit 4.2. See also Grabbe, S. and Schwarz, T, Immun. Today 19 (1): 37-44 (1998).

An animal model for arthritis is collagen-induced arthritis. This model shares clinical, histological and immunological characteristics of human autoimmune rheumatoid arthritis and is an acceptable model for human autoimmune arthritis. Mouse and rat models are characterized by synovitis, erosion of cartilage and subchondral bone. The compounds of the invention can be tested for activity against autoimmune arthritis using the protocols described in *Current Protocols in Immunology,* above, units 15.5. See also the model using a monoclonal antibody to CD18 and VLA-4 integrins described in Issekutz, A.C. et al., Immunology (1996) 88:569.

A model of asthma has been described in which antigen-induced airway hyper-reactivity, pulmonary eosinophilia and inflammation are induced by sensitizing an animal with ovalbumin and then challenging the animal with the same protein delivered by aerosol. Several animal models (guinea pig, rat, non-human primate) show symptoms similar to atopic asthma in humans upon challenge with aerosol antigens. Murine models have many of the features of human asthma. Suitable procedures to test the compounds of the invention for activity and effectiveness in the treatment of asthma are described by Wolyniec, W. W. et al, Am. J. Respir. Cell Mol. Biol. (1998) 18:777 and the references cited therein.

Additionally, the compounds of the invention can be tested on animal models for psoriasis like diseases. Evidence suggests a T cell pathogenesis for psoriasis. The compounds of the invention can be tested in the scid/scid mouse model described by Schon, M. P. et al, Nat. Med. (1997) 3:183, in which the mice demonstrate histopathologic skin lesions resembling psoriasis. Another suitable model is the human skin/scid mouse chimera prepared as described by Nickoloff, B. J. et al, Am. J. Path. (1995) 146:580.

Recombinant (transgenic) animal models can be engineered by introducing the coding portion of the genes identified herein into the genome of animals of interest, using standard techniques for producing transgenic animals. Animals that can serve as a target for transgenic manipulation include, without limitation, mice, rats, rabbits, guinea pigs, sheep, goats, pigs, and non-human primates, *e*.*g*., baboons, chimpanzees and monkeys. Techniques known in the art to introduce a transgene into such animals include pronucleic microinjection (Hoppe and Wanger, U.S. Patent No. 4,873,191); retrovirus-mediated gene transfer into germ lines (*e*.*g*., Van der Putten et al., Proc. Natl. Acad. Sci. USA 82, 6148-615 [1985]); gene targeting in embryonic stem cells (Thompson et al., Cell 56, 313-321 [1989]); electroporation of embryos (Lo, Mol. Cel. Biol. 3, 1803-1814 [1983]); sperm-mediated gene transfer (Lavitrano et al., Cell 57, 717-73 [1989]). For review, see, for example, U.S. Patent No. 4,736,866.

For the purpose of the present invention, transgenic animals include those that carry the transgene only in part of their cells ("mosaic animals"). The transgene can be integrated either as a single transgene, or in concatamers, *e*.*g*., head-to-head or head-to-tail tandems. Selective introduction of a transgene into a particular cell type is also possible by following, for example, the technique of Lasko et al., Proc. Natl. Acad. Sci. USA 89, 6232-636 (1992).

The expression of the transgene in transgenic animals can be monitored by standard techniques. For example, Southern blot analysis or PCR amplification can be used to verify the integration of the transgene. The level of mRNA expression can then be analyzed using techniques such as *in situ* hybridization, Northern blot analysis, PCR, or immunocytochemistry.

The animals may be further examined for signs of immune disease pathology, for example by histological examination to determine infiltration of immune cells into specific tissues. Blocking experiments can also be performed in which the transgenic animals are treated with the compounds of the invention to determine the extent of the T cell proliferation stimulation or inhibition of the compounds. In these experiments, blocking antibodies which bind to the PRO polypeptide, prepared as described above, are administered to the animal and the effect on immune function is determined.

Alternatively, "knock out" animals can be constructed which have a defective or altered gene encoding a polypeptide identified herein, as a result of homologous recombination between the endogenous gene encoding the polypeptide and altered genomic DNA encoding the same polypeptide introduced into an embryonic cell of the animal. For example, cDNA encoding a particular polypeptide can be used to clone genomic DNA encoding that polypeptide in accordance with established techniques. A portion of the genomic DNA encoding a particular polypeptide can be deleted or replaced with another gene, such as a gene encoding a selectable marker which can be used to monitor integration. Typically, several kilobases of unaltered flanking DNA (both at the 5' and 3' ends) are included in the vector [see *e*.*g*., Thomas and Capecchi, Cell, 51:503 (1987) for a description of homologous recombination vectors]. The vector is introduced into an embryonic stem cell line (*e*.*g*., by electroporation) and cells in which the introduced DNA has homologously recombined with the endogenous DNA are selected [see *e*.*g*., Li et al., Cell, 69:915 (1992)]. The selected cells are then injected into a blastocyst of an animal (*e*.*g*., a mouse or rat) to form aggregation chimeras [see *e*.*g*., Bradley, in Teratocarcinomas and Embryonic Stem Cells: A Practical Approach, E. J. Robertson, ed. (IRL, Oxford, 1987), pp. 113-152]. A chimeric embryo can then be implanted into a suitable pseudopregnant female foster animal and the embryo brought to term to create a "knock out" animal. Progeny harboring the homologously recombined DNA in their germ cells can be identified by standard techniques and used to breed animals in which all cells of the animal contain the homologously recombined DNA. Knockout animals can be characterized for instance, for their ability to defend against certain pathological conditions and for their development of pathological conditions due to absence of the polypeptide.

### I. ImmunoAdjuvant Therapy

In one embodiment, the immunostimulating compounds of the invention can be used in immunoadjuvant therapy for the treatment of tumors (cancer). It is now well established that T cells recognize human tumor specific antigens. One group of tumor antigens, encoded by the MAGE, BAGE and GAGE families of genes, are silent in all adult normal tissues , but are expressed in significant amounts in tumors, such as melanomas, lung tumors, head and neck tumors, and bladder carcinomas. DeSmet, C. et al., (1996) Proc. Natl. Acad Sci. USA, 93:7149. It has been shown that costimulation of T cells induces tumor regression and an antitumor response both *in vitro* and *in vivo.* Melero, I. et al., Nature Medicine (1997) 3:682; Kwon, E. D. et al., Proc. Natl. Acad. Sci. USA (1997) 94: 8099; Lynch, D. H. et al, Nature Medicine (1997) 3:625; Finn, O. J. and Lotze, M. T., J. Immunol. (1998) 21:114. The stimulatory compounds of the invention can be administered as adjuvants, alone or together with a growth regulating agent, cytotoxic agent or chemotherapeutic agent, to stimulate T cell proliferation/activation and an antitumor response to tumor antigens. The growth regulating, cytotoxic, or chemotherapeutic agent may be administered in conventional amounts using known administration regimes. Immunostimulating activity by the compounds of the invention allows reduced amounts of the growth regulating, cytotoxic, or chemotherapeutic agents thereby potentially lowering the toxicity to the patient.

### J. Screening Assays for Drug Candidates

Screening assays for drug candidates are designed to identify compounds that bind to or complex with the polypeptides encoded by the genes identified herein or a biologically active fragment thereof, or otherwise interfere with the interaction of the encoded polypeptides with other cellular proteins. Such screening assays will include assays amenable to high-throughput screening of chemical libraries, making them particularly suitable for identifying small molecule drug candidates. Small molecules contemplated include synthetic organic or inorganic compounds, including peptides, preferably soluble peptides, (poly)peptide-immunoglobulin fusions, and, in particular, antibodies including, without limitation, poly- and monoclonal antibodies and antibody fragments, single-chain antibodies, anti-idiotypic antibodies, and chimeric or humanized versions of such antibodies or fragments, as well as human antibodies and antibody fragments. The assays can be performed in a variety of formats, including protein-protein binding assays, biochemical screening assays, immunoassays and cell based assays, which are well characterized in the art. All assays are common in that they call for contacting the drug candidate with a polypeptide encoded by a nucleic acid identified herein under conditions and for a time sufficient to allow these two components to interact.

In binding assays, the interaction is binding and the complex formed can be isolated or detected in the reaction mixture. In a particular embodiment, the polypeptide encoded by the gene identified herein or the drug candidate is immobilized on a solid phase, *e.g.,* on a microtiter plate, by covalent or non-covalent attachments. Non-covalent attachment generally is accomplished by coating the solid surface with a solution of the polypeptide and drying. Alternatively, an immobilized antibody, *e*.*g*., a monoclonal antibody, specific for the polypeptide to be immobilized can be used to anchor it to a solid surface. The assay is performed by adding the non-immobilized component, which may be labeled by a detectable label, to the immobilized component, *e*.*g*., the coated surface containing the anchored component. When the reaction is complete, the non-reacted components are removed, *e.g.,* by washing, and complexes anchored on the solid surface are detected. When the originally non-immobilized component carries a detectable label, the detection of label immobilized on the surface indicates that complexing occurred. Where the originally non-immobilized component does not carry a label, complexing can be detected, for example, by using a labelled antibody specifically binding the immobilized complex.

If the candidate compound interacts with but does not bind to a particular protein encoded by a gene identified herein, its interaction with that protein can be assayed by methods well known for detecting protein-protein interactions. Such assays include traditional approaches, such as, cross-linking, co-immunoprecipitation, and co-purification through gradients or chromatographic columns. In addition, protein-protein interactions can be monitored by using a yeast-based genetic system described by Fields and co-workers [Fields and Song, Nature (London) 340, 245-246 (1989); Chien et al., Proc. Natl. Acad. Sci. USA 88, 9578-9582 (1991)] as disclosed by Chevray and Nathans, Proc. Natl. Acad. Sci. USA 89, 5789-5793 (1991). Many transcriptional activators, such as yeast GAL4, consist of two physically discrete modular domains, one acting as the DNA-binding domain, while the other one functioning as the transcription activation domain. The yeast expression system described in the foregoing publications (generally referred to as the "two-hybrid system") takes advantage of this property, and employs two hybrid proteins, one in which the target protein is fused to the DNA-binding domain of GAL4, and another, in which candidate activating proteins are fused to the activation domain. The expression of a GAL1-*lac*Z reporter gene under control of a GAL4-activated promoter depends on reconstitution of GAL4 activity via protein-protein interaction. Colonies containing interacting polypeptides are detected with a chromogenic substrate for β-galactosidase. A complete kit (MATCHMAKER™) for identifying protein-protein interactions between two specific proteins using the two-hybrid technique is commercially available from Clontech. This system can also be extended to map protein domains involved in specific protein interactions as well as to pinpoint amino acid residues that are crucial for these interactions.

In order to find compounds that interfere with the interaction of a gene identified herein and other intra- or extracellular components can be tested, a reaction mixture is usually prepared containing the product of the gene and the intra- or extracellular component under conditions and for a time allowing for the interaction and binding of the two products. To test the ability of a test compound to inhibit binding, the reaction is run in the absence and in the presence of the test compound. In addition, a placebo may be added to a third reaction mixture, to serve as positive control. The binding (complex formation) between the test compound and the intra- or extracellular component present in the mixture is monitored as described above. The formation of a complex in the control reaction(s) but not in the reaction mixture containing the test compound indicates that the test compound interferes with the interaction of the test compound and its reaction partner.

### K. Compositions and Methods for the Treatment of Immune Related Diseases

The compositions useful in the treatment of immune related diseases include, without limitation, proteins, antibodies, small organic molecules, peptides, phosphopeptides, antisense and ribozyme molecules, triple helix molecules, *etc*. that inhibit or stimulate immune function, for example, T cell proliferation/activation, lymphokine release, or immune cell infiltration.

For example, antisense RNA and RNA molecules act to directly block the translation of mRNA by hybridizing to targeted mRNA and preventing protein translation. When antisense DNA is used, oligodeoxyribonucleotides derived from the translation initiation site, *e*.*g*., between about -10 and +10 positions of the target gene nucleotide sequence, are preferred.

Ribozymes are enzymatic RNA molecules capable of catalyzing the specific cleavage of RNA. Ribozymes act by sequence-specific hybridization to the complementary target RNA, followed by endonucleolytic cleavage. Specific ribozyme cleavage sites within a potential RNA target can be identified by known techniques. For further details see, *e*.*g*., Rossi, Current Biology 4, 469-471 (1994), and PCT publication No. WO 97/33551 (published September 18, 1997).

Nucleic acid molecules in triple helix formation used to inhibit transcription should be single-stranded and composed of deoxynucleotides. The base composition of these oligonucleotides is designed such that it promotes triple helix formation via Hoogsteen base pairing rules, which generally require sizeable stretches of purines or pyrimidines on one strand of a duplex. For further details see, *e*.*g*., PCT publication No. WO 97/33551, *supra.*
These molecules can be identified by any or any combination of the screening assays discussed above and/or by any other screening techniques well known for those skilled in the art.

### L. Anti-PRO Antibodies

The present invention further provides anti-PRO antibodies. Exemplary antibodies include polyclonal, monoclonal, humanized, bispecific, and heteroconjugate antibodies.

### 1. Polyclonal Antibodies

The anti-PRO antibodies may comprise polyclonal antibodies. Methods of preparing polyclonal antibodies are known to the skilled artisan. Polyclonal antibodies can be raised in a mammal, for example, by one or more injections of an immunizing agent and, if desired, an adjuvant. Typically, the immunizing agent and/or adjuvant will be injected in the mammal by multiple subcutaneous or intraperitoneal injections. The immunizing agent may include the PRO polypeptide or a fusion protein thereof. It may be useful to conjugate the immunizing agent to a protein known to be immunogenic in the mammal being immunized. Examples of such immunogenic proteins include but are not limited to keyhole limpet hemocyanin, serum albumin, bovine thyroglobulin, and soybean trypsin inhibitor. Examples of adjuvants which may be employed include Freund's complete adjuvant and MPL-TDM adjuvant (monophosphoryl Lipid A, synthetic trehalose dicorynomycolate). The immunization protocol may be selected by one skilled in the art without undue experimentation.

### 2. Monoclonal Antibodies

The anti-PRO antibodies may, alternatively, be monoclonal antibodies. Monoclonal antibodies may be prepared using hybridoma methods, such as those described by Kohler and Milstein, Nature, 256:495 (1975). In a hybridoma method, a mouse, hamster, or other appropriate host animal, is typically immunized with an immunizing agent to elicit lymphocytes that produce or are capable of producing antibodies that will specifically bind to the immunizing agent. Alternatively, the lymphocytes may be immunized *in vitro.*

The immunizing agent will typically include the PRO polypeptide or a fusion protein thereof. Generally, either peripheral blood lymphocytes ("PBLs") are used if cells of human origin are desired, or spleen cells or lymph node cells are used if non-human mammalian sources are desired. The lymphocytes are then fused with an immortalized cell line using a suitable fusing agent, such as polyethylene glycol, to form a hybridoma cell [Goding, Monoclonal Antibodies: Principles and Practice, Academic Press, (1986) pp. 59-103]. Immortalized cell lines are usually transformed mammalian cells, particularly myeloma cells of rodent, bovine and human origin. Usually, rat or mouse myeloma cell lines are employed. The hybridoma cells may be cultured in a suitable culture medium that preferably contains one or more substances that inhibit the growth or survival of the unfused, immortalized cells. For example, if the parental cells lack the enzyme hypoxanthine guanine phosphoribosyl transferase (HGPRT or HPRT), the culture medium for the hybridomas typically will include hypoxanthine, aminopterin, and thymidine ("HAT medium"), which substances prevent the growth of HGPRT-deficient cells.

Preferred immortalized cell lines are those that fuse efficiently, support stable high level expression of antibody by the selected antibody-producing cells, and are sensitive to a medium such as HAT medium. More preferred immortalized cell lines are murine myeloma lines, which can be obtained, for instance, from the Salk Institute Cell Distribution Center, San Diego, California and the American Type Culture Collection, Manassas, Virginia. Human myeloma and mouse-human heteromyeloma cell lines also have been described for the production of human monoclonal antibodies [Kozbor, J. Immunol., 133:3001 (1984); Brodeur et al., Monoclonal Antibody Production Techniques and Applications, Marcel Dekker, Inc., New York, (1987) pp. 51-63].

The culture medium in which the hybridoma cells are cultured can then be assayed for the presence of monoclonal antibodies directed against PRO. Preferably, the binding specificity of monoclonal antibodies produced by the hybridoma cells is determined by immunoprecipitation or by an *in vitro* binding assay, such as radioimmunoassay (RIA) or enzyme-linked immunoabsorbent assay (ELISA). Such techniques and assays are known in the art. The binding affinity of the monoclonal antibody can, for example, be determined by the Scatchard analysis of Munson and Pollard, Anal. Biochem., 107:220 (1980).

After the desired hybridoma cells are identified, the clones may be subcloned by limiting dilution procedures and grown by standard methods [Goding, supra]. Suitable culture media for this purpose include, for example, Dulbecco's Modified Eagle's Medium and RPMI-1640 medium. Alternatively, the hybridoma cells may be grown *in vivo* as ascites in a mammal.

The monoclonal antibodies secreted by the subclones may be isolated or purified from the culture medium or ascites fluid by conventional immunoglobulin purification procedures such as, for example, protein A-Sepharose, hydroxylapatite chromatography, gel electrophoresis, dialysis, or affinity chromatography.

The monoclonal antibodies may also be made by recombinant DNA methods, such as those described in U.S. Patent No. 4,816,567. DNA encoding the monoclonal antibodies of the invention can be readily isolated and sequenced using conventional procedures (e.g., by using oligonucleotide probes that are capable of binding specifically to genes encoding the heavy and light chains of murine antibodies). The hybridoma cells of the invention serve as a preferred source of such DNA. Once isolated, the DNA may be placed into expression vectors, which are then transfected into host cells such as simian COS cells, Chinese hamster ovary (CHO) cells, or myeloma cells that do not otherwise produce immunoglobulin protein, to obtain the synthesis of monoclonal antibodies in the recombinant host cells. The DNA also may be modified, for example, by substituting the coding sequence for human heavy and light chain constant domains in place of the homologous murine sequences [U.S. Patent No. 4,816,567; Morrison et al., supra] or by covalently joining to the immunoglobulin coding sequence all or part of the coding sequence for a non-immunoglobulin polypeptide. Such a non-immunoglobulin polypeptide can be substituted for the constant domains of an antibody of the invention, or can be substituted for the variable domains of one antigen-combining site of an antibody of the invention to create a chimeric bivalent antibody. ,

The antibodies may be monovalent antibodies. Methods for preparing monovalent antibodies are well known in the art. For example, one method involves recombinant expression of immunoglobulin light chain and modified heavy chain. The heavy chain is truncated generally at any point in the Fc region so as to prevent heavy chain crosslinking. Alternatively, the relevant cysteine residues are substituted with another amino acid residue or are deleted so as to prevent crosslinking.

*In vitro* methods are also suitable for preparing monovalent antibodies. Digestion of antibodies to produce fragments thereof, particularly, Fab fragments, can be accomplished using routine techniques known in the art.

### 3. Human and Humanized Antibodies

The anti-PRO antibodies of the invention may further comprise humanized antibodies or human antibodies. Humanized forms of non-human (e.g., murine) antibodies are chimeric immunoglobulins, immunoglobulin chains or fragments thereof (such as Fv, Fab, Fab', F(ab')₂ or other antigen-binding subsequences of antibodies) which contain minimal sequence derived from non-human immunoglobulin. Humanized antibodies include human immunoglobulins (recipient antibody) in which residues from a complementary determining region (CDR) of the recipient are replaced by residues from a CDR of a non-human species (donor antibody) such as mouse, rat or rabbit having the desired specificity, affinity and capacity. In some instances, Fv framework residues of the human immunoglobulin are replaced by corresponding non-human residues. Humanized antibodies may also comprise residues which are found neither in the recipient antibody nor in the imported CDR or framework sequences. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulin consensus sequence. The humanized antibody optimally also will comprise at least a portion of an immunoglobulin constant region (Fc), typically that of a human immunoglobulin [Jones et al., Nature, 321:522-525 (1986); Riechmann et al., Nature, 332:323-329 (1988); and Presta, Curr. Op. Struct. Biol., 2:593-596 (1992)].

Methods for humanizing non-human antibodies are well known in the art. Generally, a humanized antibody has one or more amino acid residues introduced into it from a source which is non-human. These non-human amino acid residues are often referred to as "import" residues, which are typically taken from an "import" variable domain. Humanization can be essentially performed following the method of Winter and co-workers [Jones et al., Nature, 321:522-525 (1986); Riechmann et al., Nature, 332:323-327 (1988); Verhoeyen et al., Science, 239:1534-1536 (1988)], by substituting rodent CDRs or CDR sequences for the corresponding sequences of a human antibody. Accordingly, such "humanized" antibodies are chimeric antibodies (U.S. Patent No. 4,816,567), wherein substantially less than an intact human variable domain has been substituted by the corresponding sequence from a non-human species. In practice, humanized antibodies are typically human antibodies in which some CDR residues and possibly some FR residues are substituted by residues from analogous sites in rodent antibodies.

Human antibodies can also be produced using various techniques known in the art, including phage display libraries [Hoogenboom and Winter, J. Mol. Biol., 227:381 (1991); Marks et al., J. Mol. Biol. 222:581 (1991)]. The techniques of Cole et al. and Boerner et al. are also available for the preparation of human monoclonal antibodies (Cole et al., Monoclonal Antibodies and Cancer Therapy, Alan R. Liss, p. 77 (1985) and Boerner et al., J. Immunol., 147(1):86-95 (1991)]. Similarly, human antibodies can be made by introducing of human immunoglobulin loci into transgenic animals, e.g., mice in which the endogenous immunoglobulin genes have been partially or completely inactivated. Upon challenge, human antibody production is observed, which closely resembles that seen in humans in all respects, including gene rearrangement, assembly, and antibody repertoire. This approach is described, for example, in U.S. Patent Nos. 5,545,807; 5,545,806; 5,569,825; 5,625,126; 5,633,425; 5,661,016, and in the following scientific publications: Marks et al., Bio/Technology 10, 779-783 (1992); Lonberg et al., Nature 368 856-859 (1994); Morrison, Nature 368, 812-13 (1994); Fishwild et al., Nature Biotechnology 14, 845-51 (1996); Neuberger, Nature Biotechnology 14, 826 (1996); Lonberg and Huszar, Intern. Rev. Immunol. 13 65-93 (1995).

The antibodies may also be affinity matured using known selection and/or mutagenesis methods as described above. Preferred affinity matured antibodies have an affinity which is five times, more preferably 10 times, even more preferably 20 or 30 times greater than the starting antibody (generally murine, humanized or human) from which the matured antibody is prepared.

### 4. Bispecific Antibodies

Bispecific antibodies are monoclonal, preferably human or humanized, antibodies that have binding specificities for at least two different antigens. In the present case, one of the binding specificities is for the PRO, the other one is for any other antigen, and preferably for a cell-surface protein or receptor or receptor subunit.

Methods for making bispecific antibodies are known in the art. Traditionally, the recombinant production of bispecific antibodies is based on the co-expression of two immunoglobulin heavy-chain/light-chain pairs, where the two heavy chains have different specificities [Milstein and Cuello, Nature, 305:537-539 (1983)]. Because of the random assortment of immunoglobulin heavy and light chains, these hybridomas (quadromas) produce a potential mixture of ten different antibody molecules, of which only one has the correct bispecific structure. The purification of the correct molecule is usually accomplished by affinity chromatography steps. Similar procedures are disclosed in WO 93/08829, published 13 May 1993, and in Traunecker et al., EMBO J., 10:3655-3659 (1991).

Antibody variable domains with the desired binding specificities (antibody-antigen combining sites) can be fused to immunoglobulin constant domain sequences. The fusion preferably is with an immunoglobulin heavy-chain constant domain, comprising at least part of the hinge, CH2, and CH3 regions. It is preferred to have the first heavy-chain constant region (CH1) containing the site necessary for light-chain binding present in at least one of the fusions. DNAs encoding the immunoglobulin heavy-chain fusions and, if desired, the immunoglobulin light chain, are inserted into separate expression vectors, and are co-transfected into a suitable host organism. For further details of generating bispecific antibodies see, for example, Suresh et al., Methods in Enzymology, 121:210 (1986).

According to another approach described in WO 96/27011, the interface between a pair of antibody molecules can be engineered to maximize the percentage of heterodimers which are recovered from recombinant cell culture. The preferred interface comprises at least a part of the CH3 region of an antibody constant domain. In this method, one or more small amino acid side chains from the interface of the first antibody molecule are replaced with larger side chains (e.g. tyrosine or tryptophan). Compensatory "cavities" of identical or similar size to the large side chain(s) are created on the interface of the second antibody molecule by replacing large amino acid side chains with smaller ones (e.g. alanine or threonine). This provides a mechanism for increasing the yield of the heterodimer over other unwanted end-products such as homodimers.

Bispecific antibodies can be prepared as full length antibodies or antibody fragments (e.g. F(ab')₂ bispecific antibodies). Techniques for generating bispecific antibodies from antibody fragments have been described in the literature. For example, bispecific antibodies can be prepared can be prepared using chemical linkage. Brennan et al., Science 229:81 (1985) describe a procedure wherein intact antibodies are proteolytically cleaved to generate F(ab')₂ fragments. These fragments are reduced in the presence of the dithiol complexing agent sodium arsenite to stabilize vicinal dithiols and prevent intermolecular disulfide formation. The Fab' fragments generated are then converted to thionitrobenzoate (TNB) derivatives. One of the Fab'-TNB derivatives is then reconverted to the Fab'-thiol by reduction with mercaptoethylamine and is mixed with an equimolar amount of the other Fab'-TNB derivative to form the bispecific antibody. The bispecific antibodies produced can be used as agents for the selective immobilization of enzymes.

Fab' fragments may be directly recovered from *E. coli* and chemically coupled to form bispecific antibodies. Shalaby et al., J. Exp. Med. 175:217-225 (1992) describe the production of a fully humanized bispecific antibody F(ab')₂ molecule. Each Fab' fragment was separately secreted from *E. coli* and subjected to directed chemical coupling *in vitro* to form the bispecific antibody. The bispecific antibody thus formed was able to bind to cells overexpressing the ErbB2 receptor and normal human T cells, as well as trigger the lytic activity of human cytotoxic lymphocytes against human breast tumor targets.

Various technique for making and isolating bispecific antibody fragments directly from recombinant cell culture have also been described. For example, bispecific antibodies have been produced using leucine zippers. Kostelny et al., J. Immunol. 148(5):1547-1553 (1992). The leucine zipper peptides from the Fos and Jun proteins were linked to the Fab' portions of two different antibodies by gene fusion. The antibody homodimers were reduced at the hinge region to form monomers and then re-oxidized to form the antibody heterodimers. This method can also be utilized for the production of antibody homodimers. The "diabody" technology described by Hollinger et al., Proc. Natl. Acad. Sci. USA 90:6444-6448 (1993) has provided an alternative mechanism for making bispecific antibody fragments. The fragments comprise a heavy-chain variable domain (V_{H}) connected to a light-chain variable domain (V_{L}) by a linker which is too short to allow pairing between the two domains on the same chain. Accordingly, the V_{H} and V_{L} domains of one fragment are forced to pair with the complementary V_{L} and V_{H} domains of another fragment, thereby forming two antigen-binding sites. Another strategy for making bispecific antibody fragments by the use of single-chain Fv (sFv) dimers has also been reported. See, Gruber et al., J. Immunol. 152:5368 (1994). Antibodies with more than two valencies are contemplated. For example, trispecific antibodies can be prepared. Tutt et al., J. Immunol. 147:60 (1991).

Exemplary bispecific antibodies may bind to two different epitopes on a given PRO polypeptide herein. Alternatively, an anti-PRO polypeptide arm may be combined with an arm which binds to a triggering molecule on a leukocyte such as a T-cell receptor molecule (e.g. CD2, CD3, CD28, or B7), or Fc receptors for IgG (FcγR), such as FcγRI (CD64), FcγRII (CD32) and FcγRIII (CD16) so as to focus cellular defense mechanisms to the cell expressing the particular PRO polypeptide. Bispecific antibodies may also be used to localize cytotoxic agents to cells which express a particular PRO polypeptide. These antibodies possess a PRO-binding arm and an arm which binds a cytotoxic agent or a radionuclide chelator, such as EOTUBE, DPTA, DOTA, or TETA. Another bispecific antibody of interest binds the PRO polypeptide and further binds tissue factor (TF).

### 5. Heteroconjugate Antibodies

Heteroconjugate antibodies are also within the scope of the present invention. Heteroconjugate antibodies are composed of two covalently joined antibodies. Such antibodies have, for example, been proposed to target immune system cells to unwanted cells [U.S. Patent No. 4,676,980], and for treatment of HIV infection [WO 91/00360; WO 92/200373; EP 03089]. It is contemplated that the antibodies may be prepared *in vitro* using known methods in synthetic protein chemistry, including those involving crosslinking agents. For example, immunotoxins may be constructed using a disulfide exchange reaction or by forming a thioether bond. Examples of suitable reagents for this purpose include iminothiolate and methyl-4-mercaptobutyrimidate and those disclosed, for example, in U.S. Patent No. 4,676,980.

### 6. Effector Function Engineering

It may be desirable to modify the antibody of the invention with respect to effector function, so as to enhance, *e*.*g*., the effectiveness of the antibody in treating cancer. For example, cysteine residue(s) may be introduced into the Fc region, thereby allowing interchain disulfide bond formation in this region. The homodimeric antibody thus generated may have improved internalization capability and/or increased complement-mediated cell killing and antibody-dependent cellular cytotoxicity (ADCC). See Caron et al., J. Exp Med., 176: 1191-1195 (1992) and Shopes, J. Immunol., 148: 2918-2922 (1992). Homodimeric antibodies with enhanced anti-tumor activity may also be prepared using heterobifunctional cross-linkers as described in Wolff et al. Cancer Research, 53: 2560-2565 (1993). Alternatively, an antibody can be engineered that has dual Fc regions and may thereby have enhanced complement lysis and ADCC capabilities. See Stevenson et al., Anti-Cancer Drug Design, 3: 219-230 (1989).

### 7. Immunoconjugates

The invention also pertains to immunoconjugates comprising an antibody conjugated to a cytotoxic agent such as a chemotherapeutic agent, toxin (*e*.*g*., an enzymatically active toxin of bacterial, fungal, plant, or animal origin, or fragments thereof), or a radioactive isotope (*i*.*e*., a radioconjugate).

Chemotherapeutic agents useful in the generation of such immunoconjugates have been described above. Enzymatically active toxins and fragments thereof that can be used include diphtheria A chain, nonbinding active fragments of diphtheria toxin, exotoxin A chain (from *Pseudomonas aeruginosa*), ricin A chain, abrin A chain, modeccin A chain, alpha-sarcin, *Aleurites fordii* proteins, dianthin proteins, *Phytolaca americana* proteins (PAPI, PAPII, and PAP-S), momordica charantia inhibitor, curcin, crotin, sapaonaria officinalis inhibitor, gelonin, mitogellin, restrictocin, phenomycin, enomycin, and the tricothecenes. A variety of radionuclides are available for the production of radioconjugated antibodies. Examples include ²¹²Bi, ¹³¹I, ¹³¹In, ⁹⁰Y, and ¹⁸⁶Re.

Conjugates of the antibody and cytotoxic agent are made using a variety of bifunctional protein-coupling agents such as N-succinimidyl-3-(2-pyridyldithiol) propionate (SPDP), iminothiolane (IT), bifunctional derivatives of imidoesters (such as dimethyl adipimidate HCL), active esters (such as disuccinimidyl suberate), aldehydes (such as glutareldehyde), bis-azido compounds (such as bis (p-azidobenzoyl) hexanediamine), bis-diazonium derivatives (such as bis-(p-diazoniumbenzoyl)-ethylenediamine), diisocyanates (such as tolyene 2,6-diisocyanate), and bis-active fluorine compounds (such as 1,5-difluoro-2,4-dinitrobenzene). For example, a ricin immunotoxin can be prepared as described in Vitetta et al., Science, 238: 1098 (1987). Carbon-14-labeled 1-isothiocyanatobenzyl-3-methyldiethylene triaminepentaacetic acid (MX-DTPA) is an exemplary chelating agent for conjugation of radionucleotide to the antibody. See WO94/11026.

In another embodiment, the antibody may be conjugated to a "receptor" (such streptavidin) for utilization in tumor pretargeting wherein the antibody-receptor conjugate is administered to the patient, followed by removal of unbound conjugate from the circulation using a clearing agent and then administration of a "ligand" (*e*.*g*., avidin) that is conjugated to a cytotoxic agent (*e*.*g*., a radionucleotide).

### 8. Immunoliposomes

The antibodies disclosed herein may also be formulated as immunoliposomes. Liposomes containing the antibody are prepared by methods known in the art, such as described in Epstein et al., Proc. Natl. Acad. Sci. USA, 82: 3688 (1985); Hwang et al., Proc. Natl Acad. Sci. USA, 77: 4030 (1980); and U.S. Pat. Nos. 4,485,045 and 4,544,545. Liposomes with enhanced circulation time are disclosed in U.S. Patent No. 5,013,556.

Particularly useful liposomes can be generated by the reverse-phase evaporation method with a lipid composition comprising phosphatidylcholine, cholesterol, and PEG-derivatized phosphatidylethanolamine (PEG-PE). Liposomes are extruded through filters of defined pore size to yield liposomes with the desired diameter. Fab' fragments of the antibody of the present invention can be conjugated to the liposomes as described in Martin et al ., J. Biol. Chem., 257: 286-288 (1982) via a disulfide-interchange reaction. A chemotherapeutic agent (such as Doxorubicin) is optionally contained within the liposome. See Gabizon et al., J. National Cancer Inst., 81(19): 1484 (1989).

### M. Pharmaceutical Compositions

The active PRO molecules of the invention (*e*.*g*., PRO polypeptides, anti-PRO antibodies, and/or variants of each) as well as other molecules identified by the screening assays disclosed above, can be administered for the treatment of immune related diseases, in the form of pharmaceutical compositions.

Therapeutic formulations of the active PRO molecule, preferably a polypeptide or antibody of the invention, are prepared for storage by mixing the active molecule having the desired degree of purity with optional pharmaceutically acceptable carriers, excipients or stabilizers (Remington's Pharmaceutical Sciences 16th edition, Osol, A. Ed. [1980]), in the form of lyophilized formulations or aqueous solutions. Acceptable carriers, excipients, or stabilizers are nontoxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (*e*.*g*., Zn-protein complexes); and/or non-ionic surfactants such as TWEEN^{™}, PLURONICS^{™} or polyethylene glycol (PEG).

Compounds identified by the screening assays disclosed herein can be formulated in an analogous manner, using standard techniques well known in the art.

Lipofections or liposomes can also be used to deliver the PRO molecule into cells. Where antibody fragments are used, the smallest inhibitory fragment which specifically binds to the binding domain of the target protein is preferred. For example, based upon the variable region sequences of an antibody, peptide molecules can be designed which retain the ability to bind the target protein sequence. Such peptides can be synthesized chemically and/or produced by recombinant DNA technology (see, *e*.*g*., Marasco et al., Proc. Natl. Acad. Sci. USA 90, 7889-7893 [1993]).

The formulation herein may also contain more than one active compound as necessary for the particular indication being treated, preferably those with complementary activities that do not adversely affect each other. Alternatively, or in addition, the composition may comprise a cytotoxic agent, cytokine or growth inhibitory agent. Such molecules are suitably present in combination in amounts that are effective for the purpose intended.

The active PRO molecules may also be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nano-particles and nanocapsules) or in macroemulsions. Such techniques are disclosed in Remington's Pharmaceutical Sciences 16th edition, Osol, A. Ed. (1980).

The formulations to be used for *in vivo* administration must be sterile. This is readily accomplished by filtration through sterile filtration membranes.

Sustained-release preparations or the PRO molecules may be prepared. Suitable examples of sustained-release preparations include semipermeable matrices of solid hydrophobic polymers containing the antibody, which matrices are in the form of shaped articles, *e.g.,* films, or microcapsules. Examples of sustained-release matrices include polyesters, hydrogels (for example, poly(2-hydroxyethyl-methacrylate), or poly(vinylalcohol)), polylactides (U.S. Pat. No. 3,773,919), copolymers of L-glutamic acid and γ-ethyl-L-glutamate, non-degradable ethylene-vinyl acetate, degradable lactic acid-glycolic acid copolymers such as the LUPRON DEPOT^{™} (injectable microspheres composed of lactic acid-glycolic acid copolymer and leuprolide acetate), and poly-D-(-)-3-hydroxybutyric acid. While polymers such as ethylene-vinyl acetate and lactic acid-glycolic acid enable release of molecules for over 100 days, certain hydrogels release proteins for shorter time periods. When encapsulated antibodies remain in the body for a long time, they may denature or aggregate as a result of exposure to moisture at 37°C, resulting in a loss of biological activity and possible changes in immunogenicity. Rational strategies can be devised for stabilization depending on the mechanism involved. For example, if the aggregation mechanism is discovered to be intermolecular S-S bond formation through thio-disulfide interchange, stabilization may be achieved by modifying sulfhydryl residues, lyophilizing from acidic solutions, controlling moisture content, using appropriate additives, and developing specific polymer matrix compositions.

### N. Methods of Treatment

It is contemplated that the polypeptides, antibodies and other active compounds of the present invention may be used to treat various immune related diseases and conditions, such as T cell mediated diseases, including those characterized by infiltration of inflammatory cells into a tissue, stimulation of T-cell proliferation, inhibition of T-cell proliferation, increased or decreased vascular permeability or the inhibition thereof.

Exemplary conditions or disorders to be treated with the polypeptides, antibodies and other compounds of the invention, include, but are not limited to systemic lupus erythematosis, rheumatoid arthritis, juvenile chronic arthritis, osteoarthritis, spondyloarthropathies, systemic sclerosis (scleroderma), idiopathic inflammatory myopathies (dermatomyositis, polymyositis), Sjögren's syndrome, systemic vasculitis, sarcoidosis, autoimmune hemolytic anemia (immune pancytopenia, paroxysmal nocturnal hemoglobinuria), autoimmune thrombocytopenia (idiopathic thrombocytopenic purpura, immune-mediated thrombocytopenia), thyroiditis (Grave's disease, Hashimoto's thyroiditis, juvenile lymphocytic thyroiditis, atrophic thyroiditis), diabetes mellitus, immune-mediated renal disease (glomerulonephritis, tubulointerstitial nephritis), demyelinating diseases of the central and peripheral nervous systems such as multiple sclerosis, idiopathic demyelinating polyneuropathy or Guillain-Barré syndrome, and chronic inflammatory demyelinating polyneuropathy, hepatobiliary diseases such as infectious hepatitis (hepatitis A, B, C, D, E and other non-hepatotropic viruses), autoimmune chronic active hepatitis, primary biliary cirrhosis, granulomatous hepatitis, and sclerosing cholangitis, inflammatory bowel disease (ulcerative colitis: Crohn's disease), gluten-sensitive enteropathy, and Whipple's disease, autoimmune or immune-mediated skin diseases including bullous skin diseases, erythema multiforme and contact dermatitis, psoriasis, allergic diseases such as asthma, allergic rhinitis, atopic dermatitis, food hypersensitivity and urticaria, immunologic diseases of the lung such as eosinophilic pneumonias, idiopathic pulmonary fibrosis and hypersensitivity pneumonitis, transplantation associated diseases including graft rejection and graft -versus-host-disease.

In systemic lupus erythematosus, the central mediator of disease is the production of auto-reactive antibodies to self proteins/tissues and the subsequent generation of immune-mediated inflammation. Antibodies either directly or indirectly mediate tissue injury. Though T lymphocytes have not been shown to be directly involved in tissue damage, T lymphocytes are required for the development of auto-reactive antibodies. The genesis of the disease is thus T lymphocyte dependent. Multiple organs and systems are affected clinically including kidney, lung, musculoskeletal system, mucocutaneous, eye, central nervous system, cardiovascular system, gastrointestinal tract, bone marrow and blood.

Rheumatoid arthritis (RA) is a chronic systemic autoimmune inflammatory disease that mainly involves the synovial membrane of multiple joints with resultant injury to the articular cartilage. The pathogenesis is T lymphocyte dependent and is associated with the production of rheumatoid factors, auto-antibodies directed against self IgG, with the resultant formation of immune complexes that attain high levels in joint fluid and blood. These complexes in the joint may induce the marked infiltrate of lymphocytes and monocytes into the synovium and subsequent marked synovial changes; the joint space/fluid if infiltrated by similar cells with the addition of numerous neutrophils. Tissues affected are primarily the joints, often in symmetrical pattern. However, extra-articular disease also occurs in two major forms. One form is the development of extra-articular lesions with ongoing progressive joint disease and typical lesions of pulmonary fibrosis, vasculitis, and cutaneous ulcers. The second form of extra-articular disease is the so called Felty's syndrome which occurs late in the RA disease course, sometimes after joint disease has become quiescent, and involves the presence of neutropenia, thrombocytopenia and splenomegaly. This can be accompanied by vasculitis in multiple organs with formations of infarcts, skin ulcers and gangrene. Patients often also develop rheumatoid nodules in the subcutis tissue overlying affected joints; the nodules late stage have necrotic centers surrounded by a mixed inflammatory cell infiltrate. Other manifestations which can occur in RA include: pericarditis, pleuritis, coronary arteritis, intestitial pneumonitis with pulmonary fibrosis, keratoconjunctivitis sicca, and rhematoid nodules.

Juvenile chronic arthritis is a chronic idiopathic inflammatory disease which begins often at less than 16 years of age. Its phenotype has some similarities to RA; some patients which are rhematoid factor positive are classified as juvenile rheumatoid arthritis. The disease is sub-classified into three major categories: pauciarticular, polyarticular, and systemic. The arthritis can be severe and is typically destructive and leads to joint ankylosis and retarded growth. Other manifestations can include chronic anterior uveitis and systemic amyloidosis.

Spondyloarthropathies are a group of disorders with some common clinical features and the common association with the expression of HLA-B27 gene product. The disorders include: ankylosing sponylitis, Reiter's syndrome (reactive arthritis), arthritis associated with inflammatory bowel disease, spondylitis associated with psoriasis, juvenile onset spondyloarthropathy and undifferentiated spondyloarthropathy. Distinguishing features include sacroileitis with or without spondylitis; inflammatory asymmetric arthritis; association with HLA-B27 (a serologically defined allele of the HLA-B locus of class I MHC); ocular inflammation, and absence of autoantibodies associated with other rheumatoid disease. The cell most implicated as key to induction of the disease is the CD8+ T lymphocyte, a cell which targets antigen presented by class I MHC molecules. CD8+ T cells may react against the class I MHC allele HLA-B27 as if it were a foreign peptide expressed by MHC class I molecules. It has been hypothesized that an epitope of HLA-B27 may mimic a bacterial or other microbial antigenic epitope and thus induce a CD8+ T cells response.

Systemic sclerosis (scleroderma) has an unknown etiology. A hallmark of the disease is induration of the skin; likely this is induced by an active inflammatory process. Scleroderma can be localized or systemic; vascular lesions are common and endothelial cell injury in the microvasculature is an early and important event in the development of systemic sclerosis; the vascular injury may be immune mediated. An immunologic basis is implied by the presence of mononuclear cell infiltrates in the cutaneous lesions and the presence of anti-nuclear antibodies in many patients. ICAM-1 is often upregulated on the cell surface of fibroblasts in skin lesions suggesting that T cell interaction with these cells may have a role in the pathogenesis of the disease. Other organs involved include: the gastrointestinal tract: smooth muscle atrophy and fibrosis resulting in abnormal peristalsis/motility; kidney: concentric subendothelial intimal proliferation affecting small arcuate and interlobular arteries with resultant reduced renal cortical blood flow, results in proteinuria, azotemia and hypertension; skeletal muscle: atrophy, interstitial fibrosis; inflammation; lung: interstitial pneumonitis and interstitial fibrosis; and heart: contraction band necrosis, scarring/fibrosis.

Idiopathic inflammatory myopathies including dermatomyositis, polymyositis and others are disorders of chronic muscle inflammation of unknown etiology resulting in muscle weakness. Muscle injury/inflammation is often symmetric and progressive. Autoantibodies are associated with most forms. These myositis-specific autoantibodies are directed against and inhibit the function of components, proteins and RNA's, involved in protein synthesis.

Sjögren's syndrome is due to immune-mediated inflammation and subsequent functional destruction of the tear glands and salivary glands. The disease can be associated with or accompanied by inflammatory connective tissue diseases. The disease is associated with autoantibody production against Ro and La antigens, both of which are small RNA-protein complexes. Lesions result in keratoconjunctivitis sicca, xerostomia, with other manifestations or associations including bilary cirrhosis, peripheral or sensory neuropathy, and palpable purpura.

Systemic vasculitis are diseases in which the primary lesion is inflammation and subsequent damage to blood vessels which results in ischemia/necrosis/degeneration to tissues supplied by the affected vessels and eventual end-organ dysfunction in some cases. Vasculitides can also occur as a secondary lesion or sequelae to other immune-inflammatory mediated diseases such as rheumatoid arthritis, systemic sclerosis, etc., particularly in diseases also associated with the formation of immune complexes. Diseases in the primary systemic vasculitis group include: systemic necrotizing vasculitis: polyarteritis nodosa, allergic angiitis and granulomatosis, polyangiitis; Wegener's granulomatosis; lymphomatoid granulomatosis; and giant cell arteritis. Miscellaneous vasculitides include: mucocutaneous lymph node syndrome (MLNS or Kawasaki's disease), isolated CNS vasculitis, Behet's disease, thromboangiitis obliterans (Buerger's disease) and cutaneous necrotizing venulitis. The pathogenic mechanism of most of the types of vasculitis listed is believed to be primarily due to the deposition of immunoglobulin complexes in the vessel wall and subsequent induction of an inflammatory response either via ADCC, complement activation, or both.

Sarcoidosis is a condition of unknown etiology which is characterized by the presence of epithelioid granulomas in nearly any tissue in the body; involvement of the lung is most common. The pathogenesis involves the persistence of activated macrophages and lymphoid cells at sites of the disease with subsequent chronic sequelae resultant from the release of locally and systemically active products released by these cell types.

Autoimmune hemolytic anemia including autoimmune hemolytic anemia, immune pancytopenia, and paroxysmal noctural hemoglobinuria is a result of production of antibodies that react with antigens expressed on the surface of red blood cells (and in some cases other blood cells including platelets as well) and is a reflection of the removal of those antibody coated cells via complement mediated lysis and/or ADCC/Fc-receptor-mediated mechanisms.

In autoimmune thrombocytopenia including thrombocytopenic purpura, and immune-mediated thrombocytopenia in other clinical settings, platelet destruction/removal occurs as a result of either antibody or complement attaching to platelets and subsequent removal by complement lysis, ADCC or FC-receptor mediated mechanisms.

Thyroiditis including Grave's disease, Hashimoto's thyroiditis, juvenile lymphocytic thyroiditis, and atrophic thyroiditis, are the result of an autoimmune response against thyroid antigens with production of antibodies that react with proteins present in and often specific for the thyroid gland. Experimental models exist including spontaneous models: rats (BUF and BB rats) and chickens (obese chicken strain); inducible models: immunization of animals with either thyroglobulin, thyroid microsomal antigen (thyroid peroxidase).

Type I diabetes mellitus or insulin-dependent diabetes is the autoimmune destruction of pancreatic islet β cells; this destruction is mediated by auto-antibodies and auto-reactive T cells. Antibodies to insulin or the insulin receptor can also produce the phenotype of insulin-non-responsiveness.

Immune mediated renal diseases, including glomerulonephritis and tubulointerstitial nephritis, are the result of antibody or T lymphocyte mediated injury to renal tissue either directly as a result of the production of autoreactive antibodies or T cells against renal antigens or indirectly as a result of the deposition of antibodies and/or immune complexes in the kidney that are reactive against other, non-renal antigens. Thus other immune-mediated diseases that result in the formation of immune-complexes can also induce immune mediated renal disease as an indirect sequelae. Both direct and indirect immune mechanisms result in inflammatory response that produces/induces lesion development in renal tissues with resultant organ function impairment and in some cases progression to renal failure. Both humoral and cellular immune mechanisms can be involved in the pathogenesis of lesions.

Demyelinating diseases of the central and peripheral nervous systems, including Multiple Sclerosis; idiopathic demyelinating polyneuropathy or Guillain-Barré syndrome; and Chronic Inflammatory Demyelinating Polyneuropathy, are believed to have an autoimmune basis and result in nerve demyelination as a result of damage caused to oligodendrocytes or to myelin directly. In MS there is evidence to suggest that disease induction and progression is dependent on T lymphocytes. Multiple Sclerosis is a demyelinating disease that is T lymphocyte-dependent and has either a relapsing-remitting course or a chronic progressive course. The etiology is unknown; however, viral infections, genetic predisposition, environment, and autoimmunity all contribute. Lesions contain infiltrates of predominantly T lymphocyte mediated, microglial cells and infiltrating macrophages; CD4+ T lymphocytes are the predominant cell type at lesions. The mechanism of oligodendrocyte cell death and subsequent demyelination is not known but is likely T lymphocyte driven.

Inflammatory and Fibrotic Lung Disease, including Eosinophilic Pneumonias; Idiopathic Pulmonary Fibrosis, and Hypersensitivity Pneumonitis may involve a disregulated immune-inflammatory response. Inhibition of that response would be of therapeutic benefit.

Autoimmune or Immune-mediated Skin Disease including Bullous Skin Diseases, Erythema Multiforme, and Contact Dermatitis are mediated by auto-antibodies, the genesis of which is T lymphocyte-dependent.

Psoriasis is a T lymphocyte-mediated inflammatory disease. Lesions contain infiltrates of T lymphocytes, macrophages and antigen processing cells, and some neutrophils.

Allergic diseases, including asthma; allergic rhinitis; atopic dermatitis; food hypersensitivity; and urticaria are T lymphocyte dependent. These diseases are predominantly mediated by T lymphocyte induced inflammation, IgE mediated-inflammation or a combination of both.

Transplantation associated diseases, including Graft rejection and Graft-Versus-Host-Disease (GVHD) are T lymphocyte-dependent; inhibition of T lymphocyte function is ameliorative. Other diseases in which intervention of the immune and/or inflammatory response have benefit are infectious disease including but not limited to viral infection (including but not limited to AIDS, hepatitis A, B, C, D, E and herpes) bacterial infection, fungal infections, and protozoal and parasitic infections (molecules (or derivatives/agonists) which stimulate the MLR can be utilized therapeutically to enhance the immune response to infectious agents), diseases of immunodeficiency (molecules/derivatives/agonists) which stimulate the MLR can be utilized therapeutically to enhance the immune response for conditions of inherited, acquired, infectious induced (as in HIV infection), or iatrogenic (*i*.*e*., as from chemotherapy) immunodeficiency, and neoplasia.

It has been demonstrated that some human cancer patients develop an antibody and/or T lymphocyte response to antigens on neoplastic cells. It has also been shown in animal models of neoplasia that enhancement of the immune response can result in rejection or regression of that particular neoplasm. Molecules that enhance the T lymphocyte response in the MLR have utility *in vivo* in enhancing the immune response against neoplasia. Molecules which enhance the T lymphocyte proliferative response in the MLR (or small molecule agonists or antibodies that affected the same receptor in an agonistic fashion) can be used therapeutically to treat cancer. Molecules that inhibit the lymphocyte response in the MLR also function *in vivo* during neoplasia to suppress the immune response to a neoplasm; such molecules can either be expressed by the neoplastic cells themselves or their expression can be induced by the neoplasm in other cells. Antagonism of such inhibitory molecules (either with antibody, small molecule antagonists or other means) enhances immune-mediated tumor rejection.

Additionally, inhibition of molecules with proinflammatory properties may have therapeutic benefit in reperfusion injury; stroke; myocardial infarction; atherosclerosis; acute lung injury; hemorrhagic shock; burn; sepsis/septic shock; acute tubular necrosis; endometriosis; degenerative joint disease and pancreatis.

The compounds of the present invention, *e.g.,* polypeptides or antibodies, are administered to a mammal, preferably a human, in accord with known methods, such as intravenous administration as a bolus or by continuous infusion over a period of time, by intramuscular, intraperitoneal, intracerobrospinal, subcutaneous, intra-articular, intrasynovial, intrathecal, oral, topical, or inhalation (intranasal, intrapulmonary) routes. Intravenous or inhaled administration of polypeptides and antibodies is preferred.

In immunoadjuvant therapy, other therapeutic regimens, such administration of an anti-cancer agent, may be combined with the administration of the proteins, antibodies or compounds of the instant invention. For example, the patient to be treated with a the immunoadjuvant of the invention may also receive an anti-cancer agent (chemotherapeutic agent) or radiation therapy. Preparation and dosing schedules for such chemotherapeutic agents may be used according to manufacturers' instructions or as determined empirically by the skilled practitioner. Preparation and dosing schedules for such chemotherapy are also described in Chemotherapy Service Ed., M.C. Perry, Williams & Wilkins, Baltimore, MD (1992). The chemotherapeutic agent may precede, or follow administration of the immunoadjuvant or may be given simultaneously therewith. Additionally, an anti-estrogen compound such as tamoxifen or an anti-progesterone such as onapristone (see, EP 616812) may be given in dosages known for such molecules.

It may be desirable to also administer antibodies against other immune disease associated or tumor associated antigens, such as antibodies which bind to CD20, CD11a, CD18, ErbB2, EGFR, ErbB3, ErbB4, or vascular endothelial factor (VEGF). Alternatively, or in addition, two or more antibodies binding the same or two or more different antigens disclosed herein may be coadministered to the patient. Sometimes, it may be beneficial to also administer one or more cytokines to the patient. In one embodiment, the PRO polypeptides are coadministered with a growth inhibitory agent. For example, the growth inhibitory agent may be administered first, followed by a PRO polypeptide. However, simultaneous administration or administration first is also contemplated. Suitable dosages for the growth inhibitory agent are those presently used and may be lowered due to the combined action (synergy) of the growth inhibitory agent and the PRO polypeptide.

For the treatment or reduction in the severity of immune related disease, the appropriate dosage of an a compound of the invention will depend on the type of disease to be treated, as defined above, the severity and course of the disease, whether the agent is administered for preventive or therapeutic purposes, previous therapy, the patient's clinical history and response to the compound, and the discretion of the attending physician. The compound is suitably administered to the patient at one time or over a series of treatments.

For example, depending on the type and severity of the disease, about 1 µg/kg to 15 mg/kg (e.g., 0.1-20 mg/kg) of polypeptide or antibody is an initial candidate dosage for administration to the patient, whether, for example, by one or more separate administrations, or by continuous infusion. A typical daily dosage might range from about 1 µg/kg to 100 mg/kg or more, depending on the factors mentioned above. For repeated administrations over several days or longer, depending on the condition, the treatment is sustained until a desired suppression of disease symptoms occurs. However, other dosage regimens may be useful. The progress of this therapy is easily monitored by conventional techniques and assays.

### O. Articles of Manufacture

In another embodiment of the invention, an article of manufacture containing materials (e.g., comprising a PRO molecule) useful for the diagnosis or treatment of the disorders described above is provided. The article of manufacture comprises a container and an instruction. Suitable containers include, for example, bottles, vials, syringes, and test tubes. The containers may be formed from a variety of materials such as glass or plastic. The container holds a composition which is effective for diagnosing or treating the condition and may have a sterile access port (for example the container may be an intravenous solution bag or a vial having a stopper pierceable by a hypodermic injection needle). The active agent in the composition is usually a polypeptide or an antibody of the invention. An instruction or label on, or associated with, the container indicates that the composition is used for diagnosing or treating the condition of choice. The article of manufacture may further comprise a second container comprising a pharmaceutically-acceptable buffer, such as phosphate-buffered saline, Ringer's solution and dextrose solution. It may further include other materials desirable from a commercial and user standpoint, including other buffers, diluents, filters, needles, syringes, and package inserts with instructions for use.

### P. Diagnosis and Prognosis of Immune Related Disease

Cell surface proteins, such as proteins which are overexpressed in certain immune related diseases, are excellent targets for drug candidates or disease treatment. The same proteins along with secreted proteins encoded by the genes amplified in immune related disease states find additional use in the diagnosis and prognosis of these diseases. For example, antibodies directed against the protein products of genes amplified in multiple sclerosis, rheumatoid arthritis, or another immune related disease, can be used as diagnostics or prognostics.

For example, antibodies, including antibody fragments, can be used to qualitatively or quantitatively detect the expression of proteins encoded by amplified or overexpressed genes ("marker gene products"). The antibody preferably is equipped with a detectable, e.g., fluorescent label, and binding can be monitored by light microscopy, flow cytometry, fluorimetry, or other techniques known in the art. These techniques are particularly suitable, if the overexpressed gene encodes a cell surface protein Such binding assays are performed essentially as described above.

*In situ* detection of antibody binding to the marker gene products can be performed, for example, by immunofluorescence or immunoelectron microscopy. For this purpose, a histological specimen is removed from the patient, and a labeled antibody is applied to it, preferably by overlaying the antibody on a biological sample. This procedure also allows for determining the distribution of the marker gene product in the tissue examined. It will be apparent for those skilled in the art that a wide variety of histological methods are readily available for *in situ* detection.

The following examples are offered for illustrative purposes only, and are not intended to limit the scope of the present invention in any way.

All patent and literature references cited in the present specification are hereby incorporated by reference in their entirety.

### EXAMPLES

Commercially available reagents referred to in the examples were used according to manufacturer's instructions unless otherwise indicated. The source of those cells identified in the following examples, and throughout the specification, by ATCC accession numbers is the American Type Culture Collection, Manassas, VA.

### EXAMPLE 1: Microarray analysis of stimulated T-cells

Nucleic acid microarrays, often containing thousands of gene sequences, are useful for identifying differentially expressed genes in diseased tissues as compared to their normal counterparts. Using nucleic acid microarrays, test and control mRNA samples from test and control tissue samples are reverse transcribed and labeled to generate cDNA probes. The cDNA probes are then hybridized to an array of nucleic acids immobilized on a solid support. The array is configured such that the sequence and position of each member of the array is known. For example, a selection of genes known to be expressed in certain disease states may be arrayed on a solid support. Hybridization of a labeled probe with a particular array member indicates that the sample from which the probe was derived expresses that gene. If the hybridization signal of a probe from a test (in this instance, activated CD4+ T cells) sample is greater than hybridization signal of a probe from a control (in this instance, non-stimulated CD4 + T cells) sample, the gene or genes overexpressed in the test tissue are identified. The implication of this result is that an overexpressed protein in a test tissue is useful not only as a diagnostic marker for the presence of the disease condition, but also as a therapeutic target for treatment of the disease condition.

The methodology of hybridization of nucleic acids and microarray technology is well known in the art. In one example, the specific preparation of nucleic acids for hybridization and probes, slides, and hybridization conditions are all detailed in PCT Patent Application Serial No. PCT/US01/10482, filed on March 30, 2001 and which is herein incorporated by reference.

In this experiment, CD4+ T cells were purified from a single donor using the RossetteSep™ protocol from (Stem Cell Technologies, Vancouver BC) which contains anti-CD8, anti-CD16, anti-CD19, anti-CD36 and anti-CD56 antibodies used to produce a population of isolated CD4 + T cells. Isolated CD4+ T cells were activated with an anti-CD3 antibody (used at a concentration that does not stimulate proliferation) together with either ICAM-1 or anti-CD28 antibody. At 24 or 72 hours cells were harvested, RNA extracted and analysis run on Affimax (Affymetrix Inc. Santa Clara, CA) microarrays. Non-stimulated (resting) cells were harvested immediately after purification, and subjected to the same analysis. Genes were compared whose expression was upregulated at either of the two timepoints in activated vs. resting cells.

Below are the results of these experiments, demonstrating that various PRO polypeptides of the present invention are significantly overexpressed in isolated CD4 + T cells activated by anti-CD3/ICAM-1 or anti-CD3/anti-CD28 as compared to isolated resting CD4+ T cells. As described above, these data demonstrate that the PRO polypeptides of the present invention are useful not only as diagnostic markers for the presence of one or more immune disorders, but also serve as therapeutic targets for the treatment of those immune disorders.

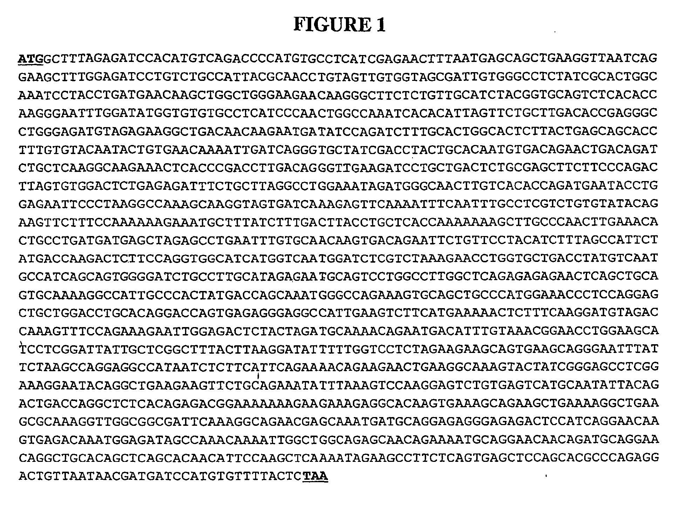

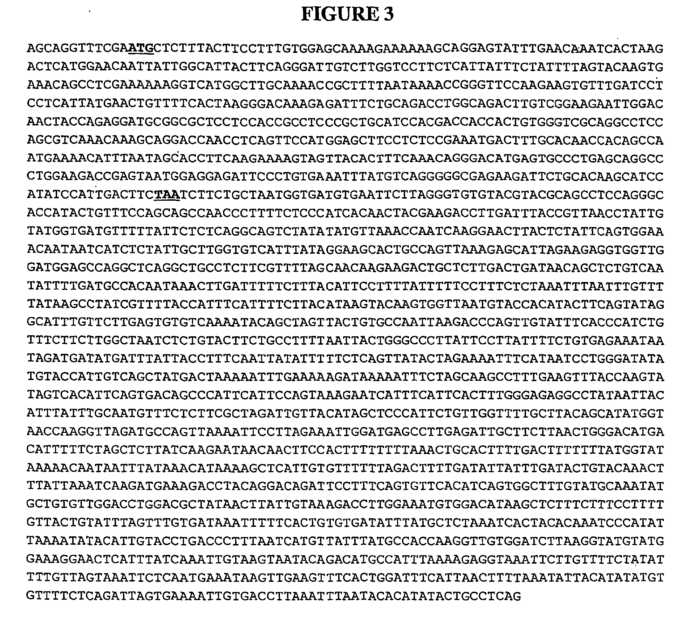

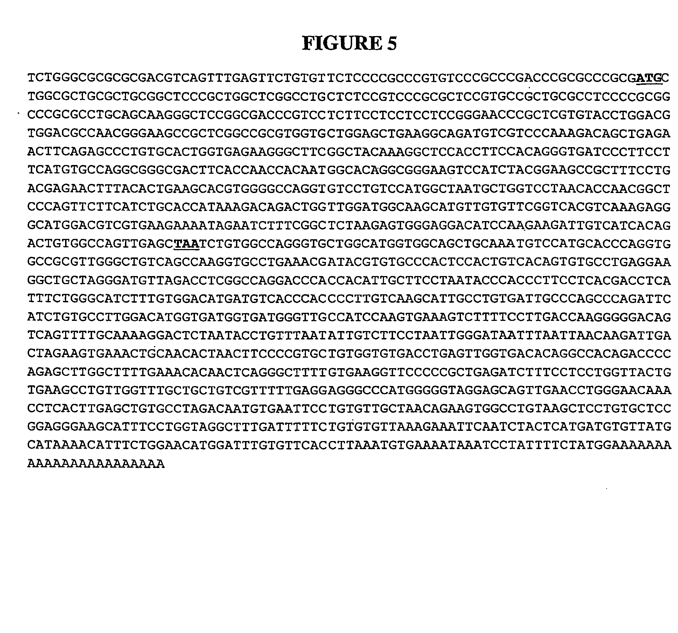

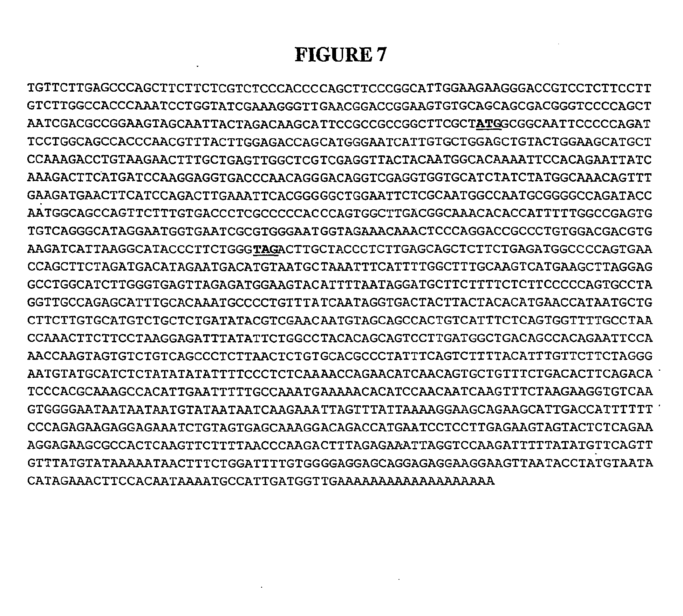

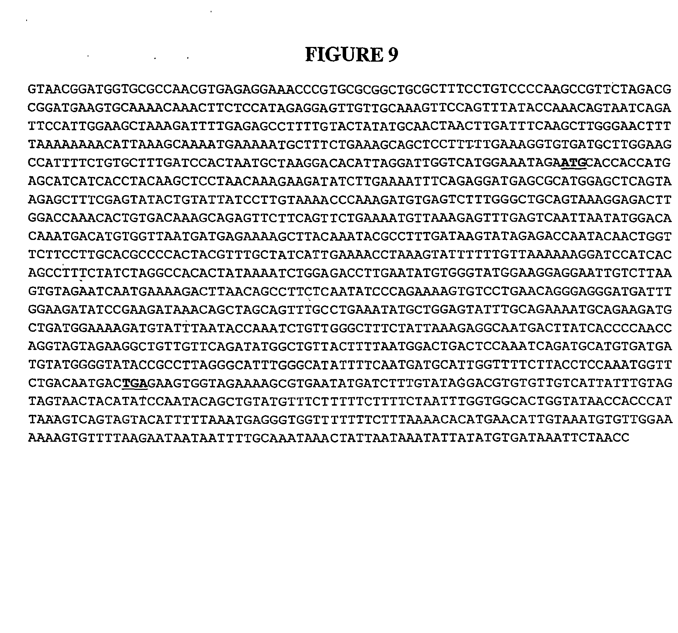

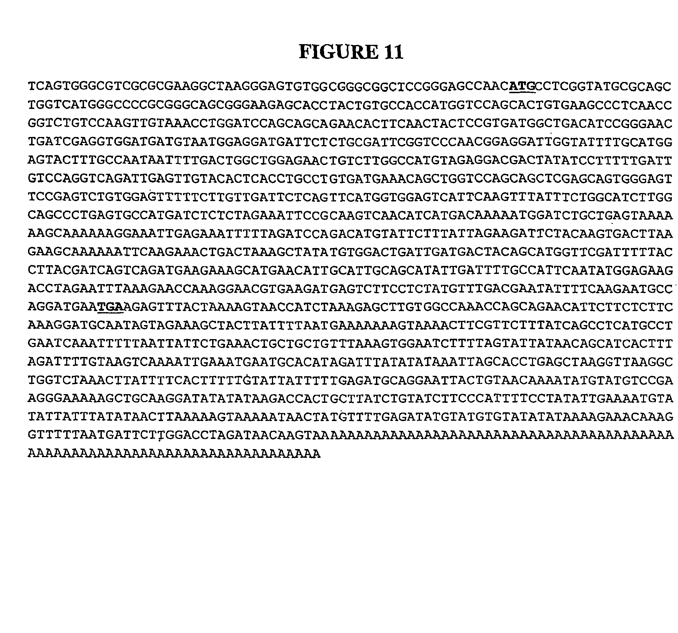

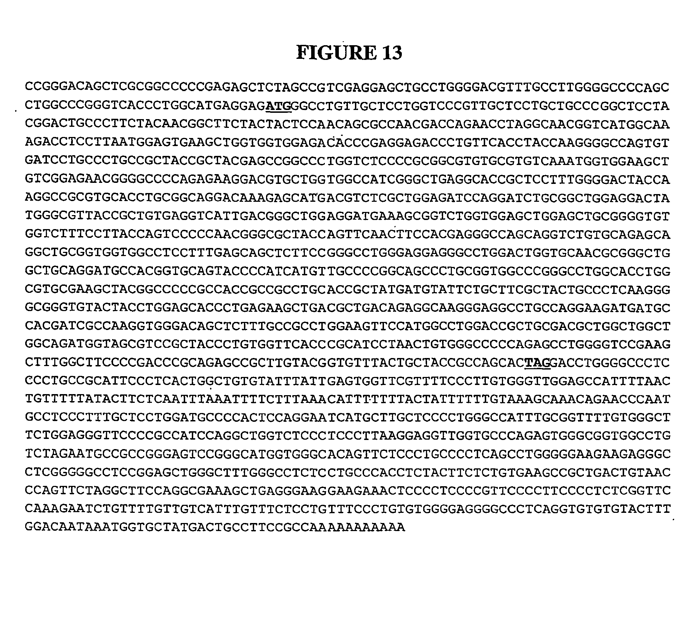

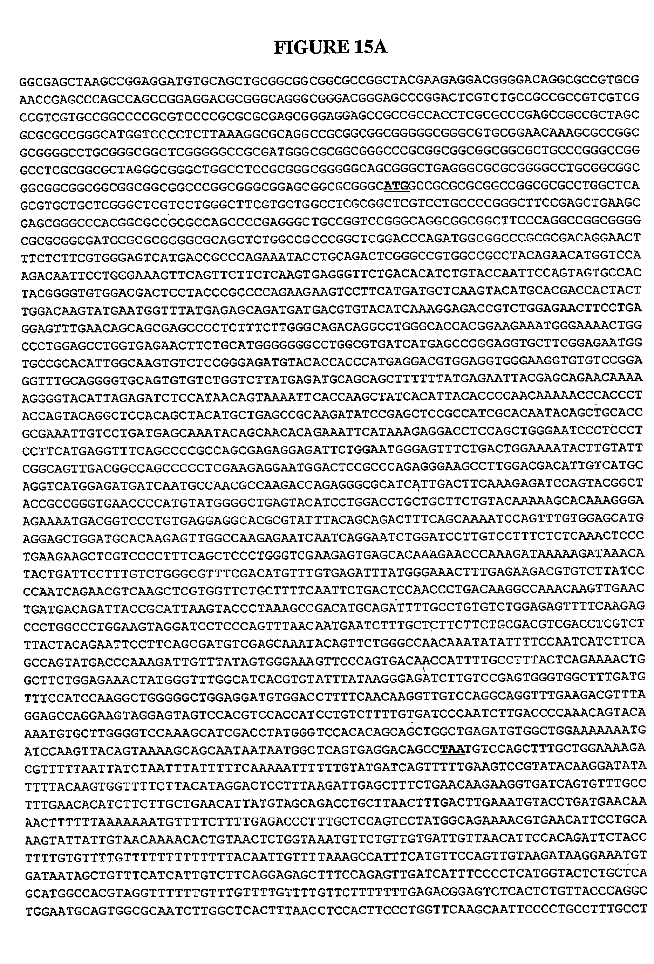

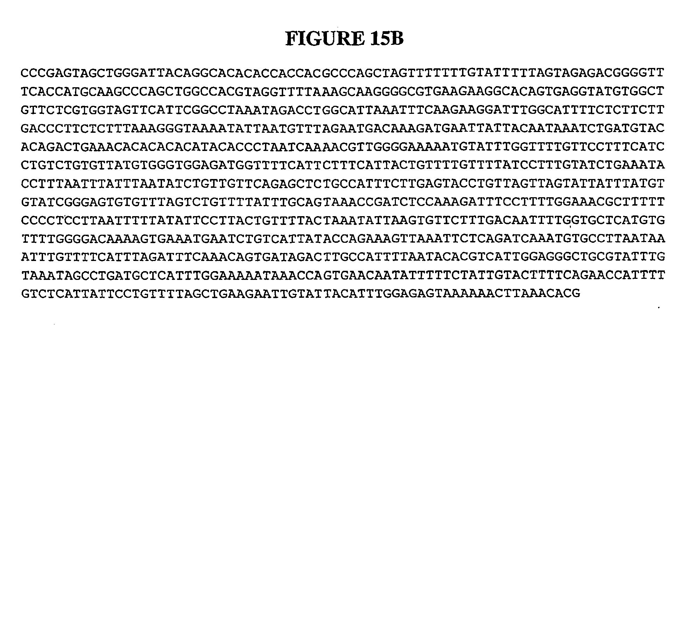

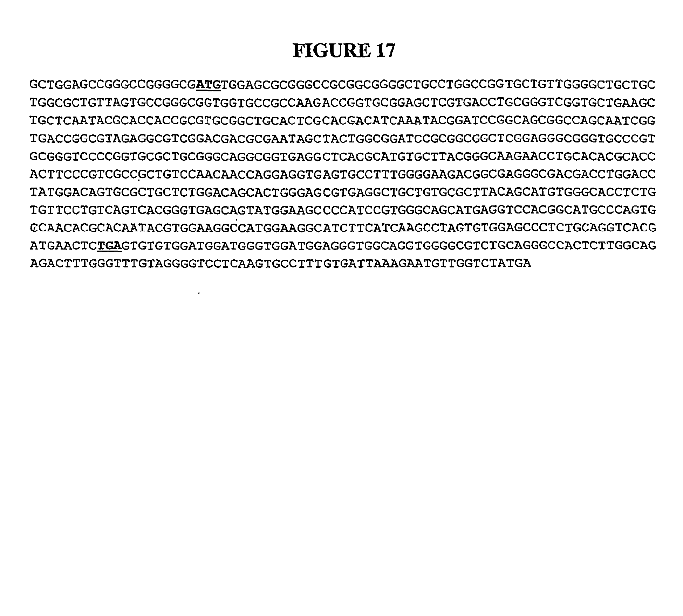

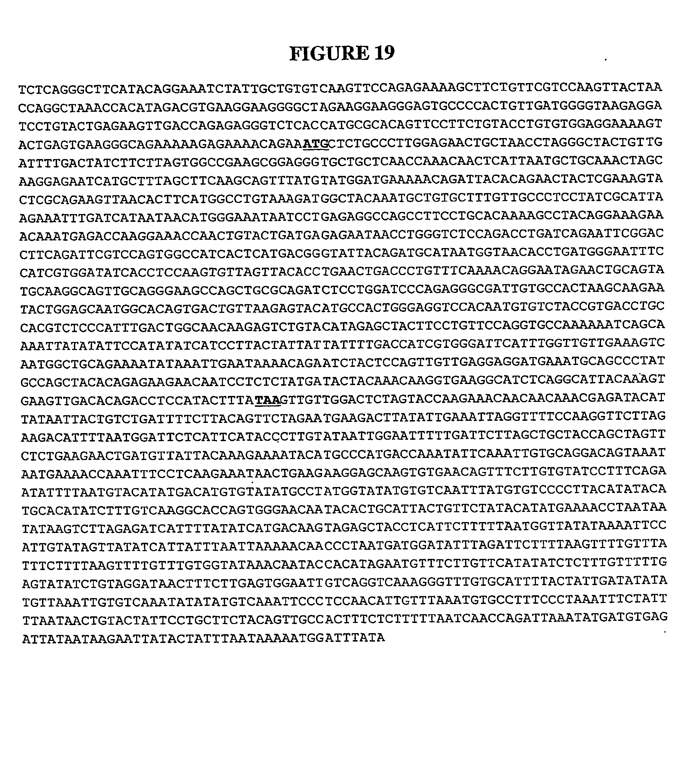

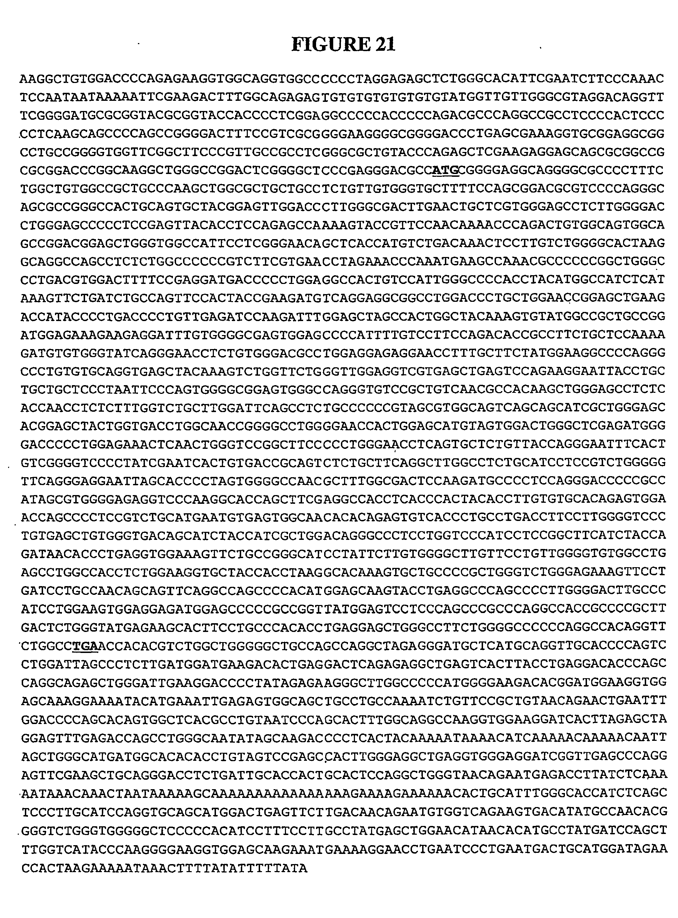

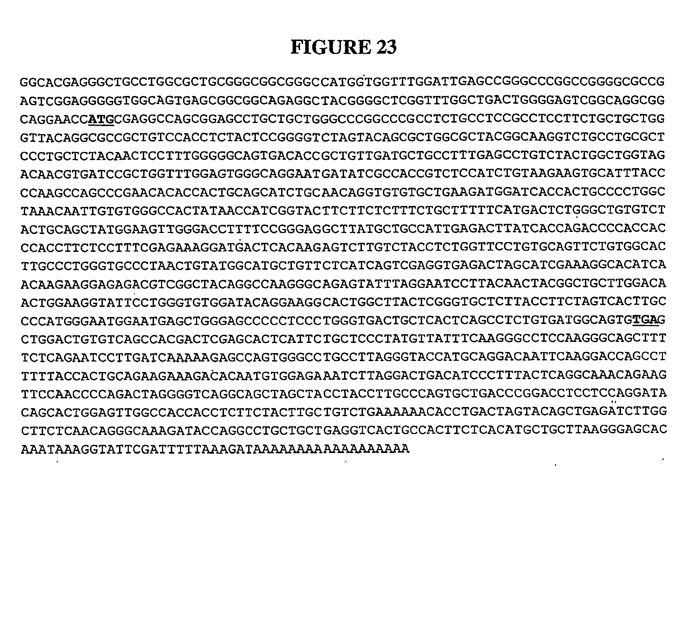

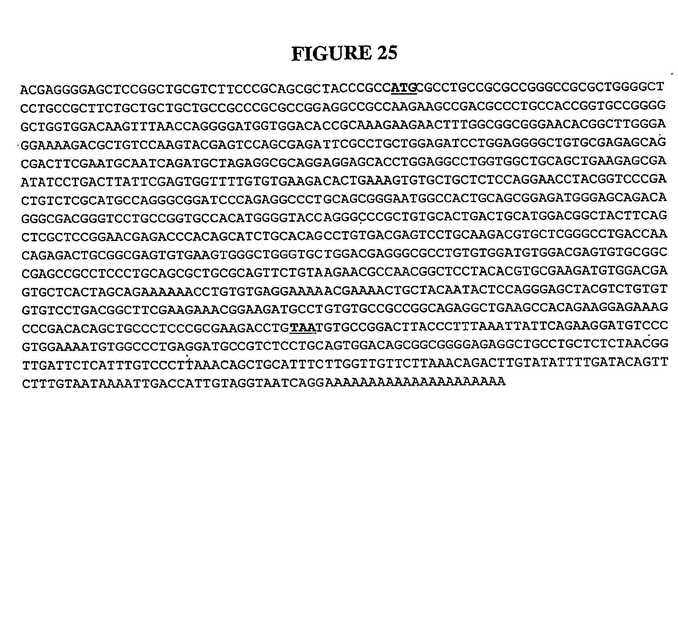

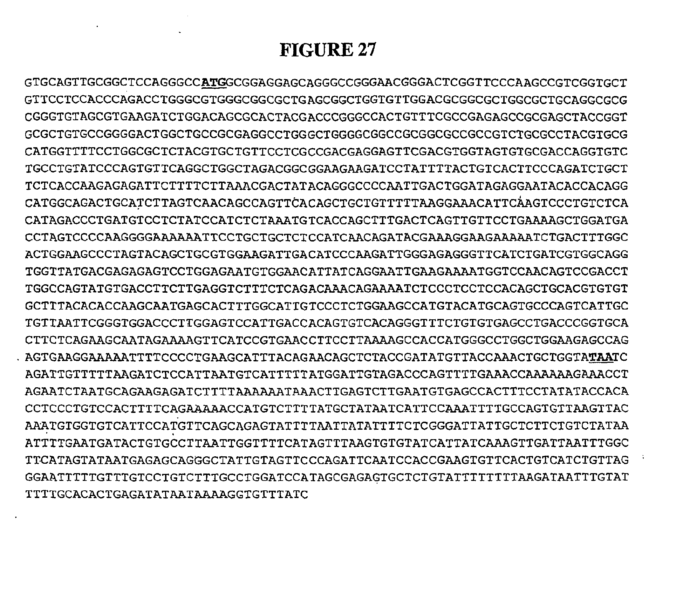

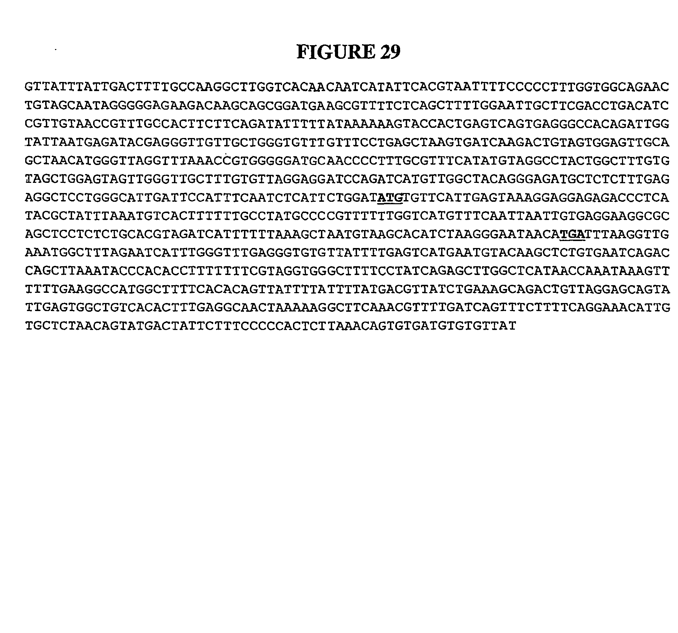

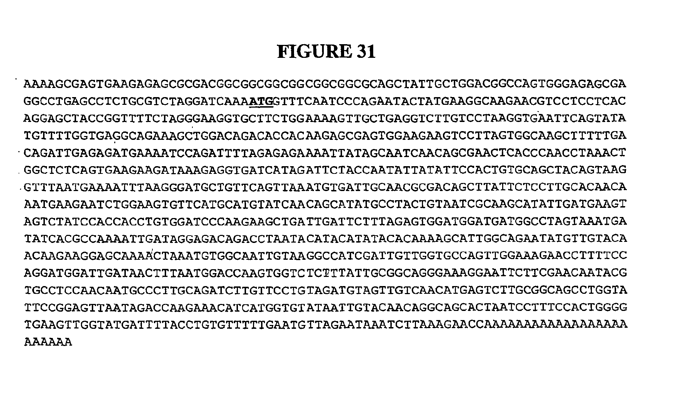

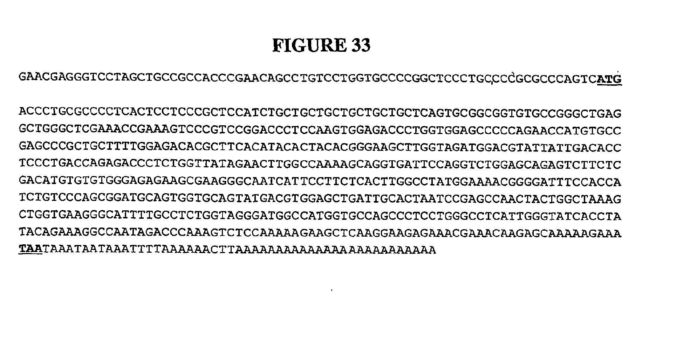

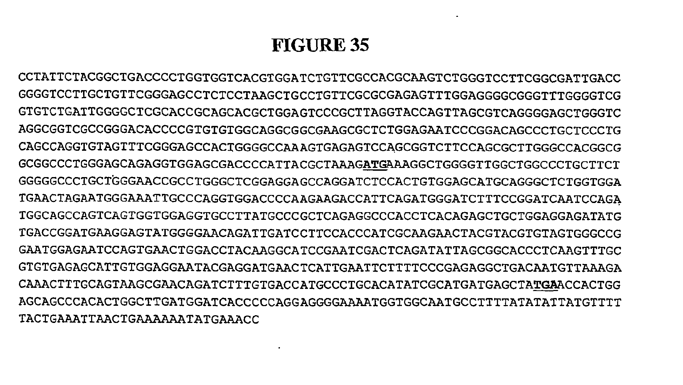

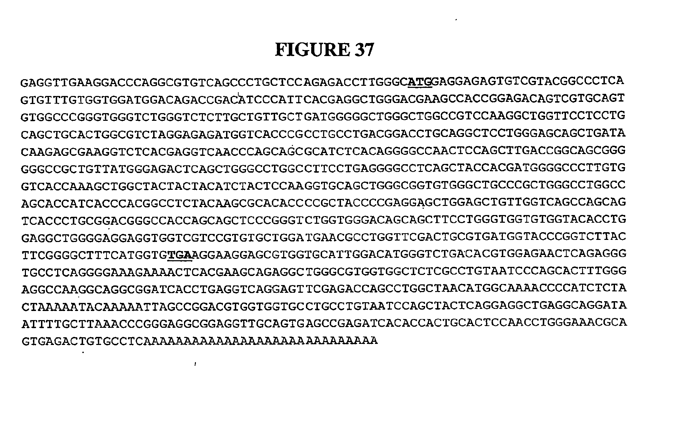

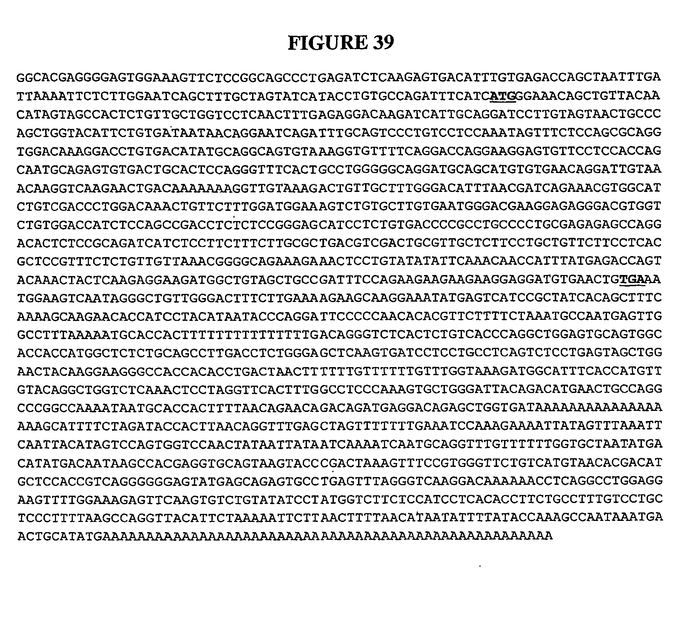

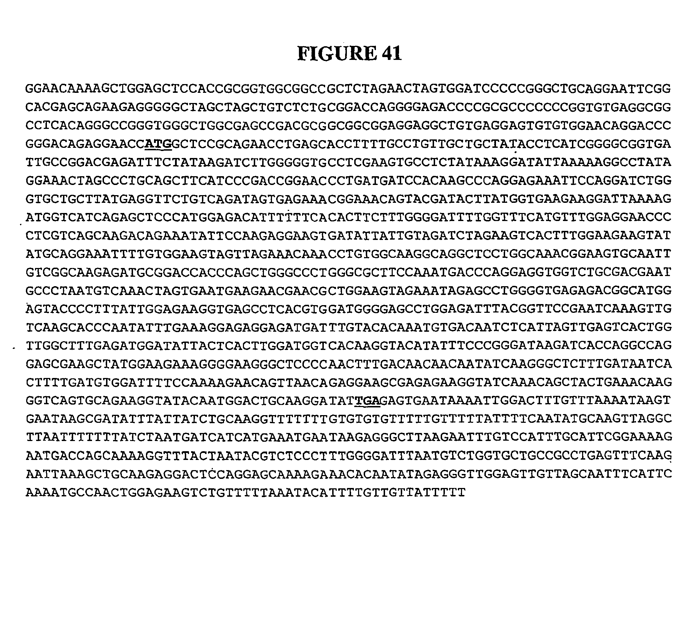

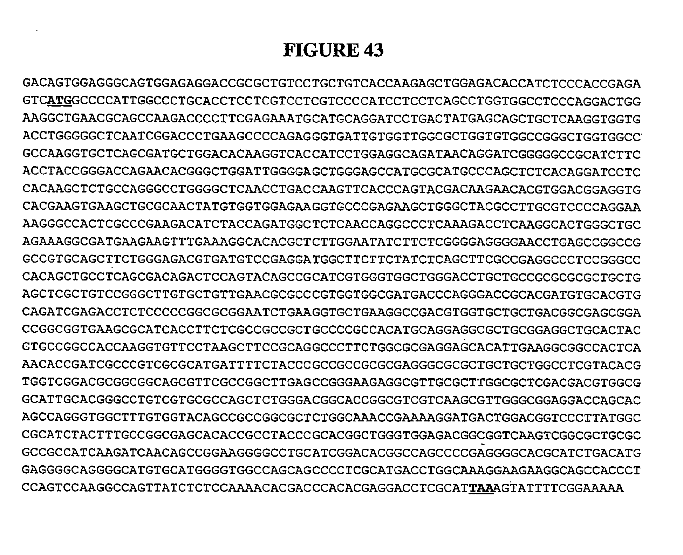

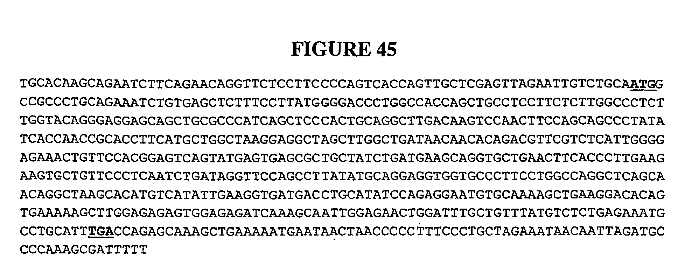

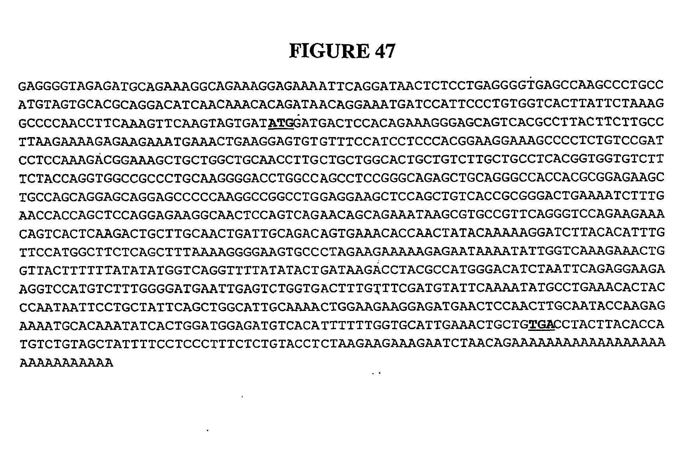

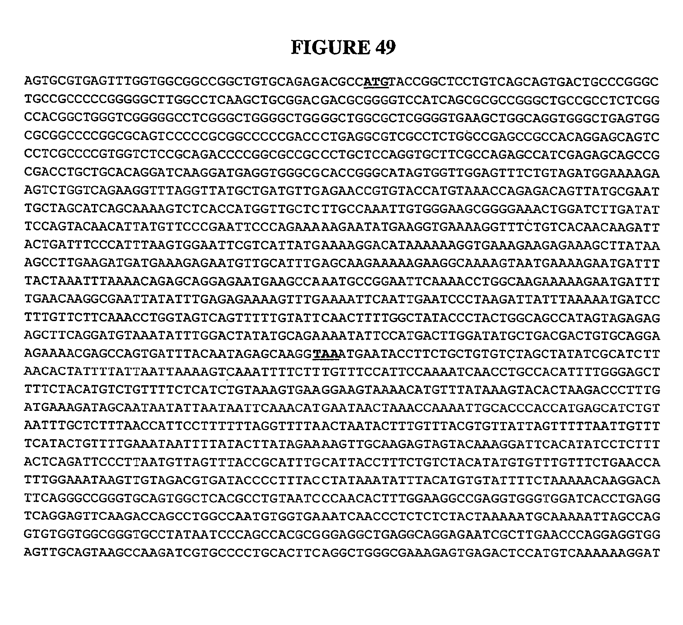

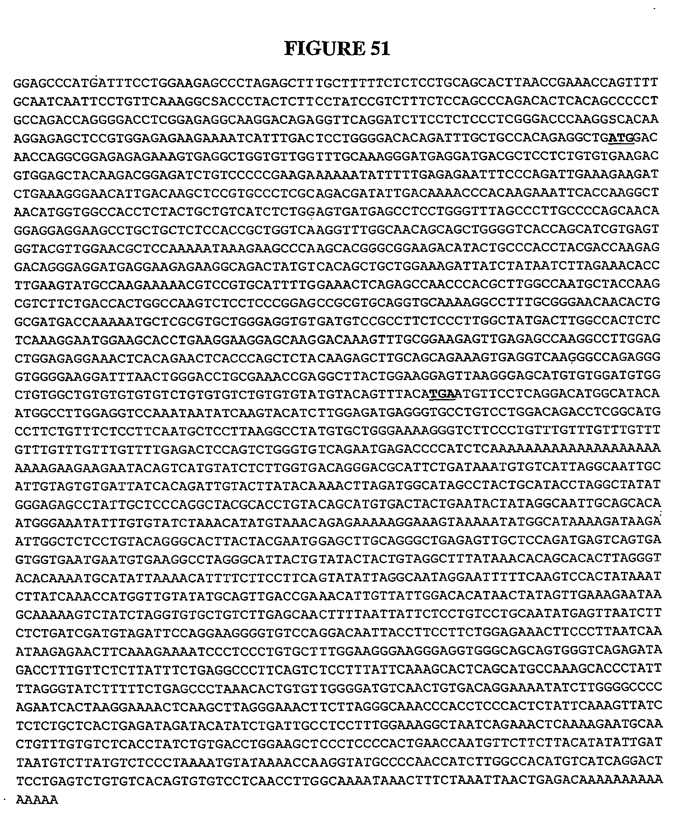

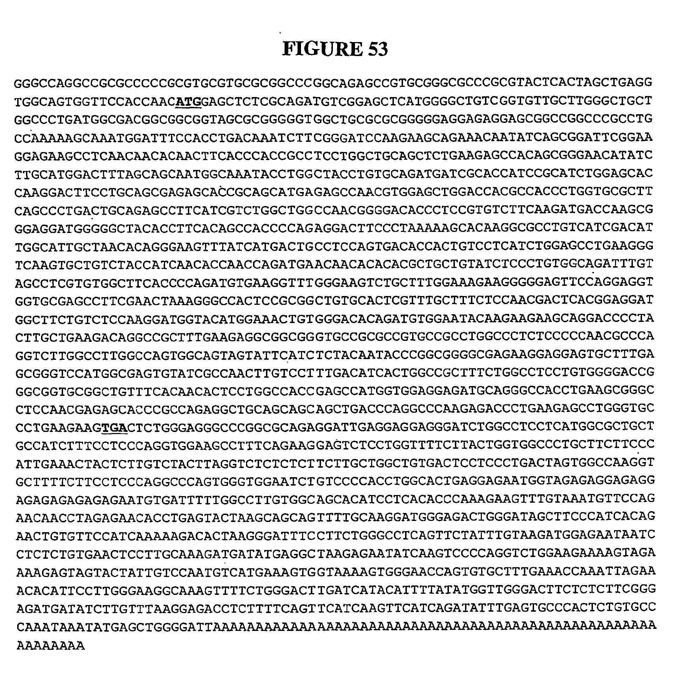

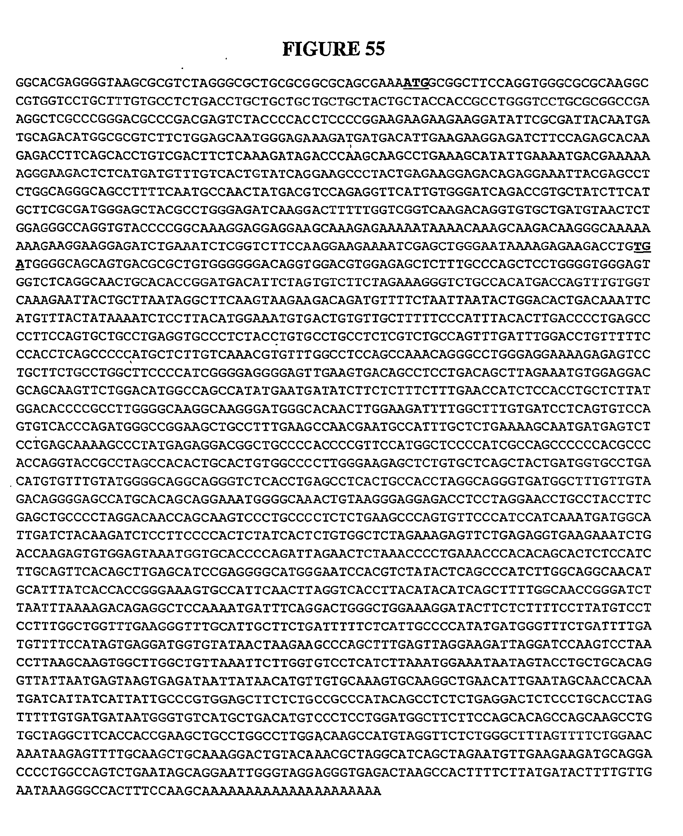

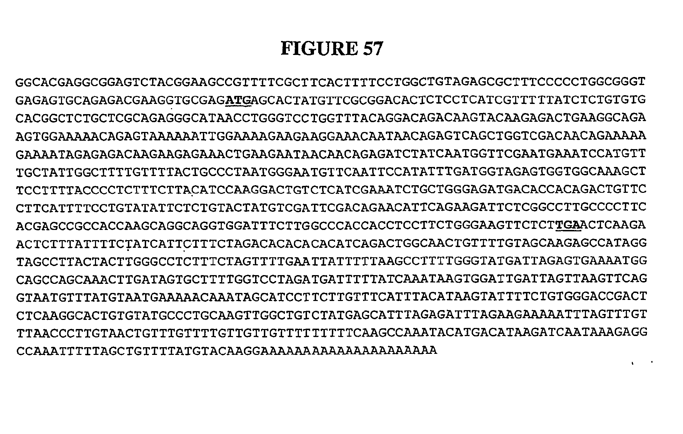

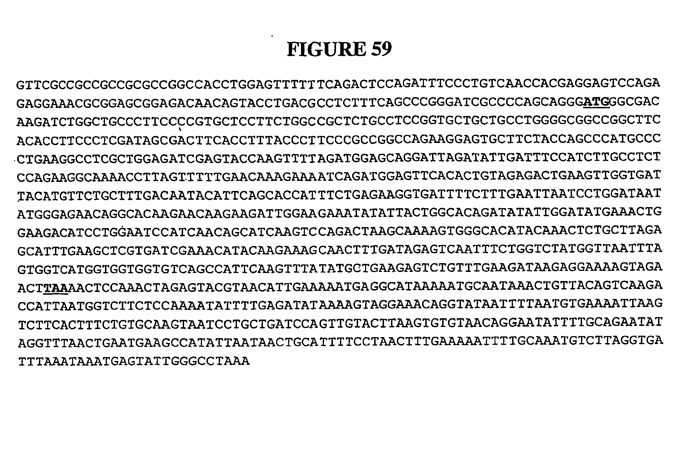

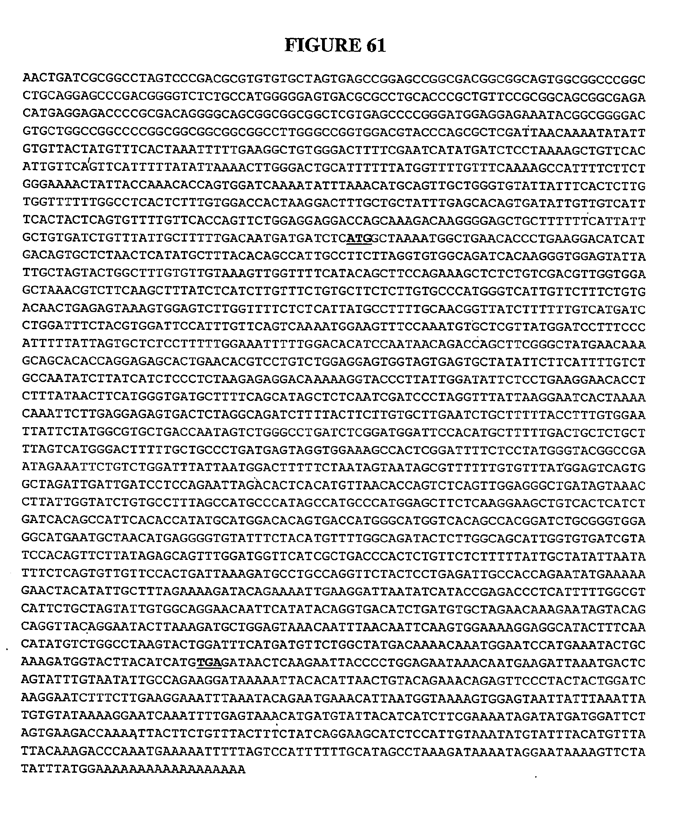

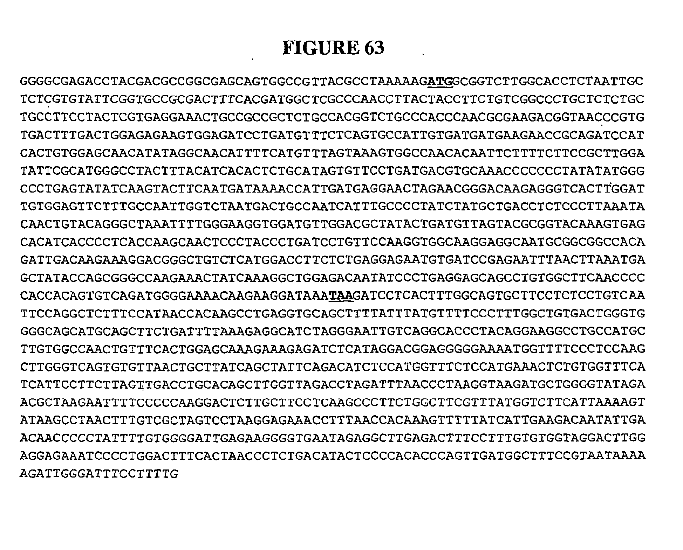

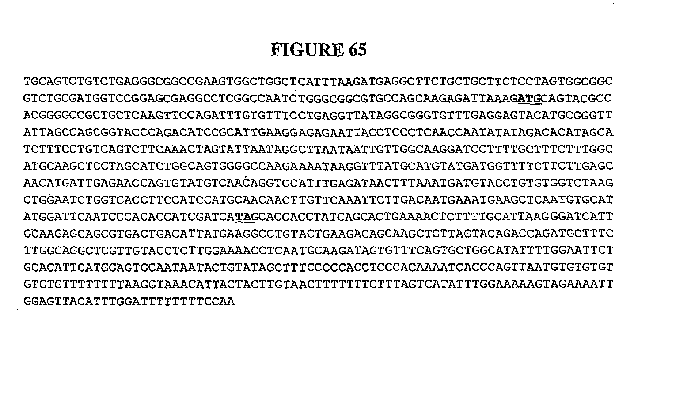

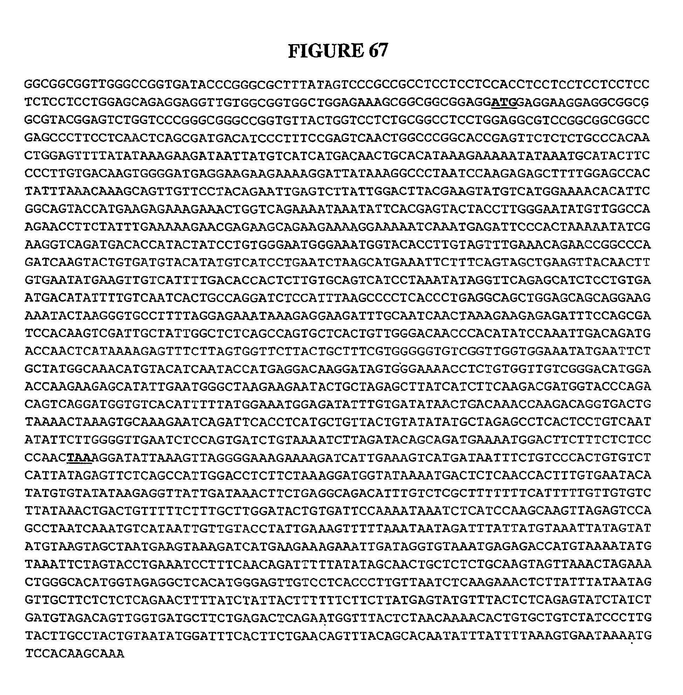

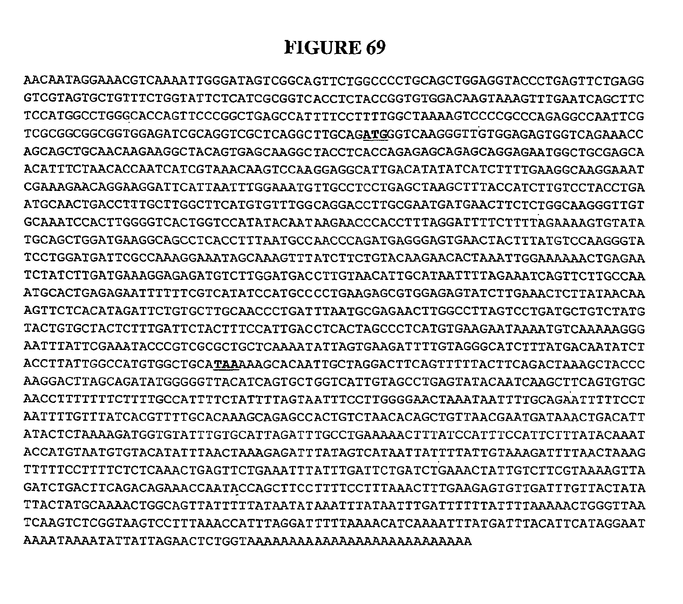

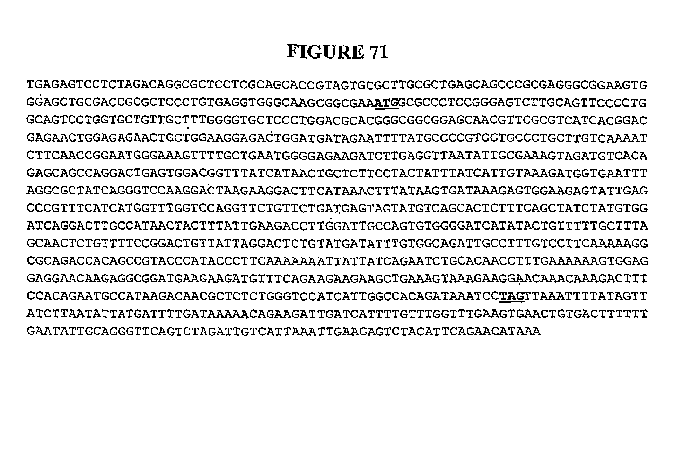

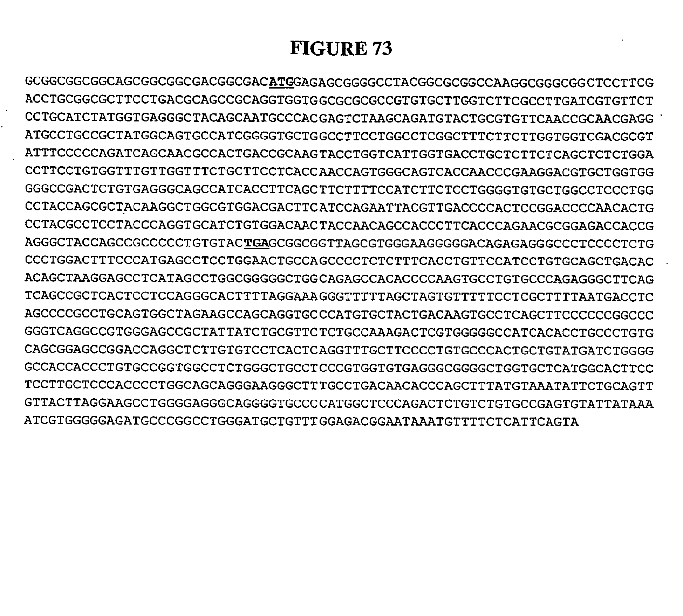

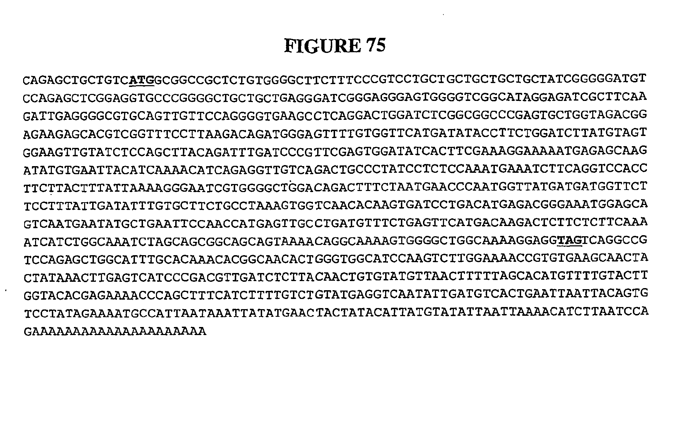

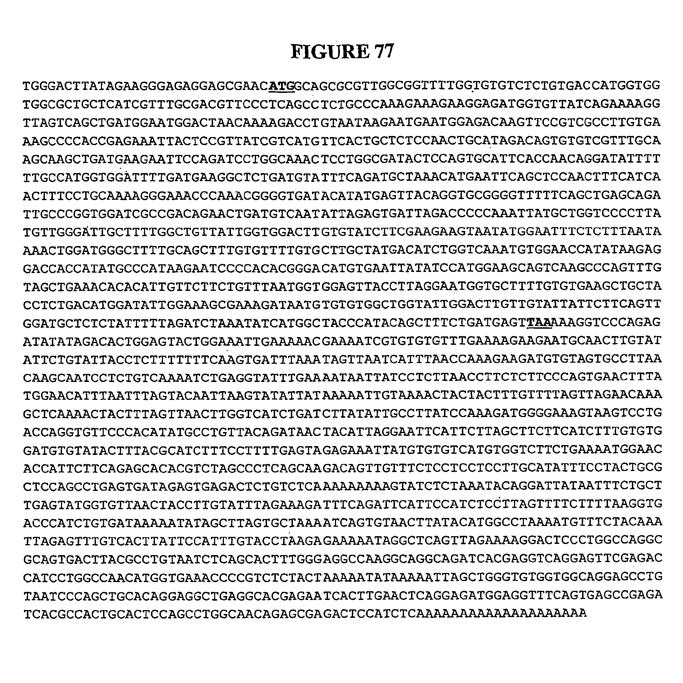

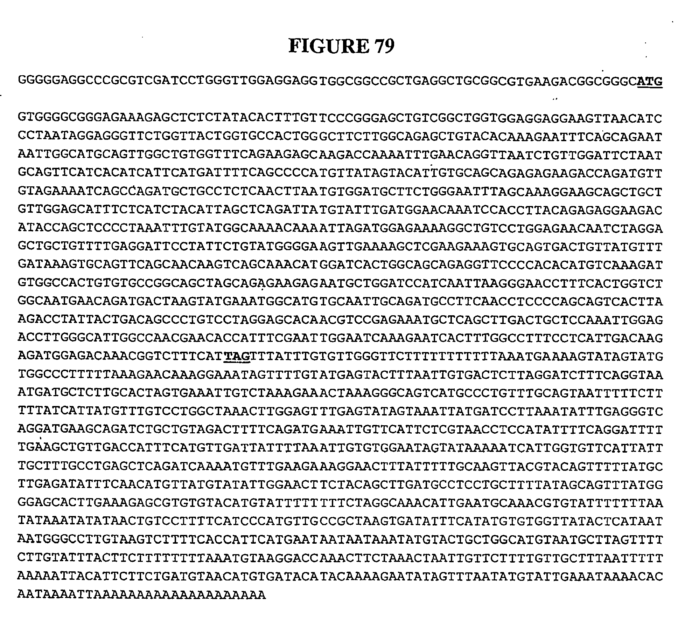

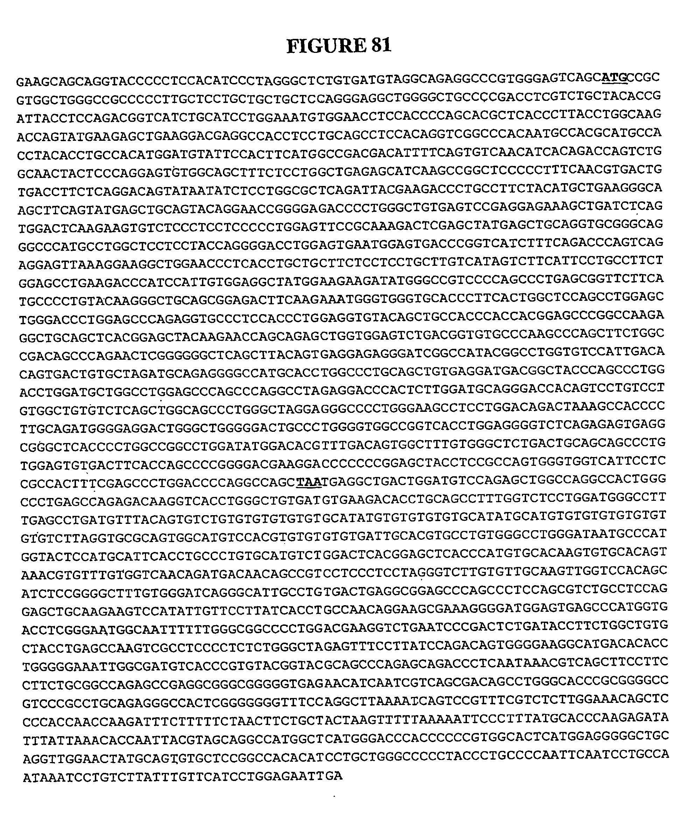

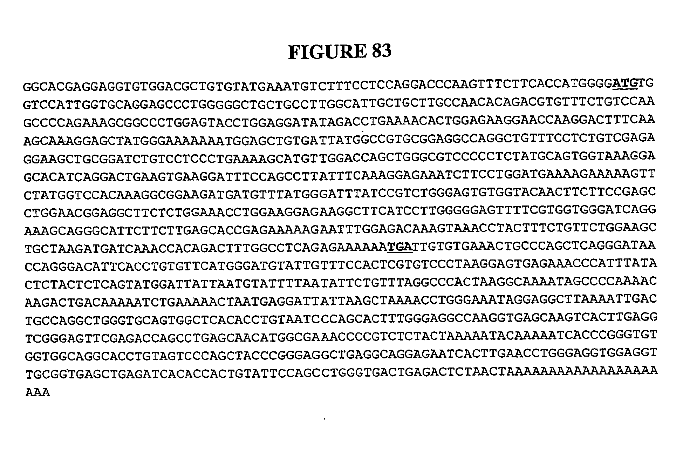

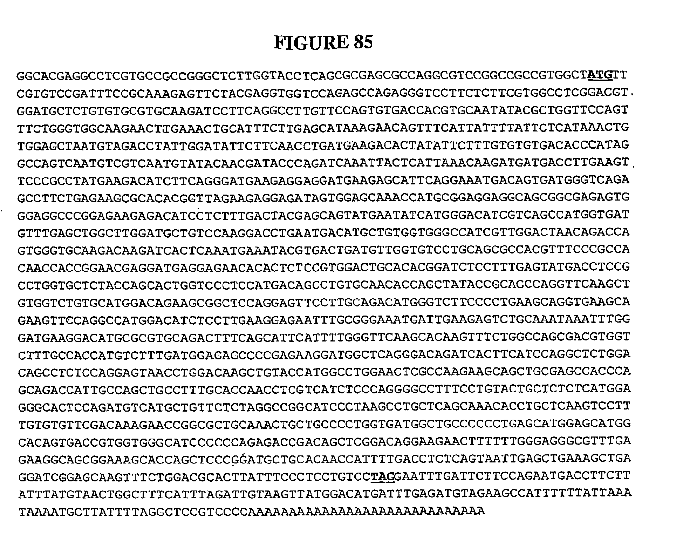

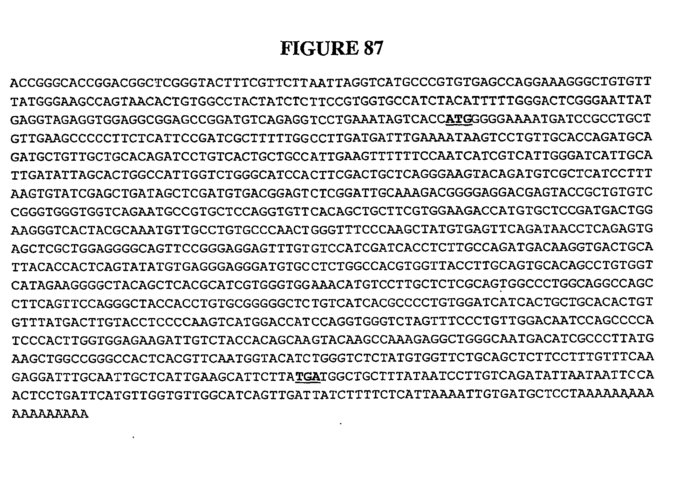

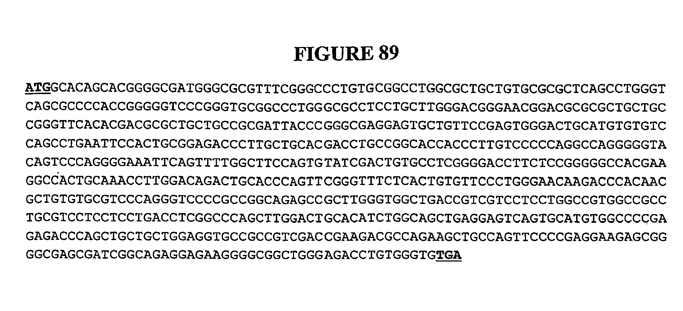

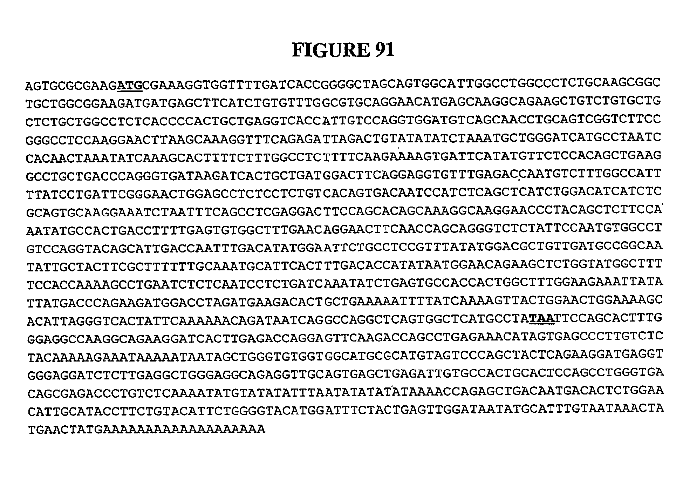

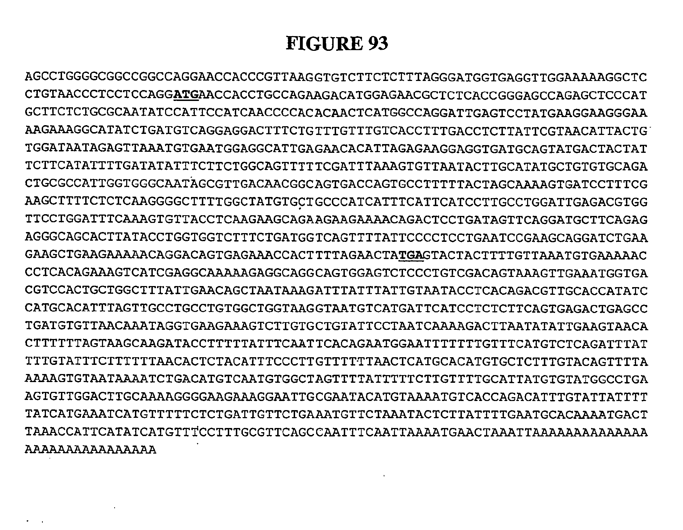

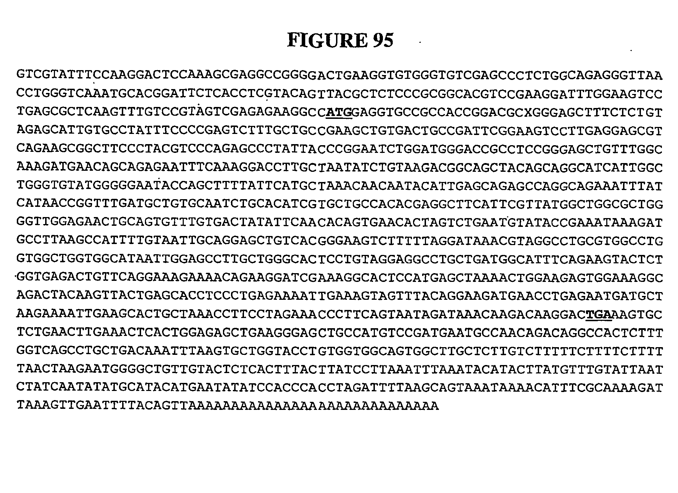

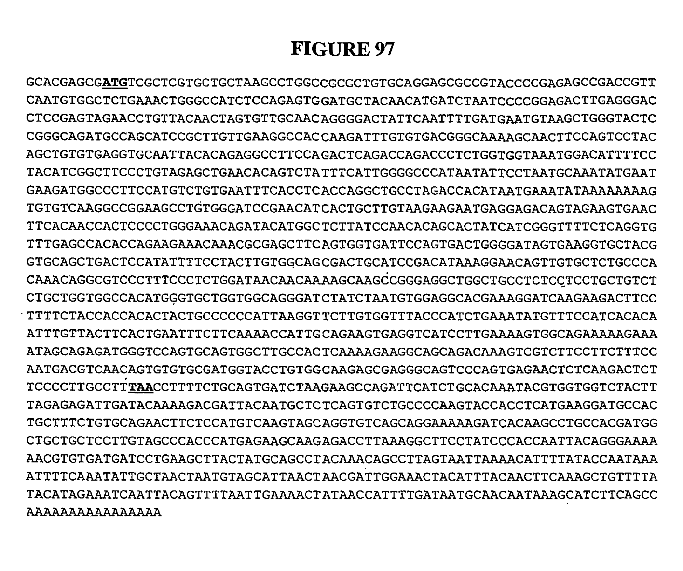

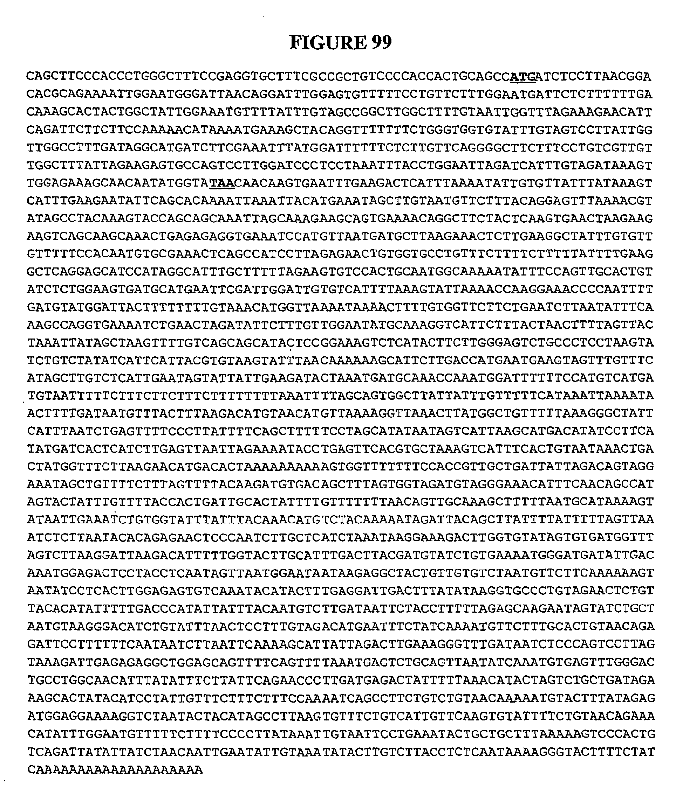

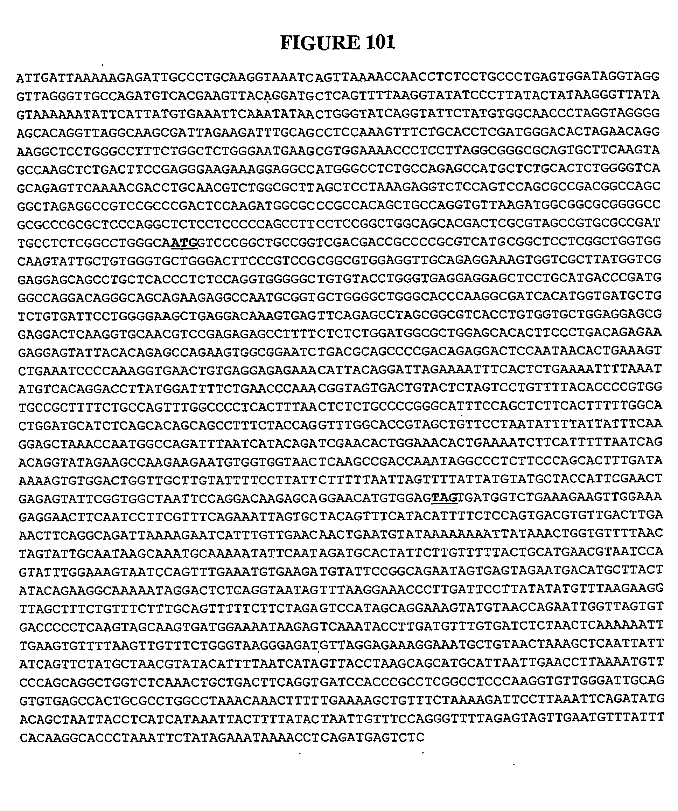

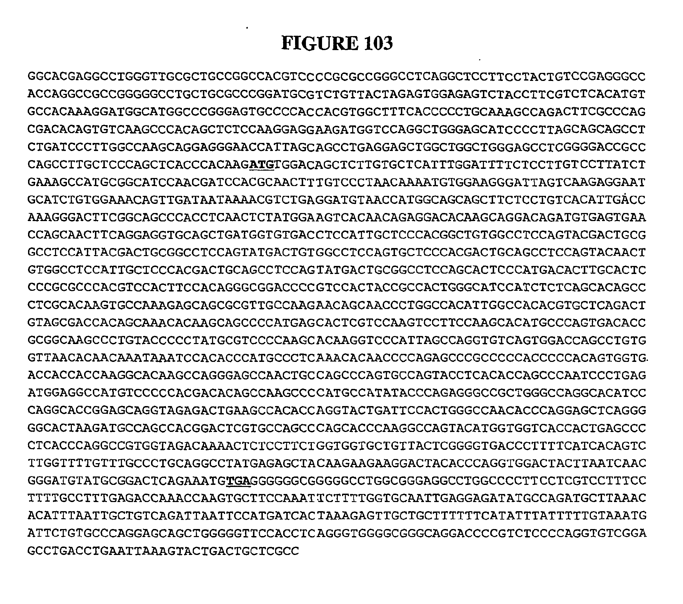

The nucleic acids of Figure 1 (SEQ ID NO:1), Figure 3 (SEQ ID NO:3), Figure 5 (SEQ ID NO:5), Figure 7 (SEQ ID NO:7), Figure 9 (SEQ ID NO:9), Figure 11 (SEQ ID NO:11), Figure 13 (SEQ ID NO:13), Figure 15A-B (SEQ ID NO:15), Figure 17 (SEQ ID NO:17), Figure 19 (SEQ ID NO:19), Figure 21 (SEQ ID NO:21), Figure 23 (SEQ ID NO:23), Figure 25 (SEQ ID NO:25), Figure 27 (SEQ ID NO:27), Figure 29 (SEQ ID NO:29), Figure 31 (SEQ ID NO:31), Figure 33 (SEQ ID NO:33), Figure 35 (SEQ ID NO:35), Figure 37 (SEQ ID NO:37), Figure 39 (SEQ ID NO:39), Figure 41 (SEQ ID NO:41), Figure 43 (SEQ ID NO:43), Figure 45 (SEQ ID NO:45), Figure 47 (SEQ ID NO:47), Figure 49 (SEQ ID NO:49), Figure 51 (SEQ ID NO:51), Figure 53 (SEQ ID NO:53), Figure 55 (SEQ ID NO:55), Figure 57 (SEQ ID NO:57), Figure 59 (SEQ ID NO:59), Figure 61 (SEQ ID NO:61), Figure 63 (SEQ ID NO:63), Figure 65 (SEQ ID NO:65), Figure 67 (SEQ ID NO:67), Figure 69 (SEQ ID NO:69), Figure 71 (SEQ ID NO:71), Figure 73 (SEQ ID NO:73), Figure 75 (SEQ ID NO:75), Figure 77 (SEQ ID NO:77), Figure 79 (SEQ ID NO:79), Figure 81 (SEQ ID NO:81), Figure 83 (SEQ ID NO:83), Figure 85 (SEQ ID NO:85), Figure 87 (SEQ ID NO:87), Figure 89 (SEQ ID NO:89), Figure 91 (SEQ ID NO:91), Figure 93 (SEQ ID NO:93), Figure 95 (SEQ ID NO:95), Figure 97 (SEQ ID NO:97), Figure 99 (SEQ ID NO:99), Figure 101 (SEQ ID NO:101) and Figure 103 (SEQ ID NO:103) show increase in expression upon stimulation with anti-CD3/ICAM1 and also show increase in expression upon stimulation with anti-CD3/anti-CD28.

### EXAMPLE 2: Use of PRO as a hybridization probe

The following method describes use of a nucleotide sequence encoding PRO as a hybridization probe.

DNA comprising the coding sequence of full-length or mature PRO as disclosed herein is employed as a probe to screen for homologous DNAs (such as those encoding naturally-occurring variants of PRO) in human tissue cDNA libraries or human tissue genomic libraries.

Hybridization and washing of filters containing either library DNAs is performed under the following high stringency conditions. Hybridization of radiolabeled PRO-derived probe to the filters is performed in a solution of 50% formamide, 5x SSC, 0.1% SDS, 0.1% sodium pyrophosphate, 50 mM sodium phosphate, pH 6.8, 2x Denhardt's solution, and 10% dextran sulfate at 42°C for 20 hours. Washing of the filters is performed in an aqueous solution of 0.1x SSC and 0.1% SDS at 42°C.

DNAs having a desired sequence identity with the DNA encoding full-length native sequence PRO can then be identified using standard techniques known in the art.

### EXAMPLE 3: Expression of PRO in E. coli

This example illustrates preparation of an unglycosylated form of PRO by recombinant expression in E. *coli.*

The DNA sequence encoding PRO is initially amplified using selected PCR primers. The primers should contain restriction enzyme sites which correspond to the restriction enzyme sites on the selected expression vector. A variety of expression vectors may be employed. An example of a suitable vector is pBR322 (derived from *E. coli;* see Bolivar et al., Gene, 2:95 (1977)) which contains genes for ampicillin and tetracycline resistance. The vector is digested with restriction enzyme and dephosphorylated. The PCR amplified sequences are then ligated into the vector. The vector will preferably include sequences which encode for an antibiotic resistance gene, a trp promoter, a polyhis leader (including the first six STII codons, polyhis sequence, and enterokinase cleavage site), the PRO coding region, lambda transcriptional terminator, and an argU gene.

The ligation mixture is then used to transform a selected E. *coli* strain using the methods described in Sambrook et al., supra. Transformants are identified by their ability to grow on LB plates and antibiotic resistant colonies are then selected. Plasmid DNA can be isolated and confirmed by restriction analysis and DNA sequencing.

Selected clones can be grown overnight in liquid culture medium such as LB broth supplemented with antibiotics. The overnight culture may subsequently be used to inoculate a larger scale culture. The cells are then grown to a desired optical density, during which the expression promoter is turned on.

After culturing the cells for several more hours, the cells can be harvested by centrifugation. The cell pellet obtained by the centrifugation can be solubilized using various agents known in the art, and the solubilized PRO protein can then be purified using a metal chelating column under conditions that allow tight binding of the protein.

PRO may be expressed in E. *coli* in a poly-His tagged form, using the following procedure. The DNA encoding PRO is initially amplified using selected PCR primers. The primers will contain restriction enzyme sites which correspond to the restriction enzyme sites on the selected expression vector, and other useful sequences providing for efficient and reliable translation initiation, rapid purification on a metal chelation column, and proteolytic removal with enterokinase. The PCR-amplified, poly-His tagged sequences are then ligated into an expression vector, which is used to transform an E. *coli* host based on strain 52 (W3110 fuhA(tonA) lon galE rpoHts(htpRts) clpP(lacIq). Transformants are first grown in LB containing 50 mg/ml carbenicillin at 30°C with shaking until an O.D.600 of 3-5 is reached. Cultures are then diluted 50-100 fold into CRAP media (prepared by mixing 3.57 g (NH₄)₂SO₄, 0.71 g sodium citrate•2H2O, 1.07 g KCl, 5.36 g Difco yeast extract, 5.36 g Sheffield hycase SF in 500 mL water, as well as 110 mM MPOS, pH 7.3, 0.55% (w/v) glucose and 7 mM MgSO₄ and grown for approximately 20-30 hours at 30°C with shaking. Samples are removed to verify expression by SDS-PAGE analysis, and the bulk culture is centrifuged to pellet the cells. Cell pellets are frozen until purification and refolding.

*E. coli* paste from 0.5 to 1 L fermentations (6-10 g pellets) is resuspended in 10 volumes (w/v) in 7 M guanidine, 20 mM Tris, pH 8 buffer. Solid sodium sulfite and sodium tetrathionate is added to make final concentrations of 0.1M and 0.02 M, respectively, and the solution is stirred overnight at 4°C. This step results in a denatured protein with all cysteine residues blocked by sulfitolization. The solution is centrifuged at 40,000 rpm in a Beckman Ultracentifuge for 30 min. The supernatant is diluted with 3-5 volumes of metal chelate column buffer (6 M guanidine, 20 mM Tris, pH 7.4) and filtered through 0.22 micron filters to clarify. The clarified extract is loaded onto a 5 ml Qiagen Ni-NTA metal chelate column equilibrated in the metal chelate column buffer. The column is washed with additional buffer containing 50 mM imidazole (Calbiochem, Utrol grade), pH 7.4. The protein is eluted with buffer containing 250 mM imidazole. Fractions containing the desired protein are pooled and stored at 4°C. Protein concentration is estimated by its absorbance at 280 nm using the calculated extinction coefficient based on its amino acid sequence.

The proteins are refolded by diluting the sample slowly into freshly prepared refolding buffer consisting of: 20 mM Tris, pH 8.6, 0.3 M NaCl, 2.5 M urea, 5 mM cysteine, 20 mM glycine and 1 mM EDTA. Refolding volumes are chosen so that the final protein concentration is between 50 to 100 micrograms/ml. The refolding solution is stirred gently at 4°C for 12-36 hours. The refolding reaction is quenched by the addition of TFA to a final concentration of 0.4% (pH of approximately 3). Before further purification of the protein, the solution is filtered through a 0.22 micron filter and acetonitrile is added to 2-10% final concentration.' The refolded protein is chromatographed on a Poros R1/H reversed phase column using a mobile buffer of 0.1% TFA with elution with a gradient of acetonitrile from 10 to 80%. Aliquots of fractions with A280 absorbance are analyzed on SDS polyacrylamide gels and fractions containing homogeneous refolded protein are pooled. Generally, the properly refolded species of most proteins are eluted at the lowest concentrations of acetonitrile since those species are the most compact with their hydrophobic interiors shielded from interaction with the reversed phase resin. Aggregated species are usually eluted at higher acetonitrile concentrations. In addition to resolving misfolded forms of proteins from the desired form, the reversed phase step also removes endotoxin from the samples.

Fractions containing the desired folded PRO polypeptide are pooled and the acetonitrile removed using a gentle stream of nitrogen directed at the solution. Proteins are formulated into 20 mM Hepes, pH 6.8 with 0.14 M sodium chloride and 4% mannitol by dialysis or by gel filtration using G25 Superfine (Pharmacia) resins equilibrated in the formulation buffer and sterile filtered.

Many of the PRO polypeptides disclosed herein were successfully expressed as described above.

### EXAMPLE 4: Expression of PRO in mammalian cells

This example illustrates preparation of a potentially glycosylated form of PRO by recombinant expression in mammalian cells.

The vector, pRK5 (see EP 307,247, published March 15, 1989), is employed as the expression vector. Optionally, the PRO DNA is ligated into pRK5 with selected restriction enzymes to allow insertion of the PRO DNA using ligation methods such as described in Sambrook et al., supra. The resulting vector is called pRK5-PRO.

In one embodiment, the selected host cells may be 293 cells. Human 293 cells (ATCC CCL 1573) are grown to confluence in tissue culture plates in medium such as DMEM supplemented with fetal calf serum and optionally, nutrient components and/or antibiotics. About 10 µg pRK5-PRO DNA is mixed with about 1 µg DNA encoding the VA RNA gene [Thimmappaya et al., Cell, 31:543 (1982)] and dissolved in 500 µl of 1 mM Tris-HCl, 0.1 mM EDTA, 0.227 M CaCl₂. To this mixture is added, dropwise, 500 µl of 50 mM HEPES (pH 7.35), 280 mM NaCl, 1.5 mM NaPO₄ and a precipitate is allowed to form for 10 minutes at 25°C. The precipitate is suspended and added to the 293 cells and allowed to settle for about four hours at 37°C. The culture medium is aspirated off and 2 ml of 20% glycerol in PBS is added for 30 seconds. The 293 cells are then washed with serum free medium, fresh medium is added and the cells are incubated for about 5 days.

Approximately 24 hours after the transfections, the culture medium is removed and replaced with culture medium (alone) or culture medium containing 200 µCi/ml ³⁵S-cysteine and 200 µCi/ml ³⁵S-methionine. After a 12 hour incubation, the conditioned medium is collected, concentrated on a spin filter, and loaded onto a 15% SDS gel. The processed gel may be dried and exposed to film for a selected period of time to reveal the presence of PRO polypeptide. The cultures containing transfected cells may undergo further incubation (in serum free medium) and the medium is tested in selected bioassays.

In an alternative technique, PRO may be introduced into 293 cells transiently using the dextran sulfate method described by Somparyrac et al., Proc. Natl. Acad. Sci., 12:7575 (1981). 293 cells are grown to maximal density in a spinner flask and 700 µg pRK5-PRO DNA is added. The cells are first concentrated from the spinner flask by centrifugation and washed with PBS. The DNA-dextran precipitate is incubated on the cell pellet for four hours. The cells are treated with 20% glycerol for 90 seconds, washed with tissue culture medium, and re-introduced into the spinner flask containing tissue culture medium, 5 µg/ml bovine insulin and 0.1 µg/ml bovine transferrin. After about four days, the conditioned media is centrifuged and filtered to remove cells and debris. The sample containing expressed PRO can then be concentrated and purified by any selected method, such as dialysis and/or column chromatography.

In another embodiment, PRO can be expressed in CHO cells. The pRK5-PRO can be transfected into CHO cells using known reagents such as CaPO₄ or DEAE-dextran. As described above, the cell cultures can be incubated, and the medium replaced with culture medium (alone) or medium containing a radiolabel such as ³⁵S-methionine. After determining the presence of PRO polypeptide, the culture medium may be replaced with serum free medium. Preferably, the cultures are incubated for about 6 days, and then the conditioned medium is harvested. The medium containing the expressed PRO can then be concentrated and purified by any selected method.

Epitope-tagged PRO may also be expressed in host CHO cells. The PRO may be subcloned out of the pRK5 vector. The subclone insert can undergo PCR to fuse in frame with a selected epitope tag such as a poly-his tag into a Baculovirus expression vector. The poly-his tagged PRO insert can then be subcloned into a SV40 promoter/enhancer containing vector containing a selection marker such as DHFR for selection of stable clones. Finally, the CHO cells can be transfected (as described above) with the SV40 promoter/enhancer containing vector. Labeling may be performed, as described above, to verify expression: The culture medium containing the expressed poly-His tagged PRO can then be concentrated and purified by any selected method, such as by Ni²⁺-chelate affinity chromatography.

PRO may also be expressed in CHO and/or COS cells by a transient expression procedure or in CHO cells by another stable expression procedure.

Stable expression in CHO cells is performed using the following procedure. The proteins are expressed as an IgG construct (immunoadhesin), in which the coding sequences for the soluble forms (e.g. extracellular domains) of the respective proteins are fused to an IgGl constant region sequence containing the hinge, CH2 and CH2 domains and/or is a poly-His tagged form.

Following PCR amplification, the respective DNAs are subcloned in a CHO expression vector using standard techniques as described in Ausubel et al., Current Protocols of Molecular Biology, Unit 3.16, John Wiley and Sons (1997). CHO expression vectors are constructed to have compatible restriction sites 5' and 3' of the DNA of interest to allow the convenient shuttling of cDNA's. The vector used expression in CHO cells is as described in Lucas et al., Nucl. Acids Res. 24:9 (1774-1779 (1996), and uses the SV40 early promoter/enhancer to drive expression of the cDNA of interest and dihydrofolate reductase (DHFR). DHFR expression permits selection for stable maintenance of the plasmid following transfection.

Twelve micrograms of the desired plasmid DNA is introduced into approximately 10 million CHO cells using commercially available transfection reagents Superfect^{®} (Quiagen), Dosper^{®} or Fugene^{®} (Boehringer Mannheim). The cells are grown as described in Lucas et al., supra. Approximately 3 x 10⁻⁷ cells are frozen in an ampule for further growth and production as described below.

The ampules containing the plasmid DNA are thawed by placement into water bath and mixed by vortexing. The contents are pipetted into a centrifuge tube containing 10 mL of media and centrifuged at 1000 rpm for 5 minutes. The supernatant is aspirated and the cells are resuspended in 10 mL of selective media (0.2 µm filtered PS20 with 5% 0.2 µm diafiltered fetal bovine serum). The cells are then aliquoted into a 100 mL spinner containing 90 mL of selective media. After 1-2 days, the cells are transferred into a 250 mL spinner filled with 150 mL selective growth medium and incubated at 37°C. After another 2-3 days, 250 mL, 500 mL and 2000 mL spinners are seeded with 3 x 10⁵ cells/mL. The cell media is exchanged with fresh media by centrifugation and resuspension in production medium. Although any suitable CHO media may be employed, a production medium described in U.S. Patent No. 5,122,469, issued June 16, 1992 may actually be used. A 3L production spinner is seeded at 1.2 x 10⁶ cells/mL. On day 0, pH is determined. On day 1, the spinner is sampled and sparging with filtered air is commenced. On day 2, the spinner is sampled, the temperature shifted to 33°C, and 30 mL of 500 g/L glucose and 0.6 mL of 10% antifoam (e.g., 35% polydimethylsiloxane emulsion, Dow Corning 365 Medical Grade Emulsion) taken. Throughout the production, the pH is adjusted as necessary to keep it at around 7.2, After 10 days, or until the viability dropped below 70%, the cell culture is harvested by centrifugation and filtering through a 0.22 µm filter. The filtrate was either stored at 4°C or immediately loaded onto columns for purification.

For the poly-His tagged constructs, the proteins are purified using a Ni-NTA column (Qiagen). Before purification, imidazole is added to the conditioned media to a concentration of 5 mM. The conditioned media is pumped onto a 6 ml Ni-NTA column equilibrated in 20 mM Hepes, pH 7.4, buffer containing 0.3 M NaCl and 5 mM imidazole at a flow rate of 4-5 ml/min, at 4°C. After loading, the column is washed with additional equilibration buffer and the protein eluted with equilibration buffer containing 0.25 M imidazole. The highly purified protein is subsequently desalted into a storage buffer containing 10 mM Hepes, 0.14 M NaCl and 4% mannitol, pH 6.8, with a 25 ml G25 Superfine (Pharmacia) column and stored at -80°C.

Immunoadhesin (Fc-containing) constructs are purified from the conditioned media as follows. The conditioned medium is pumped onto a 5 ml Protein A column (Pharmacia) which had been equilibrated in 20 mM Na phosphate buffer, pH 6.8. After loading, the column is washed extensively with equilibration buffer before elution with 100 mM citric acid, pH 3.5. The eluted protein is immediately neutralized by collecting 1 ml fractions into tubes containing 275 µl of 1 M Tris buffer, pH 9. The highly purified protein is subsequently desalted into storage buffer as described above for the poly-His tagged proteins. The homogeneity is assessed by SDS polyacrylamide gels and by N-terminal amino acid sequencing by Edman degradation.

Many of the PRO polypeptides disclosed herein were successfully expressed as described above.

### EXAMPLE 5: Expression of PRO in Yeast

The following method describes recombinant expression of PRO in yeast.

First, yeast expression vectors are constructed for intracellular production or secretion of PRO from the ADH2/GAPDH promoter. DNA encoding PRO and the promoter is inserted into suitable restriction enzyme sites in the selected plasmid to direct intracellular expression of PRO. For secretion, DNA encoding PRO can be cloned into the selected plasmid, together with DNA encoding the ADH2/GAPDH promoter, a native PRO signal peptide or other mammalian signal peptide, or, for example, a yeast alpha-factor or invertase secretory signal/leader sequence, and linker sequences (if needed) for expression of PRO.

Yeast cells, such as yeast strain AB110, can then be transformed with the expression plasmids described above and cultured in selected fermentation media. The transformed yeast supernatants can be analyzed by precipitation with 10% trichloroacetic acid and separation by SDS-PAGE, followed by staining of the gels with Coomassie Blue stain.

Recombinant PRO can subsequently be isolated and purified by removing the yeast cells from the fermentation medium by centrifugation and then concentrating the medium using selected cartridge filters. The concentrate containing PRO may further be purified using selected column chromatography resins.

Many of the PRO polypeptides disclosed herein were successfully expressed as described above.

### EXAMPLE 6: Expression of PRO in Baculovirus-Infected Insect Cells

The following method describes recombinant expression of PRO in Baculovirus-infected insect cells.

The sequence coding for PRO is fused upstream of an epitope tag contained within a baculovirus expression vector. Such epitope tags include poly-his tags and immunoglobulin tags (like Fc regions of IgG). A variety of plasmids may be employed, including plasmids derived from commercially available plasmids such as pVL1393 (Novagen). Briefly, the sequence encoding PRO or the desired portion of the coding sequence of PRO such as the sequence encoding the extracellular domain of a transmembrane protein or the sequence encoding the mature protein if the protein is extracellular is amplified by PCR with primers complementary to the 5' and 3' regions. The 5' primer may incorporate flanking (selected) restriction enzyme sites. The product is then digested with those selected restriction enzymes and subcloned into the expression vector.

Recombinant baculovirus is generated by co-transfecting the above plasmid and BaculoGold^{™} virus DNA (Pharmingen) into *Spodoptera frugiperda* ("Sf9") cells (ATCC CRL 1711) using lipofectin (commercially available from GIBCO-BRL). After 4 - 5 days of incubation at 28°C, the released viruses are harvested and used for further amplifications. Viral infection and protein expression are performed as described by O'Reilley et al., Baculovirus expression vectors: A Laboratory Manual, Oxford: Oxford University Press (1994).

Expressed poly-his tagged PRO can then be purified, for example, by Ni²⁺-chelate affinity chromatography as follows. Extracts are prepared from recombinant virus-infected Sf9 cells as described by Rupert et al., Nature, 362:175-179 (1993). Briefly, Sf9 cells are washed, resuspended in sonication buffer (25 mL Hepes, pH 7.9; 12.5 mM MgCl₂; 0.1 mM EDTA; 10% glycerol; 0.1% NP-40; 0.4M KCl), and sonicated twice for 20 seconds on ice. The sonicates are cleared by centrifugation, and the supernatant is diluted 50-fold in loading buffer (50 mM phosphate, 300 mM NaCl, 10% glycerol, pH 7.8) and filtered through a 0.45 µm filter. A Ni²⁺-NTA agarose column (commercially available from Qiagen) is prepared with a bed volume of 5 mL, washed with 25 mL of water and equilibrated with 25 mL of loading buffer. The filtered cell extract is loaded onto the column at 0.5 mL per minute. The column is washed to baseline A₂₈₀ with loading buffer, at which point fraction collection is started. Next, the column is washed with a secondary wash buffer (50 mM phosphate; 300 mM NaCl, 10% glycerol, pH 6.0), which elutes nonspecifically bound protein. After reaching A₂₈₀ baseline again, the column is developed with a 0 to 500 mM Imidazole gradient in the secondary wash buffer. One mL fractions are collected and analyzed by SDS-PAGE and silver staining or Western blot with Ni²⁺-NTA-conjugated to alkaline phosphatase (Qiagen). Fractions containing the eluted His₁₀-tagged PRO are pooled and dialyzed against loading buffer.

Alternatively, purification of the IgG tagged (or Fc tagged) PRO can be performed using known chromatography techniques, including for instance, Protein A or protein G column chromatography.

Many of the PRO polypeptides disclosed herein were successfully expressed as described above.

### EXAMPLE 7: Preparation of Antibodies that Bind PRO

This example illustrates preparation of monoclonal antibodies which can specifically bind PRO.

Techniques for producing the monoclonal antibodies are known in the art and are described, for instance, in Goding, supra. Immunogens that may be employed include purified PRO, fusion proteins containing PRO, and cells expressing recombinant PRO on the cell surface. Selection of the immunogen can be made by the skilled artisan without undue experimentation.

Mice, such as Balb/c, are immunized with the PRO immunogen emulsified in complete Freund's adjuvant and injected subcutaneously or intraperitoneally in an amount from 1-100 micrograms. Alternatively, the immunogen is emulsified in MPL-TDM adjuvant (Ribi Immunochemical Research, Hamilton, MT) and injected into the animal's hind foot pads. The immunized mice are then boosted 10 to 12 days later with additional immunogen emulsified in the selected adjuvant. Thereafter, for several weeks, the mice may also be boosted with additional immunization injections. Serum samples may be periodically obtained from the mice by retro-orbital bleeding for testing in ELISA assays to detect anti-PRO antibodies.

After a suitable antibody titer has been detected, the animals "positive" for antibodies can be injected with a final intravenous injection of PRO. Three to four days later, the mice are sacrificed and the spleen cells are harvested. The spleen cells are then fused (using 35% polyethylene glycol) to a selected murine myeloma cell line such as P3X63AgU.1, available from ATCC, No. CRL 1597. The fusions generate hybridoma cells which can then be plated in 96 well tissue culture plates containing HAT (hypoxanthine, aminopterin, and thymidine) medium to inhibit proliferation of non-fused cells, myeloma hybrids, and spleen cell hybrids.

The hybridoma cells will be screened in an ELISA for reactivity against PRO. Determination of "positive" hybridoma cells secreting the desired monoclonal antibodies against PRO is within the skill in the art.

The positive hybridoma cells can be injected intraperitoneally into syngeneic Balb/c mice to produce ascites containing the anti-PRO monoclonal antibodies. Alternatively, the hybridoma cells can be grown in tissue culture flasks or roller bottles. Purification of the monoclonal antibodies produced in the ascites can be accomplished using ammonium sulfate precipitation, followed by gel exclusion chromatography. Alternatively, affinity chromatography based upon binding of antibody to protein A or protein G can be employed.

### EXAMPLE 8: Purification of PRO Polypeptides Using Specific Antibodies

Native or recombinant PRO polypeptides may be purified by a variety of standard techniques in the art of protein purification. For example, pro-PRO polypeptide, mature PRO polypeptide, or pre-PRO polypeptide is purified by immunoaffinity chromatography using antibodies specific for the PRO polypeptide of interest. In general, an immunoaffinity column is constructed by covalently coupling the anti-PRO polypeptide antibody to an activated chromatographic resin.

Polyclonal immunoglobulins are prepared from immune sera either by precipitation with ammonium sulfate or by purification on immobilized Protein A (Pharmacia LKB Biotechnology, Piscataway, N.J.). Likewise, monoclonal antibodies are prepared from mouse ascites fluid by ammonium sulfate precipitation or chromatography on immobilized Protein A. Partially purified immunoglobulin is covalently attached to a chromatographic resin such as CnBr-activated SEPHAROSE^{™} (Pharmacia LKB Biotechnology). The antibody is coupled to the resin, the resin is blocked, and the derivative resin is washed according to the manufacturer's instructions.

Such an immunoaffinity column is utilized in the purification of PRO polypeptide by preparing a fraction from cells containing PRO polypeptide in a soluble form. This preparation is derived by solubilization of the whole cell or of a subcellular fraction obtained via differential centrifugation by the addition of detergent or by other methods well known in the art. Alternatively, soluble PRO polypeptide containing a signal sequence may be secreted in useful quantity into the medium in which the cells are grown.

A soluble PRO polypeptide-containing preparation is passed over the immunoaffinity column, and the column is washed under conditions that allow the preferential absorbance of PRO polypeptide (*e*.*g*., high ionic strength buffers in the presence of detergent). Then, the column is eluted under conditions that disrupt antibody/PRO polypeptide binding (*e*.*g*., a low pH buffer such as approximately pH 2-3, or a high concentration of a chaotrope such as urea or thiocyanate ion), and PRO polypeptide is collected.

### EXAMPLE 9: Drug Screening

This invention is particularly useful for screening compounds by using PRO polypeptides or binding fragment thereof in any of a variety of drug screening techniques. The PRO polypeptide or fragment employed in such a test may either be free in solution, affixed to a solid support, borne on a cell surface, or located intracellularly. One method of drug screening utilizes eukaryotic or prokaryotic host cells which are stably transformed with recombinant nucleic acids expressing the PRO polypeptide or fragment. Drugs are screened against such transformed cells in competitive binding assays. Such cells, either in viable or fixed form, can be used for standard binding assays. One may measure, for example, the formation of complexes between PRO polypeptide or a fragment and the agent being tested. Alternatively, one can examine the diminution in complex formation between the PRO polypeptide and its target cell or target receptors caused by the agent being tested.

Thus, the present invention provides methods of screening for drugs or any other agents which can affect a PRO polypeptide-associated disease or disorder. These methods comprise contacting such an agent with an PRO polypeptide or fragment thereof and assaying (I) for the presence of a complex between the agent and the PRO polypeptide or fragment, or (ii) for the presence of a complex between the PRO polypeptide or fragment and the cell, by methods well known in the art. In such competitive binding assays, the PRO polypeptide or fragment is typically labeled. After suitable incubation, free PRO polypeptide or fragment is separated from that present in bound form, and the amount of free or uncomplexed label is a measure of the ability of the particular agent to bind to PRO polypeptide or to interfere with the PRO polypeptide/cell complex.

Another technique for drug screening provides high throughput screening for compounds having suitable binding affinity to a polypeptide and is described in detail in WO 84/03564, published on September 13, 1984. Briefly stated, large numbers of different small peptide test compounds are synthesized on a solid substrate, such as plastic pins or some other surface. As applied to a PRO polypeptide, the peptide test compounds are reacted with PRO polypeptide and washed. Bound PRO polypeptide is detected by methods well known in the art. Purified PRO polypeptide can also be coated directly onto plates for use in the aforementioned drug screening techniques. In addition, non-neutralizing antibodies can be used to capture the peptide and immobilize it on the solid support.

This invention also contemplates the use of competitive drug screening assays in which neutralizing antibodies capable of binding PRO polypeptide specifically compete with a test compound for binding to PRO polypeptide or fragments thereof. In this manner, the antibodies can be used to detect the presence of any peptide which shares one or more antigenic determinants with PRO polypeptide.

### EXAMPLE 10: Rational Drug Design

The goal of rational drug design is to produce structural analogs of biologically active polypeptide of interest (*i*.*e.,* a PRO polypeptide) or of small molecules with which they interact, *e*.*g*., agonists, antagonists, or inhibitors. Any of these examples can be used to fashion drugs which are more active or stable forms of the PRO polypeptide or which enhance or interfere with the function of the PRO polypeptide *in vivo* (*c*.*f*., Hodgson, Bio/Technology, 9: 19-21 (1991)).

In one approach, the three-dimensional structure of the PRO polypeptide, or of a PRO polypeptide-inhibitor complex, is determined by x-ray crystallography, by computer modeling or, most typically, by a combination of the two approaches. Both the shape and charges of the PRO polypeptide must be ascertained to elucidate the structure and to determine active site(s) of the molecule. Less often, useful information regarding the structure of the PRO polypeptide may be gained by modeling based on the structure of homologous proteins. In both cases, relevant structural information is used to design analogous PRO polypeptide-like molecules or to identify efficient inhibitors. Useful examples of rational drug design may include molecules which have improved activity or stability as shown by Braxton and Wells, Biochemistry. 31:7796-7801 (1992) or which act as inhibitors, agonists, or antagonists of native peptides as shown by Athauda et al., J. Biochem., 113:742-746 (1993).

It is also possible to isolate a target-specific antibody, selected by functional assay, as described above, and then to solve its crystal structure. This approach, in principle, yields a pharmacore upon which subsequent drug design can be based. It is possible to bypass protein crystallography altogether by generating anti-idiotypic antibodies (anti-ids) to a functional, pharmacologically active antibody. As a mirror image of a mirror image, the binding site of the anti-ids would be expected to be an analog of the original receptor. The anti-id could then be used to identify and isolate peptides from banks of chemically or biologically produced peptides. The isolated peptides would then act as the pharmacore.

By virtue of the present invention, sufficient amounts of the PRO polypeptide may be made available to perform such analytical studies as X-ray crystallography. In addition, knowledge of the PRO polypeptide amino acid sequence provided herein will provide guidance to those employing computer modeling techniques in place of or in addition to x-ray crystallography.

The foregoing written specification is considered to be sufficient to enable one skilled in the art to practice the invention. The present invention is not to be limited in scope by the construct deposited, since the deposited embodiment is intended as a single illustration of certain aspects of the invention and any constructs that are functionally equivalent are within the scope of this invention. The deposit of material herein does not constitute an admission that the written description herein contained is inadequate to enable the practice of any aspect of the invention, including the best mode thereof, nor is it to be construed as limiting the scope of the claims to the specific illustrations that it represents. Indeed, various modifications of the invention in addition to those shown and described herein will become apparent to those skilled in the art from the foregoing description and fall within the scope of the appended claims.

### Additional statements of invention

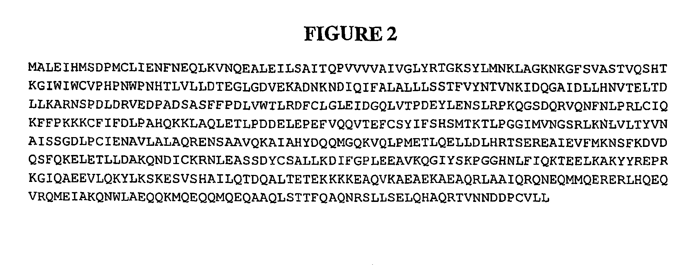

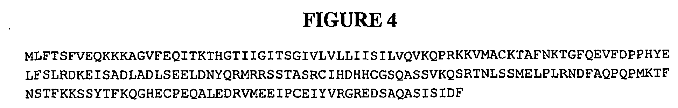

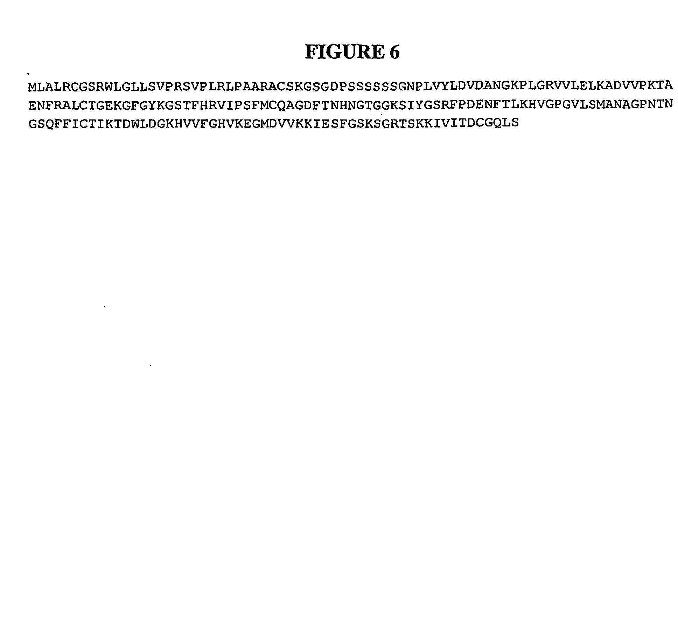

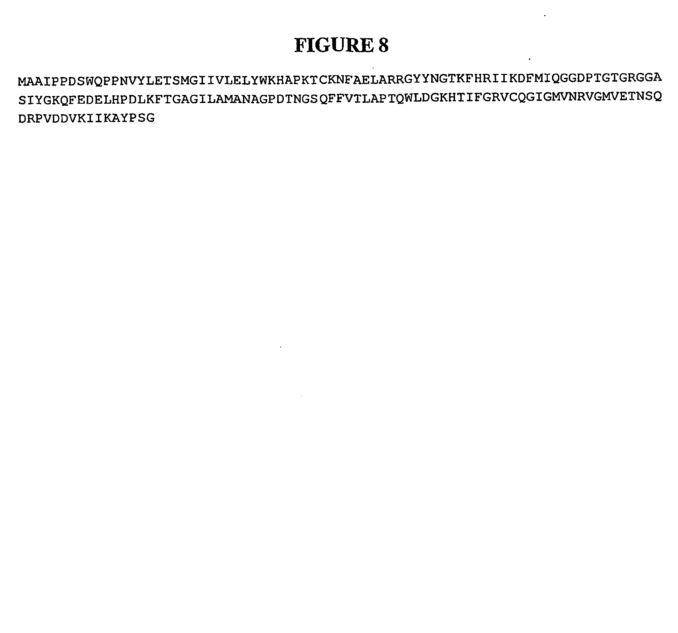

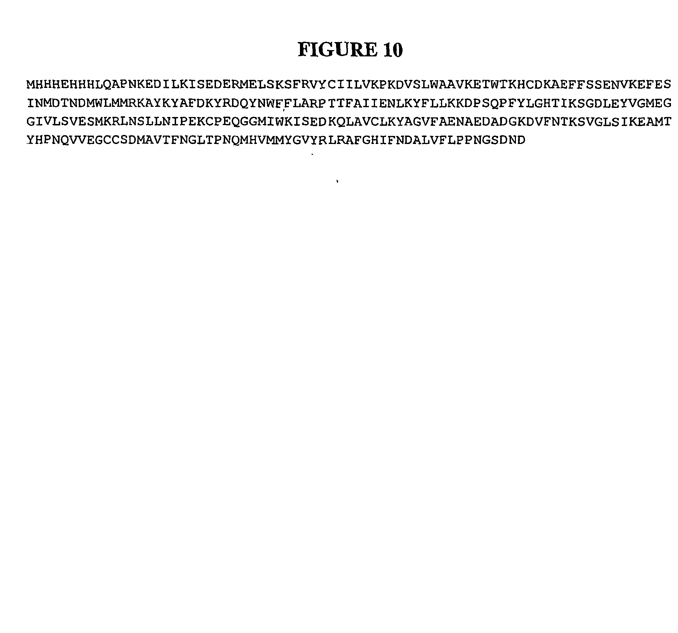

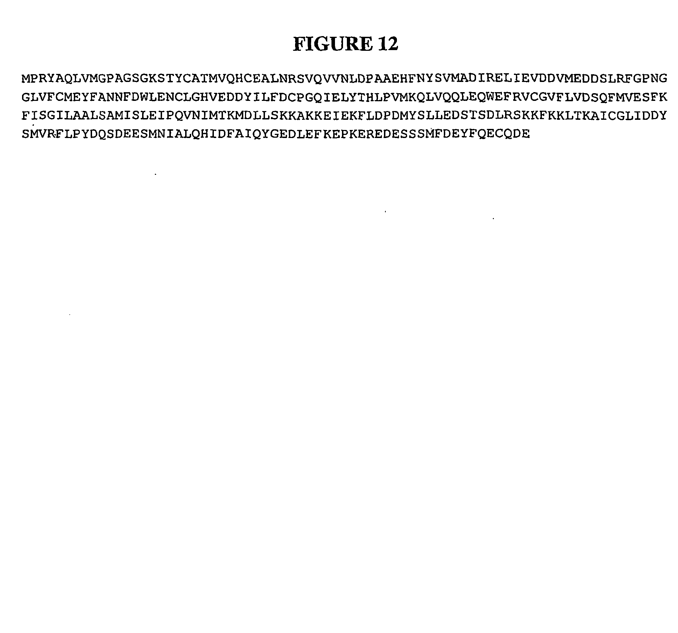

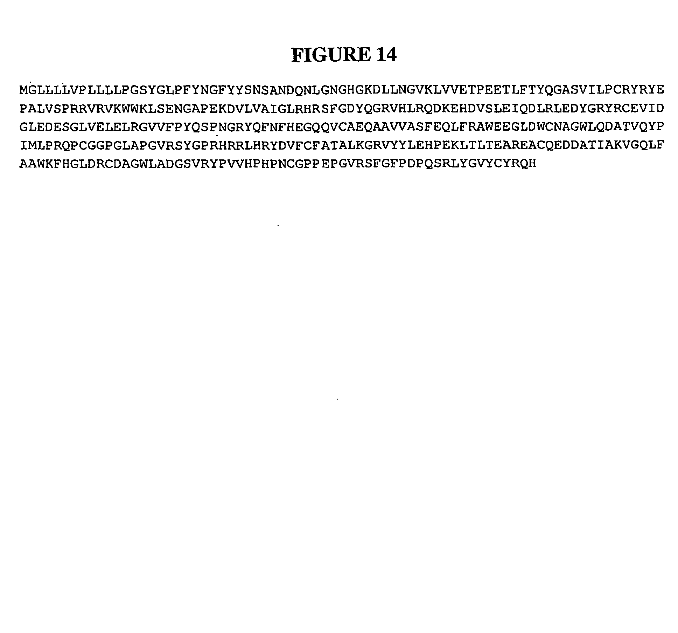

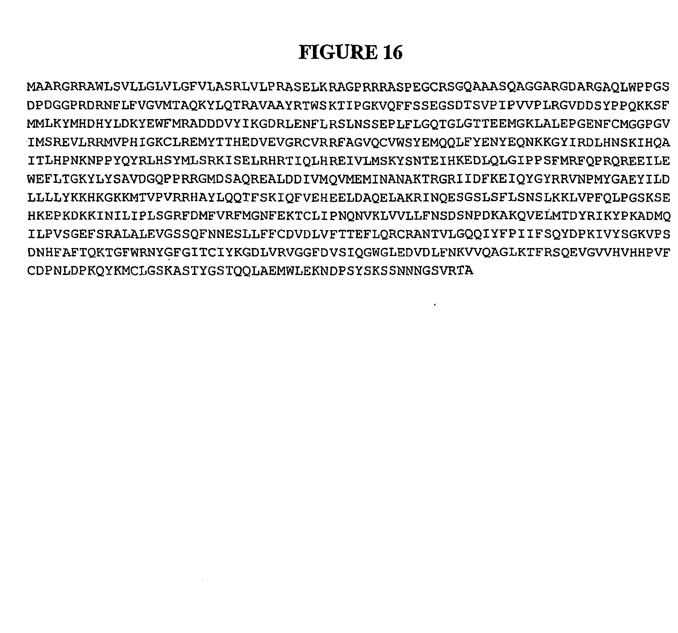

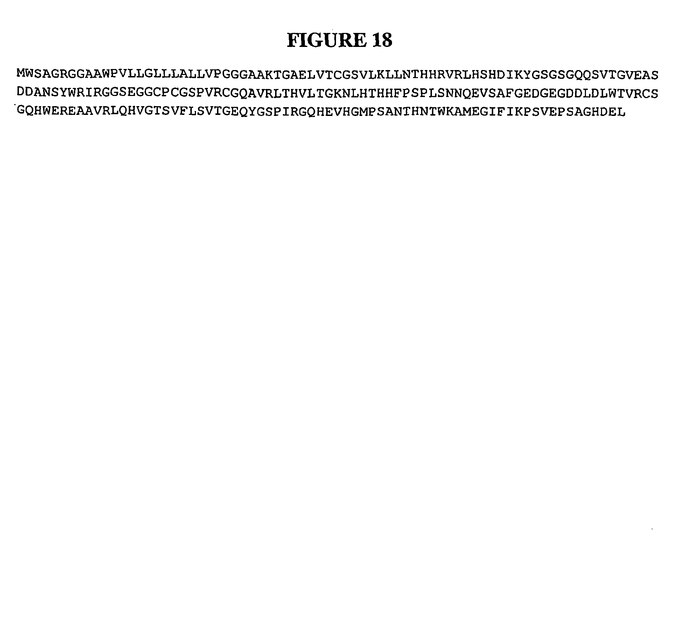

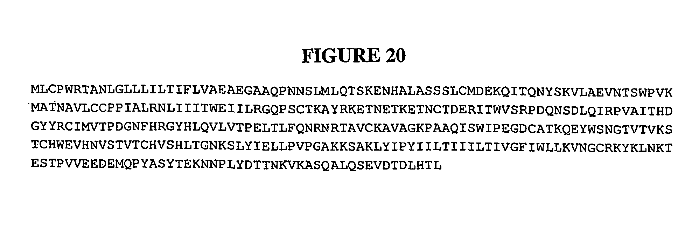

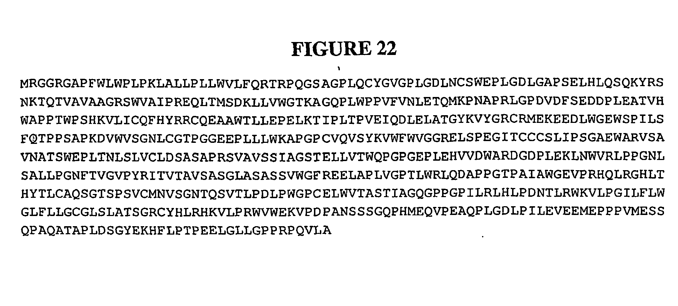

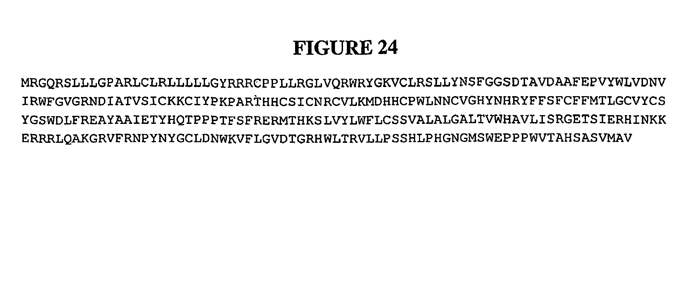

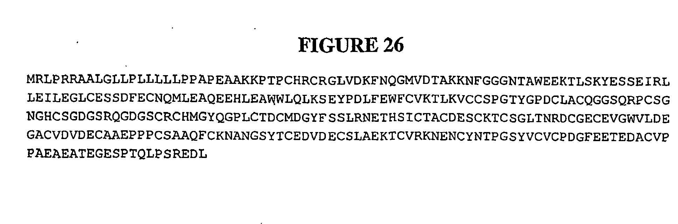

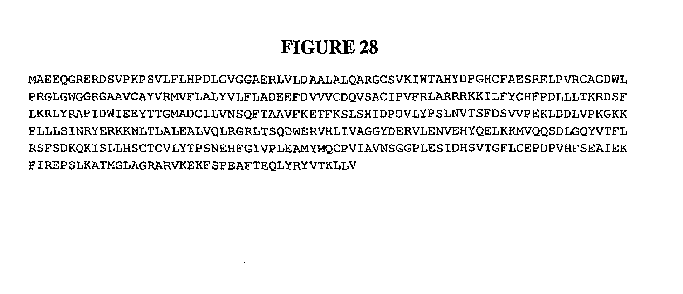

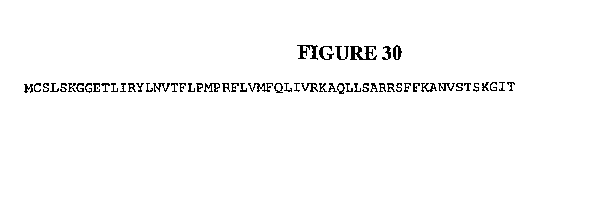

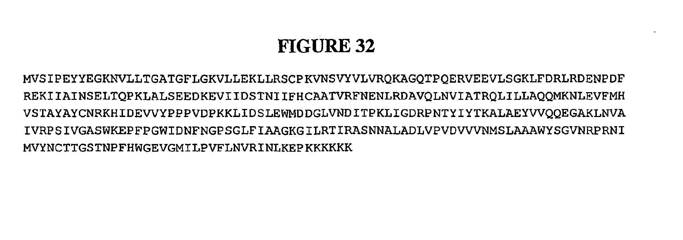

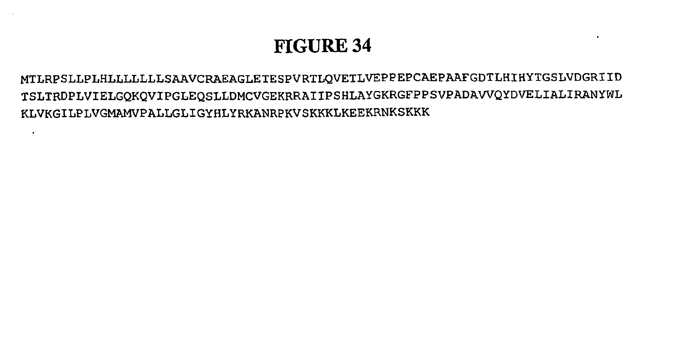

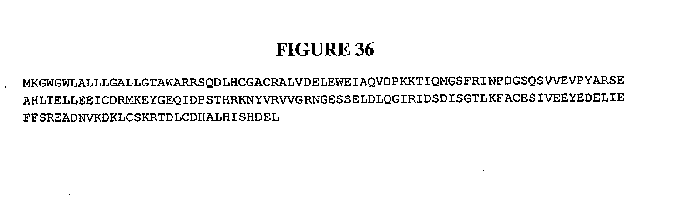

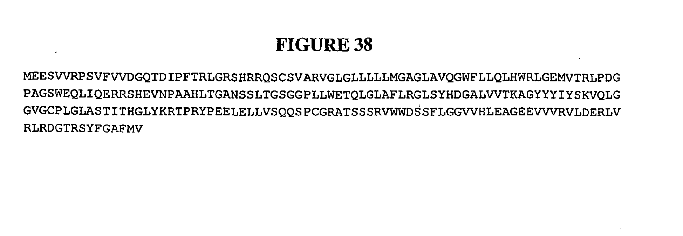

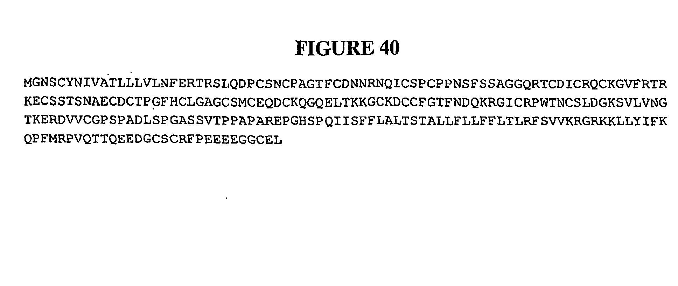

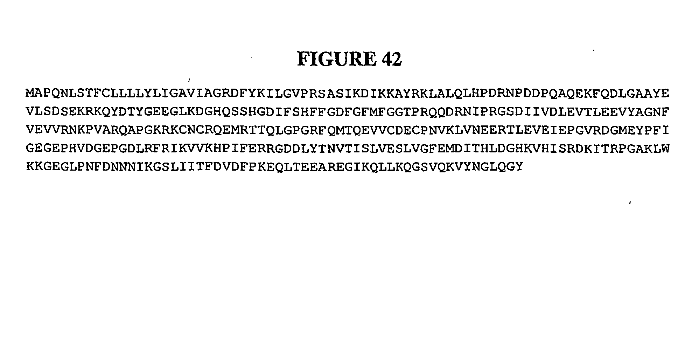

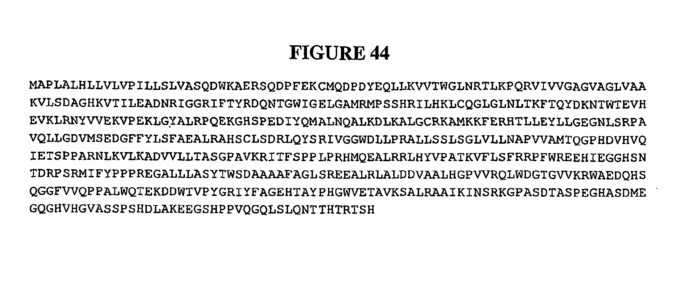

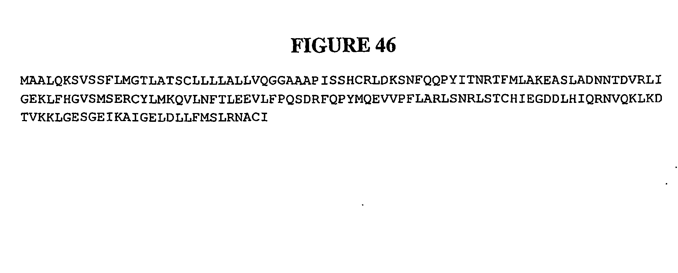

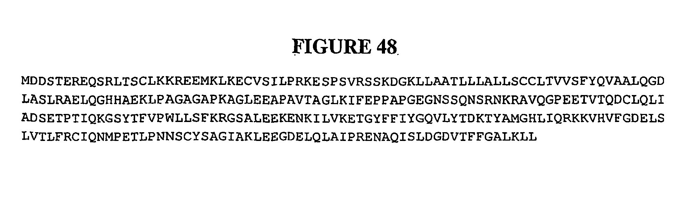

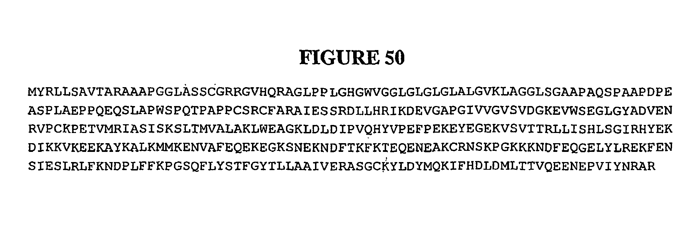

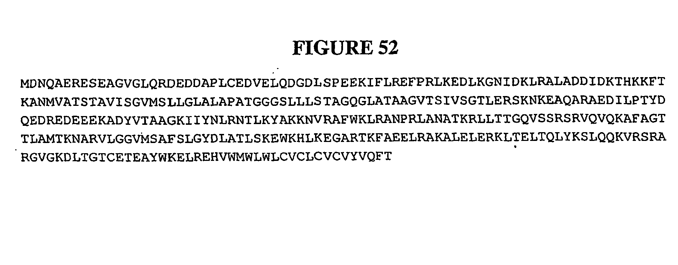

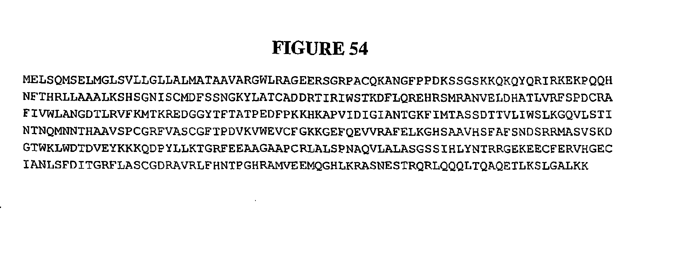

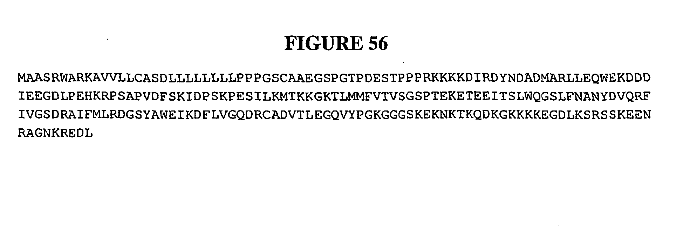

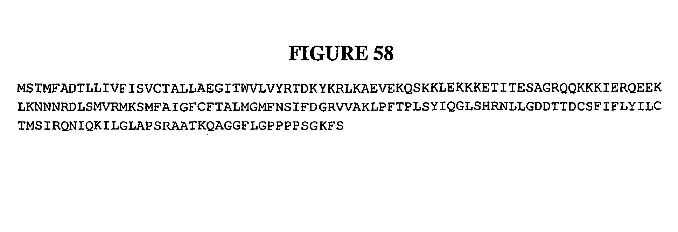

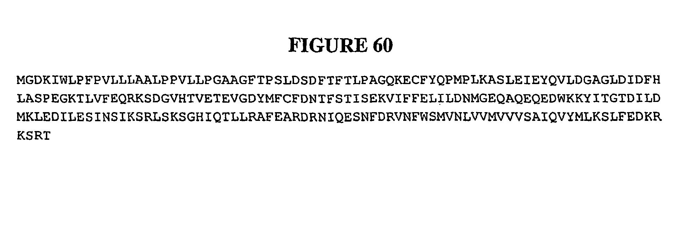

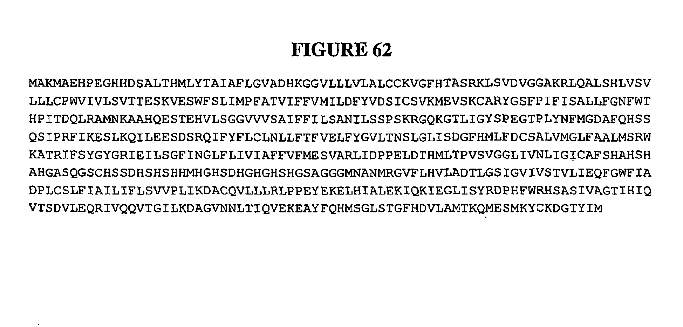

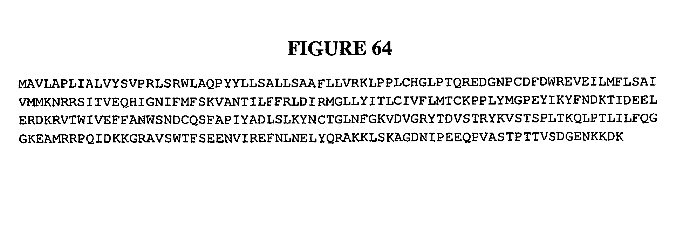

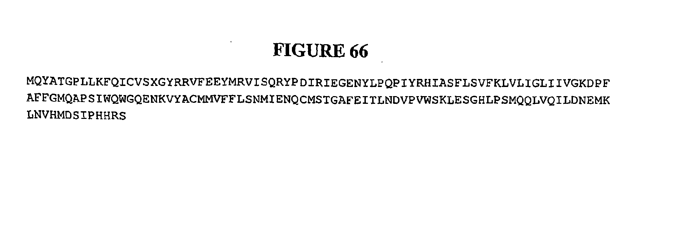

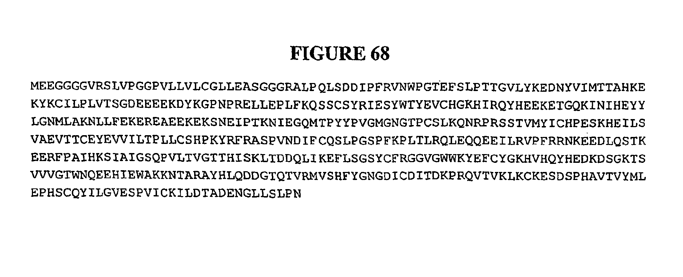

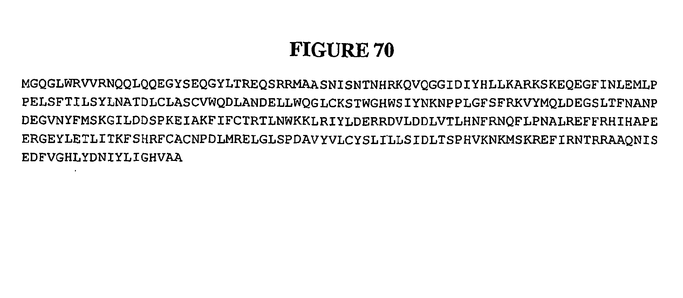

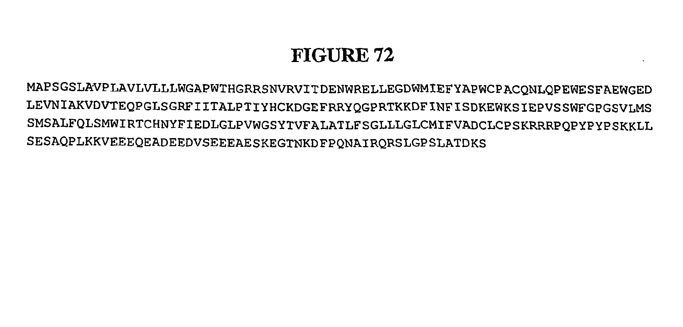

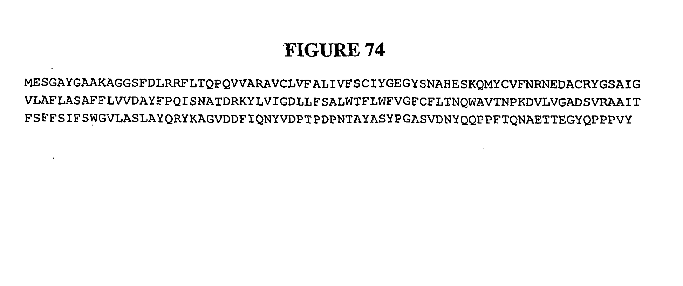

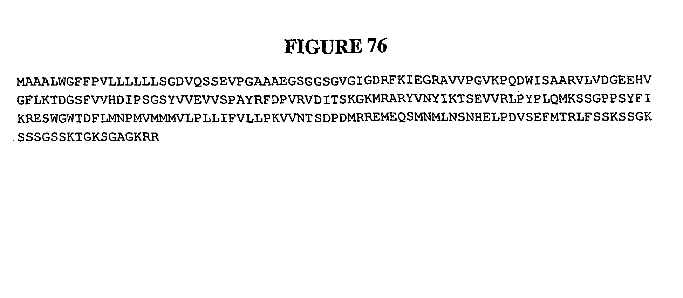

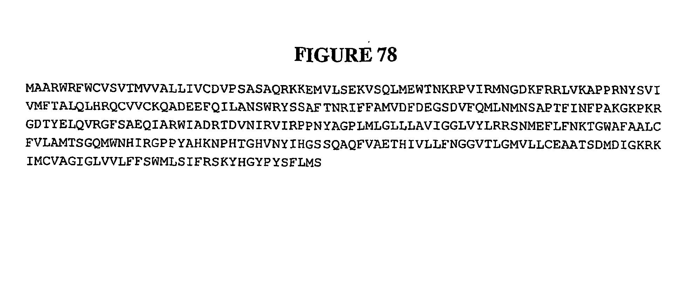

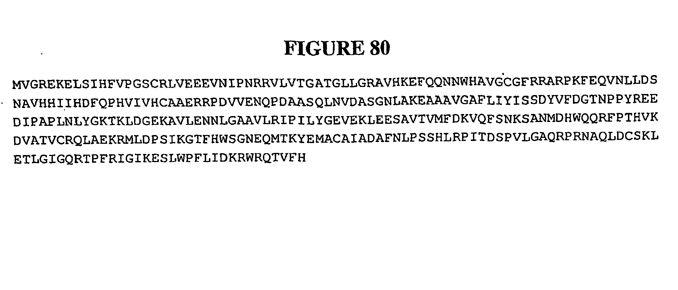

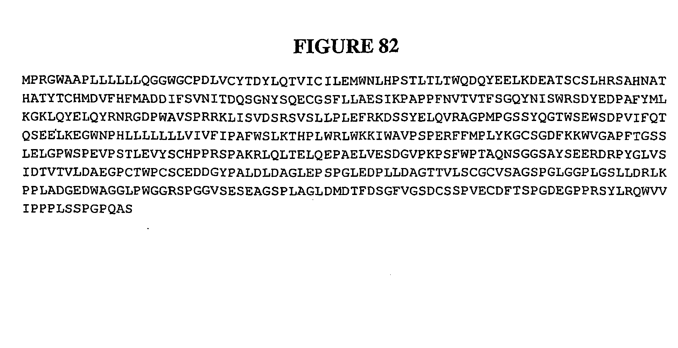

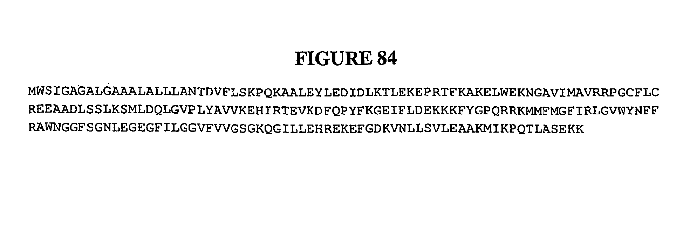

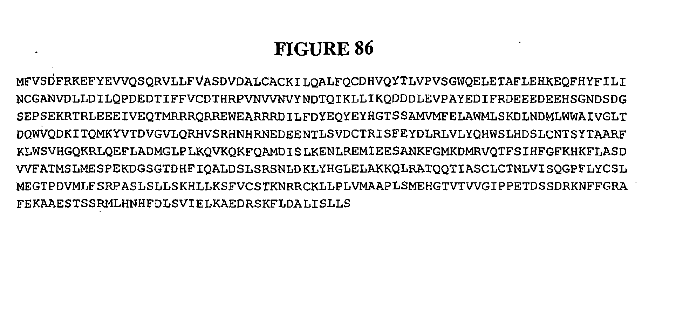

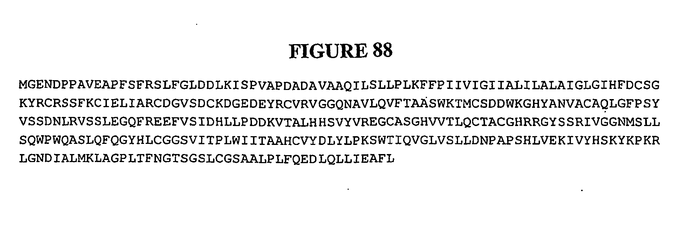

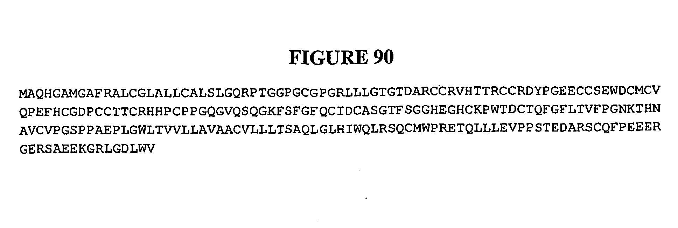

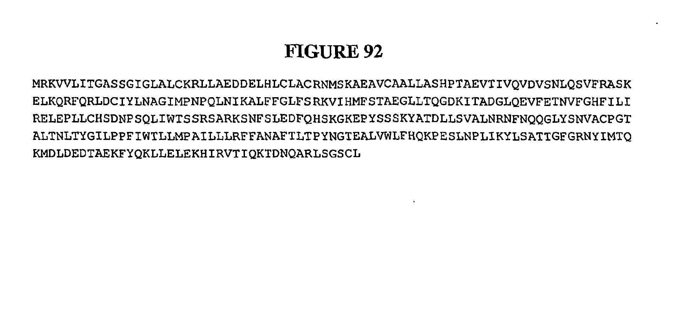

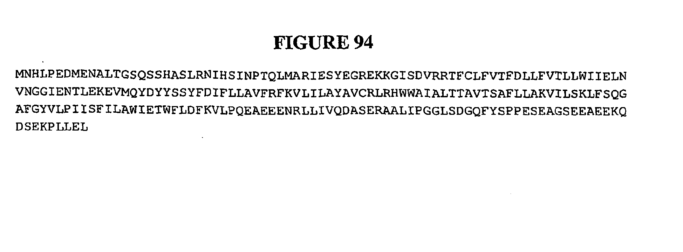

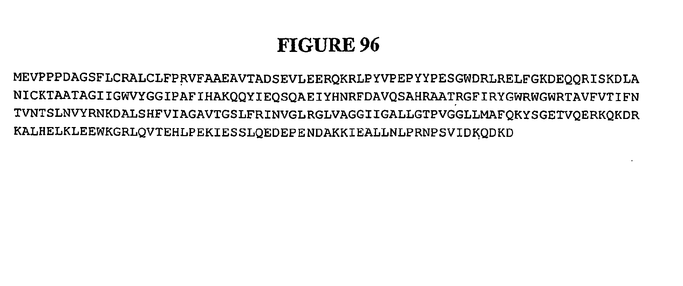

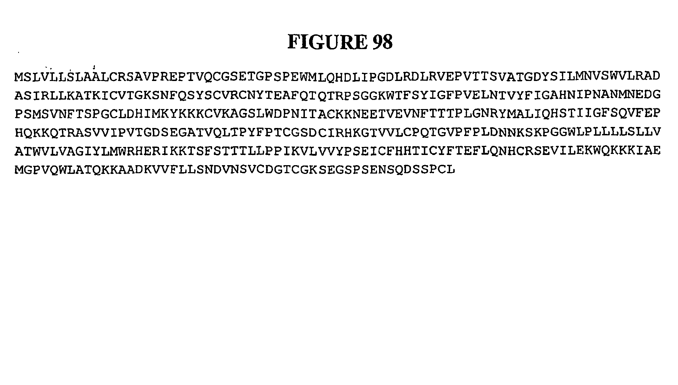

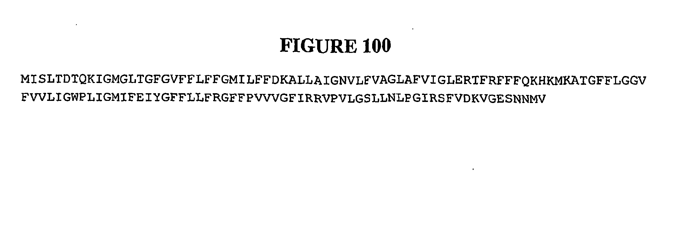

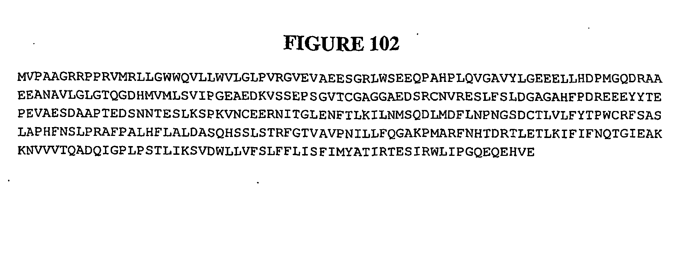

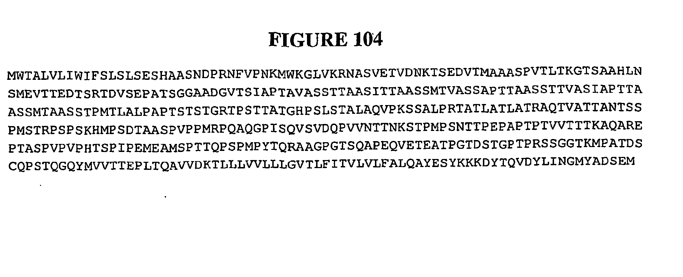

1. Isolated nucleic acid having at least 80% nucleic acid sequence identity to:
   (a) a nucleotide sequence encoding the polypeptide shown in Figure 2 (SEQ ID NO:2), Figure 4 (SEQ ID NO:4), Figure 6 (SEQ ID NO:6), Figure 8 (SEQ ID NO:8), Figure 10 (SEQ ID NO:10), Figure 12 (SEQ ID NO:12), Figure 14 (SEQ ID NO:14), Figure 16 (SEQ ID NO:16), Figure 18 (SEQ ID NO:18), Figure 20 (SEQ ID NO:20), Figure 22 (SEQ ID NO:22), Figure 24 (SEQ ID NO:24), Figure 26 (SEQ ID NO:26), Figure 28 (SEQ ID NO:28), Figure 30 (SEQ ID NO:30), Figure 32 (SEQ ID NO:32), Figure 34 (SEQ ID NO:34), Figure 36 (SEQ ID NO:36), Figure 38 (SEQ ID NO:38), Figure 40 (SEQ ID NO:40), Figure 42 (SEQ ID NO:42), Figure 44 (SEQ ID NO:44), Figure 46 (SEQ ID NO:46), Figure 48 (SEQ ID NO:48), Figure 50 (SEQ ID NO:50), Figure 52 (SEQ ID NO:52), Figure 54 (SEQ ID NO:54), Figure 56 (SEQ ID NO:56), Figure 58 (SEQ ID NO:58), Figure 60 (SEQ ID NO:60), Figure 62 (SEQ ID NO:62), Figure 64 (SEQ ID NO:64), Figure 66 (SEQ ID NO:66), Figure 68 (SEQ ID NO:68), Figure 70 (SEQ ID NO:70), Figure 72 (SEQ ID NO:72), Figure 74 (SEQ ID NO:74), Figure 76 (SEQ ID NO:76), Figure 78 (SEQ ID NO:78), Figure 80 (SEQ ID NO:80), Figure 82 (SEQ ID NO:82), Figure 84 (SEQ ID NO:84), Figure 86 (SEQ ID NO:86), Figure 88 (SEQ ID NO:88), Figure 90 (SEQ ID NO:90), Figure 92 (SEQ ID NO:92), Figure 94 (SEQ ID NO:94), Figure 96 (SEQ ID NO:96), Figure 98 (SEQ ID NO:98), Figure 100 (SEQ ID NO:100), Figure 102 (SEQ ID NO:102) or Figure 104 (SEQ ID NO:104).
2. Isolated nucleic acid having at least 80% nucleic acid sequence identity to a nucleotide sequence selected from the group consisting of the nucleotide sequence shown in Figure 1 (SEQ ID NO:1), Figure 3 (SEQ ID NO:3), Figure 5 (SEQ ID NO:5), Figure 7 (SEQ ID NO:7), Figure 9 (SEQ ID NO:9), Figure 11 (SEQ ID NO:11), Figure 13 (SEQ ID NO:13), Figure 15A-B (SEQ ID NO:15), Figure 17 (SEQ ID NO:17), Figure 19 (SEQ ID NO:19), Figure 21 (SEQ ID NO:21), Figure 23 (SEQ ID NO:23), Figure 25 (SEQ ID NO:25), Figure 27 (SEQ ID NO:27), Figure 29 (SEQ ID NO:29), Figure 31 (SEQ ID NO:31), Figure 33 (SEQ ID NO:33), Figure 35 (SEQ ID NO:35), Figure 37 (SEQ ID NO:37), Figure 39 (SEQ ID NO:39), Figure 41 (SEQ ID NO:41), Figure 43 (SEQ ID NO:43), Figure 45 (SEQ ID NO:45), Figure 47 (SEQ ID NO:47), Figure 49 (SEQ ID NO:49), Figure 51 (SEQ ID NO:51), Figure 53 (SEQ ID NO:53), Figure 55 (SEQ ID NO:55), Figure 57 (SEQ ID NO:57), Figure 59 (SEQ ID NO:59), Figure 61 (SEQ ID NO:61), Figure 63 (SEQ ID NO:63), Figure 65 (SEQ ID NO:65), Figure 67 (SEQ ID NO:67), Figure 69 (SEQ ID NO:69), Figure 71 (SEQ ID NO:71), Figure 73 (SEQ ID NO:73), Figure 75 (SEQ ID NO:75), Figure 77 (SEQ ID NO:77), Figure 79 (SEQ ID NO:79), Figure 81 (SEQ ID NO:81), Figure 83 (SEQ ID NO:83), Figure 85 (SEQ ID NO:85), Figure 87 (SEQ ID NO:87), Figure 89 (SEQ ID NO:89), Figure 91 (SEQ ID NO:91), Figure 93 (SEQ ID NO:93), Figure 95 (SEQ ID NO:95), Figure 97 (SEQ ID NO:97), Figure 99 (SEQ ID NO:99), Figure 101 (SEQ ID NO:101) and Figure 103 (SEQ ID NO:103).
3. Isolated nucleic acid having at least 80% nucleic acid sequence identity to a nucleotide sequence selected from the group consisting of the full-length coding sequence of the nucleotide sequence shown in Figure 1 (SEQ ID NO:1), Figure 3 (SEQ ID NO:3), Figure 5 (SEQ ID NO:5), Figure 7 (SEQ ID NO:7), Figure 9 (SEQ ID NO:9), Figure 11 (SEQ ID NO:11), Figure 13 (SEQ ID NO:13), Figure 15A-B (SEQ ID NO:15), Figure 17 (SEQ ID NO:17), Figure 19 (SEQ ID NO:19), Figure 21 (SEQ ID NO:21), Figure 23 (SEQ ID NO:23), Figure 25 (SEQ ID NO:25), Figure 27 (SEQ ID NO:27), Figure 29 (SEQ ID NO:29), Figure 31 (SEQ ID NO:31), Figure 33 (SEQ ID NO:33), Figure 35 (SEQ ID NO:35), Figure 37 (SEQ ID NO:37), Figure 39 (SEQ ID NO:39), Figure 41 (SEQ ID NO:41), Figure 43 (SEQ ID NO:43), Figure 45 (SEQ ID NO:45), Figure 47 (SEQ ID NO:47), Figure 49 (SEQ ID NO:49), Figure 51 (SEQ ID NO:51), Figure 53 (SEQ ID NO:53), Figure 55 (SEQ ID NO:55), Figure 57 (SEQ ID NO:57), Figure 59 (SEQ ID NO:59), Figure 61 (SEQ ID NO:61), Figure 63 (SEQ ID NO:63), Figure 65 (SEQ ID NO:65), Figure 67 (SEQ ID NO:67), Figure 69 (SEQ ID NO:69), Figure 71 (SEQ ID NO:71), Figure 73 (SEQ ID NO:73), Figure 75 (SEQ ID NO;75), Figure 77 (SEQ ID NO:77), Figure 79 (SEQ ID NO:79), Figure 81 (SEQ ID NO:81), Figure 83 (SEQ ID NO:83), Figure 85 (SEQ ID NO:85), Figure 87 (SEQ ID NO:87), Figure 89 (SEQ ID NO:89), Figure 91 (SEQ ID NO:91), Figure 93 (SEQ ID NO:93), Figure 95 (SEQ ID NO:95), Figure 97 (SEQ ID NO:97), Figure 99 (SEQ ID NO:99), Figure 101 (SEQ ID NO:101), and Figure 103 (SEQ ID NO: 103).
4. A vector comprising the nucleic acid of 1.
5. The vector of 4 operably linked to control sequences recognized by a host cell transformed with the vector.
6. A host cell comprising the vector of 4.
7. The host cell of 6, wherein said cell is a CHO cell, an *E.coli* cell or a yeast cell.
8. A process for producing a PRO polypeptide comprising culturing the host cell of 6 under conditions suitable for expression of said PRO polypeptide and recovering said PRO polypeptide from the cell culture.
9. An isolated polypeptide having at least 80% amino acid sequence identity to:
   (a) an amino acid sequence of the polypeptide shown in Figure 2 (SEQ ID NO:2), Figure 4 (SEQ ID NO:4), Figure 6 (SEQ ID NO:6), Figure 8 (SEQ ID NO:8), Figure 10 (SEQ ID NO:10), Figure 12 (SEQ ID NO:12), Figure 14 (SEQ ID NO:14), Figure 16 (SEQ ID NO:16), Figure 18 (SEQ ID NO:18), Figure 20 (SEQ ID NO:20), Figure 22 (SEQ ID NO:22), Figure 24 (SEQ ID NO:24), Figure 26 (SEQ ID NO:26), Figure 28 (SEQ ID NO:28), Figure 30 (SEQ ID NO:30), Figure 32 (SEQ ID NO:32), Figure 34 (SEQ ID NO:34), Figure 36 (SEQ ID NO:36), Figure 38 (SEQ ID NO:38), Figure 40 (SEQ ID NO:40), Figure 42 (SEQ ID NO:42), Figure 44 (SEQ ID NO:44), Figure 46 (SEQ ID NO:46), Figure 48 (SEQ ID NO:48), Figure 50 (SEQ ID NO:50), Figure 52 (SEQ ID NO:52), Figure 54 (SEQ ID NO:54), Figure 56 (SEQ ID NO:56), Figure 58 (SEQ ID NO:58), Figure 60 (SEQ ID NO:60), Figure 62 (SEQ ID NO:62), Figure 64 (SEQ ID NO:64), Figure 66 (SEQ ID NO:66), Figure 68 (SEQ ID NO:68), Figure 70 (SEQ ID NO:70), Figure 72 (SEQ ID NO:72), Figure 74 (SEQ ID NO:74), Figure 76 (SEQ ID NO:76), Figure 78 (SEQ ID NO:78), Figure 80 (SEQ ID NO:80), Figure 82 (SEQ ID NO:82), Figure 84 (SEQ ID NO:84), Figure 86 (SEQ ID NO:86), Figure 88 (SEQ ID NO:88), Figure 90 (SEQ ID NO:90), Figure 92 (SEQ ID NO:92), Figure 94 (SEQ ID NO:94), Figure 96 (SEQ ID NO:96), Figure 98 (SEQ ID NO:98), Figure 100 (SEQ ID NO:100), Figure 102 (SEQ ID NO:102), or Figure 104 (SEQ ID NO:104).
10. A chimeric molecule comprising a polypeptide according to 9 fused to a heterologous amino acid sequence.
11. The chimeric molecule of 10, wherein said heterologous amino acid sequence is an epitope tag sequence or an Fc region of an immunoglobulin.
12. An antibody which specifically binds to a polypeptide according to 9.
13. The antibody of 12, wherein said antibody is a monoclonal antibody, a humanized antibody or a single-chain antibody.
14. A composition of matter comprising (a) a polypeptide of 9, (b) an agonist of said polypeptide, (c) an antagonist of said polypeptide, or (d) an antibody that binds to said polypeptide, in combination with a carrier.
15. The composition of matter of 14, wherein said carrier is a pharmaceutically acceptable carrier.
16. The composition of matter of 14 comprising a therapeutically effective amount of (a), (b), (c) or (d).
17. An article of manufacture, comprising:
   a container;
   a label on said container; and
   a composition of matter comprising (a) a polypeptide of 9, (b) an agonist of said polypeptide, (c) an antagonist of said polypeptide, or (d) an antibody that binds to said polypeptide, contained within said container, wherein label on said container indicates that said composition of matter can be used for treating an immune related disease.
18. A method of treating an immune related disorder in a mammal in need thereof comprising administering to said mammal a therapeutically effective amount of (a) a polypeptide of 9, (b) an agonist of said polypeptide, (c) an antagonist of said polypeptide, or (d) an antibody that binds to said polypeptide.
19. The method of 18, wherein the immune related disorder is systemic lupus erythematosis, rheumatoid arthritis, osteoarthritis, juvenile chronic arthritis, a spondyloarthropathy, systemic sclerosis, an idiopathic inflammatory myopathy, Sjögren's syndrome, systemic vasculitis, sarcoidosis, autoimmune hemolytic anemia, autoimmune thrombocytopenia, thyroiditis, diabetes mellitus, immune-mediated renal disease, a demyelinating disease of the central or peripheral nervous system, idiopathic demyelinating polyneuropathy, Guillain-Barré syndrome, a chronic inflammatory demyelinating polyneuropathy, a hepatobiliary disease, infectious or autoimmune chronic active hepatitis, primary biliary cirrhosis, granulomatous hepatitis, sclerosing cholangitis, inflammatory bowel disease, gluten-sensitive enteropathy, Whipple's disease, an autoimmune or immune-mediated skin disease, a bullous skin disease, erythema multiforme, contact dermatitis, psoriasis, an allergic disease, asthma, allergic rhinitis, atopic dermatitis, food hypersensitivity, urticaria, an immunologic disease of the lung, eosinophilic pneumonias, idiopathic pulmonary fibrosis, hypersensitivity pneumonitis, a transplantation associated disease, graft rejection or graft-versus-host-disease.
20. A method for determining the presence of a PRO polypeptide in a sample suspected of containing said polypeptide, said method comprising exposing said sample to an anti-PRO83478 anti-PRO69889, anti-PRO37073, anti-PRO4984, anti-PRO71039, anti-PRO71191, anti-PRO271, anti-PRO71042, anti-PRO71242, anti-PRO6015, anti-PRO34336, anti-PRO71043 anti-PRO71044, anti-PRO71045, anti-PRO71046, anti-PRO83479, anti-PRO535, anti-PRO4426, anti-PRO34447, anti-PRO2023, anti-PRO25349 anti-PRO1265, anti-PRO34298, anti-PRO738, anti-PRO71049, anti-PRO21341, anti-PRO1125, anti-PRO4369, anti-PRO177, anti-PRO83480, anti-PRO71052 anti-PRO37125, anti-PRO83481, anti-PRO71054, anti-PRO69462, ariti-PRO268, anti-PRO615, anti-PRO25402, anti-PRO71055, anti-PRO71056, anti-PRO34332, anti-PRO71057, anti-PRO71058, anti-PRO83482, anti-PRO34454, anti-PRO6243, anti-PRO1864, anti-PRO71060, anti-PRO71061, anti-PRO793, anti-PRO83483 or anti-PRO60929 antibody and determining binding of said antibody to a component of said sample.
21. A method of diagnosing an immune related disease in a mammal, said method comprising detecting the level of expression of a gene encoding PRO83478, PRO69889, PRO37073, PRO4984, PRO71039, PRO71191, PRO271, PRO71042, PRO71242, PRO6015, PRO34336, PRO71043, PRO71044, PRO71045, PRO71046, PRO83479, PRO535, PRO4426, PRO34447, PRO2023, PRO25349, PRO1265, PRO34298, PRO738, PRO71049, PRO21341, PRO1125, PRO4369, PRO177, PRO83480, PRO71052, PRO37125, PRO83481, PRO71054, PRO69462, PRO268, PRO615, PRO25402, PRO71055, PRO71056, PRO34332, PRO71057, PRO71058, PRO83482, PRO34454, PRO6243, PRO1864, PRO71060, PRO71061, PRO793, PRO83483, or PRO60929 polypeptide (a) in a test sample of tissue cells obtained from the mammal, and (b) in a control sample of known normal tissue cells of the same cell type, wherein a higher or lower level of expression of said gene in the test sample as compared to the control sample is indicative of the presence of an immune related disease in the mammal from which the test tissue cells were obtained.
22. A method of diagnosing an immune related disease in a mammal, said method comprising
   (a) contacting an anti-PRO83478 anti-PRO69889, anti-PRO37073, anti-PRO4984, anti-PRO71039, anti-PRO71191, anti-PRO271, anti-PRO71042, anti-PRO71242, anti-PRO6015, anti-PRO34336, anti-PRO71043, anti-PRO71044, anti-PRO71045, anti-PRO71046, anti-PRO83479, anti-PRO535, antiPRO4426, anti-PRO34447, anti-PRO2023, anti-PRO25349, anti-PRO1265, anti-PRO34298, anti-PRO738, anti-PRO71049 anti-PRO21341, anti-PRO1125, anti-PRO4369, anti-PRO177, anti-PRO83480, anti-PRO71052, anti-PRO37125, anti-PRO83481, anti-PRO71054, anti-PRO69462, anti-PRO268, anti-PRO615 anti-PRO25402, anti-PRO71055, anti-PRO71056, anti-PRO34332, anti-PRO71057, anti-PRO71058, anti-PRO83482, anti-PRO34454, anti-PRO6243, anti-PRO1864, anti-PRO71060, anti-PRO71061, anti-PRO793, anti-PRO3483 or anti-PRO60929 antibody with a test sample of tissue cells obtained from said mammal and (b) detecting the formation of a complex between the antibody and the polypeptide in the test sample,
   wherein formation of said complex is indicative of the presence of an immune related disease in the mammal from which the test tissue cells were obtained.
23. A method of identifying a compound that inhibits the activity of PRO83478, PRO69889, PRO37073, PRO4984, PRO71039, PRO71191, PRO271, PRO71042, PRO71242, PRO6015, PRO34336, PRO71043, PRO71044, PRO71045, PRO71046, PRO83479, PRO535, PRO4426, PRO34447, PRO2023, PRO25349, PRO1265 PRO4298, PRO38, PRO71049, PRO21341, PRO1125, PRO4369, PRO177, PRO83480, PRO71052, PRO37125, PRO83481, PRO71054, PRO69462, PRO268, PRO615, PRO25402, PRO71055, PRO71056, PRO34332, PRO71057, PRO71058, PRO83482, PRO34454, PRO6243, PRO1864, PRO71060, PRO71061, PRO793, PRO83483, or, PRO60929 polypeptide, said method comprising contacting cells which normally respond to said polypeptide with (a) said polypeptide and (b) a candidate compound, and determining the lack responsiveness by said cell to (a).
24. A method of identifying a compound that inhibits the expression of a gene encoding a PRO83478 PRO69889, PRO37073, PRO4984, PRO71039, PRO71191, PRO271, PRO71042 PRO71242, PRO6015, PRO34336, PRO71043, PRO71044, PRO71045, PRO71046, PRO83479, PRO535, PRO4426, PRO34447, PRO2023, PRO25349, PRO1265, PRO34298, PRO738, PRO71049, PRO21341, PRO1125, PRO4369, PRO177, PRO83480, PRO71052, PRO37125, PRO83481, PRO71054, PRO69462, PRO268, PRO615, PRO25402, PRO71055, PRO71056, PRO34332, PRO71057, PRO71058, PRO83482, PRO34454, PRO6243, PRO1864, PRO71060, PRO71061, PRO793, PRO83483, or PRO60929 polypeptide, said method comprising contacting cells which normally express said polypeptide with a candidate compound, and determining the lack of expression said gene.
25. The method of 24, wherein said candidate compound is an antisense nucleic acid.
26. A method of identifying a compound that mimics the activity of a PRO83478, PRO69889, PRO37073, PRO4984, PRO71039, PRO71191 PRO271, PRO71042, PRO71242, PRO6015, PRO34336, PRO71043, PRO71044, PRO71045, PRO71046, PRO83479, PRO535, PRO4426, PRO34447, PRO2023, PRO25349, PRO1265, PRO34298, PRO738, PRO71049, PRO21341, PRO1125, PRO4369, PRO177, PRO83480, PRO71052, PRO37125, PRO83481, PRO71054, PRO69462, PRO268, PRO615, PRO25402, PRO71055, PRO71056, PRO34332, PRO71057, PRO71058, PRO83482, PRO34454, PRO6243, PRO1864, PRO71060, PRO71061, PRO793, PRO83483, or PRO60929 polypeptide, said method comprising contacting cells which normally respond to said polypeptide with a candidate compound, and determining the responsiveness by said cell to said candidate compound.
27. A method of stimulating the immune response in a mammal, said method comprising administering to said mammal an effective amount of a PRO83478, PRO69889, PRO37073, PRO4984, PRO71039, PRO71191, PRO271, PRO71042, PRO71242, PRO6015, PRO34336, PRO71043, PRO71044, PRO71045, PRO71046, PRO83479, PRO535, PRO4426, PRO34447, PRO2023, PRO25349, PRO1265, PRO34298, PRO738, PRO71049, PRO21341, PRO1125, PRO4369, PRO177, PRO83480, PRO71052, PRO37125, PRO83481, PRO71054, PRO69462, PRO268, PRO615, PRO25402, PRO71055, PRO71056, PRO34332, PRO71057, PRO71058, PRO83482, PRO34454, PRO6243, PRO1864, PRO71060, PRO71061, PRO793, PRO83483, or PRO60929 polypeptide antagonist, wherein said immune response is stimulated.
28. A method of diagnosing an inflammatory immune response in a mammal, said method comprising detecting the level of expression of a gene encoding PRO83478, PRO69889, PRO37073, PRO4984, PRO71039 PRO71191, PRO271, PRO71042, PRO71242, PRO6015, PRO34336, PRO71043, PRO71044, PRO71045, PRO71046, PRO83479, PRO535, PRO4426, PRO34447, PRO2023, PRO25349, PRO1265 PRO34298, PRO738, PRO71O49, PRO21341, PRO1125, PRO4369, PRO177, PRO83480, PRO71052, PRO37125, PRO83481, PRO71054, PRO69462, PRO268, PRO615, PRO25402, PRO71055, PRO71056, PRO34332, PRO71057, PRO71058, PRO83482, PRO34454, PRO6243, PRO1864, PRO71060, PRO71061, PRO793, PRO83483, or PRO60929 polypeptide (a) in a test sample of tissue cells obtained from the mammal, and (b) in a control sample of known normal tissue cells of the same cell type, wherein a higher or lower level of expression of said gene in the test sample as compared to the control sample is indicative of the presence of an inflammatory immune response in the mammal from which the test tissue cells were obtained.

## Claims

1. Isolated nucleic acid having at least 80% nucleic acid sequence identity to:
a nucleotide sequence encoding the polypeptide shown in Figure 44 (SEQ ID NO:44).

2. Isolated nucleic acid having at least 80% nucleic acid sequence identity to a nucleotide sequence selected from the group consisting of the nucleotide sequence shown in Figure 43 (SEQ ID NO:43),.

3. Isolated nucleic acid having at least 80% nucleic acid sequence identity to a nucleotide sequence selected from the group consisting of the full-length coding sequence of the nucleotide sequence shown in Figure 43 (SEQ ID NO:43),.

4. A vector comprising the nucleic acid of Claim 1.

5. The vector of Claim 4 operably linked to control sequences recognized by a host cell transformed with the vector.

6. A host cell comprising the vector of Claim 4.

7. The host cell of Claim 6, wherein said cell is a CHO cell, an *E.coli* cell or a yeast cell.

8. A process for producing a polypeptide comprising culturing the host cell of Claim 6 under conditions suitable for expression of said polypeptide and recovering said polypeptide from the cell culture.

9. An isolated polypeptide having at least 80% amino acid sequence identity to:
an amino acid sequence of the polypeptide shown in Figure 44 (SEQ ID NO:44).

10. A chimeric molecule comprising a polypeptide according to Claim 9 fused to a heterologous amino acid sequence.

11. The chimeric molecule of Claim 10, wherein said heterologous amino acid sequence is an epitope tag sequence or an Fc region of an immunoglobulin.

12. An antibody which specifically binds to a polypeptide according to Claim 9.

13. The antibody of Claim 12, wherein said antibody is a monoclonal antibody, a humanized antibody or a single-chain antibody.

14. A composition of matter comprising (a) a polypeptide of Claim 9, (b) an agonist of said polypeptide, (c) an antagonist of said polypeptide, or (d) an antibody that binds to said polypeptide, in combination with a carrier.

15. The composition of matter of Claim 14, wherein said carrier is a pharmaceutically acceptable carrier.

16. The composition of matter of Claim 14 comprising a therapeutically effective amount of (a), (b), (c) or (d).

17. An article of manufacture, comprising:
a container;
a label on said container; and
a composition of matter comprising (a) a polypeptide of Claim 9, (b) an agonist of said polypeptide, (c) an antagonist of said polypeptide, or (d) an antibody that binds to said polypeptide, contained within said container, wherein label on said container indicates that said composition of matter can be used for treating an immune related disease.

18. A composition comprising (a) a polypeptide of Claim 9, (b) an agonist of said polypeptide, (c) an antagonist of said polypeptide, or (d) an antibody that binds to said polypeptide, for use in a method of treating an immune related disorder in a mammal in need thereof.

19. Use of (a) a polypeptide of Claim 9, (b) an agonist of said polypeptide, (c) an antagonist of said polypeptide, or (d) an antibody that binds to said polypeptide, in the manufacture of a medicament for treating an immune related disorder in a mammal in need thereof.

20. The composition of Claim 18 or use according to Claim 19, wherein the immune related disorder is systemic lupus erythematosis, rheumatoid arthritis, osteoarthritis, juvenile chronic arthritis, a spondyloarthropathy, systemic sclerosis, an idiopathic inflammatory myopathy, Sjögren's syndrome, systemic vasculitis, sarcoidosis, autoimmune hemolytic anemia, autoimmune thrombocytopenia, thyroiditis, diabetes mellitus, immune-mediated renal disease, a demyelinating disease of the central or peripheral nervous system, idiopathic demyelinating polyneuropathy, Guillain-Barré syndrome, a chronic inflammatory demyelinating polyneuropathy, a hepatobiliary disease, infectious or autoimmune chronic active hepatitis, primary biliary cirrhosis, granulomatous hepatitis, sclerosing cholangitis, inflammatory bowel disease, gluten-sensitive enteropathy, Whipple's disease, an autoimmune or immune-mediated skin disease, a bullous skin disease, erythema multiforme, contact dermatitis, psoriasis, an allergic disease, asthma, allergic rhinitis, atopic dermatitis, food hypersensitivity, urticaria, an immunologic disease of the lung, eosinophilic pneumonias, idiopathic pulmonary fibrosis, hypersensitivity pneumonitis, a transplantation associated disease, graft rejection or graft-versus-host-disease.

21. A method for determining the presence of a polypeptide in a sample suspected of containing said polypeptide, said method comprising exposing said sample to an -antibody according to claim 12 and determining binding of said antibody to a component of said sample.

22. A method of diagnosing an immune related disease in a mammal, said method comprising detecting the level of expression of a gene encoding the polypeptide of claim 9 (a) in a test sample of tissue cells obtained from the mammal, and (b) in a control sample of known normal tissue cells of the same cell type, wherein a higher or lower level of expression of said gene in the test sample as compared to the control sample is indicative of the presence of an immune related disease in the mammal from which the test tissue cells were obtained.

23. A method of diagnosing an immune related disease in a mammal, said method comprising (a) contacting an antibody according to claim 12 with a test sample of tissue cells obtained from said mammal and (b) detecting the formation of a complex between the antibody and the polypeptide in the test sample, wherein formation of said complex is indicative of the presence of an immune related disease in the mammal from which the test tissue cells were obtained.

24. A method of identifying a compound that inhibits the activity of a polypeptide according to claim 9, said method comprising contacting cells which normally respond to said polypeptide with (a) said polypeptide and (b) a candidate compound, and determining the lack responsiveness by said cell to (a).

25. A method of identifying a compound that inhibits the expression of a gene encoding a polypeptide according to claim 9, said method comprising contacting cells which normally express said polypeptide with a candidate compound, and determining the lack of expression said gene.

26. The method of Claim 25, wherein said candidate compound is an antisense nucleic acid.

27. A method of identifying a compound that mimics the activity of a polypeptide according to claim 9, said method comprising contacting cells which normally respond to said polypeptide with a candidate compound, and determining the responsiveness by said cell to said candidate compound.

28. A composition comprising a antagonist of a polypeptide according to claim 9, for use in a method of stimulating the immune response in a mammal.

29. Use of an antagonist of a polypeptide according to claim 9 in the manufacture of a medicament for stimulating the immune response in a mammal.

30. A method of diagnosing an inflammatory immune response in a mammal, said method comprising detecting the level of expression of a gene encoding a polypeptide according to claim 9 (a) in a test sample of tissue cells obtained from the mammal, and (b) in a control sample of known normal tissue cells of the same cell type, wherein a higher or lower level of expression of said gene in the test sample as compared to the control sample is indicative of the presence of an inflammatory immune response in the mammal from which the test tissue cells were obtained.


Sequence Listing

<110> GENENTECH, INC.

<120> NOVEL COMPOSITIONS AND METHODS FOR THE TREATMENT OF IMMUNE
      RELATED DISEASES

<130> SMW/FP6624209

<140> EP
<141> 2003-09-10

<150> EP 03749572.8
<151> 2003-09-10

<150> PCT/US03/028361
<151> 2003-09-10

<150> US 60/410,174
<151> 2002-09-11

<160> 104

<210> 1
<211> 1761
<212> DNA
<213> Homo sapiens

<400> 1
 atggctttag agatccacat gtcagacccc atgtgcctca tcgagaactt 50

 taatgagcag ctgaaggtta atcaggaagc tttggagatc ctgtctgcca 100

 ttacgcaacc tgtagttgtg gtagcgattg tgggcctcta tcgcactggc 150

 aaatcctacc tgatgaacaa gctggctggg aagaacaagg gcttctctgt 200

 tgcatctacg gtgcagtctc acaccaaggg aatttggata tggtgtgtgc 250

 ctcatcccaa ctggccaaat cacacattag ttctgcttga caccgagggc 300

 ctgggagatg tagagaaggc tgacaacaag aatgatatcc agatctttgc 350

 actggcactc ttactgagca gcacctttgt gtacaatact gtgaacaaaa 400

 ttgatcaggg tgctatcgac ctactgcaca atgtgacaga actgacagat 450

 ctgctcaagg caagaaactc acccgacctt gacagggttg aagatcctgc 500

 tgactctgcg agcttcttcc cagacttagt gtggactctg agagatttct 550

 gcttaggcct ggaaatagat gggcaacttg tcacaccaga tgaatacctg 600

 gagaattccc taaggccaaa gcaaggtagt gatcaaagag ttcaaaattt 650

 caatttgcct cgtctgtgta tacagaagtt ctttccaaaa aagaaatgct 700

 ttatctttga cttacctgct caccaaaaaa agcttgccca acttgaaaca 750

 ctgcctgatg atgagctaga gcctgaattt gtgcaacaag tgacagaatt 800

 ctgttcctac atctttagcc attctatgac caagactctt ccaggtggca 850

 tcatggtcaa tggatctcgt ctaaagaacc tggtgctgac ctatgtcaat 900

gccatcagca gtggggatct gccttgcata gagaatgcag tcctggcctt 950
ggctcagaga gagaactcag ctgcagtgca aaaggccatt gcccactatg 1000
accagcaaat gggccagaaa gtgcagctgc ccatggaaac cctccaggag 1050
ctgctggacc tgcacaggac cagtgagagg gaggccattg aagtcttcat 1100
gaaaaactct ttcaaggatg tagaccaaag tttccagaaa gaattggaga 1150
ctctactaga tgcaaaacag aatgacattt gtaaacggaa cctggaagca 1200
tcctcggatt attgctcggc tttacttaag gatatttttg gtcctctaga 1250
agaagcagtg aagcagggaa tttattctaa gccaggaggc cataatctct 1300
tcattcagaa aacagaagaa ctgaaggcaa agtactatcg ggagcctcgg 1350
aaaggaatac aggctgaaga agttctgcag aaatatttaa agtccaagga 1400
gtctgtgagt catgcaatat tacagactga ccaggctctc acagagacgg 1450
aaaaaaagaa gaaagaggca caagtgaaag cagaagctga aaaggctgaa 1500
gcgcaaaggt tggcggcgat tcaaaggcag aacgagcaaa tgatgcagga 1550
gagggagaga ctccatcagg aacaagtgag acaaatggag atagccaaac 1600
aaaattggct ggcagagcaa cagaaaatgc aggaacaaca gatgcaggaa 1650
caggctgcac agctcagcac aacattccaa gctcaaaata gaagccttct 1700
cagtgagctc cagcacgccc agaggactgt taataacgat gatccatgtg 1750
ttttactcta a 1761

<210> 2
<211> 586
<212> PRT
<213> Homo sapiens

<400> 2
Met Ala Leu Glu Ile His Met Ser Asp Pro Met Cys Leu Ile Glu
1               5                   10                  15

Asn Phe Asn Glu Gln Leu Lys Val Asn Gln Glu Ala Leu Glu Ile
                20                  25                  30

Leu Ser Ala Ile Thr Gln Pro Val Val Val Val Ala Ile Val Gly
                35                  40                  45

Leu Tyr Arg Thr Gly Lys Ser Tyr Leu Met Asn Lys Leu Ala Gly
                50                  55                  60

Lys Asn Lys Gly Phe Ser Val Ala Ser Thr Val Gln Ser His Thr
                65                  70                  75

Lys Gly Ile Trp Ile Trp Cys Val Pro His Pro Asn Trp Pro Asn
                80                  85                  90

His Thr Leu Val Leu Leu Asp Thr Glu Gly Leu Gly Asp Val Glu
                95                  100                 105

Lys Ala Asp Asn Lys Asn Asp Ile Gln Ile Phe Ala Leu Ala Leu
                110                 115                 120

Leu Leu Ser Ser Thr Phe Val Tyr Asn Thr Val Asn Lys Ile Asp
                125                 130                 135

Gln Gly Ala Ile Asp Leu Leu His Asn Val Thr Glu Leu Thr Asp
                140                 145                 150

Leu Leu Lys Ala Arg Asn Ser Pro Asp Leu Asp Arg Val Glu Asp
                155                 160                 165

Pro Ala Asp Ser Ala Ser Phe Phe Pro Asp Leu Val Trp Thr Leu
                170                 175                 180

Arg Asp Phe Cys Leu Gly Leu Glu Ile Asp Gly Gln Leu Val Thr
                185                 190                 195

Pro Asp Glu Tyr Leu Glu Asn Ser Leu Arg Pro Lys Gln Gly Ser
                200                 205                 210

Asp Gln Arg Val Gln Asn Phe Asn Leu Pro Arg Leu Cys Ile Gln
                215                 220                 225

Lys Phe Phe Pro Lys Lys Lys Cys Phe Ile Phe Asp Leu Pro Ala
                230                 235                 240

His Gln Lys Lys Leu Ala Gln Leu Glu Thr Leu Pro Asp Asp Glu
                245                 250                 255

Leu Glu Pro Glu Phe Val Gln Gln Val Thr Glu Phe Cys Ser Tyr
                260                 265                 270

Ile Phe Ser His Ser Met Thr Lys Thr Leu Pro Gly Gly Ile Met
                275                 280                 285

Val Asn Gly Ser Arg Leu Lys Asn Leu Val Leu Thr Tyr Val Asn
                290                 295                 300

Ala Ile Ser Ser Gly Asp Leu Pro Cys Ile Glu Asn Ala Val Leu
                305                 310                 315

Ala Leu Ala Gln Arg Glu Asn Ser Ala Ala Val Gln Lys Ala Ile
                320                 325                 330

Ala His Tyr Asp Gln Gln Met Gly Gln Lys Val Gln Leu Pro Met
                335                 340                 345

Glu Thr Leu Gln Glu Leu Leu Asp Leu His Arg Thr Ser Glu Arg
                350                 355                 360

Glu Ala Ile Glu Val Phe Met Lys Asn Ser Phe Lys Asp Val Asp
                365                 370                 375

Gln Ser Phe Gln Lys Glu Leu Glu Thr Leu Leu Asp Ala Lys Gln
                380                 385                 390

Asn Asp Ile Cys Lys Arg Asn Leu Glu Ala Ser Ser Asp Tyr Cys
                395                 400                 405

Ser Ala Leu Leu Lys Asp Ile Phe Gly Pro Leu Glu Glu Ala Val
                410                 415                 420

Lys Gln Gly Ile Tyr Ser Lys Pro Gly Gly His Asn Leu Phe Ile
                425                 430                 435

Gln Lys Thr Glu Glu Leu Lys Ala Lys Tyr Tyr Arg Glu Pro Arg
                440                 445                 450

Lys Gly Ile Gln Ala Glu Glu Val Leu Gln Lys Tyr Leu Lys Ser
                455                 460                 465

Lys Glu Ser Val Ser His Ala Ile Leu Gln Thr Asp Gln Ala Leu

470                 475                 480

Thr Glu Thr Glu Lys Lys Lys Lys Glu Ala Gln Val Lys Ala Glu
                485                 490                 495

Ala Glu Lys Ala Glu Ala Gln Arg Leu Ala Ala Ile Gln Arg Gln
                500                 505                 510

Asn Glu Gln Met Met Gln Glu Arg Glu Arg Leu His Gln Glu Gln
                515                 520                 525

Val Arg Gln Met Glu Ile Ala Lys Gln Asn Trp Leu Ala Glu Gln
                530                 535                 540

Gln Lys Met Gln Glu Gln Gln Met Gln Glu Gln Ala Ala Gln Leu
                545                 550                 555

Ser Thr Thr Phe Gln Ala Gln Asn Arg Ser Leu Leu Ser Glu Leu
                560                 565                 570

Gln His Ala Gln Arg Thr Val Asn Asn Asp Asp Pro Cys Val Leu
                575                 580                 585

Leu

<210> 3
<211> 2308
<212> DNA
<213> Homo sapiens

<400> 3
agcaggtttc gaatgctctt tacttccttt gtggagcaaa agaaaaaagc 50

aggagtattt gaacaaatca ctaagactca tggaacaatt attggcatta 100

cttcagggat tgtcttggtc cttctcatta tttctatttt agtacaagtg 150

aaacagcctc gaaaaaaggt catggcttgc aaaaccgctt ttaataaaac 200

cgggttccaa gaagtgtttg atcctcctca ttatgaactg ttttcactaa 250

gggacaaaga gatttctgca gacctggcag acttgtcgga agaattggac 300

aactaccaga ggatgcggcg ctcctccacc gcctcccgct gcatccacga 350

ccaccactgt gggtcgcagg cctccagcgt caaacaaagc aggaccaacc 400

tcagttccat ggagcttcct ctccgaaatg actttgcaca accacagcca 450

atgaaaacat ttaatagcac cttcaagaaa agtagttaca ctttcaaaca 500

gggacatgag tgccctgagc aggccctgga agaccgagta atggaggaga 550

ttccctgtga aatttatgtc agggggcgag aagattctgc acaagcatcc 600

atatccattg acttctaatc ttctgctaat ggtgatgtga attcttaggg 650

tgtgtacgta cgcagcctcc agggcaccat actgtttcca gcagccaacc 700

cttttctccc atcacaacta cgaagacctt gatttaccgt taacctattg 750

tatggtgatg tttttattct ctcaggcagt ctatatatgt taaaccaatc 800

aaggaactta ctctattcag tggaaacaat aatcatctct attgcttggt 850

gtcatttata ggaagcactg ccagttaaag agcattagaa gaggtggttg 900

gatggagcca ggctcaggct gcctcttcgt tttagcaaca agaagactgc 950
tcttgactga taacagctct gtcaatattt tgatgccaca ataaacttga 1000
tttttcttta cattcctttt attttccttt tctctaaatt taatttgttt 1050
tataagccta tcgttttacc atttcatttt cttacataag tacaagtggt 1100
taatgtacca catacttcag tataggcatt tgttcttgag tgtgtcaaaa 1150
tacagctagt tactgtgcca attaagaccc agttgtattt cacccatctg 1200
tttcttcttg gctaatctct gtacttctgc cttttaatta ctgggccctt 1250
attccttatt ttctgtgaga aataatagat gatatgattt attacctttc 1300
aattatattt ttctcagtta tactagaaaa tttcataatc ctgggatata 1350
tgtaccattg tcagctatga ctaaaaattt gaaaaagata aaaatttcta 1400
gcaagccttt gaagtttacc aagtatagtc acattcagtg acagcccatt 1450
cattccagta aagaatcatt tcattcactt tgggagaggc ctataattac 1500
atttatttgc aatgtttctc ttcgctagat tgttacatag ctcccattct 1550
gttggttttg cttacagcat atggtaacca aggttagatg ccagttaaaa 1600
ttccttagaa attggatgag ccttgagatt gcttcttaac tgggacatga 1650
catttttcta gctcttatca agaataacaa cttccacttt tttttaaact 1700
gcacttttga ctttttttat ggtataaaaa caataattta taaacataaa 1750
agctcattgt gttttttaga cttttgatat tatttgatac tgtacaaact 1800
ttattaaatc aagatgaaag acctacagga cagattcctt tcagtgttca 1850
catcagtggc tttgtatgca aatatgctgt gttggacctg gacgctataa 1900
cttattgtaa agaccttgga aatgtggaca taagctcttt ctttcctttt 1950
gttactgtat ttagtttgtg ataaattttt cactgtgtga tatttatgct 2000
ctaaatcact acacaaatcc catattaaaa tatacattgt acctgaccct 2050
ttaatcatgt tatttatgcc accaaggttg tggatcttaa ggtatgtatg 2100
gaaaggaact catttatcaa attgtaagta atacagacat gccatttaaa 2150
agaggtaaat tcttgttttc tatattttgt tagtaaattc tcaatgaaat 2200
aagttgaagt ttcactggat ttcattaact tttaaatatt acatatatgt 2250
gttttctcag attagtgaaa attgtgacct taaatttaat acacatatac 2300
tgcctcag 2308

<210> 4
<211> 201
<212> PRT
<213> Homo sapiens

<400> 4
Met Leu Phe Thr Ser Phe Val Glu Gln Lys Lys Lys Ala Gly Val
1               5                   10                  15

Phe Glu Gln Ile Thr Lys Thr His Gly Thr Ile Ile Gly Ile Thr

                    20                  25                  30

Ser Gly Ile Val Leu Val Leu Leu Ile Ile Ser Ile Leu Val Gln
                35                  40                  45

Val Lys Gln Pro Arg Lys Lys Val Met Ala Cys Lys Thr Ala Phe
                50                  55                  60

Asn Lys Thr Gly Phe Gln Glu Val Phe Asp Pro Pro His Tyr Glu
                65                  70                  75

Leu Phe Ser Leu Arg Asp Lys Glu Ile Ser Ala Asp Leu Ala Asp
                80                  85                  90

Leu Ser Glu Glu Leu Asp Asn Tyr Gln Arg Met Arg Arg Ser Ser
                95                  100                 105

Thr Ala Ser Arg Cys Ile His Asp His His Cys Gly Ser Gln Ala
                110                 115                 120

Ser Ser Val Lys Gln Ser Arg Thr Asn Leu Ser Ser Met Glu Leu
                125                 130                 135

Pro Leu Arg Asn Asp Phe Ala Gln Pro Gln Pro Met Lys Thr Phe
                140                 145                 150

Asn Ser Thr Phe Lys Lys Ser Ser Tyr Thr Phe Lys Gln Gly His
                155                 160                 165

Glu Cys Pro Glu Gln Ala Leu Glu Asp Arg Val Met Glu Glu Ile
                170                 175                 180

Pro Cys Glu Ile Tyr Val Arg Gly Arg Glu Asp Ser Ala Gln Ala
                185                 190                 195

Ser Ile Ser Ile Asp Phe
                200

<210> 5
<211> 1591
<212> DNA
<213> Homo sapiens

<400> 5
tctgggcgcg cgcgacgtca gtttgagttc tgtgttctcc ccgcccgtgt 50

cccgcccgac ccgcgcccgc gatgctggcg ctgcgctgcg gctcccgctg 100

gctcggcctg ctctccgtcc cgcgctccgt gccgctgcgc ctccccgcgg 150

cccgcgcctg cagcaagggc tccggcgacc cgtcctcttc ctcctcctcc 200

gggaacccgc tcgtgtacct ggacgtggac gccaacggga agccgctcgg 250

ccgcgtggtg ctggagctga aggcagatgt cgtcccaaag acagctgaga 300

acttcagagc cctgtgcact ggtgagaagg gcttcggcta caaaggctcc 350

accttccaca gggtgatccc ttccttcatg tgccaggcgg gcgacttcac 400

caaccacaat ggcacaggcg ggaagtccat ctacggaagc cgctttcctg 450

acgagaactt tacactgaag cacgtggggc caggtgtcct gtccatggct 500

aatgctggtc ctaacaccaa cggctcccag ttcttcatct gcaccataaa 550

gacagactgg ttggatggca agcatgttgt gttcggtcac gtcaaagagg 600

gcatggacgt cgtgaagaaa atagaatctt tcggctctaa gagtgggagg 650
acatccaaga agattgtcat cacagactgt ggccagttga gctaatctgt 700
ggccagggtg ctggcatggt ggcagctgca aatgtccatg cacccaggtg 750
gccgcgttgg gctgtcagcc aaggtgcctg aaacgatacg tgtgcccact 800
ccactgtcac agtgtgcctg aggaaggctg ctagggatgt tagacctcgg 850
ccaggaccca ccacattgct tcctaatacc cacccttcct cacgacctca 900
tttctgggca tctttgtgga catgatgtca cccacccctt gtcaagcatt 950
gcctgtgatt gcccagccca gattcatctg tgccttggac atggtgatgg 1000
tgatgggttg ccatccaagt gaaagtcttt tccttgacca agggggacag 1050
tcagttttgc aaaaggactc taatacctgt ttaatattgt cttcctaatt 1100
gggataattt aattaacaag attgactaga agtgaaactg caacactaac 1150
ttccccgtgc tgtggtgtga cctgagttgg tgacacaggc cacagacccc 1200
agagcttggc ttttgaaaca caactcaggg cttttgtgaa ggttcccccg 1250
ctgagatctt tcctcctggt tactgtgaag cctgttggtt tgctgctgtc 1300
gtttttgagg agggcccatg ggggtaggag cagttgaacc tgggaacaaa 1350
cctcacttga gctgtgccta gacaatgtga attcctgtgt tgctaacaga 1400
agtggcctgt aagctcctgt gctccggagg gaagcatttc ctggtaggct 1450
ttgatttttc tgtgtgttaa agaaattcaa tctactcatg atgtgttatg 1500
cataaaacat ttctggaaca tggatttgtg ttcaccttaa atgtgaaaat 1550
aaatcctatt ttctatggaa aaaaaaaaaa aaaaaaaaaa a 1591

<210> 6
<211> 207
<212> PRT
<213> Homo sapiens

<400> 6
Met Leu Ala Leu Arg Cys Gly Ser Arg Trp Leu Gly Leu Leu Ser
1               5                   10                  15

Val Pro Arg Ser Val Pro Leu Arg Leu Pro Ala Ala Arg Ala Cys
                20                  25                  30

Ser Lys Gly Ser Gly Asp Pro Ser Ser Ser Ser Ser Ser Gly Asn
                35                  40                  45

Pro Leu Val Tyr Leu Asp Val Asp Ala Asn Gly Lys Pro Leu Gly
                50                  55                  60

Arg Val Val Leu Glu Leu Lys Ala Asp Val Val Pro Lys Thr Ala
                65                  70                  75

Glu Asn Phe Arg Ala Leu Cys Thr Gly Glu Lys Gly Phe Gly Tyr
                80                  85                  90

Lys Gly Ser Thr Phe His Arg Val Ile Pro Ser Phe Met Cys Gln
                95                  100                 105

Ala Gly Asp Phe Thr Asn His Asn Gly Thr Gly Gly Lys Ser Ile

110                 115                 120

Tyr Gly Ser Arg Phe Pro Asp Glu Asn Phe Thr Leu Lys His Val
                125                 130                 135

Gly Pro Gly Val Leu Ser Met Ala Asn Ala Gly Pro Asn Thr Asn
                140                 145                 150

Gly Ser Gln Phe Phe Ile Cys Thr Ile Lys Thr Asp Trp Leu Asp
                155                 160                 165

Gly Lys His Val Val Phe Gly His Val Lys Glu Gly Met Asp Val
                170                 175                 180

Val Lys Lys Ile Glu Ser Phe Gly Ser Lys Ser Gly Arg Thr Ser
                185                 190                 195

Lys Lys Ile Val Ile Thr Asp Cys Gly Gln Leu Ser
                200                 205

<210> 7
<211> 1706
<212> DNA
<213> Homo sapiens

<400> 7
tgttcttgag cccagcttct tctcgtctcc caccccagct tcccggcatt 50

ggaagaaggg accgtcctct tccttgtctt ggccacccaa atcctggtat 100

cgaaagggtt gaacggaccg gaagtgtgca gcagcgacgg gtccccagct 150

aatcgacgcc ggaagtagca attactagac aagcattccg ccgccggctt 200

cgctatggcg gcaattcccc cagattcctg gcagccaccc aacgtttact 250

tggagaccag catgggaatc attgtgctgg agctgtactg gaagcatgct 300

ccaaagacct gtaagaactt tgctgagttg gctcgtcgag gttactacaa 350

tggcacaaaa ttccacagaa ttatcaaaga cttcatgatc caaggaggtg 400

acccaacagg gacaggtcga ggtggtgcat ctatctatgg caaacagttt 450

gaagatgaac ttcatccaga cttgaaattc acgggggctg gaattctcgc 500

aatggccaat gcggggccag ataccaatgg cagccagttc tttgtgaccc 550

tcgcccccac ccagtggctt gacggcaaac acaccatttt tggccgagtg 600

tgtcagggca taggaatggt gaatcgcgtg ggaatggtag aaacaaactc 650

ccaggaccgc cctgtggacg acgtgaagat cattaaggca taccctcctg 700

ggtagacttg ctaccctctt gagcagctct tctgagatgg ccccagtgaa 750

ccagcttcta gatgacatag aatgacatgt aatgctaaat ttcattttgg 800

ctttgcaagt catgaagctt aggaggcctg gcatcttggg tgagttagag 850

atggaagtac attttaatag gatgcttctt ttctcttccc ccagtgccta 900

ggttgccaga gcatttgcac aaatgcccct gtttatcaat aggtgactac 950

ttactacaca tgaaccataa tgctgcttct tgtgcatgtc tgctctgata 1000

tacgtcgaac aatgtagcag ccactgtcat ttctcagtgg ttttgcctaa 1050

ccaaacttct tcctaaggag atttatattc tggcctacac agcagtcctt 1100

gatggctgac agccacagaa ttccaaacca agtagtgtct gtcagccctc 1150

ttaactctgt gcacgcccta tttcagtctt ttacatttgt tcttctaggg 1200

aatgtatgca tctctatata tattttccct ctcaaaacca gaacatcaac 1250

agtgctgttt ctgacacttc agacatccca cgcaaagcca cattgaattt 1300

ttgccaaatg aaaaacacat ccaacaatca agtttctaag aaggtgtcaa 1350

gtggggaata ataataatgt ataataatca agaaattagt ttattaaaag 1400

gaagcagaag cattgaccat tttttcccag agaagaggag aaatctgtag 1450

tgagcaaagg acagaccatg aatcctcctt gagaagtagt actctcagaa 1500

aggagaagcg ccactcaagt tcttttaacc caagacttta gagaaattag 1550

gtccaagatt tttatatgtt cagttgttta tgtataaaaa taactttctg 1600

gattttgtgg ggaggagcag gagaggaagg aagttaatac ctatgtaata 1650

catagaaact tccacaataa aatgccattg atggttgaaa aaaaaaaaaa 1700

aaaaaa 1706

<210> 8
<211> 166
<212> PRT
<213> Homo sapiens

<400> 8
Met Ala Ala Ile Pro Pro Asp Ser Trp Gln Pro Pro Asn Val Tyr
1               5                   10                  15

Leu Glu Thr Ser Met Gly Ile Ile Val Leu Glu Leu Tyr Trp Lys
                20                  25                  30

His Ala Pro Lys Thr Cys Lys Asn Phe Ala Glu Leu Ala Arg Arg
                35                  40                  45

Gly Tyr Tyr Asn Gly Thr Lys Phe His Arg Ile Ile Lys Asp Phe
                50                  55                  60

Met Ile Gln Gly Gly Asp Pro Thr Gly Thr Gly Arg Gly Gly Ala
                65                  70                  75

Ser Ile Tyr Gly Lys Gln Phe Glu Asp Glu Leu His Pro Asp Leu
                80                  85                  90

Lys Phe Thr Gly Ala Gly Ile Leu Ala Met Ala Asn Ala Gly Pro
                95                  100                 105

Asp Thr Asn Gly Ser Gln Phe Phe Val Thr Leu Ala Pro Thr Gln
                110                 115                 120

Trp Leu Asp Gly Lys His Thr Ile Phe Gly Arg Val Cys Gln Gly
                125                 130                 135

Ile Gly Met Val Asn Arg Val Gly Met Val Glu Thr Asn Ser Gln
                140                 145                 150

Asp Arg Pro Val Asp Asp Val Lys Ile Ile Lys Ala Tyr Pro Ser
                155                 160                 165

Gly

<210> 9
<211> 1495
<212> DNA
<213> Homo sapiens

<400> 9
gtaacggatg gtgcgccaac gtgagaggaa acccgtgcgc ggctgcgctt 50

tcctgtcccc aagccgttct agacgcggat gaagtgcaaa acaaacttct 100

ccatagagga gttgttgcaa agttccagtt tataccaaac agtaatcaga 150

ttccattgga agctaaagat tttgagagcc ttttgtacta tatgcaacta 200

acttgatttc aagcttggga acttttaaaa aaaacattaa agcaaaatga 250

aaaatgcttt ctgaaagcag ctcctttttg aaaggtgtga tgcttggaag 300

ccattttctg tgctttgatc cactaatgct aaggacacat taggattggt 350

catggaaata gaatgcacca ccatgagcat catcacctac aagctcctaa 400

caaagaagat atcttgaaaa tttcagagga tgagcgcatg gagctcagta 450

agagctttcg agtatactgt attatccttg taaaacccaa agatgtgagt 500

ctttgggctg cagtaaagga gacttggacc aaacactgtg acaaagcaga 550

gttcttcagt tctgaaaatg ttaaagagtt tgagtcaatt aatatggaca 600

caaatgacat gtggttaatg atgagaaaag cttacaaata cgcctttgat 650

aagtatagag accaatacaa ctggttcttc cttgcacgcc ccactacgtt 700

tgctatcatt gaaaacctaa agtatttttt gttaaaaaag gatccatcac 750

agcctttcta tctaggccac actataaaat ctggagacct tgaatatgtg 800

ggtatggaag gaggaattgt cttaagtgta gaatcaatga aaagacttaa 850

cagccttctc aatatcccag aaaagtgtcc tgaacaggga gggatgattt 900

ggaagatatc cgaagataaa cagctagcag tttgcctgaa atatgctgga 950

gtatttgcag aaaatgcaga agatgctgat ggaaaagatg tatttaatac 1000

caaatctgtt gggctttcta ttaaagaggc aatgacttat caccccaacc 1050

aggtagtaga aggctgttgt tcagatatgg ctgttacttt taatggactg 1100

actccaaatc agatgcatgt gatgatgtat ggggtatacc gccttagggc 1150

atttgggcat attttcaatg atgcattggt tttcttacct ccaaatggtt 1200

ctgacaatga ctgagaagtg gtagaaaagc gtgaatatga tctttgtata 1250

ggacgtgtgt tgtcattatt tgtagtagta actacatatc caatacagct 1300

gtatgtttct ttttcttttc taatttggtg gcactggtat aaccacccat 1350

taaagtcagt agtacatttt taaatgaggg tggttttttt ctttaaaaca 1400

catgaacatt gtaaatgtgt tggaaaaaag tgttttaaga ataataattt 1450

tgcaaataaa ctattaataa atattatatg tgataaattc taacc 1495

<210> 10
<211> 283
<212> PRT
<213> Homo sapiens

<400> 10
Met His His His Glu His His His Leu Gln Ala Pro Asn Lys Glu
1               5                   10                  15

Asp Ile Leu Lys Ile Ser Glu Asp Glu Arg Met Glu Leu Ser Lys
                20                  25                  30

Ser Phe Arg Val Tyr Cys Ile Ile Leu Val Lys Pro Lys Asp Val
                35                  40                  45

Ser Leu Trp Ala Ala Val Lys Glu Thr Trp Thr Lys His Cys Asp
                50                  55                  60

Lys Ala Glu Phe Phe Ser Ser Glu Asn Val Lys Glu Phe Glu Ser
                65                  70                  75

Ile Asn Met Asp Thr Asn Asp Met Trp Leu Met Met Arg Lys Ala
                80                  85                  90

Tyr Lys Tyr Ala Phe Asp Lys Tyr Arg Asp Gln Tyr Asn Trp Phe
                95                  100                 105

Phe Leu Ala Arg Pro Thr Thr Phe Ala Ile Ile Glu Asn Leu Lys
                110                 115                 120

Tyr Phe Leu Leu Lys Lys Asp Pro Ser Gln Pro Phe Tyr Leu Gly
                125                 130                 135

His Thr Ile Lys Ser Gly Asp Leu Glu Tyr Val Gly Met Glu Gly
                140                 145                 150

Gly Ile Val Leu Ser Val Glu Ser Met Lys Arg Leu Asn Ser Leu
                155                 160                 165

Leu Asn Ile Pro Glu Lys Cys Pro Glu Gln Gly Gly Met Ile Trp
                170                 175                 180

Lys Ile Ser Glu Asp Lys Gln Leu Ala Val Cys Leu Lys Tyr Ala
                185                 190                 195

Gly Val Phe Ala Glu Asn Ala Glu Asp Ala Asp Gly Lys Asp Val
                200                 205                 210

Phe Asn Thr Lys Ser Val Gly Leu Ser Ile Lys Glu Ala Met Thr
                215                 220                 225

Tyr His Pro Asn Gln Val Val Glu Gly Cys Cys Ser Asp Met Ala
                230                 235                 240

Val Thr Phe Asn Gly Leu Thr Pro Asn Gln Met His Val Met Met
                245                 250                 255

Tyr Gly Val Tyr Arg Leu Arg Ala Phe Gly His Ile Phe Asn Asp
                260                 265                 270

Ala Leu Val Phe Leu Pro Pro Asn Gly Ser Asp Asn Asp
                275                 280

<210> 11
<211> 1534
<212> DNA
<213> Homo sapiens

<400> 11
tcagtgggcg tcgcgcgaag gctaagggag tgtggcgggc ggctccggga 50
gccaacatgc ctcggtatgc gcagctggtc atgggccccg cgggcagcgg 100
gaagagcacc tactgtgcca ccatggtcca gcactgtgaa gccctcaacc 150
ggtctgtcca agttgtaaac ctggatccag cagcagaaca cttcaactac 200
tccgtgatgg ctgacatccg ggaactgatc gaggtggatg atgtaatgga 250
ggatgattct ctgcgattcg gtcccaacgg aggattggta ttttgcatgg 300
agtactttgc caataatttt gactggctgg agaactgtct tggccatgta 350
gaggacgact atatcctttt tgattgtcca ggtcagattg agttgtacac 400
tcacctgcct gtgatgaaac agctggtcca gcagctcgag cagtgggagt 450
tccgagtctg tggagttttt cttgttgatt ctcagttcat ggtggagtca 500
ttcaagttta tttctggcat cttggcagcc ctgagtgcca tgatctctct 550
agaaattccg caagtcaaca tcatgacaaa aatggatctg ctgagtaaaa 600
aagcaaaaaa ggaaattgag aaatttttag atccagacat gtattcttta 650
ttagaagatt ctacaagtga cttaagaagc aaaaaattca agaaactgac 700
taaagctata tgtggactga ttgatgacta cagcatggtt cgatttttac 750
cttacgatca gtcagatgaa gaaagcatga acattgcatt gcagcatatt 800
gattttgcca ttcaatatgg agaagaccta gaatttaaag aaccaaagga 850
acgtgaagat gagtcttcct ctatgtttga cgaatatttt caagaatgcc 900
aggatgaatg aagagtttac taaaagtaac catctaaaga gcttgtggcc 950
aaaccagcag aacattcttc tcttcaaagg atgcaatagt agaaagctac 1000
ttattttaat gaaaaaaagt aaaacttcgt tctttatcag cctcatgcct 1050
gaatcaaatt tttaattatt ctgaaactgc tgctgtttaa agtggaatct 1100
tttagtatta taacagcatc actttagatt ttgtaagtca aaattgaaat 1150
gaatgcacat agatttatat ataaattagc acctgagcta aggttaaggc 1200
tggtctaaac ttattttcac tttttgtatt atttttgaga tgcaggaatt 1250
actgtaacaa aatatgtatg tccgaaggga aaaagctgca aggatatata 1300
taagaccact gcttatctgt atcttcccat tttcctatat tgaaaatgta 1350
tattatttat ataacttaaa aagtaaaaat aactatgttt tgagatatgt 1400
atgtgtatat ataaaagaaa caaaggtttt taatgattct tggacctaga 1450
taacaagtaa aaaaaaaaaa aaaaaaaaaa aaaaaaaaaa aaaaaaaaaa 1500
aaaaaaaaaa aaaaaaaaaa aaaaaaaaaa aaaa 1534

<210> 12
<211> 284
<212> PRT
<213> Homo sapiens

<400> 12
Met Pro Arg Tyr Ala His Cys Val Met Gly Pro Ala His Ala Lys
1               5                   10                  15

Arg Ser Thr Tyr Cys Ala Thr Met Val Gln His Cys Glu Ala Leu
                20                  25                  30

Asn Arg Ser Val Gln Val Val Asn Leu Asp Pro Ala Ala Glu His
                35                  40                  45

Phe Asn Tyr Ser Val Met Ala Asp Ile Arg Glu Leu Ile Glu Val
                50                  55                  60

Asp Asp Val Met Glu Asp Asp Ser Leu Arg Phe Gly Pro Asn Gly
                65                  70                  75

Gly Leu Val Phe Cys Met Glu Tyr Phe Ala Asn Asn Phe Asp Trp
                80                  85                  90

Leu Glu Asn Cys Leu Gly His Val Glu Asp Asp Tyr Ile Leu Phe
                95                  100                 105

Asp Cys Pro Gly Gln Ile Glu Leu Tyr Thr His Leu Pro Val Met
                110                 115                 120

Lys Gln Leu Val Gln Gln Leu Glu Gln Trp Glu Phe Arg Val Cys
                125                 130                 135

Gly Val Phe Leu Val Asp Ser Gln Phe Met Val Glu Ser Phe Lys
                140                 145                 150

Phe Ile Ser Gly Ile Leu Ala Ala Leu Ser Ala Met Ile Ser Leu
                155                 160                 165

Glu Ile Pro Gln Val Asn Ile Met Thr Lys Met Asp Leu Leu Ser
                170                 175                 180

Lys Lys Ala Lys Lys Glu Ile Glu Lys Phe Leu Asp Pro Asp Met
                185                 190                 195

Tyr Ser Leu Leu Glu Asp Ser Thr Ser Asp Leu Arg Ser Lys Lys
                200                 205                 210

Phe Lys Lys Leu Thr Lys Ala Ile Cys Gly Leu Ile Asp Asp Tyr
                215                 220                 225

Ser Met Val Arg Phe Leu Pro Tyr Asp Gln Ser Asp Glu Glu Ser
                230                 235                 240

Met Asn Ile Val Leu Gln His Ile Asp Phe Ala Ile Gln Tyr Gly
                245                 250                 255

Glu Asp Leu Glu Phe Lys Glu Pro Lys Glu Arg Glu Asp Glu Ser
                260                 265                 270

Ser Ser Met Phe Asp Glu Tyr Phe Gln Glu Cys Gln Asp Glu
                275                 280

<210> 13
<211> 1844
<212> DNA
<213> Homo sapiens

<400> 13
ccgggacagc tcgcggcccc cgagagctct agccgtcgag gagctgcctg 50

gggacgtttg ccttggggcc ccagcctggc ccgggtcacc ctggcatgag 100

gagatgggcc tgttgctcct ggtcccgttg ctcctgctgc ccggctccta 150
cggactgccc ttctacaacg gcttctacta ctccaacagc gccaacgacc 200
agaacctagg caacggtcat ggcaaagacc tccttaatgg agtgaagctg 250
gtggtggaga cacccgagga gaccctgttc acctaccaag gggccagtgt 300
gatcctgccc tgccgctacc gctacgagcc ggccctggtc tccccgcggc 350
gtgtgcgtgt caaatggtgg aagctgtcgg agaacggggc cccagagaag 400
gacgtgctgg tggccatcgg gctgaggcac cgctcctttg gggactacca 450
aggccgcgtg cacctgcggc aggacaaaga gcatgacgtc tcgctggaga 500
tccaggatct gcggctggag gactatgggc gttaccgctg tgaggtcatt 550
gacgggctgg aggatgaaag cggtctggtg gagctggagc tgcggggtgt 600
ggtctttcct taccagtccc ccaacgggcg ctaccagttc aacttccacg 650
agggccagca ggtctgtgca gagcaggctg cggtggtggc ctcctttgag 700
cagctcttcc gggcctggga ggagggcctg gactggtgca acgcgggctg 750
gctgcaggat gccacggtgc agtaccccat catgttgccc cggcagccct 800
gcggtggccc gggcctggca cctggcgtgc gaagctacgg cccccgccac 850
cgccgcctgc accgctatga tgtattctgc ttcgctactg ccctcaaggg 900
gcgggtgtac tacctggagc accctgagaa gctgacgctg acagaggcaa 950
gggaggcctg ccaggaagat gatgccacga tcgccaaggt gggacagctc 1000
tttgccgcct ggaagttcca tggcctggac cgctgcgacg ctggctggct 1050
ggcagatggt agcgtccgct accctgtggt tcacccgcat cctaactgtg 1100
ggcccccaga gcctggggtc cgaagctttg gcttccccga cccgcagagc 1150
cgcttgtacg gtgtttactg ctaccgccag cactaggacc tggggccctc 1200
ccctgccgca ttccctcact ggctgtgtat ttattgagtg gttcgttttc 1250
ccttgtgggt tggagccatt ttaactgttt ttatacttct caatttaaat 1300
tttctttaaa cattttttta ctattttttg taaagcaaac agaacccaat 1350
gcctcccttt gctcctggat gccccactcc aggaatcatg cttgctcccc 1400
tgggccattt gcggttttgt gggcttctgg agggttcccc gccatccagg 1450
ctggtctccc tcccttaagg aggttggtgc ccagagtggg cggtggcctg 1500
tctagaatgc cgccgggagt ccgggcatgg tgggcacagt tctccctgcc 1550
cctcagcctg ggggaagaag agggcctcgg gggcctccgg agctgggctt 1600
tgggcctctc ctgcccacct ctacttctct gtgaagccgc tgactgtaac 1650
ccagttctag gcttccaggc gaaagctgag ggaaggaaga aactcccctc 1700
cccgttcccc ttcccctctc ggttccaaag aatctgtttt gttgtcattt 1750
gtttctcctg tttccctgtg tggggagggg ccctcaggtg tgtgtacttt 1800

ggacaataaa tggtgctatg actgccttcc gccaaaaaaa aaaa 1844

<210> 14
<211> 360
<212> PRT
<213> Homo sapiens

<400> 14
Met Gly Leu Leu Leu Leu Val Pro Leu Leu Leu Leu Pro Gly Ser
1               5                   10                  15

Tyr Gly Leu Pro Phe Tyr Asn Gly Phe Tyr Tyr Ser Asn Ser Ala
                20                  25                  30

Asn Asp Gln Asn Leu Gly Asn Gly His Gly Lys Asp Leu Leu Asn
                35                  40                  45

Gly Val Lys Leu Val Val Glu Thr Pro Glu Glu Thr Leu Phe Thr
                50                  55                  60

Tyr Gln Gly Ala Ser Val Ile Leu Pro Cys Arg Tyr Arg Tyr Glu
                65                  70                  75

Pro Ala Leu Val Ser Pro Arg Arg Val Arg Val Lys Trp Trp Lys
                80                  85                  90

Leu Ser Glu Asn Gly Ala Pro Glu Lys Asp Val Leu Val Ala Ile
                95                  100                 105

Gly Leu Arg His Arg Ser Phe Gly Asp Tyr Gln Gly Arg Val His
                110                 115                 120

Leu Arg Gln Asp Lys Glu His Asp Val Ser Leu Glu Ile Gln Asp
                125                 130                 135

Leu Arg Leu Glu Asp Tyr Gly Arg Tyr Arg Cys Glu Val Ile Asp
                140                 145                 150

Gly Leu Glu Asp Glu Ser Gly Leu Val Glu Leu Glu Leu Arg Gly
                155                 160                 165

Val Val Phe Pro Tyr Gln Ser Pro Asn Gly Arg Tyr Gln Phe Asn
                170                 175                 180

Phe His Glu Gly Gln Gln Val Cys Ala Glu Gln Ala Ala Val Val
                185                 190                 195

Ala Ser Phe Glu Gln Leu Phe Arg Ala Trp Glu Glu Gly Leu Asp
                200                 205                 210

Trp Cys Asn Ala Gly Trp Leu Gln Asp Ala Thr Val Gln Tyr Pro
                215                 220                 225

Ile Met Leu Pro Arg Gln Pro Cys Gly Gly Pro Gly Leu Ala Pro
                230                 235                 240

Gly Val Arg Ser Tyr Gly Pro Arg His Arg Arg Leu His Arg Tyr
                245                 250                 255

Asp Val Phe Cys Phe Ala Thr Ala Leu Lys Gly Arg Val Tyr Tyr
                260                 265                 270

Leu Glu His Pro Glu Lys Leu Thr Leu Thr Glu Ala Arg Glu Ala
                275                 280                 285

Cys Gln Glu Asp Asp Ala Thr Ile Ala Lys Val Gly Gln Leu Phe
                290                 295                 300

Ala Ala Trp Lys Phe His Gly Leu Asp Arg Cys Asp Ala Gly Trp
                305                 310                 315

Leu Ala Asp Gly Ser Val Arg Tyr Pro Val Val His Pro His Pro
                320                 325                 330

Asn Cys Gly Pro Pro Glu Pro Gly Val Arg Ser Phe Gly Phe Pro
                335                 340                 345

Asp Pro Gln Ser Arg Leu Tyr Gly Val Tyr Cys Tyr Arg Gln His
                350                 355                 360

<210> 15
<211> 4565
<212> DNA
<213> Homo sapiens

<400> 15
ggcgagctaa gccggaggat gtgcagctgc ggcggcggcg ccggctacga 50

agaggacggg gacaggcgcc gtgcgaaccg agcccagcca gccggaggac 100

gcgggcaggg cgggacggga gcccggactc gtctgccgcc gccgtcgtcg 150

ccgtcgtgcc ggccccgcgt ccccgcgcgc gagcgggagg agccgccgcc 200

acctcgcgcc cgagccgccg ctagcgcgcg ccgggcatgg tcccctctta 250

aaggcgcagg ccgcggcggc gggggcgggc gtgcggaaca aagcgccggc 300

gcggggcctg cgggcggctc gggggccgcg atgggcgcgg cgggcccgcg 350

gcggcggcgg cgctgcccgg gccgggcctc gcggcgctag ggcgggctgg 400

cctccgcggg cgggggcagc gggctgaggg cgcgcggggc ctgcggcggc 450

ggcggcggcg gcggcggcgg cccggcgggc ggagcggcgc gggcatggcc 500

gcgcgcggcc ggcgcgcctg gctcagcgtg ctgctcgggc tcgtcctggg 550

cttcgtgctg gcctcgcggc tcgtcctgcc ccgggcttcc gagctgaagc 600

gagcgggccc acggcgccgc gccagccccg agggctgccg gtccgggcag 650

gcggcggctt cccaggccgg cggggcgcgc ggcgatgcgc gcggggcgca 700

gctctggccg cccggctcgg acccagatgg cggcccgcgc gacaggaact 750

ttctcttcgt gggagtcatg accgcccaga aatacctgca gactcggccc 800

gtggccgcct acagaacatg gtccaagaca attcctggga aagttcagtt 850

cttctcaagt gagggttctg acacatctgt accaattcca gtagtgccac 900

tacggggtgt ggacgactcc tacccgcccc agaagaagtc cttcatgatg 950

ctcaagtaca tgcacgacca ctacttggac aagtatgaat ggtttatgag 1000

agcagatgat gacgtgtaca tcaaaggaga ccgtctggag aacttcctga 1050

ggagtttgaa cagcagcgag cccctctttc ttgggcagac aggcctgggc 1100

accacggaag aaatgggaaa actggccctg gagcctggtg agaacttctg 1150

catggggggg cctggcgtga tcatgagccg ggaggtgctt cggagaatgg 1200

tgccgcacat tggcaagtgt ctccgggaga tgtacaccac ccatgaggac 1250

gtggaggtgg gaaggtgtgt ccggaggttt gcaggggtgc agtgtgtctg 1300
gtcttatgag atgcagcagc ttttttatga gaattacgag cagaacaaaa 1350
aggggtacat tagagatctc cataacagta aaattcacca agctatcaca 1400
ttacacccca acaaaaaccc accctaccag tacaggctcc acagctacat 1450
gctgagccgc aagatatccg agctccgcca tcgcacaata cagctgcacc 1500
gcgaaattgt cctgatgagc aaatacagca acacagaaat tcataaagag 1550
gacctccagc tgggaatccc tccctccttc atgaggtttc agccccgcca 1600
gcgagaggag attctggaat gggagtttct gactggaaaa tacttgtatt 1650
cggcagttga cggccagccc cctcgaagag gaatggactc cgcccagagg 1700
gaagccttgg acgacattgt catgcaggtc atggagatga tcaatgccaa 1750
cgccaagacc agagggcgca tcattgactt caaagagatc cagtacggct 1800
accgccgggt gaaccccatg tatggggctg agtacatcct ggacctgctg 1850
cttctgtaca aaaagcacaa agggaagaaa atgacggtcc ctgtgaggag 1900
gcacgcgtat ttacagcaga ctttcagcaa aatccagttt gtggagcatg 1950
aggagctgga tgcacaagag ttggccaaga gaatcaatca ggaatctgga 2000
tccttgtcct ttctctcaaa ctccctgaag aagctcgtcc cctttcagct 2050
ccctgggtcg aagagtgagc acaaagaacc caaagataaa aagataaaca 2100
tactgattcc tttgtctggg cgtttcgaca tgtttgtgag atttatggga 2150
aactttgaga agacgtgtct tatccccaat cagaacgtca agctcgtggt 2200
tctgcttttc aattctgact ccaaccctga caaggccaaa caagttgaac 2250
tgatgacaga ttaccgcatt aagtacccta aagccgacat gcagattttg 2300
cctgtgtctg gagagttttc aagagccctg gccctggaag taggatcctc 2350
ccagtttaac aatgaatctt tgctcttctt ctgcgacgtc gacctcgtct 2400
ttactacaga attccttcag cgatgtcgag caaatacagt tctgggccaa 2450
caaatatatt ttccaatcat cttcagccag tatgacccaa agattgttta 2500
tagtgggaaa gttcccagtg acaaccattt tgcctttact cagaaaactg 2550
gcttctggag aaactatggg tttggcatca cgtgtattta taagggagat 2600
cttgtccgag tgggtggctt tgatgtttcc atccaaggct gggggctgga 2650
ggatgtggac cttttcaaca aggttgtcca ggcaggtttg aagacgttta 2700
ggagccagga agtaggagta gtccacgtcc accatcctgt cttttgtgat 2750
cccaatcttg accccaaaca gtacaaaatg tgcttggggt ccaaagcatc 2800
gacctatggg tccacacagc agctggctga gatgtggctg gaaaaaaatg 2850
atccaagtta cagtaaaagc agcaataata atggctcagt gaggacagcc 2900
taatgtccag ctttgctgga aaagacgttt ttaattatct aatttatttt 2950

tcaaaaattt tttgtatgat cagtttttga agtccgtata caaggatata 3000
ttttacaagt ggttttctta cataggactc ctttaagatt gagctttctg 3050
aacaagaagg tgatcagtgt ttgcctttga acacatcttc ttgctgaaca 3100
ttatgtagca gacctgctta actttgactt gaaatgtacc tgatgaacaa 3150
aactttttta aaaaaatgtt ttcttttgag accctttgct ccagtcctat 3200
ggcagaaaac gtgaacattc ctgcaaagta ttattgtaac aaaacactgt 3250
aactctggta aatgttctgt tgtgattgtt aacattccac agattctacc 3300
ttttgtgttt tgtttttttt tttttacaat tgttttaaag ccatttcatg 3350
ttccagttgt aagataagga aatgtgataa tagctgtttc atcattgtct 3400
tcaggagagc tttccagagt tgatcatttc ccctcatggt actctgctca 3450
gcatggccac gtaggttttt tgtttgtttt gttttgttct ttttttgaga 3500
cggagtctca ctctgttacc caggctggaa tgcagtggcg caatcttggc 3550
tcactttaac ctccacttcc ctggttcaag caattcccct gcctttgcct 3600
cccgagtagc tgggattaca ggcacacacc accacgccca gctagttttt 3650
ttgtattttt agtagagacg gggtttcacc atgcaagccc agctggccac 3700
gtaggtttta aagcaagggg cgtgaagaag gcacagtgag gtatgtggct 3750
gttctcgtgg tagttcattc ggcctaaata gacctggcat taaatttcaa 3800
gaaggatttg gcattttctc ttcttgaccc ttctctttaa agggtaaaat 3850
attaatgttt agaatgacaa agatgaatta ttacaataaa tctgatgtac 3900
acagactgaa acacacacac atacacccta atcaaaacgt tggggaaaaa 3950
tgtatttggt tttgttcctt tcatcctgtc tgtgttatgt gggtggagat 4000
ggttttcatt ctttcattac tgttttgttt tatcctttgt atctgaaata 4050
cctttaattt atttaatatc tgttgttcag agctctgcca tttcttgagt 4100
acctgttagt tagtattatt tatgtgtatc gggagtgtgt ttagtctgtt 4150
ttatttgcag taaaccgatc tccaaagatt tccttttgga aacgcttttt 4200
cccctcctta atttttatat tccttactgt tttactaaat attaagtgtt 4250
ctttgacaat tttggtgctc atgtgttttg gggacaaaag tgaaatgaat 4300
ctgtcattat accagaaagt taaattctca gatcaaatgt gccttaataa 4350
atttgttttc atttagattt caaacagtga tagacttgcc attttaatac 4400
acgtcattgg agggctgcgt atttgtaaat agcctgatgc tcatttggaa 4450
aaataaacca gtgaacaata tttttctatt gtacttttca gaaccatttt 4500
gtctcattat tcctgtttta gctgaagaat tgtattacat ttggagagta 4550
aaaaacttaa acacg 4565

<210> 16
<211> 802

<212> PRT
<213> Homo sapiens

<400> 16
Met Ala Ala Arg Gly Arg Arg Ala Trp Leu Ser Val Leu Leu Gly
1               5                   10                  15

Leu Val Leu Gly Phe Val Leu Ala Ser Arg Leu Val Leu Pro Arg
                20                  25                  30

Ala Ser Glu Leu Lys Arg Ala Gly Pro Arg Arg Arg Ala Ser Pro
                35                  40                  45

Glu Gly Cys Arg Ser Gly Gln Ala Ala Ala Ser Gln Ala Gly Gly
                50                  55                  60

Ala Arg Gly Asp Ala Arg Gly Ala Gln Leu Trp Pro Pro Gly Ser
                65                  70                  75

Asp Pro Asp Gly Gly Pro Arg Asp Arg Asn Phe Leu Phe Val Gly
                80                  85                  90

Val Met Thr Ala Gln Lys Tyr Leu Gln Thr Arg Ala Val Ala Ala
                95                  100                 105

Tyr Arg Thr Trp Ser Lys Thr Ile Pro Gly Lys Val Gln Phe Phe
                110                 115                 120

Ser Ser Glu Gly Ser Asp Thr Ser Val Pro Ile Pro Val Val Pro
                125                 130                 135

Leu Arg Gly Val Asp Asp Ser Tyr Pro Pro Gln Lys Lys Ser Phe
                140                 145                 150

Met Met Leu Lys Tyr Met His Asp His Tyr Leu Asp Lys Tyr Glu
                155                 160                 165

Trp Phe Met Arg Ala Asp Asp Asp Val Tyr Ile Lys Gly Asp Arg
                170                 175                 180

Leu Glu Asn Phe Leu Arg Ser Leu Asn Ser Ser Glu Pro Leu Phe
                185                 190                 195

Leu Gly Gln Thr Gly Leu Gly Thr Thr Glu Glu Met Gly Lys Leu
                200                 205                 210

Ala Leu Glu Pro Gly Glu Asn Phe Cys Met Gly Gly Pro Gly Val
                215                 220                 225

Ile Met Ser Arg Glu Val Leu Arg Arg Met Val Pro His Ile Gly
                230                 235                 240

Lys Cys Leu Arg Glu Met Tyr Thr Thr His Glu Asp Val Glu Val
                245                 250                 255

Gly Arg Cys Val Arg Arg Phe Ala Gly Val Gln Cys Val Trp Ser
                260                 265                 270

Tyr Glu Met Gln Gln Leu Phe Tyr Glu Asn Tyr Glu Gln Asn Lys
                275                 280                 285

Lys Gly Tyr Ile Arg Asp Leu His Asn Ser Lys Ile His Gln Ala
                290                 295                 300

Ile Thr Leu His Pro Asn Lys Asn Pro Pro Tyr Gln Tyr Arg Leu
                305                 310                 315

His Ser Tyr Met Leu Ser Arg Lys Ile Ser Glu Leu Arg His Arg

                320                 325                 330

Thr Ile Gln Leu His Arg Glu Ile Val Leu Met Ser Lys Tyr Ser
                335                 340                 345

Asn Thr Glu Ile His Lys Glu Asp Leu Gln Leu Gly Ile Pro Pro
                350                 355                 360

Ser Phe Met Arg Phe Gln Pro Arg Gln Arg Glu Glu Ile Leu Glu
                365                 370                 375

Trp Glu Phe Leu Thr Gly Lys Tyr Leu Tyr Ser Ala Val Asp Gly
                380                 385                 390

Gln Pro Pro Arg Arg Gly Met Asp Ser Ala Gln Arg Glu Ala Leu
                395                 400                 405

Asp Asp Ile Val Met Gln Val Met Glu Met Ile Asn Ala Asn Ala
                410                 415                 420

Lys Thr Arg Gly Arg Ile Ile Asp Phe Lys Glu Ile Gln Tyr Gly
                425                 430                 435

Tyr Arg Arg Val Asn Pro Met Tyr Gly Ala Glu Tyr Ile Leu Asp
                440                 445                 450

Leu Leu Leu Leu Tyr Lys Lys His Lys Gly Lys Lys Met Thr Val
                455                 460                 465

Pro Val Arg Arg His Ala Tyr Leu Gln Gln Thr Phe Ser Lys Ile
                470                 475                 480

Gln Phe Val Glu His Glu Glu Leu Asp Ala Gln Glu Leu Ala Lys
                485                 490                 495

Arg Ile Asn Gln Glu Ser Gly Ser Leu Ser Phe Leu Ser Asn Ser
                500                 505                 510

Leu Lys Lys Leu Val Pro Phe Gln Leu Pro Gly Ser Lys Ser Glu
                515                 520                 525

His Lys Glu Pro Lys Asp Lys Lys Ile Asn Ile Leu Ile Pro Leu
                530                 535                 540

Ser Gly Arg Phe Asp Met Phe Val Arg Phe Met Gly Asn Phe Glu
                545                 550                 555

Lys Thr Cys Leu Ile Pro Asn Gln Asn Val Lys Leu Val Val Leu
                560                 565                 570

Leu Phe Asn Ser Asp Ser Asn Pro Asp Lys Ala Lys Gln Val Glu
                575                 580                 585

Leu Met Thr Asp Tyr Arg Ile Lys Tyr Pro Lys Ala Asp Met Gln
                590                 595                 600

Ile Leu Pro Val Ser Gly Glu Phe Ser Arg Ala Leu Ala Leu Glu
                605                 610                 615

Val Gly Ser Ser Gln Phe Asn Asn Glu Ser Leu Leu Phe Phe Cys
                620                 625                 630

Asp Val Asp Leu Val Phe Thr Thr Glu Phe Leu Gln Arg Cys Arg
                635                 640                 645

Ala Asn Thr Val Leu Gly Gln Gln Ile Tyr Phe Pro Ile Ile Phe
                650                 655                 660

Ser Gln Tyr Asp Pro Lys Ile Val Tyr Ser Gly Lys Val Pro Ser
                665                 670                 675

Asp Asn His Phe Ala Phe Thr Gln Lys Thr Gly Phe Trp Arg Asn
                680                 685                 690

Tyr Gly Phe Gly Ile Thr Cys Ile Tyr Lys Gly Asp Leu Val Arg
                695                 700                 705

Val Gly Gly Phe Asp Val Ser Ile Gln Gly Trp Gly Leu Glu Asp
                710                 715                 720

Val Asp Leu Phe Asn Lys Val Val Gln Ala Gly Leu Lys Thr Phe
                725                 730                 735

Arg Ser Gln Glu Val Gly Val Val His Val His His Pro Val Phe
                740                 745                 750

Cys Asp Pro Asn Leu Asp Pro Lys Gln Tyr Lys Met Cys Leu Gly
                755                 760                 765

Ser Lys Ala Ser Thr Tyr Gly Ser Thr Gln Gln Leu Ala Glu Met
                770                 775                 780

Trp Leu Glu Lys Asn Asp Pro Ser Tyr Ser Lys Ser Ser Asn Asn
                785                 790                 795

Asn Gly Ser Val Arg Thr Ala
                800

<210> 17
<211> 810
<212> DNA
<213> Homo sapiens

<400> 17
gctggagccg ggccggggcg atgtggagcg cgggccgcgg cggggctgcc 50

tggccggtgc tgttggggct gctgctggcg ctgttagtgc cgggcggtgg 100

tgccgccaag accggtgcgg agctcgtgac ctgcgggtcg gtgctgaagc 150

tgctcaatac gcaccaccgc gtgcggctgc actcgcacga catcaaatac 200

ggatccggca gcggccagca atcggtgacc ggcgtagagg cgtcggacga 250

cgcgaatagc tactggcgga tccgcggcgg ctcggagggc gggtgcccgt 300

gcgggtcccc ggtgcgctgc gggcaggcgg tgaggctcac gcatgtgctt 350

acgggcaaga acctgcacac gcaccacttc ccgtcgccgc tgtccaacaa 400

ccaggaggtg agtgcctttg gggaagacgg cgagggcgac gacctggacc 450

tatggacagt gcgctgctct ggacagcact gggagcgtga ggctgctgtg 500

cgcttacagc atgtgggcac ctctgtgttc ctgtcagtca cgggtgagca 550

gtatggaagc cccatccgtg ggcagcatga ggtccacggc atgcccagtg 600

ccaacacgca caatacgtgg aaggccatgg aaggcatctt catcaagcct 650

agtgtggagc cctctgcagg tcacgatgaa ctctgagtgt gtggatggat 700

gggtggatgg agggtggcag gtggggcgtc tgcagggcca ctcttggcag 750

agactttggg tttgtagggg tcctcaagtg cctttgtgat taaagaatgt 800

tggtctatga 810

<210> 18
<211> 221
<212> PRT
<213> Homo sapiens

<400> 18
Met Trp Ser Ala Gly Arg Gly Gly Ala Ala Trp Pro Val Leu Leu
1               5                   10                  15

Gly Leu Leu Leu Ala Leu Leu Val Pro Gly Gly Gly Ala Ala Lys
                20                  25                  30

Thr Gly Ala Glu Leu Val Thr Cys Gly Ser Val Leu Lys Leu Leu
                35                  40                  45

Asn Thr His His Arg Val Arg Leu His Ser His Asp Ile Lys Tyr
                50                  55                  60

Gly Ser Gly Ser Gly Gln Gln Ser Val Thr Gly Val Glu Ala Ser
                65                  70                  75

Asp Asp Ala Asn Ser Tyr Trp Arg Ile Arg Gly Gly Ser Glu Gly
                80                  85                  90

Gly Cys Pro Cys Gly Ser Pro Val Arg Cys Gly Gln Ala Val Arg
                95                  100                 105

Leu Thr His Val Leu Thr Gly Lys Asn Leu His Thr His His Phe
                110                 115                 120

Pro Ser Pro Leu Ser Asn Asn Gln Glu Val Ser Ala Phe Gly Glu
                125                 130                 135

Asp Gly Glu Gly Asp Asp Leu Asp Leu Trp Thr Val Arg Cys Ser
                140                 145                 150

Gly Gln His Trp Glu Arg Glu Ala Ala Val Arg Leu Gln His Val
                155                 160                 165

Gly Thr Ser Val Phe Leu Ser Val Thr Gly Glu Gln Tyr Gly Ser
                170                 175                 180

Pro Ile Arg Gly Gln His Glu Val His Gly Met Pro Ser Ala Asn
                185                 190                 195

Thr His Asn Thr Trp Lys Ala Met Glu Gly Ile Phe Ile Lys Pro
                200                 205                 210

Ser Val Glu Pro Ser Ala Gly His Asp Glu Leu
                215                 220

<210> 19
<211> 2292
<212> DNA
<213> Homo sapiens

<400> 19
tctcagggct tcatacagga aatctattgc tgtgtcaagt tccagagaaa 50

agcttctgtt cgtccaagtt actaaccagg ctaaaccaca tagacgtgaa 100

ggaaggggct agaaggaagg gagtgcccca ctgttgatgg ggtaagagga 150

tcctgtactg agaagttgac cagagagggt ctcaccatgc gcacagttcc 200

ttctgtacct gtgtggagga aaagtactga gtgaagggca gaaaaagaga 250

aaacagaaat gctctgccct tggagaactg ctaacctagg gctactgttg 300
attttgacta tcttcttagt ggccgaagcg gagggtgctg ctcaaccaaa 350
caactcatta atgctgcaaa ctagcaagga gaatcatgct ttagcttcaa 400
gcagtttatg tatggatgaa aaacagatta cacagaacta ctcgaaagta 450
ctcgcagaag ttaacacttc atggcctgta aagatggcta caaatgctgt 500
gctttgttgc cctcctatcg cattaagaaa tttgatcata ataacatggg 550
aaataatcct gagaggccag ccttcctgca caaaagccta caggaaagaa 600
acaaatgaga ccaaggaaac caactgtact gatgagagaa taacctgggt 650
ctccagacct gatcagaatt cggaccttca gattcgtcca gtggccatca 700
ctcatgacgg gtattacaga tgcataatgg taacacctga tgggaatttc 750
catcgtggat atcacctcca agtgttagtt acacctgaac tgaccctgtt 800
tcaaaacagg aatagaactg cagtatgcaa ggcagttgca gggaagccag 850
ctgcgcagat ctcctggatc ccagagggcg attgtgccac taagcaagaa 900
tactggagca atggcacagt gactgttaag agtacatgcc actgggaggt 950
ccacaatgtg tctaccgtga cctgccacgt ctcccatttg actggcaaca 1000
agagtctgta catagagcta cttcctgttc caggtgccaa aaaatcagca 1050
aaattatata ttccatatat catccttact attattattt tgaccatcgt 1100
gggattcatt tggttgttga aagtcaatgg ctgcagaaaa tataaattga 1150
ataaaacaga atctactcca gttgttgagg aggatgaaat gcagccctat 1200
gccagctaca cagagaagaa caatcctctc tatgatacta caaacaaggt 1250
gaaggcatct caggcattac aaagtgaagt tgacacagac ctccatactt 1300
tataagttgt tggactctag taccaagaaa caacaacaaa cgagatacat 1350
tataattact gtctgatttt cttacagttc tagaatgaag acttatattg 1400
aaattaggtt ttccaaggtt cttagaagac attttaatgg attctcattc 1450
atacccttgt ataattggaa tttttgattc ttagctgcta ccagctagtt 1500
ctctgaagaa ctgatgttat tacaaagaaa atacatgccc atgaccaaat 1550
attcaaattg tgcaggacag taaataatga aaaccaaatt tcctcaagaa 1600
ataactgaag aaggagcaag tgtgaacagt ttcttgtgta tcctttcaga 1650
atattttaat gtacatatga catgtgtata tgcctatggt atatgtgtca 1700
atttatgtgt ccccttacat atacatgcac atatctttgt caaggcacca 1750
gtgggaacaa tacactgcat tactgttcta tacatatgaa aacctaataa 1800
tataagtctt agagatcatt ttatatcatg acaagtagag ctacctcatt 1850
ctttttaatg gttatataaa attccattgt atagttatat cattatttaa 1900
ttaaaaacaa ccctaatgat ggatatttag attcttttaa gttttgttta 1950

tttcttttaa gttttgtttg tggtataaac aataccacat agaatgtttc 2000

ttgttcatat atctctttgt ttttgagtat atctgtagga taactttctt 2050

gagtggaatt gtcaggtcaa agggtttgtg cattttacta ttgatatata 2100

tgttaaattg tgtcaaatat atatgtcaaa ttccctccaa cattgtttaa 2150

atgtgccttt ccctaaattt ctattttaat aactgtacta ttcctgcttc 2200

tacagttgcc actttctctt tttaatcaac cagattaaat atgatgtgag 2250

attataataa gaattatact atttaataaa aatggattta ta 2292

<210> 20
<211> 348
<212> PRT
<213> Homo sapiens

<400> 20

<210> 21
<211> 3258
<212> DNA
<213> Homo sapiens

<400> 21
aaggctgtgg accccagaga aggtggcagg tggcccccct aggagagctc 50

tgggcacatt cgaatcttcc caaactccaa taataaaaat tcgaagactt 100

tggcagagag tgtgtgtgtg tgtgtatggt tgttgggcgt aggacaggtt 150

tcggggatgc gcggtacgcg gtaccacccc tcggaggccc ccacccccag 200

acgcccaggc cgcctcccca ctccccctca agcagcccca gccggggact 250

ttccgtcgcg gggaaggggc ggggaccctg agcgaaaggt gcggaggcgg 300

cctgccgggg tggttcggct tcccgttgcc gcctcgggcg ctgtacccag 350

agctcgaaga ggagcagcgc ggccgcgcgg acccggcaag gctgggccgg 400

actcggggct cccgagggac gccatgcggg gaggcagggg cgcccctttc 450

tggctgtggc cgctgcccaa gctggcgctg ctgcctctgt tgtgggtgct 500

tttccagcgg acgcgtcccc agggcagcgc cgggccactg cagtgctacg 550

gagttggacc cttgggcgac ttgaactgct cgtgggagcc tcttggggac 600

ctgggagccc cctccgagtt acacctccag agccaaaagt accgttccaa 650

caaaacccag actgtggcag tggcagccgg acggagctgg gtggccattc 700

ctcgggaaca gctcaccatg tctgacaaac tccttgtctg gggcactaag 750

gcaggccagc ctctctggcc ccccgtcttc gtgaacctag aaacccaaat 800

gaagccaaac gccccccggc tgggccctga cgtggacttt tccgaggatg 850

accccctgga ggccactgtc cattgggccc cacctacatg gccatctcat 900

aaagttctga tctgccagtt ccactaccga agatgtcagg aggcggcctg 950

tgtaatccca gcactttggc aggccaaggt ggaaggatca cttagagcta 2700

ggagtttgag accagcctgg gcaatatagc aagacccctc actacaaaaa 2750

taaaacatca aaaacaaaaa caattagctg ggcatgatgg cacacacctg 2800

tagtccgagc cacttgggag gctgaggtgg gaggatcggt tgagcccagg 2850

agttcgaagc tgcagggacc tctgattgca ccactgcact ccaggctggg 2900

taacagaatg agaccttatc tcaaaaataa acaaactaat aaaaagcaaa 2950

aaaaaaaaaa aaagaaaaga aaaaacactg catttgggca ccatctcagc 3000

tcccttgcat ccaggtgcag catggactga gttcttgaca acagaatgtg 3050

gtcagaagtg acatatgcca acacggggtc tgggtggggg ctcccccaca 3100

tcctttcctt gcctatgagc tggaacataa cacatgccta tgatccagct 3150

ttggtcatac ccaaggggaa ggtggagcaa gaaatgaaaa ggaacctgaa 3200

tccctgaatg actgcatgga tagaaccact aagaaaaata aacttttata 3250

ttttata 3258

<210> 22
<211> 636
<212> PRT
<213> Homo sapiens

<400> 22
Met Arg Gly Gly Arg Gly Ala Pro Phe Trp Leu Trp Pro Leu Pro
1               5                   10                  15

Lys Leu Ala Leu Leu Pro Leu Leu Trp Val Leu Phe Gln Arg Thr
                20                  25                  30

Arg Pro Gln Gly Ser Ala Gly Pro Leu Gln Cys Tyr Gly Val Gly
                35                  40                  45

Pro Leu Gly Asp Leu Asn Cys Ser Trp Glu Pro Leu Gly Asp Leu
                50                  55                  60

Gly Ala Pro Ser Glu Leu His Leu Gln Ser Gln Lys Tyr Arg Ser
                65                  70                  75

Asn Lys Thr Gln Thr Val Ala Val Ala Ala Gly Arg Ser Trp Val
                80                  85                  90

Ala Ile Pro Arg Glu Gln Leu Thr Met Ser Asp Lys Leu Leu Val
                95                  100                 105

Trp Gly Thr Lys Ala Gly Gln Pro Leu Trp Pro Pro Val Phe Val
                110                 115                 120

Asn Leu Glu Thr Gln Met Lys Pro Asn Ala Pro Arg Leu Gly Pro
                125                 130                 135

Asp Val Asp Phe Ser Glu Asp Asp Pro Leu Glu Ala Thr Val His
                140                 145                 150

Trp Ala Pro Pro Thr Trp Pro Ser His Lys Val Leu Ile Cys Gln
                155                 160                 165

Phe His Tyr Arg Arg Cys Gln Glu Ala Ala Trp Thr Leu Leu Glu
                170                 175                 180

Pro Glu Leu Lys Thr Ile Pro Leu Thr Pro Val Glu Ile Gln Asp
                185                 190                 195

Leu Glu Leu Ala Thr Gly Tyr Lys Val Tyr Gly Arg Cys Arg Met
                200                 205                 210

Glu Lys Glu Glu Asp Leu Trp Gly Glu Trp Ser Pro Ile Leu Ser
                215                 220                 225

Phe Gln Thr Pro Pro Ser Ala Pro Lys Asp Val Trp Val Ser Gly
                230                 235                 240

Asn Leu Cys Gly Thr Pro Gly Gly Glu Glu Pro Leu Leu Leu Trp
                245                 250                 255

Lys Ala Pro Gly Pro Cys Val Gln Val Ser Tyr Lys Val Trp Phe
                260                 265                 270

Trp Val Gly Gly Arg Glu Leu Ser Pro Glu Gly Ile Thr Cys Cys
                275                 280                 285

Cys Ser Leu Ile Pro Ser Gly Ala Glu Trp Ala Arg Val Ser Ala
                290                 295                 300

Val Asn Ala Thr Ser Trp Glu Pro Leu Thr Asn Leu Ser Leu Val
                305                 310                 315

Cys Leu Asp Ser Ala Ser Ala Pro Arg Ser Val Ala Val Ser Ser
                320                 325                 330

Ile Ala Gly Ser Thr Glu Leu Leu Val Thr Trp Gln Pro Gly Pro
                335                 340                 345

Gly Glu Pro Leu Glu His Val Val Asp Trp Ala Arg Asp Gly Asp
                350                 355                 360

Pro Leu Glu Lys Leu Asn Trp Val Arg Leu Pro Pro Gly Asn Leu
                365                 370                 375

Ser Ala Leu Leu Pro Gly Asn Phe Thr Val Gly Val Pro Tyr Arg
                380                 385                 390

Ile Thr Val Thr Ala Val Ser Ala Ser Gly Leu Ala Ser Ala Ser
                395                 400                 405

Ser Val Trp Gly Phe Arg Glu Glu Leu Ala Pro Leu Val Gly Pro
                410                 415                 420

Thr Leu Trp Arg Leu Gln Asp Ala Pro Pro Gly Thr Pro Ala Ile
                425                 430                 435

Ala Trp Gly Glu Val Pro Arg His Gln Leu Arg Gly His Leu Thr
                440                 445                 450

His Tyr Thr Leu Cys Ala Gln Ser Gly Thr Ser Pro Ser Val Cys
                455                 460                 465

Met Asn Val Ser Gly Asn Thr Gln Ser Val Thr Leu Pro Asp Leu
                470                 475                 480

Pro Trp Gly Pro Cys Glu Leu Trp Val Thr Ala Ser Thr Ile Ala
                485                 490                 495

Gly Gln Gly Pro Pro Gly Pro Ile Leu Arg Leu His Leu Pro Asp
                500                 505                 510

Asn Thr Leu Arg Trp Lys Val Leu Pro Gly Ile Leu Phe Leu Trp

                515                 520                 525

Gly Leu Phe Leu Leu Gly Cys Gly Leu Ser Leu Ala Thr Ser Gly
                530                 535                 540

Arg Cys Tyr His Leu Arg His Lys Val Leu Pro Arg Trp Val Trp
                545                 550                 555

Glu Lys Val Pro Asp Pro Ala Asn Ser Ser Ser Gly Gln Pro His
                560                 565                 570

Met Glu Gln Val Pro Glu Ala Gln Pro Leu Gly Asp Leu Pro Ile
                575                 580                 585

Leu Glu Val Glu Glu Met Glu Pro Pro Pro Val Met Glu Ser Ser
                590                 595                 600

Gln Pro Ala Gln Ala Thr Ala Pro Leu Asp Ser Gly Tyr Glu Lys
                605                 610                 615

His Phe Leu Pro Thr Pro Glu Glu Leu Gly Leu Leu Gly Pro Pro
                620                 625                 630

Arg Pro Gln Val Leu Ala
                635

<210> 23
<211> 1545
<212> DNA
<213> Homo sapiens

<400> 23
ggcacgaggg ctgcctggcg ctgcgggcgg cgggccatgg tggtttggat 50

tgagccgggc ccggccgggg cgccgagtcg gagggggtgg cagtgagcgg 100

cggcagaggc tacggggctc ggtttggctg actggggagt cggcaggcgg 150

caggaaccat gcgaggccag cggagcctgc tgctgggccc ggcccgcctc 200

tgcctccgcc tccttctgct gctgggttac aggcgccgct gtccacctct 250

actccggggt ctagtacagc gctggcgcta cggcaaggtc tgcctgcgct 300

ccctgctcta caactccttt gggggcagtg acaccgctgt tgatgctgcc 350

tttgagcctg tctactggct ggtagacaac gtgatccgct ggtttggagt 400

gggcaggaat gatatcgcca ccgtctccat ctgtaagaag tgcatttacc 450

ccaagccagc ccgaacacac cactgcagca tctgcaacag gtgtgtgctg 500

aagatggatc accactgccc ctggctaaac aattgtgtgg gccactataa 550

ccatcggtac ttcttctctt tctgcttttt catgactctg ggctgtgtct 600

actgcagcta tggaagttgg gaccttttcc gggaggctta tgctgccatt 650

gagacttatc accagacccc accacccacc ttctcctttc gagaaaggat 700

gactcacaag agtcttgtct acctctggtt cctgtgcagt tctgtggcac 750

ttgccctggg tgccctaact gtatggcatg ctgttctcat cagtcgaggt 800

gagactagca tcgaaaggca catcaacaag aaggagagac gtcggctaca 850

ggccaagggc agagtattta ggaatcctta caactacggc tgcttggaca 900

actggaaggt attcctgggt gtggatacag gaaggcactg gcttactcgg 950

gtgctcttac cttctagtca cttgccccat gggaatggaa tgagctggga 1000

gccccctccc tgggtgactg ctcactcagc ctctgtgatg gcagtgtgag 1050

ctggactgtg tcagccacga ctcgagcact cattctgctc cctatgttat 1100

ttcaagggcc tccaagggca gcttttctca gaatccttga tcaaaaagag 1150

ccagtgggcc tgccttaggg taccatgcag gacaattcaa ggaccagcct 1200

ttttaccact gcagaagaaa gacacaatgt ggagaaatct taggactgac 1250

atccctttac tcaggcaaac agaagttcca accccagact aggggtcagg 1300

cagctagcta cctaccttgc ccagtgctga cccggacctc ctccaggata 1350

cagcactgga gttggccacc acctcttcta cttgctgtct gaaaaaacac 1400

ctgactagta cagctgagat cttggcttct caacagggca aagataccag 1450

gcctgctgct gaggtcactg ccacttctca catgctgctt aagggagcac 1500

aaataaaggt attcgatttt taaagataaa aaaaaaaaaa aaaaa 1545

<210> 24
<211> 296
<212> PRT
<213> Homo sapiens

<400> 24
Met Arg Gly Gln Arg Ser Leu Leu Leu Gly Pro Ala Arg Leu Cys
1               5                   10                  15

Leu Arg Leu Leu Leu Leu Leu Gly Tyr Arg Arg Arg Cys Pro Pro
                20                  25                  30

Leu Leu Arg Gly Leu Val Gln Arg Trp Arg Tyr Gly Lys Val Cys
                35                  40                  45

Leu Arg Ser Leu Leu Tyr Asn Ser Phe Gly Gly Ser Asp Thr Ala
                50                  55                  60

Val Asp Ala Ala Phe Glu Pro Val Tyr Trp Leu Val Asp Asn Val
                65                  70                  75

Ile Arg Trp Phe Gly Val Gly Arg Asn Asp Ile Ala Thr Val Ser
                80                  85                  90

Ile Cys Lys Lys Cys Ile Tyr Pro Lys Pro Ala Arg Thr His His
                95                  100                 105

Cys Ser Ile Cys Asn Arg Cys Val Leu Lys Met Asp His His Cys
                110                 115                 120

Pro Trp Leu Asn Asn Cys Val Gly His Tyr Asn His Arg Tyr Phe
                125                 130                 135

Phe Ser Phe Cys Phe Phe Met Thr Leu Gly Cys Val Tyr Cys Ser
                140                 145                 150

Tyr Gly Ser Trp Asp Leu Phe Arg Glu Ala Tyr Ala Ala Ile Glu
                155                 160                 165

Thr Tyr His Gln Thr Pro Pro Pro Thr Phe Ser Phe Arg Glu Arg
                170                 175                 180

Met Thr His Lys Ser Leu Val Tyr Leu Trp Phe Leu Cys Ser Ser
                185                 190                 195

Val Ala Leu Ala Leu Gly Ala Leu Thr Val Trp His Ala Val Leu
                200                 205                 210

Ile Ser Arg Gly Glu Thr Ser Ile Glu Arg His Ile Asn Lys Lys
                215                 220                 225

Glu Arg Arg Arg Leu Gln Ala Lys Gly Arg Val Phe Arg Asn Pro
                230                 235                 240

Tyr Asn Tyr Gly Cys Leu Asp Asn Trp Lys Val Phe Leu Gly Val
                245                 250                 255

Asp Thr Gly Arg His Trp Leu Thr Arg Val Leu Leu Pro Ser Ser
                260                 265                 270

His Leu Pro His Gly Asn Gly Met Ser Trp Glu Pro Pro Pro Trp
                275                 280                 285

Val Thr Ala His Ser Ala Ser Val Met Ala Val
                290                 295

<210> 25
<211> 1256
<212> DNA
<213> Homo sapiens

<400> 25
acgaggggag ctccggctgc gtcttcccgc agcgctaccc gccatgcgcc 50

tgccgcgccg ggccgcgctg gggctcctgc cgcttctgct gctgctgccg 100

cccgcgccgg aggccgccaa gaagccgacg ccctgccacc ggtgccgggg 150

gctggtggac aagtttaacc aggggatggt ggacaccgca aagaagaact 200

ttggcggcgg gaacacggct tgggaggaaa agacgctgtc caagtacgag 250

tccagcgaga ttcgcctgct ggagatcctg gaggggctgt gcgagagcag 300

cgacttcgaa tgcaatcaga tgctagaggc gcaggaggag cacctggagg 350

cctggtggct gcagctgaag agcgaatatc ctgacttatt cgagtggttt 400

tgtgtgaaga cactgaaagt gtgctgctct ccaggaacct acggtcccga 450

ctgtctcgca tgccagggcg gatcccagag gccctgcagc gggaatggcc 500

actgcagcgg agatgggagc agacagggcg acgggtcctg ccggtgccac 550

atggggtacc agggcccgct gtgcactgac tgcatggacg gctacttcag 600

ctcgctccgg aacgagaccc acagcatctg cacagcctgt gacgagtcct 650

gcaagacgtg ctcgggcctg accaacagag actgcggcga gtgtgaagtg 700

ggctgggtgc tggacgaggg cgcctgtgtg gatgtggacg agtgtgcggc 750

cgagccgcct ccctgcagcg ctgcgcagtt ctgtaagaac gccaacggct 800

cctacacgtg cgaagatgtg gacgagtgct cactagcaga aaaaacctgt 850

gtgaggaaaa acgaaaactg ctacaatact ccagggagct acgtctgtgt 900

gtgtcctgac ggcttcgaag aaacggaaga tgcctgtgtg ccgccggcag 950

aggctgaagc cacagaagga gaaagcccga cacagctgcc ctcccgcgaa 1000

gacctgtaat gtgccggact taccctttaa attattcaga aggatgtccc 1050

gtggaaaatg tggccctgag gatgccgtct cctgcagtgg acagcggcgg 1100

ggagaggctg cctgctctct aacggttgat tctcatttgt cccttaaaca 1150

gctgcatttc ttggttgttc ttaaacagac ttgtatattt tgatacagtt 1200

ctttgtaata aaattgacca ttgtaggtaa tcaggaaaaa aaaaaaaaaa 1250

aaaaaa 1256

<210> 26
<211> 321
<212> PRT
<213> Homo sapiens

<400> 26
Met Arg Leu Pro Arg Arg Ala Ala Leu Gly Leu Leu Pro Leu Leu
1               5                   10                  15

Leu Leu Leu Pro Pro Ala Pro Glu Ala Ala Lys Lys Pro Thr Pro
                20                  25                  30

Cys His Arg Cys Arg Gly Leu Val Asp Lys Phe Asn Gln Gly Met
                35                  40                  45

Val Asp Thr Ala Lys Lys Asn Phe Gly Gly Gly Asn Thr Ala Trp
                50                  55                  60

Glu Glu Lys Thr Leu Ser Lys Tyr Glu Ser Ser Glu Ile Arg Leu
                65                  70                  75

Leu Glu Ile Leu Glu Gly Leu Cys Glu Ser Ser Asp Phe Glu Cys
                80                  85                  90

Asn Gln Met Leu Glu Ala Gln Glu Glu His Leu Glu Ala Trp Trp
                95                  100                 105

Leu Gln Leu Lys Ser Glu Tyr Pro Asp Leu Phe Glu Trp Phe Cys
                110                 115                 120

Val Lys Thr Leu Lys Val Cys Cys Ser Pro Gly Thr Tyr Gly Pro
                125                 130                 135

Asp Cys Leu Ala Cys Gln Gly Gly Ser Gln Arg Pro Cys Ser Gly
                140                 145                 150

Asn Gly His Cys Ser Gly Asp Gly Ser Arg Gln Gly Asp Gly Ser
                155                 160                 165

Cys Arg Cys His Met Gly Tyr Gln Gly Pro Leu Cys Thr Asp Cys
                170                 175                 180

Met Asp Gly Tyr Phe Ser Ser Leu Arg Asn Glu Thr His Ser Ile
                185                 190                 195

Cys Thr Ala Cys Asp Glu Ser Cys Lys Thr Cys Ser Gly Leu Thr
                200                 205                 210

Asn Arg Asp Cys Gly Glu Cys Glu Val Gly Trp Val Leu Asp Glu
                215                 220                 225

Gly Ala Cys Val Asp Val Asp Glu Cys Ala Ala Glu Pro Pro Pro
                230                 235                 240

Cys Ser Ala Ala Gln Phe Cys Lys Asn Ala Asn Gly Ser Tyr Thr
                245                 250                 255

Cys Glu Asp Val Asp Glu Cys Ser Leu Ala Glu Lys Thr Cys Val
                260                 265                 270

Arg Lys Asn Glu Asn Cys Tyr Asn Thr Pro Gly Ser Tyr Val Cys
                275                 280                 285

Val Cys Pro Asp Gly Phe Glu Glu Thr Glu Asp Ala Cys Val Pro
                290                 295                 300

Pro Ala Glu Ala Glu Ala Thr Glu Gly Glu Ser Pro Thr Gln Leu
                305                 310                 315

Pro Ser Arg Glu Asp Leu
                320

<210> 27
<211> 1835
<212> DNA
<213> Homo sapiens

<400> 27
gtgcagttgc ggctccaggg ccatggcgga ggagcagggc cgggaacggg 50

actcggttcc caagccgtcg gtgctgttcc tccacccaga cctgggcgtg 100

ggcggcgctg agcggctggt gttggacgcg gcgctggcgc tgcaggcgcg 150

cgggtgtagc gtgaagatct ggacagcgca ctacgacccg ggccactgtt 200

tcgccgagag ccgcgagcta ccggtgcgct gtgccgggga ctggctgccg 250

cgaggcctgg gctggggcgg ccgcggcgcc gccgtctgcg cctacgtgcg 300

catggttttc ctggcgctct acgtgctgtt cctcgccgac gaggagttcg 350

acgtggtagt gtgcgaccag gtgtctgcct gtatcccagt gttcaggctg 400

gctagacggc ggaagaagat cctattttac tgtcacttcc cagatctgct 450

tctcaccaag agagattctt ttcttaaacg actatacagg gccccaattg 500

actggataga ggaatacacc acaggcatgg cagactgcat cttagtcaac 550

agccagttca cagctgctgt ttttaaggaa acattcaagt ccctgtctca 600

catagaccct gatgtcctct atccatctct aaatgtcacc agctttgact 650

cagttgttcc tgaaaagctg gatgacctag tccccaaggg gaaaaaattc 700

ctgctgctct ccatcaacag atacgaaagg aagaaaaatc tgactttggc 750

actggaagcc ctagtacagc tgcgtggaag attgacatcc caagattggg 800

agagggttca tctgatcgtg gcaggtggtt atgacgagag agtcctggag 850

aatgtggaac attatcagga attgaagaaa atggtccaac agtccgacct 900

tggccagtat gtgaccttct tgaggtcttt ctcagacaaa cagaaaatct 950

ccctcctcca cagctgcacg tgtgtgcttt acacaccaag caatgagcac 1000

tttggcattg tccctctgga agccatgtac atgcagtgcc cagtcattgc 1050

tgttaattcg ggtggaccct tggagtccat tgaccacagt gtcacagggt 1100

ttctgtgtga gcctgacccg gtgcacttct cagaagcaat agaaaagttc 1150

atccgtgaac cttccttaaa agccaccatg ggcctggctg gaagagccag 1200

agtgaaggaa aaattttccc ctgaagcatt tacagaacag ctctaccgat 1250

atgttaccaa actgctggta taatcagatt gtttttaaga tctccattaa 1300

tgtcattttt atggattgta gacccagttt tgaaaccaaa aaagaaacct 1350

agaatctaat gcagaagaga tcttttaaaa aataaacttg agtcttgaat 1400

gtgagccact ttcctatata ccacacctcc ctgtccactt ttcagaaaaa 1450

ccatgtcttt tatgctataa tcattccaaa ttttgccagt gttaagttac 1500

aaatgtggtg tcattccatg ttcagcagag tattttaatt atattttctc 1550

gggattattg ctcttctgtc tataaatttt gaatgatact gtgccttaat 1600

tggttttcat agtttaagtg tgtatcatta tcaaagttga ttaatttggc 1650

ttcatagtat aatgagagca gggctattgt agttcccaga ttcaatccac 1700

cgaagtgttc actgtcatct gttagggaat ttttgtttgt cctgtctttg 1750

cctggatcca tagcgagagt gctctgtatt ttttttaaga taatttgtat 1800

ttttgcacac tgagatataa taaaaggtgt ttatc 1835

<210> 28
<211> 416
<212> PRT
<213> Homo sapiens

<400> 28
Met Ala Glu Glu Gln Gly Arg Glu Arg Asp Ser Val Pro Lys Pro
1               5                   10                  15

Ser Val Leu Phe Leu His Pro Asp Leu Gly Val Gly Gly Ala Glu
                20                  25                  30

Arg Leu Val Leu Asp Ala Ala Leu Ala Leu Gln Ala Arg Gly Cys
                35                  40                  45

Ser Val Lys Ile Trp Thr Ala His Tyr Asp Pro Gly His Cys Phe
                50                  55                  60

Ala Glu Ser Arg Glu Leu Pro Val Arg Cys Ala Gly Asp Trp Leu
                65                  70                  75

Pro Arg Gly Leu Gly Trp Gly Gly Arg Gly Ala Ala Val Cys Ala
                80                  85                  90

Tyr Val Arg Met Val Phe Leu Ala Leu Tyr Val Leu Phe Leu Ala
                95                  100                 105

Asp Glu Glu Phe Asp Val Val Val Cys Asp Gln Val Ser Ala Cys
                110                 115                 120

Ile Pro Val Phe Arg Leu Ala Arg Arg Arg Lys Lys Ile Leu Phe
                125                 130                 135

Tyr Cys His Phe Pro Asp Leu Leu Leu Thr Lys Arg Asp Ser Phe
                140                 145                 150

Leu Lys Arg Leu Tyr Arg Ala Pro Ile Asp Trp Ile Glu Glu Tyr
                155                 160                 165

Thr Thr Gly Met Ala Asp Cys Ile Leu Val Asn Ser Gln Phe Thr
                170                 175                 180

Ala Ala Val Phe Lys Glu Thr Phe Lys Ser Leu Ser His Ile Asp
                185                 190                 195

Pro Asp Val Leu Tyr Pro Ser Leu Asn Val Thr Ser Phe Asp Ser
                200                 205                 210

Val Val Pro Glu Lys Leu Asp Asp Leu Val Pro Lys Gly Lys Lys
                215                 220                 225

Phe Leu Leu Leu Ser Ile Asn Arg Tyr Glu Arg Lys Lys Asn Leu
                230                 235                 240

Thr Leu Ala Leu Glu Ala Leu Val Gln Leu Arg Gly Arg Leu Thr
                245                 250                 255

Ser Gln Asp Trp Glu Arg Val His Leu Ile Val Ala Gly Gly Tyr
                260                 265                 270

Asp Glu Arg Val Leu Glu Asn Val Glu His Tyr Gln Glu Leu Lys
                275                 280                 285

Lys Met Val Gln Gln Ser Asp Leu Gly Gln Tyr Val Thr Phe Leu
                290                 295                 300

Arg Ser Phe Ser Asp Lys Gln Lys Ile Ser Leu Leu His Ser Cys
                305                 310                 315

Thr Cys Val Leu Tyr Thr Pro Ser Asn Glu His Phe Gly Ile Val
                320                 325                 330

Pro Leu Glu Ala Met Tyr Met Gln Cys Pro Val Ile Ala Val Asn
                335                 340                 345

Ser Gly Gly Pro Leu Glu Ser Ile Asp His Ser Val Thr Gly Phe
                350                 355                 360

Leu Cys Glu Pro Asp Pro Val His Phe Ser Glu Ala Ile Glu Lys
                365                 370                 375

Phe Ile Arg Glu Pro Ser Leu Lys Ala Thr Met Gly Leu Ala Gly
                380                 385                 390

Arg Ala Arg Val Lys Glu Lys Phe Ser Pro Glu Ala Phe Thr Glu
                395                 400                 405

Gln Leu Tyr Arg Tyr Val Thr Lys Leu Leu Val
                410                 415

<210> 29
<211> 1032
<212> DNA
<213> Homo sapiens

<400> 29
gttatttatt gacttttgcc aaggcttggt cacaacaatc atattcacgt 50

aattttcccc ctttggtggc agaactgtag caataggggg agaagacaag 100

cagcggatga agcgttttct cagcttttgg aattgcttcg acctgacatc 150

cgttgtaacc gtttgccact tcttcagata tttttataaa aaagtaccac 200

tgagtcagtg agggccacag attggtatta atgagatacg agggttgttg 250

ctgggtgttt gtttcctgag ctaagtgatc aagactgtag tggagttgca 300

gctaacatgg gttaggttta aaccgtgggg gatgcaaccc ctttgcgttt 350

catatgtagg cctactggct ttgtgtagct ggagtagttg ggttgctttg 400

tgttaggagg atccagatca tgttggctac agggagatgc tctctttgag 450

aggctcctgg gcattgattc catttcaatc tcattctgga tatgtgttca 500

ttgagtaaag gaggagagac cctcatacgc tatttaaatg tcactttttt 550

gcctatgccc cgttttttgg tcatgtttca attaattgtg aggaaggcgc 600

agctcctctc tgcacgtaga tcatttttta aagctaatgt aagcacatct 650

aagggaataa catgatttaa ggttgaaatg gctttagaat catttgggtt 700

tgagggtgtg ttattttgag tcatgaatgt acaagctctg tgaatcagac 750

cagcttaaat acccacacct ttttttcgta ggtgggcttt tcctatcaga 800

gcttggctca taaccaaata aagttttttg aaggccatgg cttttcacac 850

agttatttta ttttatgacg ttatctgaaa gcagactgtt aggagcagta 900

ttgagtggct gtcacacttt gaggcaacta aaaaggcttc aaacgttttg 950

atcagtttct tttcaggaaa cattgtgctc taacagtatg actattcttt 1000

cccccactct taaacagtgt gatgtgtgtt at 1032

<210> 30
<211> 57
<212> PRT
<213> Homo sapiens

<400> 30
Met Cys Ser Leu Ser Lys Gly Gly Glu Thr Leu Ile Arg Tyr Leu
1               5                   10                  15

Asn Val Thr Phe Leu Pro Met Pro Arg Phe Leu Val Met Phe Gln
                20                  25                  30

Leu Ile Val Arg Lys Ala Gln Leu Leu Ser Ala Arg Arg Ser Phe
                35                  40                  45

Phe Lys Ala Asn Val Ser Thr Ser Lys Gly Ile Thr
                50                  55

<210> 31
<211> 1131
<212> DNA
<213> Homo sapiens

<400> 31
aaaagcgagt gaagagagcg cgacggcggc ggcggcggcg gcgcagctat 50

tgctggacgg ccagtgggag agcgaggcct gagcctctgc gtctaggatc 100

aaaatggttt caatcccaga atactatgaa ggcaagaacg tcctcctcac 150

aggagctacc ggttttctag ggaaggtgct tctggaaaag ttgctgaggt 200

cttgtcctaa ggtgaattca gtatatgttt tggtgaggca gaaagctgga 250

cagacaccac aagagcgagt ggaagaagtc cttagtggca agctttttga 300

cagattgaga gatgaaaatc cagattttag agagaaaatt atagcaatca 350

acagcgaact cacccaacct aaactggctc tcagtgaaga agataaagag 400

gtgatcatag attctaccaa tattatattc cactgtgcag ctacagtaag 450

gtttaatgaa aatttaaggg atgctgttca gttaaatgtg attgcaacgc 500

gacagcttat tctccttgca caacaaatga agaatctgga agtgttcatg 550

catgtatcaa cagcatatgc ctactgtaat cgcaagcata ttgatgaagt 600

agtctatcca ccacctgtgg atcccaagaa gctgattgat tctttagagt 650

ggatggatga tggcctagta aatgatatca cgccaaaatt gataggagac 700

agacctaata catacatata cacaaaagca ttggcagaat atgttgtaca 750

acaagaagga gcaaaactaa atgtggcaat tgtaaggcca tcgattgttg 800

gtgccagttg gaaagaacct tttccaggat ggattgataa ctttaatgga 850

ccaagtggtc tctttattgc ggcagggaaa ggaattcttc gaacaatacg 900

tgcctccaac aatgcccttg cagatcttgt tcctgtagat gtagttgtca 950

acatgagtct tgcggcagcc tggtattccg gagttaatag accaagaaac 1000

atcatggtgt ataattgtac aacaggcagc actaatcctt tccactgggg 1050

tgaagttggt atgattttac ctgtgttttt gaatgttaga ataaatctta 1100

aagaaccaaa aaaaaaaaaa aaaaaaaaaa a 1131

<210> 32
<211> 341
<212> PRT
<213> Homo sapiens

<400> 32
Met Val Ser Ile Pro Glu Tyr Tyr Glu Gly Lys Asn Val Leu Leu
1               5                   10                  15

Thr Gly Ala Thr Gly Phe Leu Gly Lys Val Leu Leu Glu Lys Leu
                20                  25                  30

Leu Arg Ser Cys Pro Lys Val Asn Ser Val Tyr Val Leu Val Arg
                35                  40                  45

Gln Lys Ala Gly Gln Thr Pro Gln Glu Arg Val Glu Glu Val Leu
                50                  55                  60

Ser Gly Lys Leu Phe Asp Arg Leu Arg Asp Glu Asn Pro Asp Phe
                65                  70                  75

Arg Glu Lys Ile Ile Ala Ile Asn Ser Glu Leu Thr Gln Pro Lys
                80                  85                  90

Leu Ala Leu Ser Glu Glu Asp Lys Glu Val Ile Ile Asp Ser Thr
                95                  100                 105

Asn Ile Ile Phe His Cys Ala Ala Thr Val Arg Phe Asn Glu Asn
                110                 115                 120

Leu Arg Asp Ala Val Gln Leu Asn Val Ile Ala Thr Arg Gln Leu
                125                 130                 135

Ile Leu Leu Ala Gln Gln Met Lys Asn Leu Glu Val Phe Met His

                140                 145                 150

Val Ser Thr Ala Tyr Ala Tyr Cys Asn Arg Lys His Ile Asp Glu
                155                 160                 165

Val Val Tyr Pro Pro Pro Val Asp Pro Lys Lys Leu Ile Asp Ser
                170                 175                 180

Leu Glu Trp Met Asp Asp Gly Leu Val Asn Asp Ile Thr Pro Lys
                185                 190                 195

Leu Ile Gly Asp Arg Pro Asn Thr Tyr Ile Tyr Thr Lys Ala Leu
                200                 205                 210

Ala Glu Tyr Val Val Gln Gln Glu Gly Ala Lys Leu Asn Val Ala
                215                 220                 225

Ile Val Arg Pro Ser Ile Val Gly Ala Ser Trp Lys Glu Pro Phe
                230                 235                 240

Pro Gly Trp Ile Asp Asn Phe Asn Gly Pro Ser Gly Leu Phe Ile
                245                 250                 255

Ala Ala Gly Lys Gly Ile Leu Arg Thr Ile Arg Ala Ser Asn Asn
                260                 265                 270

Ala Leu Ala Asp Leu Val Pro Val Asp Val Val Val Asn Met Ser
                275                 280                 285

Leu Ala Ala Ala Trp Tyr Ser Gly Val Asn Arg Pro Arg Asn Ile
                290                 295                 300

Met Val Tyr Asn Cys Thr Thr Gly Ser Thr Asn Pro Phe His Trp
                305                 310                 315

Gly Glu Val Gly Met Ile Leu Pro Val Phe Leu Asn Val Arg Ile
                320                 325                 330

Asn Leu Lys Glu Pro Lys Lys Lys Lys Lys Lys
                335                 340

<210> 33
<211> 727
<212> DNA
<213> Homo sapiens

<400> 33
gaacgagggt cctagctgcc gccacccgaa cagcctgtcc tggtgccccg 50

gctccctgcc ccgcgcccag tcatgaccct gcgcccctca ctcctcccgc 100

tccatctgct gctgctgctg ctgctcagtg cggcggtgtg ccgggctgag 150

gctgggctcg aaaccgaaag tcccgtccgg accctccaag tggagaccct 200

ggtggagccc ccagaaccat gtgccgagcc cgctgctttt ggagacacgc 250

ttcacataca ctacacggga agcttggtag atggacgtat tattgacacc 300

tccctgacca gagaccctct ggttatagaa cttggccaaa agcaggtgat 350

tccaggtctg gagcagagtc ttctcgacat gtgtgtggga gagaagcgaa 400

gggcaatcat tccttctcac ttggcctatg gaaaacgggg atttccacca 450

tctgtcccag cggatgcagt ggtgcagtat gacgtggagc tgattgcact 500

aatccgagcc aactactggc taaagctggt gaagggcatt ttgcctctgg 550

tagggatggc catggtgcca gccctcctgg gcctcattgg gtatcaccta 600

tacagaaagg ccaatagacc caaagtctcc aaaaagaagc tcaaggaaga 650

gaaacgaaac aagagcaaaa agaaataata aataataaat tttaaaaaac 700

ttaaaaaaaa aaaaaaaaaa aaaaaaa 727

<210> 34
<211> 201
<212> PRT
<213> Homo sapiens

<400> 34
Met Thr Leu Arg Pro Ser Leu Leu Pro Leu His Leu Leu Leu Leu
1               5                   10                  15

Leu Leu Leu Ser Ala Ala Val Cys Arg Ala Glu Ala Gly Leu Glu
                20                  25                  30

Thr Glu Ser Pro Val Arg Thr Leu Gln Val Glu Thr Leu Val Glu
                35                  40                  45

Pro Pro Glu Pro Cys Ala Glu Pro Ala Ala Phe Gly Asp Thr Leu
                50                  55                  60

His Ile His Tyr Thr Gly Ser Leu Val Asp Gly Arg Ile Ile Asp
                65                  70                  75

Thr Ser Leu Thr Arg Asp Pro Leu Val Ile Glu Leu Gly Gln Lys
                80                  85                  90

Gln Val Ile Pro Gly Leu Glu Gln Ser Leu Leu Asp Met Cys Val
                95                  100                 105

Gly Glu Lys Arg Arg Ala Ile Ile Pro Ser His Leu Ala Tyr Gly
                110                 115                 120

Lys Arg Gly Phe Pro Pro Ser Val Pro Ala Asp Ala Val Val Gln
                125                 130                 135

Tyr Asp Val Glu Leu Ile Ala Leu Ile Arg Ala Asn Tyr Trp Leu
                140                 145                 150

Lys Leu Val Lys Gly Ile Leu Pro Leu Val Gly Met Ala Met Val
                155                 160                 165

Pro Ala Leu Leu Gly Leu Ile Gly Tyr His Leu Tyr Arg Lys Ala
                170                 175                 180

Asn Arg Pro Lys Val Ser Lys Lys Lys Leu Lys Glu Glu Lys Arg
                185                 190                 195

Asn Lys Ser Lys Lys Lys
                200

<210> 35
<211> 1080
<212> DNA
<213> Homo sapiens

<400> 35
cctattctac ggctgacccc tggtggtcac gtggatctgt tcgccacgca 50

agtctgggtc cttcggcgat tgaccggggt ccttgctgtt cgggagcctc 100

tcctaagctg cctgttcgcg cgagagtttg gaggggcggg tttggggtcg 150

gtgtctgatt ggggctcgca ccgcagcacg ctggagtccc gcttaggtac 200

cagttagcgt caggggagct gggtcaggcg gtcgccggga caccccgtgt 250

gtggcaggcg gcgaagcgct ctggagaatc ccggacagcc ctgctccctg 300

cagccaggtg tagtttcggg agccactggg gccaaagtga gagtccagcg 350

gtcttccagc gcttgggcca cggcggcggc cctgggagca gaggtggagc 400

gaccccatta cgctaaagat gaaaggctgg ggttggctgg ccctgcttct 450

gggggccctg ctgggaaccg cctgggctcg gaggagccag gatctccact 500

gtggagcatg cagggctctg gtggatgaac tagaatggga aattgcccag 550

gtggacccca agaagaccat tcagatggga tctttccgga tcaatccaga 600

tggcagccag tcagtggtgg aggtgcctta tgcccgctca gaggcccacc 650

tcacagagct gctggaggag atatgtgacc ggatgaagga gtatggggaa 700

cagattgatc cttccaccca tcgcaagaac tacgtacgtg tagtgggccg 750

gaatggagaa tccagtgaac tggacctaca aggcatccga atcgactcag 800

atattagcgg caccctcaag tttgcgtgtg agagcattgt ggaggaatac 850

gaggatgaac tcattgaatt cttttcccga gaggctgaca atgttaaaga 900

caaactttgc agtaagcgaa cagatctttg tgaccatgcc ctgcacatat 950

cgcatgatga gctatgaacc actggagcag cccacactgg cttgatggat 1000

cacccccagg aggggaaaat ggtggcaatg ccttttatat attatgtttt 1050

tactgaaatt aactgaaaaa atatgaaacc 1080

<210> 36
<211> 182
<212> PRT
<213> Homo sapiens

<400> 36
Met Lys Gly Trp Gly Trp Leu Ala Leu Leu Leu Gly Ala Leu Leu
1               5                   10                  15

Gly Thr Ala Trp Ala Arg Arg Ser Gln Asp Leu His Cys Gly Ala
                20                  25                  30

Cys Arg Ala Leu Val Asp Glu Leu Glu Trp Glu Ile Ala Gln Val
                35                  40                  45

Asp Pro Lys Lys Thr Ile Gln Met Gly Ser Phe Arg Ile Asn Pro
                50                  55                  60

Asp Gly Ser Gln Ser Val Val Glu Val Pro Tyr Ala Arg Ser Glu
                65                  70                  75

Ala His Leu Thr Glu Leu Leu Glu Glu Ile Cys Asp Arg Met Lys
                80                  85                  90

Glu Tyr Gly Glu Gln Ile Asp Pro Ser Thr His Arg Lys Asn Tyr
                95                  100                 105

Val Arg Val Val Gly Arg Asn Gly Glu Ser Ser Glu Leu Asp Leu
                110                 115                 120

Gln Gly Ile Arg Ile Asp Ser Asp Ile Ser Gly Thr Leu Lys Phe
                125                 130                 135

Ala Cys Glu Ser Ile Val Glu Glu Tyr Glu Asp Glu Leu Ile Glu
                140                 145                 150

Phe Phe Ser Arg Glu Ala Asp Asn Val Lys Asp Lys Leu Cys Ser
                155                 160                 165

Lys Arg Thr Asp Leu Cys Asp His Ala Leu His Ile Ser His Asp
                170                 175                 180

Glu Leu

<210> 37
<211> 1169
<212> DNA
<213> Homo sapiens

<400> 37
gaggttgaag gacccaggcg tgtcagccct gctccagaga ccttgggcat 50

ggaggagagt gtcgtacggc cctcagtgtt tgtggtggat ggacagaccg 100

acatcccatt cacgaggctg ggacgaagcc accggagaca gtcgtgcagt 150

gtggcccggg tgggtctggg tctcttgctg ttgctgatgg gggctgggct 200

ggccgtccaa ggctggttcc tcctgcagct gcactggcgt ctaggagaga 250

tggtcacccg cctgcctgac ggacctgcag gctcctggga gcagctgata 300

caagagcgaa ggtctcacga ggtcaaccca gcagcgcatc tcacaggggc 350

caactccagc ttgaccggca gcgggggccc gctgttatgg gagactcagc 400

tgggcctggc cttcctgagg ggcctcagct accacgatgg ggcccttgtg 450

gtcaccaaag ctggctacta ctacatctac tccaaggtgc agctgggcgg 500

tgtgggctgc ccgctgggcc tggccagcac catcacccac ggcctctaca 550

agcgcacacc ccgctacccc gaggagctgg agctgttggt cagccagcag 600

tcaccctgcg gacgggccac cagcagctcc cgggtctggt gggacagcag 650

cttcctgggt ggtgtggtac acctggaggc tggggaggag gtggtcgtcc 700

gtgtgctgga tgaacgcctg gttcgactgc gtgatggtac ccggtcttac 750

ttcggggctt tcatggtgtg aaggaaggag cgtggtgcat tggacatggg 800

tctgacacgt ggagaactca gagggtgcct caggggaaag aaaactcacg 850

aagcagaggc tgggcgtggt ggctctcgcc tgtaatccca gcactttggg 900

aggccaaggc aggcggatca cctgaggtca ggagttcgag accagcctgg 950

ctaacatggc aaaaccccat ctctactaaa aatacaaaaa ttagccggac 1000

gtggtggtgc ctgcctgtaa tccagctact caggaggctg aggcaggata 1050

attttgctta aacccgggag gcggaggttg cagtgagccg agatcacacc 1100

actgcactcc aacctgggaa acgcagtgag actgtgcctc aaaaaaaaaa 1150

aaaaaaaaaa aaaaaaaaa 1169

<210> 38
<211> 240
<212> PRT
<213> Homo sapiens

<400> 38
Met Glu Glu Ser Val Val Arg Pro Ser Val Phe Val Val Asp Gly
1               5                   10                  15

Gln Thr Asp Ile Pro Phe Thr Arg Leu Gly Arg Ser His Arg Arg
                20                  25                  30

Gln Ser Cys Ser Val Ala Arg Val Gly Leu Gly Leu Leu Leu Leu
                35                  40                  45

Leu Met Gly Ala Gly Leu Ala Val Gln Gly Trp Phe Leu Leu Gln
                50                  55                  60

Leu His Trp Arg Leu Gly Glu Met Val Thr Arg Leu Pro Asp Gly
                65                  70                  75

Pro Ala Gly Ser Trp Glu Gln Leu Ile Gln Glu Arg Arg Ser His
                80                  85                  90

Glu Val Asn Pro Ala Ala His Leu Thr Gly Ala Asn Ser Ser Leu
                95                  100                 105

Thr Gly Ser Gly Gly Pro Leu Leu Trp Glu Thr Gln Leu Gly Leu
                110                 115                 120

Ala Phe Leu Arg Gly Leu Ser Tyr His Asp Gly Ala Leu Val Val
                125                 130                 135

Thr Lys Ala Gly Tyr Tyr Tyr Ile Tyr Ser Lys Val Gln Leu Gly
                140                 145                 150

Gly Val Gly Cys Pro Leu Gly Leu Ala Ser Thr Ile Thr His Gly
                155                 160                 165

Leu Tyr Lys Arg Thr Pro Arg Tyr Pro Glu Glu Leu Glu Leu Leu
                170                 175                 180

Val Ser Gln Gln Ser Pro Cys Gly Arg Ala Thr Ser Ser Ser Arg
                185                 190                 195

Val Trp Trp Asp Ser Ser Phe Leu Gly Gly Val Val His Leu Glu
                200                 205                 210

Ala Gly Glu Glu Val Val Val Arg Val Leu Asp Glu Arg Leu Val
                215                 220                 225

Arg Leu Arg Asp Gly Thr Arg Ser Tyr Phe Gly Ala Phe Met Val
                230                 235                 240

<210> 39
<211> 1937
<212> DNA
<213> Homo sapiens

<400> 39
ggcacgaggg gagtggaaag ttctccggca gccctgagat ctcaagagtg 50

acatttgtga gaccagctaa tttgattaaa attctcttgg aatcagcttt 100

gctagtatca tacctgtgcc agatttcatc atgggaaaca gctgttacaa 150

catagtagcc actctgttgc tggtcctcaa ctttgagagg acaagatcat 200
tgcaggatcc ttgtagtaac tgcccagctg gtacattctg tgataataac 250
aggaatcaga tttgcagtcc ctgtcctcca aatagtttct ccagcgcagg 300
tggacaaagg acctgtgaca tatgcaggca gtgtaaaggt gttttcagga 350
ccaggaagga gtgttcctcc accagcaatg cagagtgtga ctgcactcca 400
gggtttcact gcctgggggc aggatgcagc atgtgtgaac aggattgtaa 450
acaaggtcaa gaactgacaa aaaaaggttg taaagactgt tgctttggga 500
catttaacga tcagaaacgt ggcatctgtc gaccctggac aaactgttct 550
ttggatggaa agtctgtgct tgtgaatggg acgaaggaga gggacgtggt 600
ctgtggacca tctccagccg acctctctcc gggagcatcc tctgtgaccc 650
cgcctgcccc tgcgagagag ccaggacact ctccgcagat catctccttc 700
tttcttgcgc tgacgtcgac tgcgttgctc ttcctgctgt tcttcctcac 750
gctccgtttc tctgttgtta aacggggcag aaagaaactc ctgtatatat 800
tcaaacaacc atttatgaga ccagtacaaa ctactcaaga ggaagatggc 850
tgtagctgcc gatttccaga agaagaagaa ggaggatgtg aactgtgaaa 900
tggaagtcaa tagggctgtt gggactttct tgaaaagaag caaggaaata 950
tgagtcatcc gctatcacag ctttcaaaag caagaacacc atcctacata 1000
atacccagga ttcccccaac acacgttctt ttctaaatgc caatgagttg 1050
gcctttaaaa atgcaccact tttttttttt ttttgacagg gtctcactct 1100
gtcacccagg ctggagtgca gtggcaccac catggctctc tgcagccttg 1150
acctctggga gctcaagtga tcctcctgcc tcagtctcct gagtagctgg 1200
aactacaagg aagggccacc acacctgact aacttttttg tttttgtttt 1250
ggtaaagatg gcatttcacc atgttgtaca ggctggtctc aaactcctag 1300
gttcactttg gcctcccaaa gtgctgggat tacagacatg aactgccagg 1350
cccggccaaa ataatgcacc acttttaaca gaacagacag atgaggacag 1400
agctggtgat aaaaaaaaaa aaaaaaaagc attttctaga taccacttaa 1450
caggtttgag ctagtttttt tgaaatccaa agaaaattat agtttaaatt 1500
caattacata gtccagtggt ccaactataa ttataatcaa aatcaatgca 1550
ggtttgtttt ttggtgctaa tatgacatat gacaataagc cacgaggtgc 1600
agtaagtacc cgactaaagt ttccgtgggt tctgtcatgt aacacgacat 1650
gctccaccgt caggggggag tatgagcaga gtgcctgagt ttagggtcaa 1700
ggacaaaaaa cctcaggcct ggaggaagtt ttggaaagag ttcaagtgtc 1750
tgtatatcct atggtcttct ccatcctcac accttctgcc tttgtcctgc 1800
tcccttttaa gccaggttac attctaaaaa ttcttaactt ttaacataat 1850

attttatacc aaagccaata aatgaactgc atatgaaaaa aaaaaaaaaa 1900

aaaaaaaaaa aaaaaaaaaa aaaaaaaaaa aaaaaaa 1937

<210> 40
<211> 255
<212> PRT
<213> Homo sapiens

<400> 40
Met Gly Asn Ser Cys Tyr Asn Ile Val Ala Thr Leu Leu Leu Val
1               5                   10                  15

Leu Asn Phe Glu Arg Thr Arg Ser Leu Gln Asp Pro Cys Ser Asn
                20                  25                  30

Cys Pro Ala Gly Thr Phe Cys Asp Asn Asn Arg Asn Gln Ile Cys
                35                  40                  45

Ser Pro Cys Pro Pro Asn Ser Phe Ser Ser Ala Gly Gly Gln Arg
                50                  55                  60

Thr Cys Asp Ile Cys Arg Gln Cys Lys Gly Val Phe Arg Thr Arg
                65                  70                  75

Lys Glu Cys Ser Ser Thr Ser Asn Ala Glu Cys Asp Cys Thr Pro
                80                  85                  90

Gly Phe His Cys Leu Gly Ala Gly Cys Ser Met Cys Glu Gln Asp
                95                  100                 105

Cys Lys Gln Gly Gln Glu Leu Thr Lys Lys Gly Cys Lys Asp Cys
                110                 115                 120

Cys Phe Gly Thr Phe Asn Asp Gln Lys Arg Gly Ile Cys Arg Pro
                125                 130                 135

Trp Thr Asn Cys Ser Leu Asp Gly Lys Ser Val Leu Val Asn Gly
                140                 145                 150

Thr Lys Glu Arg Asp Val Val Cys Gly Pro Ser Pro Ala Asp Leu
                155                 160                 165

Ser Pro Gly Ala Ser Ser Val Thr Pro Pro Ala Pro Ala Arg Glu
                170                 175                 180

Pro Gly His Ser Pro Gln Ile Ile Ser Phe Phe Leu Ala Leu Thr
                185                 190                 195

Ser Thr Ala Leu Leu Phe Leu Leu Phe Phe Leu Thr Leu Arg Phe
                200                 205                 210

Ser Val Val Lys Arg Gly Arg Lys Lys Leu Leu Tyr Ile Phe Lys
                215                 220                 225

Gln Pro Phe Met Arg Pro Val Gln Thr Thr Gln Glu Glu Asp Gly
                230                 235                 240

Cys Ser Cys Arg Phe Pro Glu Glu Glu Glu Gly Gly Cys Glu Leu
                245                 250                 255

<210> 41
<211> 1701
<212> DNA
<213> Mus musculus

<400> 41
ggaacaaaag ctggagctcc accgcggtgg cggccgctct agaactagtg 50

gatcccccgg gctgcaggaa ttcggcacga gcagaagagg gggctagcta 100
gctgtctctg cggaccaggg gagaccccgc gcccccccgg tgtgaggcgg 150
cctcacaggg ccgggtgggc tggcgagccg acgcggcggc ggaggaggct 200
gtgaggagtg tgtggaacag gacccgggac agaggaacca tggctccgca 250
gaacctgagc accttttgcc tgttgctgct atacctcatc ggggcggtga 300
ttgccggacg agatttctat aagatcttgg gggtgcctcg aagtgcctct 350
ataaaggata ttaaaaaggc ctataggaaa ctagccctgc agcttcatcc 400
cgaccggaac cctgatgatc cacaagccca ggagaaattc caggatctgg 450
gtgctgctta tgaggttctg tcagatagtg agaaacggaa acagtacgat 500
acttatggtg aagaaggatt aaaagatggt catcagagct cccatggaga 550
cattttttca cacttctttg gggattttgg tttcatgttt ggaggaaccc 600
ctcgtcagca agacagaaat attccaagag gaagtgatat tattgtagat 650
ctagaagtca ctttggaaga agtatatgca ggaaattttg tggaagtagt 700
tagaaacaaa cctgtggcaa ggcaggctcc tggcaaacgg aagtgcaatt 750
gtcggcaaga gatgcggacc acccagctgg gccctgggcg cttccaaatg 800
acccaggagg tggtctgcga cgaatgccct aatgtcaaac tagtgaatga 850
agaacgaacg ctggaagtag aaatagagcc tggggtgaga gacggcatgg 900
agtacccctt tattggagaa ggtgagcctc acgtggatgg ggagcctgga 950
gatttacggt tccgaatcaa agttgtcaag cacccaatat ttgaaaggag 1000
aggagatgat ttgtacacaa atgtgacaat ctcattagtt gagtcactgg 1050
ttggctttga gatggatatt actcacttgg atggtcacaa ggtacatatt 1100
tcccgggata agatcaccag gccaggagcg aagctatgga agaaagggga 1150
agggctcccc aactttgaca acaacaatat caagggctct ttgataatca 1200
cttttgatgt ggattttcca aaagaacagt taacagagga agcgagagaa 1250
ggtatcaaac agctactgaa acaagggtca gtgcagaagg tatacaatgg 1300
actgcaagga tattgagagt gaataaaatt ggactttgtt taaaataagt 1350
gaataagcga tatttattat ctgcaaggtt tttttgtgtg tgtttttgtt 1400
tttattttca atatgcaagt taggcttaat ttttttatct aatgatcatc 1450
atgaaatgaa taagagggct taagaatttg tccatttgca ttcggaaaag 1500
aatgaccagc aaaaggttta ctaatacgtc tccctttggg gatttaatgt 1550
ctggtgctgc cgcctgagtt tcaagaatta aagctgcaag aggactccag 1600
gagcaaaaga aacacaatat agagggttgg agttgttagc aatttcattc 1650
aaaatgccaa ctggagaagt ctgtttttaa atacattttg ttgttatttt 1700
t 1701

<210> 42
<211> 358
<212> PRT
<213> Mus musculus

<400> 42

<210> 43
<211> 1798
<212> DNA
<213> Homo sapiens

<400> 43
gacagtggag ggcagtggag aggaccgcgc tgtcctgctg tcaccaagag 50

ctggagacac catctcccac cgagagtcat ggccccattg gccctgcacc 100

tcctcgtcct cgtccccatc ctcctcagcc tggtggcctc ccaggactgg 150

aaggctgaac gcagccaaga ccccttcgag aaatgcatgc aggatcctga 200

ctatgagcag ctgctcaagg tggtgacctg ggggctcaat cggaccctga 250

agccccagag ggtgattgtg gttggcgctg gtgtggccgg gctggtggcc 300

gccaaggtgc tcagcgatgc tggacacaag gtcaccatcc tggaggcaga 350

taacaggatc gggggccgca tcttcaccta ccgggaccag aacacgggct 400

ggattgggga gctgggagcc atgcgcatgc ccagctctca caggatcctc 450

cacaagctct gccagggcct ggggctcaac ctgaccaagt tcacccagta 500

cgacaagaac acgtggacgg aggtgcacga agtgaagctg cgcaactatg 550

tggtggagaa ggtgcccgag aagctgggct acgccttgcg tccccaggaa 600

aagggccact cgcccgaaga catctaccag atggctctca accaggccct 650

caaagacctc aaggcactgg gctgcagaaa ggcgatgaag aagtttgaaa 700

ggcacacgct cttggaatat cttctcgggg aggggaacct gagccggccg 750

gccgtgcagc ttctgggaga cgtgatgtcc gaggatggct tcttctatct 800

cagcttcgcc gaggccctcc gggcccacag ctgcctcagc gacagactcc 850

agtacagccg catcgtgggt ggctgggacc tgctgccgcg cgcgctgctg 900

agctcgctgt ccgggcttgt gctgttgaac gcgcccgtgg tggcgatgac 950

ccagggaccg cacgatgtgc acgtgcagat cgagacctct cccccggcgc 1000

ggaatctgaa ggtgctgaag gccgacgtgg tgctgctgac ggcgagcgga 1050

ccggcggtga agcgcatcac cttctcgccg ccgctgcccc gccacatgca 1100

ggaggcgctg cggaggctgc actacgtgcc ggccaccaag gtgttcctaa 1150

gcttccgcag gcccttctgg cgcgaggagc acattgaagg cggccactca 1200

aacaccgatc gcccgtcgcg catgattttc tacccgccgc cgcgcgaggg 1250

cgcgctgctg ctggcctcgt acacgtggtc ggacgcggcg gcagcgttcg 1300

ccggcttgag ccgggaagag gcgttgcgct tggcgctcga cgacgtggcg 1350

gcattgcacg ggcctgtcgt gcgccagctc tgggacggca ccggcgtcgt 1400

caagcgttgg gcggaggacc agcacagcca gggtggcttt gtggtacagc 1450

cgccggcgct ctggcaaacc gaaaaggatg actggacggt cccttatggc 1500

cgcatctact ttgccggcga gcacaccgcc tacccgcacg gctgggtgga 1550

gacggcggtc aagtcggcgc tgcgcgccgc catcaagatc aacagccgga 1600

aggggcctgc atcggacacg gccagccccg aggggcacgc atctgacatg 1650

gaggggcagg ggcatgtgca tggggtggcc agcagcccct cgcatgacct 1700

ggcaaaggaa gaaggcagcc accctccagt ccaaggccag ttatctctcc 1750

aaaacacgac ccacacgagg acctcgcatt aaagtatttt cggaaaaa 1798

<210> 44
<211> 567
<212> PRT
<213> Homo sapiens

<400> 44
Met Ala Pro Leu Ala Leu His Leu Leu Val Leu Val Pro Ile Leu
1               5                   10                  15

Leu Ser Leu Val Ala Ser Gln Asp Trp Lys Ala Glu Arg Ser Gln
                20                  25                  30

Asp Pro Phe Glu Lys Cys Met Gln Asp Pro Asp Tyr Glu Gln Leu
                35                  40                  45

Leu Lys Val Val Thr Trp Gly Leu Asn Arg Thr Leu Lys Pro Gln
                50                  55                  60

Arg Val Ile Val Val Gly Ala Gly Val Ala Gly Leu Val Ala Ala
                65                  70                  75

Lys Val Leu Ser Asp Ala Gly His Lys Val Thr Ile Leu Glu Ala
                80                  85                  90

Asp Asn Arg Ile Gly Gly Arg Ile Phe Thr Tyr Arg Asp Gln Asn
                95                  100                 105

Thr Gly Trp Ile Gly Glu Leu Gly Ala Met Arg Met Pro Ser Ser
                110                 115                 120

His Arg Ile Leu His Lys Leu Cys Gln Gly Leu Gly Leu Asn Leu
                125                 130                 135

Thr Lys Phe Thr Gln Tyr Asp Lys Asn Thr Trp Thr Glu Val His
                140                 145                 150

Glu Val Lys Leu Arg Asn Tyr Val Val Glu Lys Val Pro Glu Lys
                155                 160                 165

Leu Gly Tyr Ala Leu Arg Pro Gln Glu Lys Gly His Ser Pro Glu
                170                 175                 180

Asp Ile Tyr Gln Met Ala Leu Asn Gln Ala Leu Lys Asp Leu Lys
                185                 190                 195

Ala Leu Gly Cys Arg Lys Ala Met Lys Lys Phe Glu Arg His Thr
                200                 205                 210

Leu Leu Glu Tyr Leu Leu Gly Glu Gly Asn Leu Ser Arg Pro Ala
                215                 220                 225

Val Gln Leu Leu Gly Asp Val Met Ser Glu Asp Gly Phe Phe Tyr
                230                 235                 240

Leu Ser Phe Ala Glu Ala Leu Arg Ala His Ser Cys Leu Ser Asp
                245                 250                 255

Arg Leu Gln Tyr Ser Arg Ile Val Gly Gly Trp Asp Leu Leu Pro
                260                 265                 270

Arg Ala Leu Leu Ser Ser Leu Ser Gly Leu Val Leu Leu Asn Ala
                275                 280                 285

Pro Val Val Ala Met Thr Gln Gly Pro His Asp Val His Val Gln
                290                 295                 300

Ile Glu Thr Ser Pro Pro Ala Arg Asn Leu Lys Val Leu Lys Ala
                305                 310                 315

Asp Val Val Leu Leu Thr Ala Ser Gly Pro Ala Val Lys Arg Ile
                320                 325                 330

Thr Phe Ser Pro Pro Leu Pro Arg His Met Gln Glu Ala Leu Arg
                335                 340                 345

Arg Leu His Tyr Val Pro Ala Thr Lys Val Phe Leu Ser Phe Arg
                350                 355                 360

Arg Pro Phe Trp Arg Glu Glu His Ile Glu Gly Gly His Ser Asn
                365                 370                 375

Thr Asp Arg Pro Ser Arg Met Ile Phe Tyr Pro Pro Pro Arg Glu
                380                 385                 390

Gly Ala Leu Leu Leu Ala Ser Tyr Thr Trp Ser Asp Ala Ala Ala
                395                 400                 405

Ala Phe Ala Gly Leu Ser Arg Glu Glu Ala Leu Arg Leu Ala Leu
                410                 415                 420

Asp Asp Val Ala Ala Leu His Gly Pro Val Val Arg Gln Leu Trp
                425                 430                 435

Asp Gly Thr Gly Val Val Lys Arg Trp Ala Glu Asp Gln His Ser
                440                 445                 450

Gln Gly Gly Phe Val Val Gln Pro Pro Ala Leu Trp Gln Thr Glu
                455                 460                 465

Lys Asp Asp Trp Thr Val Pro Tyr Gly Arg Ile Tyr Phe Ala Gly
                470                 475                 480

Glu His Thr Ala Tyr Pro His Gly Trp Val Glu Thr Ala Val Lys
                485                 490                 495

Ser Ala Leu Arg Ala Ala Ile Lys Ile Asn Ser Arg Lys Gly Pro
                500                 505                 510

Ala Ser Asp Thr Ala Ser Pro Glu Gly His Ala Ser Asp Met Glu
                515                 520                 525

Gly Gln Gly His Val His Gly Val Ala Ser Ser Pro Ser His Asp
                530                 535                 540

Leu Ala Lys Glu Glu Gly Ser His Pro Pro Val Gln Gly Gln Leu

545                 550                 555

Ser Leu Gln Asn Thr Thr His Thr Arg Thr Ser His
                560                 565

<210> 45
<211> 690
<212> DNA
<213> Homo sapiens

<400> 45
tgcacaagca gaatcttcag aacaggttct ccttccccag tcaccagttg 50

ctcgagttag aattgtctgc aatggccgcc ctgcagaaat ctgtgagctc 100

tttccttatg gggaccctgg ccaccagctg cctccttctc ttggccctct 150

tggtacaggg aggagcagct gcgcccatca gctcccactg caggcttgac 200

aagtccaact tccagcagcc ctatatcacc aaccgcacct tcatgctggc 250

taaggaggct agcttggctg ataacaacac agacgttcgt ctcattgggg 300

agaaactgtt ccacggagtc agtatgagtg agcgctgcta tctgatgaag 350

caggtgctga acttcaccct tgaagaagtg ctgttccctc aatctgatag 400

gttccagcct tatatgcagg aggtggtgcc cttcctggcc aggctcagca 450

acaggctaag cacatgtcat attgaaggtg atgacctgca tatccagagg 500

aatgtgcaaa agctgaagga cacagtgaaa aagcttggag agagtggaga 550

gatcaaagca attggagaac tggatttgct gtttatgtct ctgagaaatg 600

cctgcatttg accagagcaa agctgaaaaa tgaataacta accccctttc 650

cctgctagaa ataacaatta gatgccccaa agcgattttt 690

<210> 46
<211> 179
<212> PRT
<213> Homo sapiens

<400> 46
Met Ala Ala Leu Gln Lys Ser Val Ser Ser Phe Leu Met Gly Thr
1               5                   10                  15

Leu Ala Thr Ser Cys Leu Leu Leu Leu Ala Leu Leu Val Gln Gly
                20                  25                  30

Gly Ala Ala Ala Pro Ile Ser Ser His Cys Arg Leu Asp Lys Ser
                35                  40                  45

Asn Phe Gln Gln Pro Tyr Ile Thr Asn Arg Thr Phe Met Leu Ala
                50                  55                  60

Lys Glu Ala Ser Leu Ala Asp Asn Asn Thr Asp Val Arg Leu Ile
                65                  70                  75

Gly Glu Lys Leu Phe His Gly Val Ser Met Ser Glu Arg Cys Tyr
                80                  85                  90

Leu Met Lys Gln Val Leu Asn Phe Thr Leu Glu Glu Val Leu Phe
                95                  100                 105

Pro Gln Ser Asp Arg Phe Gln Pro Tyr Met Gln Glu Val Val Pro
                110                 115                 120

Phe Leu Ala Arg Leu Ser Asn Arg Leu Ser Thr Cys His Ile Glu
                125                 130                 135

Gly Asp Asp Leu His Ile Gln Arg Asn Val Gln Lys Leu Lys Asp
                140                 145                 150

Thr Val Lys Lys Leu Gly Glu Ser Gly Glu Ile Lys Ala Ile Gly
                155                 160                 165

Glu Leu Asp Leu Leu Phe Met Ser Leu Arg Asn Ala Cys Ile
                170                 175

<210> 47
<211> 1136
<212> DNA
<213> Homo sapiens

<400> 47
gaggggtaga gatgcagaaa ggcagaaagg agaaaattca ggataactct 50

cctgaggggt gagccaagcc ctgccatgta gtgcacgcag gacatcaaca 100

aacacagata acaggaaatg atccattccc tgtggtcact tattctaaag 150

gccccaacct tcaaagttca agtagtgata tggatgactc cacagaaagg 200

gagcagtcac gccttacttc ttgccttaag aaaagagaag aaatgaaact 250

gaaggagtgt gtttccatcc tcccacggaa ggaaagcccc tctgtccgat 300

cctccaaaga cggaaagctg ctggctgcaa ccttgctgct ggcactgctg 350

tcttgctgcc tcacggtggt gtctttctac caggtggccg ccctgcaagg 400

ggacctggcc agcctccggg cagagctgca gggccaccac gcggagaagc 450

tgccagcagg agcaggagcc cccaaggccg gcctggagga agctccagct 500

gtcaccgcgg gactgaaaat ctttgaacca ccagctccag gagaaggcaa 550

ctccagtcag aacagcagaa ataagcgtgc cgttcagggt ccagaagaaa 600

cagtcactca agactgcttg caactgattg cagacagtga aacaccaact 650

atacaaaaag gatcttacac atttgttcca tggcttctca gctttaaaag 700

gggaagtgcc ctagaagaaa aagagaataa aatattggtc aaagaaactg 750

gttacttttt tatatatggt caggttttat atactgataa gacctacgcc 800

atgggacatc taattcagag gaagaaggtc catgtctttg gggatgaatt 850

gagtctggtg actttgtttc gatgtattca aaatatgcct gaaacactac 900

ccaataattc ctgctattca gctggcattg caaaactgga agaaggagat 950

gaactccaac ttgcaatacc aagagaaaat gcacaaatat cactggatgg 1000

agatgtcaca ttttttggtg cattgaaact gctgtgacct acttacacca 1050

tgtctgtagc tattttcctc cctttctctg tacctctaag aagaaagaat 1100

ctaacagaaa aaaaaaaaaa aaaaaaaaaa aaaaaa 1136

<210> 48
<211> 285
<212> PRT

<213> Homo sapiens

<400> 48
Met Asp Asp Ser Thr Glu Arg Glu Gln Ser Arg Leu Thr Ser Cys
1               5                   10                  15

Leu Lys Lys Arg Glu Glu Met Lys Leu Lys Glu Cys Val Ser Ile
                20                  25                  30

Leu Pro Arg Lys Glu Ser Pro Ser Val Arg Ser Ser Lys Asp Gly
                35                  40                  45

Lys Leu Leu Ala Ala Thr Leu Leu Leu Ala Leu Leu Ser Cys Cys
                50                  55                  60

Leu Thr Val Val Ser Phe Tyr Gln Val Ala Ala Leu Gln Gly Asp
                65                  70                  75

Leu Ala Ser Leu Arg Ala Glu Leu Gln Gly His His Ala Glu Lys
                80                  85                  90

Leu Pro Ala Gly Ala Gly Ala Pro Lys Ala Gly Leu Glu Glu Ala
                95                  100                 105

Pro Ala Val Thr Ala Gly Leu Lys Ile Phe Glu Pro Pro Ala Pro
                110                 115                 120

Gly Glu Gly Asn Ser Ser Gln Asn Ser Arg Asn Lys Arg Ala Val
                125                 130                 135

Gln Gly Pro Glu Glu Thr Val Thr Gln Asp Cys Leu Gln Leu Ile
                140                 145                 150

Ala Asp Ser Glu Thr Pro Thr Ile Gln Lys Gly Ser Tyr Thr Phe
                155                 160                 165

Val Pro Trp Leu Leu Ser Phe Lys Arg Gly Ser Ala Leu Glu Glu
                170                 175                 180

Lys Glu Asn Lys Ile Leu Val Lys Glu Thr Gly Tyr Phe Phe Ile
                185                 190                 195

Tyr Gly Gln Val Leu Tyr Thr Asp Lys Thr Tyr Ala Met Gly His
                200                 205                 210

Leu Ile Gln Arg Lys Lys Val His Val Phe Gly Asp Glu Leu Ser
                215                 220                 225

Leu Val Thr Leu Phe Arg Cys Ile Gln Asn Met Pro Glu Thr Leu
                230                 235                 240

Pro Asn Asn Ser Cys Tyr Ser Ala Gly Ile Ala Lys Leu Glu Glu
                245                 250                 255

Gly Asp Glu Leu Gln Leu Ala Ile Pro Arg Glu Asn Ala Gln Ile
                260                 265                 270

Ser Leu Asp Gly Asp Val Thr Phe Phe Gly Ala Leu Lys Leu Leu
                275                 280                 285

<210> 49
<211> 2025
<212> DNA
<213> Homo sapiens

<400> 49
agtgcgtgag tttggtggcg gccggctgtg cagagacgcc atgtaccggc 50

tcctgtcagc agtgactgcc cgggctgccg cccccggggg cttggcctca 100
agctgcggac gacgcggggt ccatcagcgc gccgggctgc cgcctctcgg 150
ccacggctgg gtcgggggcc tcgggctggg gctggggctg gcgctcgggg 200
tgaagctggc aggtgggctg agtggcgcgg ccccggcgca gtcccccgcg 250
gcccccgacc ctgaggcgtc gcctctggcc gagccgccac aggagcagtc 300
cctcgccccg tggtctccgc agaccccggc gccgccctgc tccaggtgct 350
tcgccagagc catcgagagc agccgcgacc tgctgcacag gatcaaggat 400
gaggtgggcg caccgggcat agtggttgga gtttctgtag atggaaaaga 450
agtctggtca gaaggtttag gttatgctga tgttgagaac cgtgtaccat 500
gtaaaccaga gacagttatg cgaattgcta gcatcagcaa aagtctcacc 550
atggttgctc ttgccaaatt gtgggaagcg gggaaactgg atcttgatat 600
tccagtacaa cattatgttc ccgaattccc agaaaaagaa tatgaaggtg 650
aaaaggtttc tgtcacaaca agattactga tttcccattt aagtggaatt 700
cgtcattatg aaaaggacat aaaaaaggtg aaagaagaga aagcttataa 750
agccttgaag atgatgaaag agaatgttgc atttgagcaa gaaaaagaag 800
gcaaaagtaa tgaaaagaat gattttacta aatttaaaac agagcaggag 850
aatgaagcca aatgccggaa ttcaaaacct ggcaagaaaa agaatgattt 900
tgaacaaggc gaattatatt tgagagaaaa gtttgaaaat tcaattgaat 950
ccctaagatt atttaaaaat gatcctttgt tcttcaaacc tggtagtcag 1000
tttttgtatt caacttttgg ctatacccta ctggcagcca tagtagagag 1050
agcttcagga tgtaaatatt tggactatat gcagaaaata ttccatgact 1100
tggatatgct gacgactgtg caggaagaaa acgagccagt gatttacaat 1150
agagcaaggt aaatgaatac cttctgctgt gtctagctat atcgcatctt 1200
aacactattt tattaattaa aagtcaaatt ttctttgttt ccattccaaa 1250
atcaacctgc cacattttgg gagcttttct acatgtctgt tttctcatct 1300
gtaaagtgaa ggaagtaaaa catgtttata aagtacacta agaccctttg 1350
atgaaagata gcaataatat taataattca aacatgaata actaaaccaa 1400
aattgcaccc accatgagca tctgtaattt gctctttaac cattcctttt 1450
ttaggtttta actaatactt tgtttacgtg ttattagttt ttaattgttt 1500
tcatactgtt ttgaaataat tttatactta tagaaaagtt gcaagagtag 1550
tacaaaggat tcacatatcc tctttactca gattccctta atgttagttt 1600
accgcatttg cattaccttt ctgtctacat atgtgtttgt ttctgaacca 1650
tttggaaata agttgtagac gtgatacccc tttacctata aatatttaca 1700
tgtgtatttt ctaaaaacaa ggacattcag ggccgggtgc agtggctcac 1750

gcctgtaatc ccaacacttt ggaaggccga ggtgggtgga tcacctgagg 1800

tcaggagttc aagaccagcc tggccaatgt ggtgaaatca accctctctc 1850

tactaaaaat gcaaaaatta gccaggtgtg gtggcgggtg cctataatcc 1900

cagccacgcg ggaggctgag gcaggagaat cgcttgaacc caggaggtgg 1950

agttgcagta agccaagatc gtgcccctgc acttcaggct gggcgaaaga 2000

gtgagactcc atgtcaaaaa aggat 2025

<210> 50
<211> 373
<212> PRT
<213> Homo sapiens

<400> 50

<210> 51
<211> 2930
<212> DNA
<213> Homo sapiens

<400> 51
ggagcccatg atttcctgga agagccctag agctttgctt tttctctcct 50

gcagcactta accgaaacca gttttgcaat caattcctgt tcaaaggcsa 100

ccctactctt cctatccgtc tttctccagc ccagacactc acagccccct 150

gccagaccag gggacctcgg agaggcaagg acagaggttc aggatcttcc 200

tctccctcgg gacccaaggs cacaaaggag agctccgtgg agagaagaaa 250

atcatttgac tcctggggac acagatttgc tgccacagag gctgatggac 300

aaccaggcgg agagagaaag tgaggctggt gttggtttgc aaagggatga 350

ggatgacgct cctctgtgtg aagacgtgga gctacaagac ggagatctgt 400

cccccgaaga aaaaatattt ttgagagaat ttcccagatt gaaagaagat 450

ctgaaaggga acattgacaa gctccgtgcc ctcgcagacg atattgacaa 500

aacccacaag aaattcacca aggctaacat ggtggccacc tctactgctg 550

tcatctctgg agtgatgagc ctcctgggtt tagcccttgc cccagcaaca 600

ggaggaggaa gcctgctgct ctccaccgct ggtcaaggtt tggcaacagc 650

agctggggtc accagcatcg tgagtggtac gttggaacgc tccaaaaata 700

aagaagccca agcacgggcg gaagacatac tgcccaccta cgaccaagag 750

gacagggagg atgaggaaga gaaggcagac tatgtcacag ctgctggaaa 800

gattatctat aatcttagaa acaccttgaa gtatgccaag aaaaacgtcc 850

gtgcattttg gaaactcaga gccaacccac gcttggccaa tgctaccaag 900

cgtcttctga ccactggcca agtctcctcc cggagccgcg tgcaggtgca 950

aaaggccttt gcgggaacaa cactggcgat gaccaaaaat gctcgcgtgc 1000
tgggaggtgt gatgtccgcc ttctcccttg gctatgactt ggccactctc 1050
tcaaaggaat ggaagcacct gaaggaagga gcaaggacaa agtttgcgga 1100
agagttgaga gccaaggcct tggagctgga gaggaaactc acagaactca 1150
cccagctcta caagagcttg cagcagaaag tgaggtcaag ggccagaggg 1200
gtggggaagg atttaactgg gacctgcgaa accgaggctt actggaagga 1250
gttaagggag catgtgtgga tgtggctgtg gctgtgtgtg tgtctgtgtg 1300
tctgtgtgta tgtacagttt acatgaatgt tcctcaggac atggcataca 1350
atggccttgg aggtccaaat aatatcaagt acatcttgga gatgagggtg 1400
cctgtcctgg acagacctcg gcatgccttc tgtttctcct tcaatgctcc 1450
ttaaggccta tgtgctggga aaagggtctt ccctgtttgt ttgtttgttt 1500
gtttgtttgt ttgttttgag actccagtct gggtgtcaga atgagacccc 1550
atctcaaaaa aaaaaaaaaa aaaaaaaaag aagaagaata cagtcatgta 1600
tctcttggtg acagggacgc attctgataa atgtgtcatt aggcaattgc 1650
attgtagtgt gattatcaca gattgtactt atacaaaact tagatggcat 1700
agcctactgc atacctaggc tatatgggag agcctattgc tcccaggcta 1750
cgcacctgta cagcatgtga ctactgaata ctataggcaa ttgcagcaca 1800
atgggaaata tttgtgtatc taaacatatg taaacagaga aaaaggaaag 1850
taaaaatatg gcataaaaga taagaattgg ctctcctgta cagggcactt 1900
actacgaatg gagcttgcag ggctgagagt tgctccagat gagtcagtga 1950
gtggtgaatg aatgtgaagg cctagggcat tactgtatac tactgtaggc 2000
tttataaaca cagcacactt agggtacaca aaatgcatat taaaacattt 2050
tcttccttca gtatattagg caataggaat ttttcaagtc cactataaat 2100
cttatcaaac catggttgta tatgcagttg accgaaacat tgttattgga 2150
cacataacta tagttgaaag aataagcaaa aagtctatct aggtgtgctg 2200
tcttgagcaa cttttaatta ttctcctgtc ctgcaatatg agttaatctt 2250
ctctgatcga tgtagattcc aggaaggggt gtccaggaca attaccttcc 2300
ttctggagaa acttccctta atcaaataag agaacttcaa agaaaatccc 2350
tccctgtgct ttggaaggga agggaggtgg gcagcagtgg gtcagagata 2400
gacctttgtt ctcttatttc tgaggccctt cagtctcctt tattcaaagc 2450
actcagcatg ccaaagcacc ctattttagg gtatcttttt ctgagcccta 2500
aacactgtgt tggggatgtc aactgtgaca ggaaaatatc ttggggcccc 2550
agaatcacta aggaaaactc aagcttaggg aaacttctta gggcaaaccc 2600
acctcccact ctattcaaag ttatctctct gctcactgag atagatacat 2650

atctgattgc ctcctttgga aaggctaatc agaaactcaa aagaatgcaa 2700

ctgtttgtgt ctcacctatc tgtgacctgg aagctccctc cccactgaac 2750

caatgttctt cttacatata ttgattaatg tcttatgtct ccctaaaatg 2800

tataaaacca aggtatgccc caaccatctt ggccacatgt catcaggact 2850

tcctgagtct gtgtcacagt gtgtcctcaa ccttggcaaa ataaactttc 2900

taaattaact gagacaaaaa aaaaaaaaaa 2930

<210> 52
<211> 343
<212> PRT
<213> Homo sapiens

<400> 52
Met Asp Asn Gln Ala Glu Arg Glu Ser Glu Ala Gly Val Gly Leu
1               5                   10                  15

Gln Arg Asp Glu Asp Asp Ala Pro Leu Cys Glu Asp Val Glu Leu
                20                  25                  30

Gln Asp Gly Asp Leu Ser Pro Glu Glu Lys Ile Phe Leu Arg Glu
                35                  40                  45

Phe Pro Arg Leu Lys Glu Asp Leu Lys Gly Asn Ile Asp Lys Leu
                50                  55                  60

Arg Ala Leu Ala Asp Asp Ile Asp Lys Thr His Lys Lys Phe Thr
                65                  70                  75

Lys Ala Asn Met Val Ala Thr Ser Thr Ala Val Ile Ser Gly Val
                80                  85                  90

Met Ser Leu Leu Gly Leu Ala Leu Ala Pro Ala Thr Gly Gly Gly
                95                  100                 105

Ser Leu Leu Leu Ser Thr Ala Gly Gln Gly Leu Ala Thr Ala Ala
                110                 115                 120

Gly Val Thr Ser Ile Val Ser Gly Thr Leu Glu Arg Ser Lys Asn
                125                 130                 135

Lys Glu Ala Gln Ala Arg Ala Glu Asp Ile Leu Pro Thr Tyr Asp
                140                 145                 150

Gln Glu Asp Arg Glu Asp Glu Glu Glu Lys Ala Asp Tyr Val Thr
                155                 160                 165

Ala Ala Gly Lys Ile Ile Tyr Asn Leu Arg Asn Thr Leu Lys Tyr
                170                 175                 180

Ala Lys Lys Asn Val Arg Ala Phe Trp Lys Leu Arg Ala Asn Pro
                185                 190                 195

Arg Leu Ala Asn Ala Thr Lys Arg Leu Leu Thr Thr Gly Gln Val
                200                 205                 210

Ser Ser Arg Ser Arg Val Gln Val Gln Lys Ala Phe Ala Gly Thr
                215                 220                 225

Thr Leu Ala Met Thr Lys Asn Ala Arg Val Leu Gly Gly Val Met
                230                 235                 240

Ser Ala Phe Ser Leu Gly Tyr Asp Leu Ala Thr Leu Ser Lys Glu

245                 250                 255
Trp Lys His Leu Lys Glu Gly Ala Arg Thr Lys Phe Ala Glu Glu
                260                 265                 270
Leu Arg Ala Lys Ala Leu Glu Leu Glu Arg Lys Leu Thr Glu Leu
                275                 280                 285
Thr Gln Leu Tyr Lys Ser Leu Gln Gln Lys Val Arg Ser Arg Ala
                290                 295                 300
Arg Gly Val Gly Lys Asp Leu Thr Gly Thr Cys Glu Thr Glu Ala
                305                 310                 315
Tyr Trp Lys Glu Leu Arg Glu His Val Trp Met Trp Leu Trp Leu
                320                 325                 330
Cys Val Cys Leu Cys Val Cys Val Tyr Val Gln Phe Thr
                335                 340

<210> 53
<211> 2333
<212> DNA
<213> Homo sapiens

<400> 53
gggccaggcc gcgcccccgc gtgcgtgcgc ggcccggcag agccgtgcgg 50
gcgcccgcgt actcactagc tgaggtggca gtggttccac caacatggag 100
ctctcgcaga tgtcggagct catggggctg tcggtgttgc ttgggctgct 150
ggccctgatg gcgacggcgg cggtagcgcg ggggtggctg cgcgcggggg 200
aggagaggag cggccggccc gcctgccaaa aagcaaatgg atttccacct 250
gacaaatctt cgggatccaa gaagcagaaa caatatcagc ggattcggaa 300
ggagaagcct caacaacaca acttcaccca ccgcctcctg gctgcagctc 350
tgaagagcca cagcgggaac atatcttgca tggactttag cagcaatggc 400
aaatacctgg ctacctgtgc agatgatcgc accatccgca tctggagcac 450
caaggacttc ctgcagcgag agcaccgcag catgagagcc aacgtggagc 500
tggaccacgc caccctggtg cgcttcagcc ctgactgcag agccttcatc 550
gtctggctgg ccaacgggga caccctccgt gtcttcaaga tgaccaagcg 600
ggaggatggg ggctacacct tcacagccac cccagaggac ttccctaaaa 650
agcacaaggc gcctgtcatc gacattggca ttgctaacac agggaagttt 700
atcatgactg cctccagtga caccactgtc ctcatctgga gcctgaaggg 750
tcaagtgctg tctaccatca acaccaacca gatgaacaac acacacgctg 800
ctgtatctcc ctgtggcaga tttgtagcct cgtgtggctt caccccagat 850
gtgaaggttt gggaagtctg ctttggaaag aagggggagt tccaggaggt 900
ggtgcgagcc ttcgaactaa agggccactc cgcggctgtg cactcgtttg 950
ctttctccaa cgactcacgg aggatggctt ctgtctccaa ggatggtaca 1000
tggaaactgt gggacacaga tgtggaatac aagaagaagc aggaccccta 1050

cttgctgaag acaggccgct ttgaagaggc ggcgggtgcc gcgccgtgcc 1100
gcctggccct ctcccccaac gcccaggtct tggccttggc cagtggcagt 1150
agtattcatc tctacaatac ccggcggggc gagaaggagg agtgctttga 1200
gcgggtccat ggcgagtgta tcgccaactt gtcctttgac atcactggcc 1250
gctttctggc ctcctgtggg gaccgggcgg tgcggctgtt tcacaacact 1300
cctggccacc gagccatggt ggaggagatg cagggccacc tgaagcgggc 1350
ctccaacgag agcacccgcc agaggctgca gcagcagctg acccaggccc 1400
aagagaccct gaagagcctg ggtgccctga agaagtgact ctgggagggc 1450
ccggcgcaga ggattgagga ggagggatct ggcctcctca tggcgctgct 1500
gccatctttc ctcccaggtg gaagcctttc agaaggagtc tcctggtttt 1550
cttactggtg gccctgcttc ttcccattga aactactctt gtctacttag 1600
gtctctctct tcttgctggc tgtgactcct ccctgactag tggccaaggt 1650
gcttttcttc ctcccaggcc cagtgggtgg aatctgtccc cacctggcac 1700
tgaggagaat ggtagagagg agaggagaga gagagagaat gtgatttttg 1750
gccttgtggc agcacatcct cacacccaaa gaagtttgta aatgttccag 1800
aacaacctag agaacacctg agtactaagc agcagttttg caaggatggg 1850
agactgggat agcttcccat cacagaactg tgttccatca aaaagacact 1900
aagggatttc cttctgggcc tcagttctat ttgtaagatg gagaataatc 1950
ctctctgtga actccttgca aagatgatat gaggctaaga gaatatcaag 2000
tccccaggtc tggaagaaaa gtagaaaaga gtagtactat tgtccaatgt 2050
catgaaagtg gtaaaagtgg gaaccagtgt gctttgaaac caaattagaa 2100
acacattcct tgggaaggca aagttttctg ggacttgatc atacatttta 2150
tatggttggg acttctctct tcgggagatg atatcttgtt taaggagacc 2200
tcttttcagt tcatcaagtt catcagatat ttgagtgccc actctgtgcc 2250
caaataaata tgagctgggg attaaaaaaa aaaaaaaaaa aaaaaaaaaa 2300
aaaaaaaaaa aaaaaaaaaa aaaaaaaaaa aaa 2333

<210> 54
<211> 447
<212> PRT
<213> Homo sapiens

<400> 54
Met Glu Leu Ser Gln Met Ser Glu Leu Met Gly Leu Ser Val Leu
1               5                   10                  15

Leu Gly Leu Leu Ala Leu Met Ala Thr Ala Ala Val Ala Arg Gly
                20                  25                  30

Trp Leu Arg Ala Gly Glu Glu Arg Ser Gly Arg Pro Ala Cys Gln
                35                  40                  45

Lys Ala Asn Gly Phe Pro Pro Asp Lys Ser Ser Gly Ser Lys Lys

50                  55                  60

Gln Lys Gln Tyr Gln Arg Ile Arg Lys Glu Lys Pro Gln Gln His
                65                  70                  75

Asn Phe Thr His Arg Leu Leu Ala Ala Ala Leu Lys Ser His Ser
                80                  85                  90

Gly Asn Ile Ser Cys Met Asp Phe Ser Ser Asn Gly Lys Tyr Leu
                95                  100                 105

Ala Thr Cys Ala Asp Asp Arg Thr Ile Arg Ile Trp Ser Thr Lys
                110                 115                 120

Asp Phe Leu Gln Arg Glu His Arg Ser Met Arg Ala Asn Val Glu
                125                 130                 135

Leu Asp His Ala Thr Leu Val Arg Phe Ser Pro Asp Cys Arg Ala
                140                 145                 150

Phe Ile Val Trp Leu Ala Asn Gly Asp Thr Leu Arg Val Phe Lys
                155                 160                 165

Met Thr Lys Arg Glu Asp Gly Gly Tyr Thr Phe Thr Ala Thr Pro
                170                 175                 180

Glu Asp Phe Pro Lys Lys His Lys Ala Pro Val Ile Asp Ile Gly
                185                 190                 195

Ile Ala Asn Thr Gly Lys Phe Ile Met Thr Ala Ser Ser Asp Thr
                200                 205                 210

Thr Val Leu Ile Trp Ser Leu Lys Gly Gln Val Leu Ser Thr Ile
                215                 220                 225

Asn Thr Asn Gln Met Asn Asn Thr His Ala Ala Val Ser Pro Cys
                230                 235                 240

Gly Arg Phe Val Ala Ser Cys Gly Phe Thr Pro Asp Val Lys Val
                245                 250                 255

Trp Glu Val Cys Phe Gly Lys Lys Gly Glu Phe Gln Glu Val Val
                260                 265                 270

Arg Ala Phe Glu Leu Lys Gly His Ser Ala Ala Val His Ser Phe
                275                 280                 285

Ala Phe Ser Asn Asp Ser Arg Arg Met Ala Ser Val Ser Lys Asp
                290                 295                 300

Gly Thr Trp Lys Leu Trp Asp Thr Asp Val Glu Tyr Lys Lys Lys
                305                 310                 315

Gln Asp Pro Tyr Leu Leu Lys Thr Gly Arg Phe Glu Glu Ala Ala
                320                 325                 330

Gly Ala Ala Pro Cys Arg Leu Ala Leu Ser Pro Asn Ala Gln Val
                335                 340                 345

Leu Ala Leu Ala Ser Gly Ser Ser Ile His Leu Tyr Asn Thr Arg
                350                 355                 360

Arg Gly Glu Lys Glu Glu Cys Phe Glu Arg Val His Gly Glu Cys
                365                 370                 375

Ile Ala Asn Leu Ser Phe Asp Ile Thr Gly Arg Phe Leu Ala Ser
                380                 385                 390

Cys Gly Asp Arg Ala Val Arg Leu Phe His Asn Thr Pro Gly His
                395                 400                 405

Arg Ala Met Val Glu Glu Met Gln Gly His Leu Lys Arg Ala Ser
                410                 415                 420

Asn Glu Ser Thr Arg Gln Arg Leu Gln Gln Gln Leu Thr Gln Ala
                425                 430                 435

Gln Glu Thr Leu Lys Ser Leu Gly Ala Leu Lys Lys
                440                 445

<210> 55
<211> 2968
<212> DNA
<213> Homo sapiens

<400> 55
ggcacgaggg gtaagcgcgt ctagggcgct gcgcggcgca gcgaaaatgg 50

cggcttccag gtgggcgcgc aaggccgtgg tcctgctttg tgcctctgac 100

ctgctgctgc tgctgctact gctaccaccg cctgggtcct gcgcggccga 150

aggctcgccc gggacgcccg acgagtctac cccacctccc cggaagaaga 200

agaaggatat tcgcgattac aatgatgcag acatggcgcg tcttctggag 250

caatgggaga aagatgatga cattgaagaa ggagatcttc cagagcacaa 300

gagaccttca gcacctgtcg acttctcaaa gatagaccca agcaagcctg 350

aaagcatatt gaaaatgacg aaaaaaggga agactctcat gatgtttgtc 400

actgtatcag gaagccctac tgagaaggag acagaggaaa ttacgagcct 450

ctggcagggc agccttttca atgccaacta tgacgtccag aggttcattg 500

tgggatcaga ccgtgctatc ttcatgcttc gcgatgggag ctacgcctgg 550

gagatcaagg actttttggt cggtcaagac aggtgtgctg atgtaactct 600

ggagggccag gtgtaccccg gcaaaggagg aggaagcaaa gagaaaaata 650

aaacaaagca agacaagggc aaaaaaaaga aggaaggaga tctgaaatct 700

cggtcttcca aggaagaaaa tcgagctggg aataaaagag aagacctgtg 750

atggggcagc agtgacgcgc tgtgggggga caggtggacg tggagagctc 800

tttgcccagc tcctggggtg ggagtggtct caggcaactg cacaccggat 850

gacattctag tgtcttctag aaagggtctg ccacatgacc agtttgtggt 900

caaagaatta ctgcttaata ggcttcaagt aagaagacag atgttttcta 950

attaatactg gacactgaca aattcatgtt tactataaaa tctccttaca 1000

tggaaatgtg actgtgttgc tttttcccat ttacacttga cccctgagcc 1050

ccttccagtg ctgcctgagg tgccctctac ctgtgcctgc ctctcgtctg 1100

ccagtttgat ttggacctgt ttttcccacc tcagccccca tgctcttgtc 1150

aaacgtgttt ggcctccagc caaacagggc ctgggaggaa aagagagtcc 1200

tgcttctgcc tggcttcccc atcggggagg ggagttgaag tgacagcctc 1250

ctgacagctt agaaatgtgg aggacgcagc aagttctgga catggccagc 1300
catatgaatg atatcttctc tttctttgaa ccatctccac ctgctcttat 1350
ggacaccccg ccttggggca aggcaaggga tgggcacaac ttggaagatt 1400
ttggctttgt gatcctcagt gtccagtgtc acccagatgg gccggaagct 1450
gcctttgaag ccaacgaatg ccatttgctc tgaaaagcaa tgatgagtct 1500
cctgagcaaa agccctatga gaggacggct gccccacccc gttccatggc 1550
tccccatcgc cagcccccca cgcccaccag gtaccgccta gccacactgc 1600
actgtggccc cttgggaaga gctctgtgct cagctactga tggtgcctga 1650
catgtgtttg tatggggcag gcagggtctc acctgagcct cactgccacc 1700
taggcagggt gatggctttg ttgtagacag gggagccatg cacagcagga 1750
aatggggcaa actgtaaggg aggagacctc ctaggaacct gcctaccttc 1800
gagctgcccc taggacaacc agcaagtccc tgcccctctc tgaagcccag 1850
tgttcccatc catcaaatga tggcattgat ctacaagatc tccttcccca 1900
ctctatcact ctgtggctct agaaagagtt ctgagaggtg aagaaatctg 1950
accaagagtg tggagtaaat ggtgcacccc agattagaac tctaaacccc 2000
tgaaacccac acagcactct ccatcttgca gttcacagct tgagcatccg 2050
aggggcatgg gaatccacgt ctatactcag cccatcttgg caggcaacat 2100
gcatttatca ccaccgggaa agtgccattc aacttaggtc accttacata 2150
catcagcttt tggcaaccgg gatcttaatt taaaagacag aggctccaaa 2200
atgatttcag gactgggctg gaaaggatac ttctcttttc cttatgtcct 2250
cctttggctg gtttgaaggg tttgcattgc ttctgatttt tctcattgcc 2300
ccatatgatg ggtttctgat tttgatgttt tccatagtga ggatggtgta 2350
taactaagaa gcccagcttt gagttaggaa gattaggatc caagtcctaa 2400
ccttaagcaa gtggcttggc tgttaaattc ttggtgtcct catcttaaat 2450
ggaaataata gtacctgctg cacaggttat taatgagtaa gtgagataat 2500
tataacatgt tgtgcaaagt gcaaggctga acattgaata gcaaccacaa 2550
tgatcattat cattattgcc cgtggagctt ctctgccgcc catacagcct 2600
ctctgaggac tctccctgca cctagttttt gtgatgataa tgggtgtcat 2650
gctgacatgt ccctcctgga tggcttcttc cagcacagcc agcaagcctg 2700
tgctaggctt caccaccgaa gctgcctggc cttggacaag ccatgtaggt 2750
tctctgggct ttagttttct ggaacaaata agagttttgc aagctgcaaa 2800
ggactgtaca aacgctaggc atcagctaga atgttgaaga agatgcagga 2850
cccctggcca gtctgaatag caggaattgg gtaggagggt gagactaagc 2900
cacttttctt atgatacttt tgttgaataa agggccactt tccaagcaaa 2950

aaaaaaaaaa aaaaaaaa 2968

<210> 56
<211> 234
<212> PRT
<213> Homo sapiens

<400> 56
Met Ala Ala Ser Arg Trp Ala Arg Lys Ala Val Val Leu Leu Cys
1               5                   10                  15

Ala Ser Asp Leu Leu Leu Leu Leu Leu Leu Leu Pro Pro Pro Gly
                20                  25                  30

Ser Cys Ala Ala Glu Gly Ser Pro Gly Thr Pro Asp Glu Ser Thr
                35                  40                  45

Pro Pro Pro Arg Lys Lys Lys Lys Asp Ile Arg Asp Tyr Asn Asp
                50                  55                  60

Ala Asp Met Ala Arg Leu Leu Glu Gln Trp Glu Lys Asp Asp Asp
                65                  70                  75

Ile Glu Glu Gly Asp Leu Pro Glu His Lys Arg Pro Ser Ala Pro
                80                  85                  90

Val Asp Phe Ser Lys Ile Asp Pro Ser Lys Pro Glu Ser Ile Leu
                95                  100                 105

Lys Met Thr Lys Lys Gly Lys Thr Leu Met Met Phe Val Thr Val
                110                 115                 120

Ser Gly Ser Pro Thr Glu Lys Glu Thr Glu Glu Ile Thr Ser Leu
                125                 130                 135

Trp Gln Gly Ser Leu Phe Asn Ala Asn Tyr Asp Val Gln Arg Phe
                140                 145                 150

Ile Val Gly Ser Asp Arg Ala Ile Phe Met Leu Arg Asp Gly Ser
                155                 160                 165

Tyr Ala Trp Glu Ile Lys Asp Phe Leu Val Gly Gln Asp Arg Cys
                170                 175                 180

Ala Asp Val Thr Leu Glu Gly Gln Val Tyr Pro Gly Lys Gly Gly
                185                 190                 195

Gly Ser Lys Glu Lys Asn Lys Thr Lys Gln Asp Lys Gly Lys Lys
                200                 205                 210

Lys Lys Glu Gly Asp Leu Lys Ser Arg Ser Ser Lys Glu Glu Asn
                215                 220                 225

Arg Ala Gly Asn Lys Arg Glu Asp Leu
                230

<210> 57
<211> 1175
<212> DNA
<213> Homo sapiens

<400> 57
ggcacgaggc ggagtctacg gaagccgttt tcgcttcact tttcctggct 50

gtagagcgct ttccccctgg cgggtgagag tgcagagacg aaggtgcgag 100

atgagcacta tgttcgcgga cactctcctc atcgttttta tctctgtgtg 150

cacggctctg ctcgcagagg gcataacctg ggtcctggtt tacaggacag 200

acaagtacaa gagactgaag gcagaagtgg aaaaacagag taaaaaattg 250

gaaaagaaga aggaaacaat aacagagtca gctggtcgac aacagaaaaa 300

gaaaatagag agacaagaag agaaactgaa gaataacaac agagatctat 350

caatggttcg aatgaaatcc atgtttgcta ttggcttttg ttttactgcc 400

ctaatgggaa tgttcaattc catatttgat ggtagagtgg tggcaaagct 450

tccttttacc cctctttctt acatccaagg actgtctcat cgaaatctgc 500

tgggagatga caccacagac tgttccttca ttttcctgta tattctctgt 550

actatgtcga ttcgacagaa cattcagaag attctcggcc ttgccccttc 600

acgagccgcc accaagcagg caggtggatt tcttggccca ccacctcctt 650

ctgggaagtt ctcttgaact caagaactct ttattttcta tcattctttc 700

tagacacaca cacatcagac tggcaactgt tttgtagcaa gagccatagg 750

tagccttact acttgggcct ctttctagtt ttgaattatt tttaagcctt 800

ttgggtatga ttagagtgaa aatggcagcc agcaaacttg atagtgcttt 850

tggtcctaga tgatttttat caaataagtg gattgattag ttaagttcag 900

gtaatgttta tgtaatgaaa aacaaatagc atccttcttg tttcatttac 950

ataagtattt tctgtgggac cgactctcaa ggcactgtgt atgccctgca 1000

agttggctgt ctatgagcat ttagagattt agaagaaaaa tttagtttgt 1050

ttaacccttg taactgtttg ttttgttgtt gttttttttt caagccaaat 1100

acatgacata agatcaataa agaggccaaa tttttagctg ttttatgtac 1150

aaggaaaaaa aaaaaaaaaa aaaaa 1175

<210> 58
<211> 188
<212> PRT
<213> Homo sapiens

<400> 58
Met Ser Thr Met Phe Ala Asp Thr Leu Leu Ile Val Phe Ile Ser
1               5                   10                  15

Val Cys Thr Ala Leu Leu Ala Glu Gly Ile Thr Trp Val Leu Val
                20                  25                  30

Tyr Arg Thr Asp Lys Tyr Lys Arg Leu Lys Ala Glu Val Glu Lys
                35                  40                  45

Gln Ser Lys Lys Leu Glu Lys Lys Lys Glu Thr Ile Thr Glu Ser
                50                  55                  60

Ala Gly Arg Gln Gln Lys Lys Lys Ile Glu Arg Gln Glu Glu Lys
                65                  70                  75

Leu Lys Asn Asn Asn Arg Asp Leu Ser Met Val Arg Met Lys Ser
                80                  85                  90

Met Phe Ala Ile Gly Phe Cys Phe Thr Ala Leu Met Gly Met Phe
                95                  100                 105

Asn Ser Ile Phe Asp Gly Arg Val Val Ala Lys Leu Pro Phe Thr
                110                 115                 120

Pro Leu Ser Tyr Ile Gln Gly Leu Ser His Arg Asn Leu Leu Gly
                125                 130                 135

Asp Asp Thr Thr Asp Cys Ser Phe Ile Phe Leu Tyr Ile Leu Cys
                140                 145                 150

Thr Met Ser Ile Arg Gln Asn Ile Gln Lys Ile Leu Gly Leu Ala
                155                 160                 165

Pro Ser Arg Ala Ala Thr Lys Gln Ala Gly Gly Phe Leu Gly Pro
                170                 175                 180

Pro Pro Pro Ser Gly Lys Phe Ser
                185

<210> 59
<211> 1152
<212> DNA
<213> Homo sapiens

<400> 59
gttcgccgcc gccgcgccgg ccacctggag ttttttcaga ctccagattt 50

ccctgtcaac cacgaggagt ccagagagga aacgcggagc ggagacaaca 100

gtacctgacg cctctttcag cccgggatcg ccccagcagg gatgggcgac 150

aagatctggc tgcccttccc cgtgctcctt ctggccgctc tgcctccggt 200

gctgctgcct ggggcggccg gcttcacacc ttccctcgat agcgacttca 250

cctttaccct tcccgccggc cagaaggagt gcttctacca gcccatgccc 300

ctgaaggcct cgctggagat cgagtaccaa gttttagatg gagcaggatt 350

agatattgat ttccatcttg cctctccaga aggcaaaacc ttagtttttg 400

aacaaagaaa atcagatgga gttcacactg tagagactga agttggtgat 450

tacatgttct gctttgacaa tacattcagc accatttctg agaaggtgat 500

tttctttgaa ttaatcctgg ataatatggg agaacaggca caagaacaag 550

aagattggaa gaaatatatt actggcacag atatattgga tatgaaactg 600

gaagacatcc tggaatccat caacagcatc aagtccagac taagcaaaag 650

tgggcacata caaactctgc ttagagcatt tgaagctcgt gatcgaaaca 700

tacaagaaag caactttgat agagtcaatt tctggtctat ggttaattta 750

gtggtcatgg tggtggtgtc agccattcaa gtttatatgc tgaagagtct 800

gtttgaagat aagaggaaaa gtagaactta aaactccaaa ctagagtacg 850

taacattgaa aaatgaggca taaaaatgca ataaactgtt acagtcaaga 900

ccattaatgg tcttctccaa aatattttga gatataaaag taggaaacag 950

gtataatttt aatgtgaaaa ttaagtcttc actttctgtg caagtaatcc 1000

tgctgatcca gttgtactta agtgtgtaac aggaatattt tgcagaatat 1050

aggtttaact gaatgaagcc atattaataa ctgcattttc ctaactttga 1100

aaaattttgc aaatgtctta ggtgatttaa ataaatgagt attgggccta 1150

aa 1152

<210> 60
<211> 229
<212> PRT
<213> Homo sapiens

<400> 60
Met Gly Asp Lys Ile Trp Leu Pro Phe Pro Val Leu Leu Leu Ala
1               5                   10                  15

Ala Leu Pro Pro Val Leu Leu Pro Gly Ala Ala Gly Phe Thr Pro
                20                  25                  30

Ser Leu Asp Ser Asp Phe Thr Phe Thr Leu Pro Ala Gly Gln Lys
                35                  40                  45

Glu Cys Phe Tyr Gln Pro Met Pro Leu Lys Ala Ser Leu Glu Ile
                50                  55                  60

Glu Tyr Gln Val Leu Asp Gly Ala Gly Leu Asp Ile Asp Phe His
                65                  70                  75

Leu Ala Ser Pro Glu Gly Lys Thr Leu Val Phe Glu Gln Arg Lys
                80                  85                  90

Ser Asp Gly Val His Thr Val Glu Thr Glu Val Gly Asp Tyr Met
                95                  100                 105

Phe Cys Phe Asp Asn Thr Phe Ser Thr Ile Ser Glu Lys Val Ile
                110                 115                 120

Phe Phe Glu Leu Ile Leu Asp Asn Met Gly Glu Gln Ala Gln Glu
                125                 130                 135

Gln Glu Asp Trp Lys Lys Tyr Ile Thr Gly Thr Asp Ile Leu Asp
                140                 145                 150

Met Lys Leu Glu Asp Ile Leu Glu Ser Ile Asn Ser Ile Lys Ser
                155                 160                 165

Arg Leu Ser Lys Ser Gly His Ile Gln Thr Leu Leu Arg Ala Phe
                170                 175                 180

Glu Ala Arg Asp Arg Asn Ile Gln Glu Ser Asn Phe Asp Arg Val
                185                 190                 195

Asn Phe Trp Ser Met Val Asn Leu Val Val Met Val Val Val Ser
                200                 205                 210

Ala Ile Gln Val Tyr Met Leu Lys Ser Leu Phe Glu Asp Lys Arg
                215                 220                 225

Lys Ser Arg Thr

<210> 61
<211> 2952
<212> DNA
<213> Homo sapiens

<400> 61
aactgatcgc ggcctagtcc cgacgcgtgt gtgctagtga gccggagccg 50

gcgacggcgg cagtggcggc ccggcctgca ggagcccgac ggggtctctg 100

ccatggggga gtgacgcgcc tgcacccgct gttccgcggc agcggcgaga 150
catgaggaga ccccgcgaca ggggcagcgg cggcggctcg tgagccccgg 200
gatggaggag aaatacggcg gggacgtgct ggccggcccc ggcggcggcg 250
gcggccttgg gccggtggac gtacccagcg ctcgattaac aaaatatatt 300
gtgttactat gtttcactaa atttttgaag gctgtgggac ttttcgaatc 350
atatgatctc ctaaaagctg ttcacattgt tcagttcatt tttatattaa 400
aacttgggac tgcatttttt atggttttgt ttcaaaagcc attttcttct 450
gggaaaacta ttaccaaaca ccagtggatc aaaatattta aacatgcagt 500
tgctgggtgt attatttcac tcttgtggtt ttttggcctc actctttgtg 550
gaccactaag gactttgctg ctatttgagc acagtgatat tgttgtcatt 600
tcactactca gtgttttgtt caccagttct ggaggaggac cagcaaagac 650
aaggggagct gcttttttca ttattgctgt gatctgttta ttgctttttg 700
acaatgatga tctcatggct aaaatggctg aacaccctga aggacatcat 750
gacagtgctc taactcatat gctttacaca gccattgcct tcttaggtgt 800
ggcagatcac aagggtggag tattattgct agtactggct ttgtgttgta 850
aagttggttt tcatacagct tccagaaagc tctctgtcga cgttggtgga 900
gctaaacgtc ttcaagcttt atctcatctt gtttctgtgc ttctcttgtg 950
cccatgggtc attgttcttt ctgtgacaac tgagagtaaa gtggagtctt 1000
ggttttctct cattatgcct tttgcaacgg ttatcttttt tgtcatgatc 1050
ctggatttct acgtggattc catttgttca gtcaaaatgg aagtttccaa 1100
atgtgctcgt tatggatcct ttcccatttt tattagtgct ctcctttttg 1150
gaaatttttg gacacatcca ataacagacc agcttcgggc tatgaacaaa 1200
gcagcacacc aggagagcac tgaacacgtc ctgtctggag gagtggtagt 1250
gagtgctata ttcttcattt tgtctgccaa tatcttatca tctccctcta 1300
agagaggaca aaaaggtacc cttattggat attctcctga aggaacacct 1350
ctttataact tcatgggtga tgcttttcag catagctctc aatcgatccc 1400
taggtttatt aaggaatcac taaaacaaat tcttgaggag agtgactcta 1450
ggcagatctt ttacttcttg tgcttgaatc tgctttttac ctttgtggaa 1500
ttattctatg gcgtgctgac caatagtctg ggcctgatct cggatggatt 1550
ccacatgctt tttgactgct ctgctttagt catgggactt tttgctgccc 1600
tgatgagtag gtggaaagcc actcggattt tctcctatgg gtacggccga 1650
atagaaattc tgtctggatt tattaatgga ctttttctaa tagtaatagc 1700
gttttttgtg tttatggagt cagtggctag attgattgat cctccagaat 1750
tagacactca catgttaaca ccagtctcag ttggagggct gatagtaaac 1800

cttattggta tctgtgcctt tagccatgcc catagccatg cccatggagc 1850

ttctcaagga agctgtcact catctgatca cagccattca caccatatgc 1900

atggacacag tgaccatggg catggtcaca gccacggatc tgcgggtgga 1950

ggcatgaatg ctaacatgag gggtgtattt ctacatgttt tggcagatac 2000

tcttggcagc attggtgtga tcgtatccac agttcttata gagcagtttg 2050

gatggttcat cgctgaccca ctctgttctc tttttattgc tatattaata 2100

tttctcagtg ttgttccact gattaaagat gcctgccagg ttctactcct 2150

gagattgcca ccagaatatg aaaaagaact acatattgct ttagaaaaga 2200

tacagaaaat tgaaggatta atatcatacc gagaccctca tttttggcgt 2250

cattctgcta gtattgtggc aggaacaatt catatacagg tgacatctga 2300

tgtgctagaa caaagaatag tacagcaggt tacaggaata cttaaagatg 2350

ctggagtaaa caatttaaca attcaagtgg aaaaggaggc atactttcaa 2400

catatgtctg gcctaagtac tggatttcat gatgttctgg ctatgacaaa 2450

acaaatggaa tccatgaaat actgcaaaga tggtacttac atcatgtgag 2500

ataactcaag aattacccct ggagaataaa caatgaagat taaatgactc 2550

agtatttgta atattgccag aaggataaaa attacacatt aactgtacag 2600

aaacagagtt ccctactact ggatcaagga atctttcttg aaggaaattt 2650

aaatacagaa tgaaacatta atggtaaaag tggagtaatt atttaaatta 2700

tgtgtataaa aggaatcaaa ttttgagtaa acatgatgta ttacatcatc 2750

ttcgaaaata gatatgatgg attctagtga agaccaaaat tacttctgtt 2800

tactttctat caggaagcat ctccattgta aatatgtatt tacatgttta 2850

ttacaaagac ccaaatgaaa aatttttagt ccattttttg catagcctaa 2900

agataaaata ggaataaaag ttctatattt atggaaaaaa aaaaaaaaaa 2950

aa 2952

<210> 62
<211> 594
<212> PRT
<213> Homo sapiens

<400> 62
Met Ala Lys Met Ala Glu His Pro Glu Gly His His Asp Ser Ala
1               5                   10                  15

Leu Thr His Met Leu Tyr Thr Ala Ile Ala Phe Leu Gly Val Ala
                20                  25                  30

Asp His Lys Gly Gly Val Leu Leu Leu Val Leu Ala Leu Cys Cys
                35                  40                  45

Lys Val Gly Phe His Thr Ala Ser Arg Lys Leu Ser Val Asp Val
                50                  55                  60

Gly Gly Ala Lys Arg Leu Gln Ala Leu Ser His Leu Val Ser Val

65                  70                  75

Leu Leu Leu Cys Pro Trp Val Ile Val Leu Ser Val Thr Thr Glu
                80                  85                  90

Ser Lys Val Glu Ser Trp Phe Ser Leu Ile Met Pro Phe Ala Thr
                95                  100                 105

Val Ile Phe Phe Val Met Ile Leu Asp Phe Tyr Val Asp Ser Ile
                110                 115                 120

Cys Ser Val Lys Met Glu Val Ser Lys Cys Ala Arg Tyr Gly Ser
                125                 130                 135

Phe Pro Ile Phe Ile Ser Ala Leu Leu Phe Gly Asn Phe Trp Thr
                140                 145                 150

His Pro Ile Thr Asp Gln Leu Arg Ala Met Asn Lys Ala Ala His
                155                 160                 165

Gln Glu Ser Thr Glu His Val Leu Ser Gly Gly Val Val Val Ser
                170                 175                 180

Ala Ile Phe Phe Ile Leu Ser Ala Asn Ile Leu Ser Ser Pro Ser
                185                 190                 195

Lys Arg Gly Gln Lys Gly Thr Leu Ile Gly Tyr Ser Pro Glu Gly
                200                 205                 210

Thr Pro Leu Tyr Asn Phe Met Gly Asp Ala Phe Gln His Ser Ser
                215                 220                 225

Gln Ser Ile Pro Arg Phe Ile Lys Glu Ser Leu Lys Gln Ile Leu
                230                 235                 240

Glu Glu Ser Asp Ser Arg Gln Ile Phe Tyr Phe Leu Cys Leu Asn
                245                 250                 255

Leu Leu Phe Thr Phe Val Glu Leu Phe Tyr Gly Val Leu Thr Asn
                260                 265                 270

Ser Leu Gly Leu Ile Ser Asp Gly Phe His Met Leu Phe Asp Cys
                275                 280                 285

Ser Ala Leu Val Met Gly Leu Phe Ala Ala Leu Met Ser Arg Trp
                290                 295                 300

Lys Ala Thr Arg Ile Phe Ser Tyr Gly Tyr Gly Arg Ile Glu Ile
                305                 310                 315

Leu Ser Gly Phe Ile Asn Gly Leu Phe Leu Ile Val Ile Ala Phe
                320                 325                 330

Phe Val Phe Met Glu Ser Val Ala Arg Leu Ile Asp Pro Pro Glu
                335                 340                 345

Leu Asp Thr His Met Leu Thr Pro Val Ser Val Gly Gly Leu Ile
                350                 355                 360

Val Asn Leu Ile Gly Ile Cys Ala Phe Ser His Ala His Ser His
                365                 370                 375

Ala His Gly Ala Ser Gln Gly Ser Cys His Ser Ser Asp His Ser
                380                 385                 390

His Ser His His Met His Gly His Ser Asp His Gly His Gly His
                395                 400                 405

Ser His Gly Ser Ala Gly Gly Gly Met Asn Ala Asn Met Arg Gly
                410                 415                 420

Val Phe Leu His Val Leu Ala Asp Thr Leu Gly Ser Ile Gly Val
                425                 430                 435

Ile Val Ser Thr Val Leu Ile Glu Gln Phe Gly Trp Phe Ile Ala
                440                 445                 450

Asp Pro Leu Cys Ser Leu Phe Ile Ala Ile Leu Ile Phe Leu Ser
                455                 460                 465

Val Val Pro Leu Ile Lys Asp Ala Cys Gln Val Leu Leu Leu Arg
                470                 475                 480

Leu Pro Pro Glu Tyr Glu Lys Glu Leu His Ile Ala Leu Glu Lys
                485                 490                 495

Ile Gln Lys Ile Glu Gly Leu Ile Ser Tyr Arg Asp Pro His Phe
                500                 505                 510

Trp Arg His Ser Ala Ser Ile Val Ala Gly Thr Ile His Ile Gln
                515                 520                 525

Val Thr Ser Asp Val Leu Glu Gln Arg Ile Val Gln Gln Val Thr
                530                 535                 540

Gly Ile Leu Lys Asp Ala Gly Val Asn Asn Leu Thr Ile Gln Val
                545                 550                 555

Glu Lys Glu Ala Tyr Phe Gln His Met Ser Gly Leu Ser Thr Gly
                560                 565                 570

Phe His Asp Val Leu Ala Met Thr Lys Gln Met Glu Ser Met Lys
                575                 580                 585

Tyr Cys Lys Asp Gly Thr Tyr Ile Met
                590

<210> 63
<211> 1669
<212> DNA
<213> Homo sapiens

<400> 63
ggggcgagac ctacgacgcc ggcgagcagt ggccgttacg cctaaaaaga 50

tggcggtctt ggcacctcta attgctctcg tgtattcggt gccgcgactt 100

tcacgatggc tcgcccaacc ttactacctt ctgtcggccc tgctctctgc 150

tgccttccta ctcgtgagga aactgccgcc gctctgccac ggtctgccca 200

cccaacgcga agacggtaac ccgtgtgact ttgactggag agaagtggag 250

atcctgatgt ttctcagtgc cattgtgatg atgaagaacc gcagatccat 300

cactgtggag caacatatag gcaacatttt catgtttagt aaagtggcca 350

acacaattct tttcttccgc ttggatattc gcatgggcct actttacatc 400

acactctgca tagtgttcct gatgacgtgc aaaccccccc tatatatggg 450

ccctgagtat atcaagtact tcaatgataa aaccattgat gaggaactag 500

aacgggacaa gagggtcact tggattgtgg agttctttgc caattggtct 550

aatgactgcc aatcatttgc ccctatctat gctgacctct cccttaaata 600

caactgtaca gggctaaatt ttgggaaggt ggatgttgga cgctatactg 650
atgttagtac gcggtacaaa gtgagcacat cacccctcac caagcaactc 700
cctaccctga tcctgttcca aggtggcaag gaggcaatgc ggcggccaca 750
gattgacaag aaaggacggg ctgtctcatg gaccttctct gaggagaatg 800
tgatccgaga atttaactta aatgagctat accagcgggc caagaaacta 850
tcaaaggctg gagacaatat ccctgaggag cagcctgtgg cttcaacccc 900
caccacagtg tcagatgggg aaaacaagaa ggataaataa gatcctcact 950
ttggcagtgc ttcctctcct gtcaattcca ggctctttcc ataaccacaa 1000
gcctgaggtg cagcttttat ttatgttttc cctttggctg tgactgggtg 1050
gggcagcatg cagcttctga ttttaaagag gcatctaggg aattgtcagg 1100
caccctacag gaaggcctgc catgcttgtg gccaactgtt tcactggagc 1150
aaagaaagag atctcatagg acggaggggg aaaatggttt tccctccaag 1200
cttgggtcag tgtgttaact gcttatcagc tattcagaca tctccatggt 1250
ttctccatga aactctgtgg tttcatcatt ccttcttagt tgacctgcac 1300
agcttggtta gacctagatt taaccctaag gtaagatgct ggggtataga 1350
acgctaagaa ttttccccca aggactcttg cttcctcaag cccttctggc 1400
ttcgtttatg gtcttcatta aaagtataag cctaactttg tcgctagtcc 1450
taaggagaaa cctttaacca caaagttttt atcattgaag acaatattga 1500
acaaccccct atttgtgggg gattgagaag gggtgaatag aggcttgaga 1550
ctttcctttg tgtggtagga cttggaggag aaatcccctg gactttcact 1600
aaccctctga catactcccc acacccagtt gatggctttc cgtaataaaa 1650
agattgggat ttcctttg 1669

<210> 64
<211> 296
<212> PRT
<213> Homo sapiens

<400> 64

<210> 65
<211> 1002
<212> DNA
<213> Homo sapiens

<400> 65
tgcagtctgt ctgagggcgg ccgaagtggc tggctcattt aagatgaggc 50

ttctgctgct tctcctagtg gcggcgtctg cgatggtccg gagcgaggcc 100

tcggccaatc tgggcggcgt gccagcaaga gattaaagat gcagtacgcc 150

acggggccgc tgctcaagtt ccagatttgt gtttcctgag gttataggcg 200

ggtgtttgag gagtacatgc gggttattag ccagcggtac ccagacatcc 250

gcattgaagg agagaattac ctccctcaac caatatatag acacatagca 300

tctttcctgt cagtcttcaa actagtatta ataggcttaa taattgttgg 350

caaggatcct tttgctttct ttggcatgca agctcctagc atctggcagt 400

ggggccaaga aaataaggtt tatgcatgta tgatggtttt cttcttgagc 450

aacatgattg agaaccagtg tatgtcaaca ggtgcatttg agataacttt 500

aaatgatgta cctgtgtggt ctaagctgga atctggtcac cttccatcca 550

tgcaacaact tgttcaaatt cttgacaatg aaatgaagct caatgtgcat 600

atggattcaa tcccacacca tcgatcatag caccacctat cagcactgaa 650

aactcttttg cattaaggga tcattgcaag agcagcgtga ctgacattat 700

gaaggcctgt actgaagaca gcaagctgtt agtacagacc agatgctttc 750

ttggcaggct cgttgtacct cttggaaaac ctcaatgcaa gatagtgttt 800

cagtgctggc atattttgga attctgcaca ttcatggagt gcaataatac 850

tgtatagctt tcccccacct cccacaaaat cacccagtta atgtgtgtgt 900

gtgtgttttt tttaaggtaa acattactac ttgtaacttt ttttctttag 950

tcatatttgg aaaaagtaga aaattggagt tacatttgga ttttttttcc 1000

aa 1002

<210> 66
<211> 163
<212> PRT
<213> Homo sapiens

<220>
<221> unsure
<222> 17
<223> unknown amino acid

<400> 66
Met Gln Tyr Ala Thr Gly Pro Leu Leu Lys Phe Gln Ile Cys Val
1               5                   10                  15

Ser Xaa Gly Tyr Arg Arg Val Phe Glu Glu Tyr Met Arg Val Ile
                20                  25                  30

Ser Gln Arg Tyr Pro Asp Ile Arg Ile Glu Gly Glu Asn Tyr Leu
                35                  40                  45

Pro Gln Pro Ile Tyr Arg His Ile Ala Ser Phe Leu Ser Val Phe
                50                  55                  60

Lys Leu Val Leu Ile Gly Leu Ile Ile Val Gly Lys Asp Pro Phe
                65                  70                  75

Ala Phe Phe Gly Met Gln Ala Pro Ser Ile Trp Gln Trp Gly Gln
                80                  85                  90

Glu Asn Lys Val Tyr Ala Cys Met Met Val Phe Phe Leu Ser Asn
                95                  100                 105

Met Ile Glu Asn Gln Cys Met Ser Thr Gly Ala Phe Glu Ile Thr
                110                 115                 120

Leu Asn Asp Val Pro Val Trp Ser Lys Leu Glu Ser Gly His Leu
                125                 130                 135

Pro Ser Met Gln Gln Leu Val Gln Ile Leu Asp Asn Glu Met Lys
                140                 145                 150

Leu Asn Val His Met Asp Ser Ile Pro His His Arg Ser
                155                 160

<210> 67

<211> 2412
<212> DNA
<213> Homo sapiens

<400> 67
ggcggcggtt gggccggtga tacccgggcg ctttatagtc ccgccgcctc 50

ctcctccacc tcctcctcct cctcctctcc tcctggagca gaggaggttg 100

tggcggtggc tggagaaagc ggcggcggag gatggaggaa ggaggcggcg 150

gcgtacggag tctggtcccg ggcgggccgg tgttactggt cctctgcggc 200

ctcctggagg cgtccggcgg cggccgagcc cttcctcaac tcagcgatga 250

catccctttc cgagtcaact ggcccggcac cgagttctct ctgcccacaa 300

ctggagtttt atataaagaa gataattatg tcatcatgac aactgcacat 350

aaagaaaaat ataaatgcat acttcccctt gtgacaagtg gggatgagga 400

agaagaaaag gattataaag gccctaatcc aagagagctt ttggagccac 450

tatttaaaca aagcagttgt tcctacagaa ttgagtctta ttggacttac 500

gaagtatgtc atggaaaaca cattcggcag taccatgaag agaaagaaac 550

tggtcagaaa ataaatattc acgagtacta ccttgggaat atgttggcca 600

agaaccttct atttgaaaaa gaacgagaag cagaagaaaa ggaaaaatca 650

aatgagattc ccactaaaaa tatcgaaggt cagatgacac catactatcc 700

tgtgggaatg ggaaatggta caccttgtag tttgaaacag aaccggccca 750

gatcaagtac tgtgatgtac atatgtcatc ctgaatctaa gcatgaaatt 800

ctttcagtag ctgaagttac aacttgtgaa tatgaagttg tcattttgac 850

accactcttg tgcagtcatc ctaaatatag gttcagagca tctcctgtga 900

atgacatatt ttgtcaatca ctgccaggat ctccatttaa gcccctcacc 950

ctgaggcagc tggagcagca ggaagaaata ctaagggtgc cttttaggag 1000

aaataaagag gaagatttgc aatcaactaa agaagagaga tttccagcga 1050

tccacaagtc gattgctatt ggctctcagc cagtgctcac tgttgggaca 1100

acccacatat ccaaattgac agatgaccaa ctcataaaag agtttcttag 1150

tggttcttac tgctttcgtg ggggtgtcgg ttggtggaaa tatgaattct 1200

gctatggcaa acatgtacat caataccatg aggacaagga tagtgggaaa 1250

acctctgtgg ttgtcgggac atggaaccaa gaagagcata ttgaatgggc 1300

taagaagaat actgctagag cttatcatct tcaagacgat ggtacccaga 1350

cagtcaggat ggtgtcacat ttttatggaa atggagatat ttgtgatata 1400

actgacaaac caagacaggt gactgtaaaa ctaaagtgca aagaatcaga 1450

ttcacctcat gctgttactg tatatatgct agagcctcac tcctgtcaat 1500

atattcttgg ggttgaatct ccagtgatct gtaaaatctt agatacagca 1550

gatgaaaatg gacttctttc tctccccaac taaaggatat taaagttagg 1600

ggaaagaaaa gatcattgaa agtcatgata atttctgtcc cactgtgtct 1650
cattatagag ttctcagcca ttggacctct tctaaaggat ggtataaaat 1700
gactctcaac cactttgtga atacatatgt gtatataaga ggttattgat 1750
aaacttctga ggcagacatt tgtctcgctt tttttcattt ttgttgtgtc 1800
ttataaactg actgtttttc tttgcttgga tactgtgatt ccaaaataaa 1850
tctcatccaa gcaagttaga gtccagccta atcaaatgtc ataattgttg 1900
tacctattga aagtttttaa ataatagatt tattatgtaa attatagtat 1950
atgtaagtag ctaatgaagt aaagatcatg aagaaagaaa ttgataggtg 2000
taaatgagag accatgtaaa atatgtaaat tctagtacct gaaatccttt 2050
caacagattt ttatatagca actgctctct gcaagtagtt aaactagaaa 2100
ctgggcacat ggtagaggct cacatgggag ttgtcctcac ccttgttaat 2150
ctcaagaaac tcttatttat aataggttgc ttctctctca gaacttttat 2200
ctattacttt tttcttctta tgagtatgtt tactctcaga gtatctatct 2250
gatgtagaca gttggtgatg cttctgagac tcagaatggt ttactctaac 2300
aaaacactgt gctgtctatc ccttgtactt gcctactgta atatggattt 2350
cacttctgaa cagtttacag cacaatattt attttaaagt gaataaaatg 2400
tccacaagca aa 2412

<210> 68
<211> 483
<212> PRT
<213> Homo sapiens

<400> 68
Met Glu Glu Gly Gly Gly Gly Val Arg Ser Leu Val Pro Gly Gly
1               5                   10                  15

Pro Val Leu Leu Val Leu Cys Gly Leu Leu Glu Ala Ser Gly Gly
                20                  25                  30

Gly Arg Ala Leu Pro Gln Leu Ser Asp Asp Ile Pro Phe Arg Val
                35                  40                  45

Asn Trp Pro Gly Thr Glu Phe Ser Leu Pro Thr Thr Gly Val Leu
                50                  55                  60

Tyr Lys Glu Asp Asn Tyr Val Ile Met Thr Thr Ala His Lys Glu
                65                  70                  75

Lys Tyr Lys Cys Ile Leu Pro Leu Val Thr Ser Gly Asp Glu Glu
                80                  85                  90

Glu Glu Lys Asp Tyr Lys Gly Pro Asn Pro Arg Glu Leu Leu Glu
                95                  100                 105

Pro Leu Phe Lys Gln Ser Ser Cys Ser Tyr Arg Ile Glu Ser Tyr
                110                 115                 120

Trp Thr Tyr Glu Val Cys His Gly Lys His Ile Arg Gln Tyr His
                125                 130                 135

Glu Glu Lys Glu Thr Gly Gln Lys Ile Asn Ile His Glu Tyr Tyr
                140                 145                 150

Leu Gly Asn Met Leu Ala Lys Asn Leu Leu Phe Glu Lys Glu Arg
                155                 160                 165

Glu Ala Glu Glu Lys Glu Lys Ser Asn Glu Ile Pro Thr Lys Asn
                170                 175                 180

Ile Glu Gly Gln Met Thr Pro Tyr Tyr Pro Val Gly Met Gly Asn
                185                 190                 195

Gly Thr Pro Cys Ser Leu Lys Gln Asn Arg Pro Arg Ser Ser Thr
                200                 205                 210

Val Met Tyr Ile Cys His Pro Glu Ser Lys His Glu Ile Leu Ser
                215                 220                 225

Val Ala Glu Val Thr Thr Cys Glu Tyr Glu Val Val Ile Leu Thr
                230                 235                 240

Pro Leu Leu Cys Ser His Pro Lys Tyr Arg Phe Arg Ala Ser Pro
                245                 250                 255

Val Asn Asp Ile Phe Cys Gln Ser Leu Pro Gly Ser Pro Phe Lys
                260                 265                 270

Pro Leu Thr Leu Arg Gln Leu Glu Gln Gln Glu Glu Ile Leu Arg
                275                 280                 285

Val Pro Phe Arg Arg Asn Lys Glu Glu Asp Leu Gln Ser Thr Lys
                290                 295                 300

Glu Glu Arg Phe Pro Ala Ile His Lys Ser Ile Ala Ile Gly Ser
                305                 310                 315

Gln Pro Val Leu Thr Val Gly Thr Thr His Ile Ser Lys Leu Thr
                320                 325                 330

Asp Asp Gln Leu Ile Lys Glu Phe Leu Ser Gly Ser Tyr Cys Phe
                335                 340                 345

Arg Gly Gly Val Gly Trp Trp Lys Tyr Glu Phe Cys Tyr Gly Lys
                350                 355                 360

His Val His Gln Tyr His Glu Asp Lys Asp Ser Gly Lys Thr Ser
                365                 370                 375

Val Val Val Gly Thr Trp Asn Gln Glu Glu His Ile Glu Trp Ala
                380                 385                 390

Lys Lys Asn Thr Ala Arg Ala Tyr His Leu Gln Asp Asp Gly Thr
                395                 400                 405

Gln Thr Val Arg Met Val Ser His Phe Tyr Gly Asn Gly Asp Ile
                410                 415                 420

Cys Asp Ile Thr Asp Lys Pro Arg Gln Val Thr Val Lys Leu Lys
                425                 430                 435

Cys Lys Glu Ser Asp Ser Pro His Ala Val Thr Val Tyr Met Leu
                440                 445                 450

Glu Pro His Ser Cys Gln Tyr Ile Leu Gly Val Glu Ser Pro Val
                455                 460                 465

Ile Cys Lys Ile Leu Asp Thr Ala Asp Glu Asn Gly Leu Leu Ser
                470                 475                 480

Leu Pro Asn

<210> 69
<211> 2004
<212> DNA
<213> Homo sapiens

<400> 69
aacaatagga aacgtcaaaa ttgggatagt cggcagttct ggcccctgca 50

gctggaggta ccctgagttc tgagggtcgt agtgctgttt ctggtattct 100

catcgcggtc acctctaccg gtgtggacaa gtaaagtttg aatcagcttc 150

tccatggcct gggcaccagt tcccggctga gccattttcc ttttggctaa 200

aagtccccgc ccagaggcca attcgtcgcg gcggcggtgg agatcgcagg 250

tcgctcaggc ttgcagatgg gtcaagggtt gtggagagtg gtcagaaacc 300

agcagctgca acaagaaggc tacagtgagc aaggctacct caccagagag 350

cagagcagga gaatggctgc gagcaacatt tctaacacca atcatcgtaa 400

acaagtccaa ggaggcattg acatatatca tcttttgaag gcaaggaaat 450

cgaaagaaca ggaaggattc attaatttgg aaatgttgcc tcctgagcta 500

agctttacca tcttgtccta cctgaatgca actgaccttt gcttggcttc 550

atgtgtttgg caggaccttg cgaatgatga acttctctgg caagggttgt 600

gcaaatccac ttggggtcac tggtccatat acaataagaa cccaccttta 650

ggattttctt ttagaaaagt gtatatgcag ctggatgaag gcagcctcac 700

ctttaatgcc aacccagatg agggagtgaa ctactttatg tccaagggta 750

tcctggatga ttcgccaaag gaaatagcaa agtttatctt ctgtacaaga 800

acactaaatt ggaaaaaact gagaatctat cttgatgaaa ggagagatgt 850

cttggatgac cttgtaacat tgcataattt tagaaatcag ttcttgccaa 900

atgcactgag agaatttttt cgtcatatcc atgcccctga agagcgtgga 950

gagtatcttg aaactcttat aacaaagttc tcacatagat tctgtgcttg 1000

caaccctgat ttaatgcgag aacttggcct tagtcctgat gctgtctatg 1050

tactgtgcta ctctttgatt ctactttcca ttgacctcac tagccctcat 1100

gtgaagaata aaatgtcaaa aagggaattt attcgaaata cccgtcgcgc 1150

tgctcaaaat attagtgaag attttgtagg gcatctttat gacaatatct 1200

accttattgg ccatgtggct gcataaaaag cacaattgct aggacttcag 1250

tttttacttc agactaaagc tacccaagga cttagcagat atgggggtta 1300

catcagtgct ggtcattgta gcctgagtat acaatcaagc ttcagtgtgc 1350

aacctttttt tcttttgcca ttttctattt tagtaatttc cttggggaac 1400

taaataattt tgcagaattt ttcctaattt tgtttatcac gttttgcaca 1450

aagcagagcc actgtctaac acagctgtta acgaatgata aactgacatt 1500

atactctaaa agatggtgta tttgtgcatt agatttgcct gaaaaacttt 1550

atccatttcc attctttata caaataccat gtaatgtgta catatttaac 1600

taaagagatt tatagtcata attattttat tgtaaagatt ttaactaaag 1650

tttttccttt tctctcaaac tgagttctga aatttatttg attctgatct 1700

gaaactattg tcttcgtaaa agttagatct gacttcagac agaaaccaat 1750

accagcttcc ttttccttta aactttgaag agtgttgatt tgttactata 1800

ttactatgca aaactggcag ttatttttat aatataaatt tataatttga 1850

tttttatttt taaaaactgg gttaatcaag tctcggtaag tcctttaaac 1900

catttaggat ttttaaaaca tcaaaattta tgatttacat tcataggaat 1950

aaaataaaat attattagaa ctctggtaaa aaaaaaaaaa aaaaaaaaaa 2000

aaaa 2004

<210> 70
<211> 319
<212> PRT
<213> Homo sapiens

<400> 70
Met Gly Gln Gly Leu Trp Arg Val Val Arg Asn Gln Gln Leu Gln
1               5                   10                  15

Gln Glu Gly Tyr Ser Glu Gln Gly Tyr Leu Thr Arg Glu Gln Ser
                20                  25                  30

Arg Arg Met Ala Ala Ser Asn Ile Ser Asn Thr Asn His Arg Lys
                35                  40                  45

Gln Val Gln Gly Gly Ile Asp Ile Tyr His Leu Leu Lys Ala Arg
                50                  55                  60

Lys Ser Lys Glu Gln Glu Gly Phe Ile Asn Leu Glu Met Leu Pro
                65                  70                  75

Pro Glu Leu Ser Phe Thr Ile Leu Ser Tyr Leu Asn Ala Thr Asp
                80                  85                  90

Leu Cys Leu Ala Ser Cys Val Trp Gln Asp Leu Ala Asn Asp Glu
                95                  100                 105

Leu Leu Trp Gln Gly Leu Cys Lys Ser Thr Trp Gly His Trp Ser
                110                 115                 120

Ile Tyr Asn Lys Asn Pro Pro Leu Gly Phe Ser Phe Arg Lys Val
                125                 130                 135

Tyr Met Gln Leu Asp Glu Gly Ser Leu Thr Phe Asn Ala Asn Pro
                140                 145                 150

Asp Glu Gly Val Asn Tyr Phe Met Ser Lys Gly Ile Leu Asp Asp
                155                 160                 165

Ser Pro Lys Glu Ile Ala Lys Phe Ile Phe Cys Thr Arg Thr Leu
                170                 175                 180

Asn Trp Lys Lys Leu Arg Ile Tyr Leu Asp Glu Arg Arg Asp Val
                185                 190                 195

Leu Asp Asp Leu Val Thr Leu His Asn Phe Arg Asn Gln Phe Leu
                200                 205                 210

Pro Asn Ala Leu Arg Glu Phe Phe Arg His Ile His Ala Pro Glu
                215                 220                 225

Glu Arg Gly Glu Tyr Leu Glu Thr Leu Ile Thr Lys Phe Ser His
                230                 235                 240

Arg Phe Cys Ala Cys Asn Pro Asp Leu Met Arg Glu Leu Gly Leu
                245                 250                 255

Ser Pro Asp Ala Val Tyr Val Leu Cys Tyr Ser Leu Ile Leu Leu
                260                 265                 270

Ser Ile Asp Leu Thr Ser Pro His Val Lys Asn Lys Met Ser Lys
                275                 280                 285

Arg Glu Phe Ile Arg Asn Thr Arg Arg Ala Ala Gln Asn Ile Ser
                290                 295                 300

Glu Asp Phe Val Gly His Leu Tyr Asp Asn Ile Tyr Leu Ile Gly
                305                 310                 315

His Val Ala Ala

<210> 71
<211> 1112
<212> DNA
<213> Homo sapiens

<400> 71
tgagagtcct ctagacaggc gctcctcgca gcaccgtagt gcgcttgcgc 50

tgagcagccc gcgagggcgg aagtgggagc tgcgaccgcg ctccctgtga 100

ggtgggcaag cggcgaaatg gcgccctccg ggagtcttgc agttcccctg 150

gcagtcctgg tgctgttgct ttggggtgct ccctggacgc acgggcggcg 200

gagcaacgtt cgcgtcatca cggacgagaa ctggagagaa ctgctggaag 250

gagactggat gatagaattt tatgccccgt ggtgccctgc ttgtcaaaat 300

cttcaaccgg aatgggaaag ttttgctgaa tggggagaag atcttgaggt 350

taatattgcg aaagtagatg tcacagagca gccaggactg agtggacggt 400

ttatcataac tgctcttcct actatttatc attgtaaaga tggtgaattt 450

aggcgctatc agggtccaag gactaagaag gacttcataa actttataag 500

tgataaagag tggaagagta ttgagcccgt ttcatcatgg tttggtccag 550

gttctgttct gatgagtagt atgtcagcac tctttcagct atctatgtgg 600

atcaggactt gccataacta ctttattgaa gaccttggat tgccagtgtg 650

gggatcatat actgtttttg ctttagcaac tctgttttcc ggactgttat 700

taggactctg tatgatattt gtggcagatt gcctttgtcc ttcaaaaagg 750

cgcagaccac agccgtaccc ataccttcaa aaaaattat tatcagaatc 800

tgcacaacct ttgaaaaaag tggaggagga acaagaggcg gatgaagaag 850

atgtttcaga agaagaagct gaaagtaaag aaggaacaaa caaagacttt 900

ccacagaatg ccataagaca acgctctctg ggtccatcat tggccacaga 950

taaatcctag ttaaatttta tagttatctt aatattatga ttttgataaa 1000

aacagaagat tgatcatttt gtttggtttg aagtgaactg tgactttttt 1050

gaatattgca gggttcagtc tagattgtca ttaaattgaa gagtctacat 1100

tcagaacata aa 1112

<210> 72
<211> 280
<212> PRT
<213> Homo sapiens

<400> 72
Met Ala Pro Ser Gly Ser Leu Ala Val Pro Leu Ala Val Leu Val
1               5                   10                  15

Leu Leu Leu Trp Gly Ala Pro Trp Thr His Gly Arg Arg Ser Asn
                20                  25                  30

Val Arg Val Ile Thr Asp Glu Asn Trp Arg Glu Leu Leu Glu Gly
                35                  40                  45

Asp Trp Met Ile Glu Phe Tyr Ala Pro Trp Cys Pro Ala Cys Gln
                50                  55                  60

Asn Leu Gln Pro Glu Trp Glu Ser Phe Ala Glu Trp Gly Glu Asp
                65                  70                  75

Leu Glu Val Asn Ile Ala Lys Val Asp Val Thr Glu Gln Pro Gly
                80                  85                  90

Leu Ser Gly Arg Phe Ile Ile Thr Ala Leu Pro Thr Ile Tyr His
                95                  100                 105

Cys Lys Asp Gly Glu Phe Arg Arg Tyr Gln Gly Pro Arg Thr Lys
                110                 115                 120

Lys Asp Phe Ile Asn Phe Ile Ser Asp Lys Glu Trp Lys Ser Ile
                125                 130                 135

Glu Pro Val Ser Ser Trp Phe Gly Pro Gly Ser Val Leu Met Ser
                140                 145                 150

Ser Met Ser Ala Leu Phe Gln Leu Ser Met Trp Ile Arg Thr Cys
                155                 160                 165

His Asn Tyr Phe Ile Glu Asp Leu Gly Leu Pro Val Trp Gly Ser
                170                 175                 180

Tyr Thr Val Phe Ala Leu Ala Thr Leu Phe Ser Gly Leu Leu Leu
                185                 190                 195

Gly Leu Cys Met Ile Phe Val Ala Asp Cys Leu Cys Pro Ser Lys
                200                 205                 210

Arg Arg Arg Pro Gln Pro Tyr Pro Tyr Pro Ser Lys Lys Leu Leu
                215                 220                 225

Ser Glu Ser Ala Gln Pro Leu Lys Lys Val Glu Glu Glu Gln Glu
                230                 235                 240

Ala Asp Glu Glu Asp Val Ser Glu Glu Glu Ala Glu Ser Lys Glu
                245                 250                 255

Gly Thr Asn Lys Asp Phe Pro Gln Asn Ala Ile Arg Gln Arg Ser
                260                 265                 270

Leu Gly Pro Ser Leu Ala Thr Asp Lys Ser
                275                 280

<210> 73
<211> 1491
<212> DNA
<213> Homo sapiens

<400> 73
gcggcggcgg cagcggcggc gacggcgaca tggagagcgg ggcctacggc 50

gcggccaagg cgggcggctc cttcgacctg cggcgcttcc tgacgcagcc 100

gcaggtggtg gcgcgcgccg tgtgcttggt cttcgccttg atcgtgttct 150

cctgcatcta tggtgagggc tacagcaatg cccacgagtc taagcagatg 200

tactgcgtgt tcaaccgcaa cgaggatgcc tgccgctatg gcagtgccat 250

cggggtgctg gccttcctgg cctcggcttt cttcttggtg gtcgacgcgt 300

atttccccca gatcagcaac gccactgacc gcaagtacct ggtcattggt 350

gacctgctct tctcagctct ctggaccttc ctgtggtttg ttggtttctg 400

cttcctcacc aaccagtggg cagtcaccaa cccgaaggac gtgctggtgg 450

gggccgactc tgtgagggca gccatcacct tcagcttctt ttccatcttc 500

tcctggggtg tgctggcctc cctggcctac cagcgctaca aggctggcgt 550

ggacgacttc atccagaatt acgttgaccc cactccggac cccaacactg 600

cctacgcctc ctacccaggt gcatctgtgg acaactacca acagccaccc 650

ttcacccaga acgcggagac caccgagggc taccagccgc cccctgtgta 700

ctgagcggcg gttagcgtgg gaagggggac agagagggcc ctcccctctg 750

ccctggactt tcccatgagc ctcctggaac tgccagcccc tctctttcac 800

ctgttccatc ctgtgcagct gacacacagc taaggagcct catagcctgg 850

cgggggctgg cagagccaca ccccaagtgc ctgtgcccag agggcttcag 900

tcagccgctc actcctccag ggcactttta ggaaagggtt tttagctagt 950

gtttttcctc gcttttaatg acctcagccc cgcctgcagt ggctagaagc 1000

cagcaggtgc ccatgtgcta ctgacaagtg cctcagcttc cccccggccc 1050

gggtcaggcc gtgggagccg ctattatctg cgttctctgc caaagactcg 1100

tgggggccat cacacctgcc ctgtgcagcg gagccggacc aggctcttgt 1150

gtcctcactc aggtttgctt cccctgtgcc cactgctgta tgatctgggg 1200

gccaccaccc tgtgccggtg gcctctgggc tgcctcccgt ggtgtgaggg 1250

cggggctggt gctcatggca cttcctcctt gctcccaccc ctggcagcag 1300

ggaagggctt tgcctgacaa cacccagctt tatgtaaata ttctgcagtt 1350

gttacttagg aagcctgggg agggcagggg tgccccatgg ctcccagact 1400

ctgtctgtgc cgagtgtatt ataaaatcgt gggggagatg cccggcctgg 1450

gatgctgttt ggagacggaa taaatgtttt ctcattcagt a 1491

<210> 74
<211> 224
<212> PRT
<213> Homo sapiens

<400> 74
Met Glu Ser Gly Ala Tyr Gly Ala Ala Lys Ala Gly Gly Ser Phe
1               5                   10                  15

Asp Leu Arg Arg Phe Leu Thr Gln Pro Gln Val Val Ala Arg Ala
                20                  25                  30

Val Cys Leu Val Phe Ala Leu Ile Val Phe Ser Cys Ile Tyr Gly
                35                  40                  45

Glu Gly Tyr Ser Asn Ala His Glu Ser Lys Gln Met Tyr Cys Val
                50                  55                  60

Phe Asn Arg Asn Glu Asp Ala Cys Arg Tyr Gly Ser Ala Ile Gly
                65                  70                  75

Val Leu Ala Phe Leu Ala Ser Ala Phe Phe Leu Val Val Asp Ala
                80                  85                  90

Tyr Phe Pro Gln Ile Ser Asn Ala Thr Asp Arg Lys Tyr Leu Val
                95                  100                 105

Ile Gly Asp Leu Leu Phe Ser Ala Leu Trp Thr Phe Leu Trp Phe
                110                 115                 120

Val Gly Phe Cys Phe Leu Thr Asn Gln Trp Ala Val Thr Asn Pro
                125                 130                 135

Lys Asp Val Leu Val Gly Ala Asp Ser Val Arg Ala Ala Ile Thr
                140                 145                 150

Phe Ser Phe Phe Ser Ile Phe Ser Trp Gly Val Leu Ala Ser Leu
                155                 160                 165

Ala Tyr Gln Arg Tyr Lys Ala Gly Val Asp Asp Phe Ile Gln Asn
                170                 175                 180

Tyr Val Asp Pro Thr Pro Asp Pro Asn Thr Ala Tyr Ala Ser Tyr
                185                 190                 195

Pro Gly Ala Ser Val Asp Asn Tyr Gln Gln Pro Pro Phe Thr Gln
                200                 205                 210

Asn Ala Glu Thr Thr Glu Gly Tyr Gln Pro Pro Pro Val Tyr
                215                 220

<210> 75
<211> 1072
<212> DNA
<213> Mus musculus

<400> 75
cagagctgct gtcatggcgg ccgctctgtg gggcttcttt cccgtcctgc 50

tgctgctgct gctatcgggg gatgtccaga gctcggaggt gcccggggct 100

gctgctgagg gatcgggagg gagtggggtc ggcataggag atcgcttcaa 150

gattgagggg cgtgcagttg ttccaggggt gaagcctcag gactggatct 200

cggcggcccg agtgctggta gacggagaag agcacgtcgg tttccttaag 250

acagatggga gttttgtggt tcatgatata ccttctggat cttatgtagt 300

ggaagttgta tctccagctt acagatttga tcccgttcga gtggatatca 350

cttcgaaagg aaaaatgaga gcaagatatg tgaattacat caaaacatca 400

gaggttgtca gactgcccta tcctctccaa atgaaatctt caggtccacc 450

ttcttacttt attaaaaggg aatcgtgggg ctggacagac tttctaatga 500

acccaatggt tatgatgatg gttcttcctt tattgatatt tgtgcttctg 550

cctaaagtgg tcaacacaag tgatcctgac atgagacggg aaatggagca 600

gtcaatgaat atgctgaatt ccaaccatga gttgcctgat gtttctgagt 650

tcatgacaag actcttctct tcaaaatcat ctggcaaatc tagcagcggc 700

agcagtaaaa caggcaaaag tggggctggc aaaaggaggt agtcaggccg 750

tccagagctg gcatttgcac aaacacggca acactgggtg gcatccaagt 800

cttggaaaac cgtgtgaagc aactactata aacttgagtc atcccgacgt 850

tgatctctta caactgtgta tgttaacttt ttagcacatg ttttgtactt 900

ggtacacgag aaaacccagc tttcatcttt tgtctgtatg aggtcaatat 950

tgatgtcact gaattaatta cagtgtccta tagaaaatgc cattaataaa 1000

ttatatgaac tactatacat tatgtatatt aattaaaaca tcttaatcca 1050

gaaaaaaaaa aaaaaaaaaa aa 1072

<210> 76
<211> 242
<212> PRT
<213> Mus musculus

<400> 76
Met Ala Ala Ala Leu Trp Gly Phe Phe Pro Val Leu Leu Leu Leu
1               5                   10                  15

Leu Leu Ser Gly Asp Val Gln Ser Ser Glu Val Pro Gly Ala Ala
                20                  25                  30

Ala Glu Gly Ser Gly Gly Ser Gly Val Gly Ile Gly Asp Arg Phe
                35                  40                  45

Lys Ile Glu Gly Arg Ala Val Val Pro Gly Val Lys Pro Gln Asp
                50                  55                  60

Trp Ile Ser Ala Ala Arg Val Leu Val Asp Gly Glu Glu His Val
                65                  70                  75

Gly Phe Leu Lys Thr Asp Gly Ser Phe Val Val His Asp Ile Pro
                80                  85                  90

Ser Gly Ser Tyr Val Val Glu Val Val Ser Pro Ala Tyr Arg Phe
                95                  100                 105

Asp Pro Val Arg Val Asp Ile Thr Ser Lys Gly Lys Met Arg Ala
                110                 115                 120

Arg Tyr Val Asn Tyr Ile Lys Thr Ser Glu Val Val Arg Leu Pro
                125                 130                 135

Tyr Pro Leu Gln Met Lys Ser Ser Gly Pro Pro Ser Tyr Phe Ile
                140                 145                 150

Lys Arg Glu Ser Trp Gly Trp Thr Asp Phe Leu Met Asn Pro Met
                155                 160                 165

Val Met Met Met Val Leu Pro Leu Leu Ile Phe Val Leu Leu Pro
                170                 175                 180

Lys Val Val Asn Thr Ser Asp Pro Asp Met Arg Arg Glu Met Glu
                185                 190                 195

Gln Ser Met Asn Met Leu Asn Ser Asn His Glu Leu Pro Asp Val
                200                 205                 210

Ser Glu Phe Met Thr Arg Leu Phe Ser Ser Lys Ser Ser Gly Lys
                215                 220                 225

Ser Ser Ser Gly Ser Ser Lys Thr Gly Lys Ser Gly Ala Gly Lys
                230                 235                 240

Arg Arg

<210> 77
<211> 2241
<212> DNA
<213> Homo sapiens

<400> 77
tgggacttat agaagggaga ggagcgaaca tggcagcgcg ttggcggttt 50

tggtgtgtct ctgtgaccat ggtggtggcg ctgctcatcg tttgcgacgt 100

tccctcagcc tctgcccaaa gaaagaagga gatggtgtta tcagaaaagg 150

ttagtcagct gatggaatgg actaacaaaa gacctgtaat aagaatgaat 200

ggagacaagt tccgtcgcct tgtgaaagcc ccaccgagaa attactccgt 250

tatcgtcatg ttcactgctc tccaactgca tagacagtgt gtcgtttgca 300

agcaagctga tgaagaattc cagatcctgg caaactcctg gcgatactcc 350

agtgcattca ccaacaggat attttttgcc atggtggatt ttgatgaagg 400

ctctgatgta tttcagatgc taaacatgaa ttcagctcca actttcatca 450

actttcctgc aaaagggaaa cccaaacggg gtgatacata tgagttacag 500

gtgcggggtt tttcagctga gcagattgcc cggtggatcg ccgacagaac 550

tgatgtcaat attagagtga ttagaccccc aaattatgct ggtcccctta 600

tgttgggatt gcttttggct gttattggtg gacttgtgta tcttcgaaga 650

agtaatatgg aatttctctt taataaaact ggatgggctt ttgcagcttt 700

gtgttttgtg cttgctatga catctggtca aatgtggaac catataagag 750

gaccaccata tgcccataag aatccccaca cgggacatgt gaattatatc 800

catggaagca gtcaagccca gtttgtagct gaaacacaca ttgttcttct 850

gtttaatggt ggagttacct taggaatggt gcttttgtgt gaagctgcta 900

cctctgacat ggatattgga aagcgaaaga taatgtgtgt ggctggtatt 950
ggacttgttg tattattctt cagttggatg ctctctattt ttagatctaa 1000
atatcatggc tacccataca gctttctgat gagttaaaaa ggtcccagag 1050
atatatagac actggagtac tggaaattga aaaacgaaaa tcgtgtgtgt 1100
ttgaaaagaa gaatgcaact tgtatattct gtattacctc tttttttcaa 1150
gtgatttaaa tagttaatca tttaaccaaa gaagatgtgt agtgccttaa 1200
caagcaatcc tctgtcaaaa tctgaggtat ttgaaaataa ttatcctctt 1250
aaccttctct tcccagtgaa ctttatggaa catttaattt agtacaatta 1300
agtatattat aaaaattgta aaactactac tttgttttag ttagaacaaa 1350
gctcaaaact actttagtta acttggtcat ctgatcttat attgccttat 1400
ccaaagatgg ggaaagtaag tcctgaccag gtgttcccac atatgcctgt 1450
tacagataac tacattagga attcattctt agcttcttca tctttgtgtg 1500
gatgtgtata ctttacgcat ctttcctttt gagtagagaa attatgtgtg 1550
tcatgtggtc ttctgaaaat ggaacaccat tcttcagagc acacgtctag 1600
ccctcagcaa gacagttgtt tctcctcctc cttgcatatt tcctactgcg 1650
ctccagcctg agtgatagag tgagactctg tctcaaaaaa aaagtatctc 1700
taaatacagg attataattt ctgcttgagt atggtgttaa ctaccttgta 1750
tttagaaaga tttcagattc attccatctc cttagttttc ttttaaggtg 1800
acccatctgt gataaaaata tagcttagtg ctaaaatcag tgtaacttat 1850
acatggccta aaatgtttct acaaattaga gtttgtcact tattccattt 1900
gtacctaaga gaaaaatagg ctcagttaga aaaggactcc ctggccaggc 1950
gcagtgactt acgcctgtaa tctcagcact ttgggaggcc aaggcaggca 2000
gatcacgagg tcaggagttc gagaccatcc tggccaacat ggtgaaaccc 2050
cgtctctact aaaaatataa aaattagctg ggtgtggtgg caggagcctg 2100
taatcccagc tgcacaggag gctgaggcac gagaatcact tgaactcagg 2150
agatggaggt ttcagtgagc cgagatcacg ccactgcact ccagcctggc 2200
aacagagcga gactccatct caaaaaaaaa aaaaaaaaaa a 2241

<210> 78
<211> 335
<212> PRT
<213> Homo sapiens

<400> 78
Met Ala Ala Arg Trp Arg Phe Trp Cys Val Ser Val Thr Met Val
1               5                   10                  15

Val Ala Leu Leu Ile Val Cys Asp Val Pro Ser Ala Ser Ala Gln
                20                  25                  30

Arg Lys Lys Glu Met Val Leu Ser Glu Lys Val Ser Gln Leu Met

35                  40                  45

Glu Trp Thr Asn Lys Arg Pro Val Ile Arg Met Asn Gly Asp Lys
                50                  55                  60

Phe Arg Arg Leu Val Lys Ala Pro Pro Arg Asn Tyr Ser Val Ile
                65                  70                  75

Val Met Phe Thr Ala Leu Gln Leu His Arg Gln Cys Val Val Cys
                80                  85                  90

Lys Gln Ala Asp Glu Glu Phe Gln Ile Leu Ala Asn Ser Trp Arg
                95                  100                 105

Tyr Ser Ser Ala Phe Thr Asn Arg Ile Phe Phe Ala Met Val Asp
                110                 115                 120

Phe Asp Glu Gly Ser Asp Val Phe Gln Met Leu Asn Met Asn Ser
                125                 130                 135

Ala Pro Thr Phe Ile Asn Phe Pro Ala Lys Gly Lys Pro Lys Arg
                140                 145                 150

Gly Asp Thr Tyr Glu Leu Gln Val Arg Gly Phe Ser Ala Glu Gln
                155                 160                 165

Ile Ala Arg Trp Ile Ala Asp Arg Thr Asp Val Asn Ile Arg Val
                170                 175                 180

Ile Arg Pro Pro Asn Tyr Ala Gly Pro Leu Met Leu Gly Leu Leu
                185                 190                 195

Leu Ala Val Ile Gly Gly Leu Val Tyr Leu Arg Arg Ser Asn Met
                200                 205                 210

Glu Phe Leu Phe Asn Lys Thr Gly Trp Ala Phe Ala Ala Leu Cys
                215                 220                 225

Phe Val Leu Ala Met Thr Ser Gly Gln Met Trp Asn His Ile Arg
                230                 235                 240

Gly Pro Pro Tyr Ala His Lys Asn Pro His Thr Gly His Val Asn
                245                 250                 255

Tyr Ile His Gly Ser Ser Gln Ala Gln Phe Val Ala Glu Thr His
                260                 265                 270

Ile Val Leu Leu Phe Asn Gly Gly Val Thr Leu Gly Met Val Leu
                275                 280                 285

Leu Cys Glu Ala Ala Thr Ser Asp Met Asp Ile Gly Lys Arg Lys
                290                 295                 300

Ile Met Cys Val Ala Gly Ile Gly Leu Val Val Leu Phe Phe Ser
                305                 310                 315

Trp Met Leu Ser Ile Phe Arg Ser Lys Tyr His Gly Tyr Pro Tyr
                320                 325                 330

Ser Phe Leu Met Ser
                335

<210> 79
<211> 2054
<212> DNA
<213> Homo sapiens

<400> 79

gggggaggcc cgcgtcgatc ctgggttgga ggaggtggcg gccgctgagg 50
ctgcggcgtg aagacggcgg gcatggtggg gcgggagaaa gagctctcta 100
tacactttgt tcccgggagc tgtcggctgg tggaggagga agttaacatc 150
cctaatagga gggttctggt tactggtgcc actgggcttc ttggcagagc 200
tgtacacaaa gaatttcagc agaataattg gcatgcagtt ggctgtggtt 250
tcagaagagc aagaccaaaa tttgaacagg ttaatctgtt ggattctaat 300
gcagttcatc acatcattca tgattttcag ccccatgtta tagtacattg 350
tgcagcagag agaagaccag atgttgtaga aaatcagcca gatgctgcct 400
ctcaacttaa tgtggatgct tctgggaatt tagcaaagga agcagctgct 450
gttggagcat ttctcatcta cattagctca gattatgtat ttgatggaac 500
aaatccacct tacagagagg aagacatacc agctccccta aatttgtatg 550
gcaaaacaaa attagatgga gaaaaggctg tcctggagaa caatctagga 600
gctgctgttt tgaggattcc tattctgtat ggggaagttg aaaagctcga 650
agaaagtgca gtgactgtta tgtttgataa agtgcagttc agcaacaagt 700
cagcaaacat ggatcactgg cagcagaggt tccccacaca tgtcaaagat 750
gtggccactg tgtgccggca gctagcagag aagagaatgc tggatccatc 800
aattaaggga acctttcact ggtctggcaa tgaacagatg actaagtatg 850
aaatggcatg tgcaattgca gatgccttca acctccccag cagtcactta 900
agacctatta ctgacagccc tgtcctagga gcacaacgtc cgagaaatgc 950
tcagcttgac tgctccaaat tggagacctt gggcattggc caacgaacac 1000
catttcgaat tggaatcaaa gaatcacttt ggcctttcct cattgacaag 1050
agatggagac aaacggtctt tcattagttt atttgtgttg ggttcttttt 1100
ttttttaaat gaaaagtata gtatgtggcc ctttttaaag aacaaaggaa 1150
atagttttgt atgagtactt taattgtgac tcttaggatc tttcaggtaa 1200
atgatgctct tgcactagtg aaattgtcta aagaaactaa agggcagtca 1250
tgccctgttt gcagtaattt ttctttttat cattatgttt gtcctggcta 1300
aacttggagt ttgagtatag taaattatga tccttaaata tttgagggtc 1350
aggatgaagc agatctgctg tagacttttc agatgaaatt gttcattctc 1400
gtaacctcca tattttcagg atttttgaag ctgttgacca tttcatgttg 1450
attattttaa attgtgtgga atagtataaa aatcattggt gttcattatt 1500
tgctttgcct gagctcagat caaaatgttt gaagaaagga actttatttt 1550
tgcaagttac gtacagtttt tatgcttgag atatttcaac atgttatgta 1600
tattggaact tctacagctt gatgcctcct gcttttatag cagtttatgg 1650
ggagcacttg aaagagcgtg tgtacatgta ttttttttct aggcaaacat 1700

tgaatgcaaa cgtgtatttt tttaatataa atatataact gtccttttca 1750

tcccatgttg ccgctaagtg atatttcata tgtgtggtta tactcataat 1800

aatgggcctt gtaagtcttt tcaccattca tgaataataa taaatatgta 1850

ctgctggcat gtaatgctta gttttcttgt atttacttct tttttttaaa 1900

tgtaaggacc aaacttctaa actaattgtt cttttgttgc tttaattttt 1950

aaaaattaca ttcttctgat gtaacatgtg atacatacaa aagaatatag 2000

tttaatatgt attgaaataa aacacaataa aattaaaaaa aaaaaaaaaa 2050

aaaa 2054

<210> 80
<211> 334
<212> PRT
<213> Homo sapiens

<400> 80
Met Val Gly Arg Glu Lys Glu Leu Ser Ile His Phe Val Pro Gly
1               5                   10                  15

Ser Cys Arg Leu Val Glu Glu Glu Val Asn Ile Pro Asn Arg Arg
                20                  25                  30

Val Leu Val Thr Gly Ala Thr Gly Leu Leu Gly Arg Ala Val His
                35                  40                  45

Lys Glu Phe Gln Gln Asn Asn Trp His Ala Val Gly Cys Gly Phe
                50                  55                  60

Arg Arg Ala Arg Pro Lys Phe Glu Gln Val Asn Leu Leu Asp Ser
                65                  70                  75

Asn Ala Val His His Ile Ile His Asp Phe Gln Pro His Val Ile
                80                  85                  90

Val His Cys Ala Ala Glu Arg Arg Pro Asp Val Val Glu Asn Gln
                95                  100                 105

Pro Asp Ala Ala Ser Gln Leu Asn Val Asp Ala Ser Gly Asn Leu
                110                 115                 120

Ala Lys Glu Ala Ala Ala Val Gly Ala Phe Leu Ile Tyr Ile Ser
                125                 130                 135

Ser Asp Tyr Val Phe Asp Gly Thr Asn Pro Pro Tyr Arg Glu Glu
                140                 145                 150

Asp Ile Pro Ala Pro Leu Asn Leu Tyr Gly Lys Thr Lys Leu Asp
                155                 160                 165

Gly Glu Lys Ala Val Leu Glu Asn Asn Leu Gly Ala Ala Val Leu
                170                 175                 180

Arg Ile Pro Ile Leu Tyr Gly Glu Val Glu Lys Leu Glu Glu Ser
                185                 190                 195

Ala Val Thr Val Met Phe Asp Lys Val Gln Phe Ser Asn Lys Ser
                200                 205                 210

Ala Asn Met Asp His Trp Gln Gln Arg Phe Pro Thr His Val Lys
                215                 220                 225

Asp Val Ala Thr Val Cys Arg Gln Leu Ala Glu Lys Arg Met Leu

                230                 235                 240

Asp Pro Ser Ile Lys Gly Thr Phe His Trp Ser Gly Asn Glu Gln
                245                 250                 255

Met Thr Lys Tyr Glu Met Ala Cys Ala Ile Ala Asp Ala Phe Asn
                260                 265                 270

Leu Pro Ser Ser His Leu Arg Pro Ile Thr Asp Ser Pro Val Leu
                275                 280                 285

Gly Ala Gln Arg Pro Arg Asn Ala Gln Leu Asp Cys Ser Lys Leu
                290                 295                 300

Glu Thr Leu Gly Ile Gly Gln Arg Thr Pro Phe Arg Ile Gly Ile
                305                 310                 315

Lys Glu Ser Leu Trp Pro Phe Leu Ile Asp Lys Arg Trp Arg Gln
                320                 325                 330

Thr Val Phe His

<210> 81
<211> 2887
<212> DNA
<213> Homo sapiens

<400> 81
gaagcagcag gtaccccctc cacatcccta gggctctgtg atgtaggcag 50

aggcccgtgg gagtcagcat gccgcgtggc tgggccgccc ccttgctcct 100

gctgctgctc cagggaggct ggggctgccc cgacctcgtc tgctacaccg 150

attacctcca gacggtcatc tgcatcctgg aaatgtggaa cctccacccc 200

agcacgctca cccttacctg gcaagaccag tatgaagagc tgaaggacga 250

ggccacctcc tgcagcctcc acaggtcggc ccacaatgcc acgcatgcca 300

cctacacctg ccacatggat gtattccact tcatggccga cgacattttc 350

agtgtcaaca tcacagacca gtctggcaac tactcccagg agtgtggcag 400

ctttctcctg gctgagagca tcaagccggc tccccctttc aacgtgactg 450

tgaccttctc aggacagtat aatatctcct ggcgctcaga ttacgaagac 500

cctgccttct acatgctgaa gggcaagctt cagtatgagc tgcagtacag 550

gaaccgggga gacccctggg ctgtgagtcc gaggagaaag ctgatctcag 600

tggactcaag aagtgtctcc ctcctccccc tggagttccg caaagactcg 650

agctatgagc tgcaggtgcg ggcagggccc atgcctggct cctcctacca 700

ggggacctgg agtgaatgga gtgacccggt catctttcag acccagtcag 750

aggagttaaa ggaaggctgg aaccctcacc tgctgcttct cctcctgctt 800

gtcatagtct tcattcctgc cttctggagc ctgaagaccc atccattgtg 850

gaggctatgg aagaagatat gggccgtccc cagccctgag cggttcttca 900

tgcccctgta caagggctgc agcggagact tcaagaaatg ggtgggtgca 950

cccttcactg gctccagcct ggagctggga ccctggagcc cagaggtgcc 1000

ctccaccctg gaggtgtaca gctgccaccc accacggagc ccggccaaga 1050
ggctgcagct cacggagcta caagaaccag cagagctggt ggagtctgac 1100
ggtgtgccca agcccagctt ctggccgaca gcccagaact cggggggctc 1150
agcttacagt gaggagaggg atcggccata cggcctggtg tccattgaca 1200
cagtgactgt gctagatgca gaggggccat gcacctggcc ctgcagctgt 1250
gaggatgacg gctacccagc cctggacctg gatgctggcc tggagcccag 1300
cccaggccta gaggacccac tcttggatgc agggaccaca gtcctgtcct 1350
gtggctgtgt ctcagctggc agccctgggc taggagggcc cctgggaagc 1400
ctcctggaca gactaaagcc accccttgca gatggggagg actgggctgg 1450
gggactgccc tggggtggcc ggtcacctgg aggggtctca gagagtgagg 1500
cgggctcacc cctggccggc ctggatatgg acacgtttga cagtggcttt 1550
gtgggctctg actgcagcag ccctgtggag tgtgacttca ccagccccgg 1600
ggacgaagga cccccccgga gctacctccg ccagtgggtg gtcattcctc 1650
cgccactttc gagccctgga ccccaggcca gctaatgagg ctgactggat 1700
gtccagagct ggccaggcca ctgggccctg agccagagac aaggtcacct 1750
gggctgtgat gtgaagacac ctgcagcctt tggtctcctg gatgggcctt 1800
tgagcctgat gtttacagtg tctgtgtgtg tgtgtgcata tgtgtgtgtg 1850
tgcatatgca tgtgtgtgtg tgtgtgtgtc ttaggtgcgc agtggcatgt 1900
ccacgtgtgt gtgtgattgc acgtgcctgt gggcctggga taatgcccat 1950
ggtactccat gcattcacct gccctgtgca tgtctggact cacggagctc 2000
acccatgtgc acaagtgtgc acagtaaacg tgtttgtggt caacagatga 2050
caacagccgt cctccctcct agggtcttgt gttgcaagtt ggtccacagc 2100
atctccgggg ctttgtggga tcagggcatt gcctgtgact gaggcggagc 2150
ccagccctcc agcgtctgcc tccaggagct gcaagaagtc catattgttc 2200
cttatcacct gccaacagga agcgaaaggg gatggagtga gcccatggtg 2250
acctcgggaa tggcaatttt ttgggcggcc cctggacgaa ggtctgaatc 2300
ccgactctga taccttctgg ctgtgctacc tgagccaagt cgcctcccct 2350
ctctgggcta gagtttcctt atccagacag tggggaaggc atgacacacc 2400
tgggggaaat tggcgatgtc acccgtgtac ggtacgcagc ccagagcaga 2450
ccctcaataa acgtcagctt ccttccttct gcggccagag ccgaggcggg 2500
cgggggtgag aacatcaatc gtcagcgaca gcctgggcac ccgcggggcc 2550
gtcccgcctg cagagggcca ctcggggggg tttccaggct taaaatcagt 2600
ccgtttcgtc tcttggaaac agctccccac caaccaagat ttctttttct 2650
aacttctgct actaagtttt taaaaattcc ctttatgcac ccaagagata 2700

tttattaaac accaattacg tagcaggcca tggctcatgg gacccacccc 2750

ccgtggcact catggagggg gctgcaggtt ggaactatgc agtgtgctcc 2800

ggccacacat cctgctgggc cccctaccct gccccaattc aatcctgcca 2850

ataaatcctg tcttatttgt tcatcctgga gaattga 2887

<210> 82
<211> 538
<212> PRT
<213> Homo sapiens

<400> 82
Met Pro Arg Gly Trp Ala Ala Pro Leu Leu Leu Leu Leu Leu Gln
1               5                   10                  15

Gly Gly Trp Gly Cys Pro Asp Leu Val Cys Tyr Thr Asp Tyr Leu
                20                  25                  30

Gln Thr Val Ile Cys Ile Leu Glu Met Trp Asn Leu His Pro Ser
                35                  40                  45

Thr Leu Thr Leu Thr Trp Gln Asp Gln Tyr Glu Glu Leu Lys Asp
                50                  55                  60

Glu Ala Thr Ser Cys Ser Leu His Arg Ser Ala His Asn Ala Thr
                65                  70                  75

His Ala Thr Tyr Thr Cys His Met Asp Val Phe His Phe Met Ala
                80                  85                  90

Asp Asp Ile Phe Ser Val Asn Ile Thr Asp Gln Ser Gly Asn Tyr
                95                  100                 105

Ser Gln Glu Cys Gly Ser Phe Leu Leu Ala Glu Ser Ile Lys Pro
                110                 115                 120

Ala Pro Pro Phe Asn Val Thr Val Thr Phe Ser Gly Gln Tyr Asn
                125                 130                 135

Ile Ser Trp Arg Ser Asp Tyr Glu Asp Pro Ala Phe Tyr Met Leu
                140                 145                 150

Lys Gly Lys Leu Gln Tyr Glu Leu Gln Tyr Arg Asn Arg Gly Asp
                155                 160                 165

Pro Trp Ala Val Ser Pro Arg Arg Lys Leu Ile Ser Val Asp Ser
                170                 175                 180

Arg Ser Val Ser Leu Leu Pro Leu Glu Phe Arg Lys Asp Ser Ser
                185                 190                 195

Tyr Glu Leu Gln Val Arg Ala Gly Pro Met Pro Gly Ser Ser Tyr
                200                 205                 210

Gln Gly Thr Trp Ser Glu Trp Ser Asp Pro Val Ile Phe Gln Thr
                215                 220                 225

Gln Ser Glu Glu Leu Lys Glu Gly Trp Asn Pro His Leu Leu Leu
                230                 235                 240

Leu Leu Leu Leu Val Ile Val Phe Ile Pro Ala Phe Trp Ser Leu
                245                 250                 255

Lys Thr His Pro Leu Trp Arg Leu Trp Lys Lys Ile Trp Ala Val
                260                 265                 270

Pro Ser Pro Glu Arg Phe Phe Met Pro Leu Tyr Lys Gly Cys Ser
                275                 280                 285

Gly Asp Phe Lys Lys Trp Val Gly Ala Pro Phe Thr Gly Ser Ser
                290                 295                 300

Leu Glu Leu Gly Pro Trp Ser Pro Glu Val Pro Ser Thr Leu Glu
                305                 310                 315

Val Tyr Ser Cys His Pro Pro Arg Ser Pro Ala Lys Arg Leu Gln
                320                 325                 330

Leu Thr Glu Leu Gln Glu Pro Ala Glu Leu Val Glu Ser Asp Gly
                335                 340                 345

Val Pro Lys Pro Ser Phe Trp Pro Thr Ala Gln Asn Ser Gly Gly
                350                 355                 360

Ser Ala Tyr Ser Glu Glu Arg Asp Arg Pro Tyr Gly Leu Val Ser
                365                 370                 375

Ile Asp Thr Val Thr Val Leu Asp Ala Glu Gly Pro Cys Thr Trp
                380                 385                 390

Pro Cys Ser Cys Glu Asp Asp Gly Tyr Pro Ala Leu Asp Leu Asp
                395                 400                 405

Ala Gly Leu Glu Pro Ser Pro Gly Leu Glu Asp Pro Leu Leu Asp
                410                 415                 420

Ala Gly Thr Thr Val Leu Ser Cys Gly Cys Val Ser Ala Gly Ser
                425                 430                 435

Pro Gly Leu Gly Gly Pro Leu Gly Ser Leu Leu Asp Arg Leu Lys
                440                 445                 450

Pro Pro Leu Ala Asp Gly Glu Asp Trp Ala Gly Gly Leu Pro Trp
                455                 460                 465

Gly Gly Arg Ser Pro Gly Gly Val Ser Glu Ser Glu Ala Gly Ser
                470                 475                 480

Pro Leu Ala Gly Leu Asp Met Asp Thr Phe Asp Ser Gly Phe Val
                485                 490                 495

Gly Ser Asp Cys Ser Ser Pro Val Glu Cys Asp Phe Thr Ser Pro
                500                 505                 510

Gly Asp Glu Gly Pro Pro Arg Ser Tyr Leu Arg Gln Trp Val Val
                515                 520                 525

Ile Pro Pro Pro Leu Ser Ser Pro Gly Pro Gln Ala Ser
                530                 535

<210> 83
<211> 1278
<212> DNA
<213> Homo sapiens

<400> 83
ggcacgagga ggtgtggacg ctgtgtatga aatgtctttc ctccaggacc 50

caagtttctt caccatgggg atgtggtcca ttggtgcagg agccctgggg 100

gctgctgcct tggcattgct gcttgccaac acagacgtgt ttctgtccaa 150

gccccagaaa gcggccctgg agtacctgga ggatatagac ctgaaaacac 200

tggagaagga accaaggact ttcaaagcaa aggagctatg ggaaaaaaat 250
ggagctgtga ttatggccgt gcggaggcca ggctgtttcc tctgtcgaga 300
ggaagctgcg gatctgtcct ccctgaaaag catgttggac cagctgggcg 350
tccccctcta tgcagtggta aaggagcaca tcaggactga agtgaaggat 400
ttccagcctt atttcaaagg agaaatcttc ctggatgaaa agaaaaagtt 450
ctatggtcca caaaggcgga agatgatgtt tatgggattt atccgtctgg 500
gagtgtggta caacttcttc cgagcctgga acggaggctt ctctggaaac 550
ctggaaggag aaggcttcat ccttggggga gttttcgtgg tgggatcagg 600
aaagcagggc attcttcttg agcaccgaga aaaagaattt ggagacaaag 650
taaacctact ttctgttctg gaagctgcta agatgatcaa accacagact 700
ttggcctcag agaaaaaatg attgtgtgaa actgcccagc tcagggataa 750
ccagggacat tcacctgtgt tcatgggatg tattgtttcc actcgtgtcc 800
ctaaggagtg agaaacccat ttatactcta ctctcagtat ggattattaa 850
tgtattttaa tattctgttt aggcccacta aggcaaaata gccccaaaac 900
aagactgaca aaaatctgaa aaactaatga ggattattaa gctaaaacct 950
gggaaatagg aggcttaaaa ttgactgcca ggctgggtgc agtggctcac 1000
acctgtaatc ccagcacttt gggaggccaa ggtgagcaag tcacttgagg 1050
tcgggagttc gagaccagcc tgagcaacat ggcgaaaccc cgtctctact 1100
aaaaatacaa aaatcacccg ggtgtggtgg caggcacctg tagtcccagc 1150
tacccgggag gctgaggcag gagaatcact tgaacctggg aggtggaggt 1200
tgcggtgagc tgagatcaca ccactgtatt ccagcctggg tgactgagac 1250
tctaactaaa aaaaaaaaaa aaaaaaaa 1278

<210> 84
<211> 216
<212> PRT
<213> Homo sapiens

<400> 84
Met Trp Ser Ile Gly Ala Gly Ala Leu Gly Ala Ala Ala Leu Ala
1               5                   10                  15

Leu Leu Leu Ala Asn Thr Asp Val Phe Leu Ser Lys Pro Gln Lys
                20                  25                  30

Ala Ala Leu Glu Tyr Leu Glu Asp Ile Asp Leu Lys Thr Leu Glu
                35                  40                  45

Lys Glu Pro Arg Thr Phe Lys Ala Lys Glu Leu Trp Glu Lys Asn
                50                  55                  60

Gly Ala Val Ile Met Ala Val Arg Arg Pro Gly Cys Phe Leu Cys
                65                  70                  75

Arg Glu Glu Ala Ala Asp Leu Ser Ser Leu Lys Ser Met Leu Asp
                80                  85                  90

Gln Leu Gly Val Pro Leu Tyr Ala Val Val Lys Glu His Ile Arg
                95                  100                 105

Thr Glu Val Lys Asp Phe Gln Pro Tyr Phe Lys Gly Glu Ile Phe
                110                 115                 120

Leu Asp Glu Lys Lys Lys Phe Tyr Gly Pro Gln Arg Arg Lys Met
                125                 130                 135

Met Phe Met Gly Phe Ile Arg Leu Gly Val Trp Tyr Asn Phe Phe
                140                 145                 150

Arg Ala Trp Asn Gly Gly Phe Ser Gly Asn Leu Glu Gly Glu Gly
                155                 160                 165

Phe Ile Leu Gly Gly Val Phe Val Val Gly Ser Gly Lys Gln Gly
                170                 175                 180

Ile Leu Leu Glu His Arg Glu Lys Glu Phe Gly Asp Lys Val Asn
                185                 190                 195

Leu Leu Ser Val Leu Glu Ala Ala Lys Met Ile Lys Pro Gln Thr
                200                 205                 210

Leu Ala Ser Glu Lys Lys
                215

<210> 85
<211> 1932
<212> DNA
<213> Homo sapiens

<400> 85
ggcacgaggc ctcgtgccgc cgggctcttg gtacctcagc gcgagcgcca 50

ggcgtccggc cgccgtggct atgttcgtgt ccgatttccg caaagagttc 100

tacgaggtgg tccagagcca gagggtcctt ctcttcgtgg cctcggacgt 150

ggatgctctg tgtgcgtgca agatccttca ggccttgttc cagtgtgacc 200

acgtgcaata tacgctggtt ccagtttctg ggtggcaaga acttgaaact 250

gcatttcttg agcataaaga acagtttcat tattttattc tcataaactg 300

tggagctaat gtagacctat tggatattct tcaacctgat gaagacacta 350

tattctttgt gtgtgacacc cataggccag tcaatgtcgt caatgtatac 400

aacgataccc agatcaaatt actcattaaa caagatgatg accttgaagt 450

tcccgcctat gaagacatct tcagggatga agaggaggat gaagagcatt 500

caggaaatga cagtgatggg tcagagcctt ctgagaagcg cacacggtta 550

gaagaggaga tagtggagca aaccatgcgg aggaggcagc ggcgagagtg 600

ggaggcccgg agaagagaca tcctctttga ctacgagcag tatgaatatc 650

atgggacatc gtcagccatg gtgatgtttg agctggcttg gatgctgtcc 700

aaggacctga atgacatgct gtggtgggcc atcgttggac taacagacca 750

gtgggtgcaa gacaagatca ctcaaatgaa atacgtgact gatgttggtg 800

tcctgcagcg ccacgtttcc cgccacaacc accggaacga ggatgaggag 850

aacacactct ccgtggactg cacacggatc tcctttgagt atgacctccg 900
cctggtgctc taccagcact ggtccctcca tgacagcctg tgcaacacca 950
gctataccgc agccaggttc aagctgtggt ctgtgcatgg acagaagcgg 1000
ctccaggagt tccttgcaga catgggtctt cccctgaagc aggtgaagca 1050
gaagttccag gccatggaca tctccttgaa ggagaatttg cgggaaatga 1100
ttgaagagtc tgcaaataaa tttgggatga aggacatgcg cgtgcagact 1150
ttcagcattc attttgggtt caagcacaag tttctggcca gcgacgtggt 1200
ctttgccacc atgtctttga tggagagccc cgagaaggat ggctcaggga 1250
cagatcactt catccaggct ctggacagcc tctccaggag taacctggac 1300
aagctgtacc atggcctgga actcgccaag aagcagctgc gagccaccca 1350
gcagaccatt gccagctgcc tttgcaccaa cctcgtcatc tcccaggggc 1400
ctttcctgta ctgctctctc atggagggca ctccagatgt catgctgttc 1450
tctaggccgg catccctaag cctgctcagc aaacacctgc tcaagtcctt 1500
tgtgtgttcg acaaagaacc ggcgctgcaa actgctgccc ctggtgatgg 1550
ctgcccccct gagcatggag catggcacag tgaccgtggt gggcatcccc 1600
ccagagaccg acagctcgga caggaagaac tttttgggga gggcgtttga 1650
gaaggcagcg gaaagcacca gctcccggat gctgcacaac cattttgacc 1700
tctcagtaat tgagctgaaa gctgaggatc ggagcaagtt tctggacgca 1750
cttatttccc tcctgtccta ggaatttgat tcttccagaa tgaccttctt 1800
atttatgtaa ctggctttca tttagattgt aagttatgga catgatttga 1850
gatgtagaag ccatttttta ttaaataaaa tgcttatttt aggctccgtc 1900
cccaaaaaaa aaaaaaaaaa aaaaaaaaaa aa 1932

<210> 86
<211> 566
<212> PRT
<213> Homo sapiens

<400> 86
Met Phe Val Ser Asp Phe Arg Lys Glu Phe Tyr Glu Val Val Gln
1               5                   10                  15

Ser Gln Arg Val Leu Leu Phe Val Ala Ser Asp Val Asp Ala Leu
                20                  25                  30

Cys Ala Cys Lys Ile Leu Gln Ala Leu Phe Gln Cys Asp His Val
                35                  40                  45

Gln Tyr Thr Leu Val Pro Val Ser Gly Trp Gln Glu Leu Glu Thr
                50                  55                  60

Ala Phe Leu Glu His Lys Glu Gln Phe His Tyr Phe Ile Leu Ile
                65                  70                  75

Asn Cys Gly Ala Asn Val Asp Leu Leu Asp Ile Leu Gln Pro Asp
                80                  85                  90

Glu Asp Thr Ile Phe Phe Val Cys Asp Thr His Arg Pro Val Asn
                95                  100                 105

Val Val Asn Val Tyr Asn Asp Thr Gln Ile Lys Leu Leu Ile Lys
                110                 115                 120

Gln Asp Asp Asp Leu Glu Val Pro Ala Tyr Glu Asp Ile Phe Arg
                125                 130                 135

Asp Glu Glu Glu Asp Glu Glu His Ser Gly Asn Asp Ser Asp Gly
                140                 145                 150

Ser Glu Pro Ser Glu Lys Arg Thr Arg Leu Glu Glu Glu Ile Val
                155                 160                 165

Glu Gln Thr Met Arg Arg Arg Gln Arg Arg Glu Trp Glu Ala Arg
                170                 175                 180

Arg Arg Asp Ile Leu Phe Asp Tyr Glu Gln Tyr Glu Tyr His Gly
                185                 190                 195

Thr Ser Ser Ala Met Val Met Phe Glu Leu Ala Trp Met Leu Ser
                200                 205                 210

Lys Asp Leu Asn Asp Met Leu Trp Trp Ala Ile Val Gly Leu Thr
                215                 220                 225

Asp Gln Trp Val Gln Asp Lys Ile Thr Gln Met Lys Tyr Val Thr
                230                 235                 240

Asp Val Gly Val Leu Gln Arg His Val Ser Arg His Asn His Arg
                245                 250                 255

Asn Glu Asp Glu Glu Asn Thr Leu Ser Val Asp Cys Thr Arg Ile
                260                 265                 270

Ser Phe Glu Tyr Asp Leu Arg Leu Val Leu Tyr Gln His Trp Ser
                275                 280                 285

Leu His Asp Ser Leu Cys Asn Thr Ser Tyr Thr Ala Ala Arg Phe
                290                 295                 300

Lys Leu Trp Ser Val His Gly Gln Lys Arg Leu Gln Glu Phe Leu
                305                 310                 315

Ala Asp Met Gly Leu Pro Leu Lys Gln Val Lys Gln Lys Phe Gln
                320                 325                 330

Ala Met Asp Ile Ser Leu Lys Glu Asn Leu Arg Glu Met Ile Glu
                335                 340                 345

Glu Ser Ala Asn Lys Phe Gly Met Lys Asp Met Arg Val Gln Thr
                350                 355                 360

Phe Ser Ile His Phe Gly Phe Lys His Lys Phe Leu Ala Ser Asp
                365                 370                 375

Val Val Phe Ala Thr Met Ser Leu Met Glu Ser Pro Glu Lys Asp
                380                 385                 390

Gly Ser Gly Thr Asp His Phe Ile Gln Ala Leu Asp Ser Leu Ser
                395                 400                 405

Arg Ser Asn Leu Asp Lys Leu Tyr His Gly Leu Glu Leu Ala Lys
                410                 415                 420

Lys Gln Leu Arg Ala Thr Gln Gln Thr Ile Ala Ser Cys Leu Cys
                425                 430                 435

Thr Asn Leu Val Ile Ser Gln Gly Pro Phe Leu Tyr Cys Ser Leu
                440                 445                 450

Met Glu Gly Thr Pro Asp Val Met Leu Phe Ser Arg Pro Ala Ser
                455                 460                 465

Leu Ser Leu Leu Ser Lys His Leu Leu Lys Ser Phe Val Cys Ser
                470                 475                 480

Thr Lys Asn Arg Arg Cys Lys Leu Leu Pro Leu Val Met Ala Ala
                485                 490                 495

Pro Leu Ser Met Glu His Gly Thr Val Thr Val Val Gly Ile Pro
                500                 505                 510

Pro Glu Thr Asp Ser Ser Asp Arg Lys Asn Phe Phe Gly Arg Ala
                515                 520                 525

Phe Glu Lys Ala Ala Glu Ser Thr Ser Ser Arg Met Leu His Asn
                530                 535                 540

His Phe Asp Leu Ser Val Ile Glu Leu Lys Ala Glu Asp Arg Ser
                545                 550                 555

Lys Phe Leu Asp Ala Leu Ile Ser Leu Leu Ser
                560                 565

<210> 87
<211> 1359
<212> DNA
<213> Homo sapiens

<400> 87
accgggcacc ggacggctcg ggtactttcg ttcttaatta ggtcatgccc 50

gtgtgagcca ggaaagggct gtgtttatgg gaagccagta acactgtggc 100

ctactatctc ttccgtggtg ccatctacat ttttgggact cgggaattat 150

gaggtagagg tggaggcgga gccggatgtc agaggtcctg aaatagtcac 200

catgggggaa aatgatccgc ctgctgttga agcccccttc tcattccgat 250

cgctttttgg ccttgatgat ttgaaaataa gtcctgttgc accagatgca 300

gatgctgttg ctgcacagat cctgtcactg ctgccattga agttttttcc 350

aatcatcgtc attgggatca ttgcattgat attagcactg gccattggtc 400

tgggcatcca cttcgactgc tcagggaagt acagatgtcg ctcatccttt 450

aagtgtatcg agctgatagc tcgatgtgac ggagtctcgg attgcaaaga 500

cggggaggac gagtaccgct gtgtccgggt gggtggtcag aatgccgtgc 550

tccaggtgtt cacagctgct tcgtggaaga ccatgtgctc cgatgactgg 600

aagggtcact acgcaaatgt tgcctgtgcc caactgggtt tcccaagcta 650

tgtgagttca gataacctca gagtgagctc gctggagggg cagttccggg 700

aggagtttgt gtccatcgat cacctcttgc cagatgacaa ggtgactgca 750

ttacaccact cagtatatgt gagggaggga tgtgcctctg gccacgtggt 800

taccttgcag tgcacagcct gtggtcatag aaggggctac agctcacgca 850

tcgtgggtgg aaacatgtcc ttgctctcgc agtggccctg gcaggccagc 900

cttcagttcc agggctacca cctgtgcggg ggctctgtca tcacgcccct 950

gtggatcatc actgctgcac actgtgttta tgacttgtac ctccccaagt 1000

catggaccat ccaggtgggt ctagtttccc tgttggacaa tccagcccca 1050

tcccacttgg tggagaagat tgtctaccac agcaagtaca agccaaagag 1100

gctgggcaat gacatcgccc ttatgaagct ggccgggcca ctcacgttca 1150

atggtacatc tgggtctcta tgtggttctg cagctcttcc tttgtttcaa 1200

gaggatttgc aattgctcat tgaagcattc ttatgatggc tgctttataa 1250

tccttgtcag atattaataa ttccaactcc tgattcatgt tggtgttggc 1300

atcagttgat tatcttttct cattaaaatt gtgatgctcc taaaaaaaaa 1350

aaaaaaaaa 1359

<210> 88
<211> 344
<212> PRT
<213> Homo sapiens

<400> 88
Met Gly Glu Asn Asp Pro Pro Ala Val Glu Ala Pro Phe Ser Phe
1               5                   10                  15

Arg Ser Leu Phe Gly Leu Asp Asp Leu Lys Ile Ser Pro Val Ala
                20                  25                  30

Pro Asp Ala Asp Ala Val Ala Ala Gln Ile Leu Ser Leu Leu Pro
                35                  40                  45

Leu Lys Phe Phe Pro Ile Ile Val Ile Gly Ile Ile Ala Leu Ile
                50                  55                  60

Leu Ala Leu Ala Ile Gly Leu Gly Ile His Phe Asp Cys Ser Gly
                65                  70                  75

Lys Tyr Arg Cys Arg Ser Ser Phe Lys Cys Ile Glu Leu Ile Ala
                80                  85                  90

Arg Cys Asp Gly Val Ser Asp Cys Lys Asp Gly Glu Asp Glu Tyr
                95                  100                 105

Arg Cys Val Arg Val Gly Gly Gln Asn Ala Val Leu Gln Val Phe
                110                 115                 120

Thr Ala Ala Ser Trp Lys Thr Met Cys Ser Asp Asp Trp Lys Gly
                125                 130                 135

His Tyr Ala Asn Val Ala Cys Ala Gln Leu Gly Phe Pro Ser Tyr
                140                 145                 150

Val Ser Ser Asp Asn Leu Arg Val Ser Ser Leu Glu Gly Gln Phe
                155                 160                 165

Arg Glu Glu Phe Val Ser Ile Asp His Leu Leu Pro Asp Asp Lys
                170                 175                 180

Val Thr Ala Leu His His Ser Val Tyr Val Arg Glu Gly Cys Ala
                185                 190                 195

Ser Gly His Val Val Thr Leu Gln Cys Thr Ala Cys Gly His Arg

                        200                         205                         210

Arg Gly Tyr Ser Ser Arg Ile Val Gly Gly Asn Met Ser Leu Leu
                215                 220                 225

Ser Gln Trp Pro Trp Gln Ala Ser Leu Gln Phe Gln Gly Tyr His
                230                 235                 240

Leu Cys Gly Gly Ser Val Ile Thr Pro Leu Trp Ile Ile Thr Ala
                245                 250                 255

Ala His Cys Val Tyr Asp Leu Tyr Leu Pro Lys Ser Trp Thr Ile
                260                 265                 270

Gln Val Gly Leu Val Ser Leu Leu Asp Asn Pro Ala Pro Ser His
                275                 280                 285

Leu Val Glu Lys Ile Val Tyr His Ser Lys Tyr Lys Pro Lys Arg
                290                 295                 300

Leu Gly Asn Asp Ile Ala Leu Met Lys Leu Ala Gly Pro Leu Thr
                305                 310                 315

Phe Asn Gly Thr Ser Gly Ser Leu Cys Gly Ser Ala Ala Leu Pro
                320                 325                 330

Leu Phe Gln Glu Asp Leu Gln Leu Leu Ile Glu Ala Phe Leu
                335                 340

<210> 89
<211> 726
<212> DNA
<213> Homo sapiens

<400> 89
atggcacagc acggggcgat gggcgcgttt cgggccctgt gcggcctggc 50

gctgctgtgc gcgctcagcc tgggtcagcg ccccaccggg ggtcccgggt 100

gcggccctgg gcgcctcctg cttgggacgg gaacggacgc gcgctgctgc 150

cgggttcaca cgacgcgctg ctgccgcgat tacccgggcg aggagtgctg 200

ttccgagtgg gactgcatgt gtgtccagcc tgaattccac tgcggagacc 250

cttgctgcac gacctgccgg caccaccctt gtcccccagg ccagggggta 300

cagtcccagg ggaaattcag ttttggcttc cagtgtatcg actgtgcctc 350

ggggaccttc tccgggggcc acgaaggcca ctgcaaacct tggacagact 400

gcacccagtt cgggtttctc actgtgttcc ctgggaacaa gacccacaac 450

gctgtgtgcg tcccagggtc cccgccggca gagccgcttg ggtggctgac 500

cgtcgtcctc ctggccgtgg ccgcctgcgt cctcctcctg acctcggccc 550

agcttggact gcacatctgg cagctgagga gtcagtgcat gtggccccga 600

gagacccagc tgctgctgga ggtgccgccg tcgaccgaag acgccagaag 650

ctgccagttc cccgaggaag agcggggcga gcgatcggca gaggagaagg 700

ggcggctggg agacctgtgg gtgtga 726

<210> 90
<211> 241
<212> PRT

<213> Homo sapiens

<400> 90
Met Ala Gln His Gly Ala Met Gly Ala Phe Arg Ala Leu Cys Gly
1               5                   10                  15

Leu Ala Leu Leu Cys Ala Leu Ser Leu Gly Gln Arg Pro Thr Gly
                20                  25                  30

Gly Pro Gly Cys Gly Pro Gly Arg Leu Leu Leu Gly Thr Gly Thr
                35                  40                  45

Asp Ala Arg Cys Cys Arg Val His Thr Thr Arg Cys Cys Arg Asp
                50                  55                  60

Tyr Pro Gly Glu Glu Cys Cys Ser Glu Trp Asp Cys Met Cys Val
                65                  70                  75

Gln Pro Glu Phe His Cys Gly Asp Pro Cys Cys Thr Thr Cys Arg
                80                  85                  90

His His Pro Cys Pro Pro Gly Gln Gly Val Gln Ser Gln Gly Lys
                95                  100                 105

Phe Ser Phe Gly Phe Gln Cys Ile Asp Cys Ala Ser Gly Thr Phe
                110                 115                 120

Ser Gly Gly His Glu Gly His Cys Lys Pro Trp Thr Asp Cys Thr
                125                 130                 135

Gln Phe Gly Phe Leu Thr Val Phe Pro Gly Asn Lys Thr His Asn
                140                 145                 150

Ala Val Cys Val Pro Gly Ser Pro Pro Ala Glu Pro Leu Gly Trp
                155                 160                 165

Leu Thr Val Val Leu Leu Ala Val Ala Ala Cys Val Leu Leu Leu
                170                 175                 180

Thr Ser Ala Gln Leu Gly Leu His Ile Trp Gln Leu Arg Ser Gln
                185                 190                 195

Cys Met Trp Pro Arg Glu Thr Gln Leu Leu Leu Glu Val Pro Pro
                200                 205                 210

Ser Thr Glu Asp Ala Arg Ser Cys Gln Phe Pro Glu Glu Glu Arg
                215                 220                 225

Gly Glu Arg Ser Ala Glu Glu Lys Gly Arg Leu Gly Asp Leu Trp
                230                 235                 240

Val


<210> 91
<211> 1453
<212> DNA
<213> Homo sapiens

<400> 91
agtgcgcgaa gatgcgaaag gtggttttga tcaccggggc tagcagtggc 50

attggcctgg ccctctgcaa gcggctgctg gcggaagatg atgagcttca 100

tctgtgtttg gcgtgcagga acatgagcaa ggcagaagct gtctgtgctg 150

ctctgctggc ctctcacccc actgctgagg tcaccattgt ccaggtggat 200

gtcagcaacc tgcagtcggt cttccgggcc tccaaggaac ttaagcaaag 250
gtttcagaga ttagactgta tatatctaaa tgctgggatc atgcctaatc 300
cacaactaaa tatcaaagca cttttctttg gcctcttttc aagaaaagtg 350
attcatatgt tctccacagc tgaaggcctg ctgacccagg gtgataagat 400
cactgctgat ggacttcagg aggtgtttga gaccaatgtc tttggccatt 450
ttatcctgat tcgggaactg gagcctctcc tctgtcacag tgacaatcca 500
tctcagctca tctggacatc atctcgcagt gcaaggaaat ctaatttcag 550
cctcgaggac ttccagcaca gcaaaggcaa ggaaccctac agctcttcca 600
aatatgccac tgaccttttg agtgtggctt tgaacaggaa cttcaaccag 650
cagggtctct attccaatgt ggcctgtcca ggtacagcat tgaccaattt 700
gacatatgga attctgcctc cgtttatatg gacgctgttg atgccggcaa 750
tattgctact tcgctttttt gcaaatgcat tcactttgac accatataat 800
ggaacagaag ctctggtatg gcttttccac caaaagcctg aatctctcaa 850
tcctctgatc aaatatctga gtgccaccac tggctttgga agaaattata 900
ttatgaccca gaagatggac ctagatgaag acactgctga aaaattttat 950
caaaagttac tggaactgga aaagcacatt agggtcacta ttcaaaaaac 1000
agataatcag gccaggctca gtggctcatg cctataattc cagcactttg 1050
ggaggccaag gcagaaggat cacttgagac caggagttca agaccagcct 1100
gagaaacata gtgagccctt gtctctacaa aaagaaataa aaataatagc 1150
tgggtgtggt ggcatgcgca tgtagtccca gctactcaga aggatgaggt 1200
gggaggatct cttgaggctg ggaggcagag gttgcagtga gctgagattg 1250
tgccactgca ctccagcctg ggtgacagcg agaccctgtc tcaaaatatg 1300
tatatattta atatatatat aaaaccagag ctgacaatga cactctggaa 1350
cattgcatac cttctgtaca ttctggggta catggatttc tactgagttg 1400
gataatatgc atttgtaata aactatgaac tatgaaaaaa aaaaaaaaaa 1450
aaa 1453

<210> 92
<211> 341
<212> PRT
<213> Homo sapiens

<400> 92
Met Arg Lys Val Val Leu Ile Thr Gly Ala Ser Ser Gly Ile Gly
1               5                   10                  15

Leu Ala Leu Cys Lys Arg Leu Leu Ala Glu Asp Asp Glu Leu His
                20                  25                  30

Leu Cys Leu Ala Cys Arg Asn Met Ser Lys Ala Glu Ala Val Cys
                35                  40                  45

Ala Ala Leu Leu Ala Ser His Pro Thr Ala Glu Val Thr Ile Val

                50                  55                  60

Gln Val Asp Val Ser Asn Leu Gln Ser Val Phe Arg Ala Ser Lys
                65                  70                  75

Glu Leu Lys Gln Arg Phe Gln Arg Leu Asp Cys Ile Tyr Leu Asn
                80                  85                  90

Ala Gly Ile Met Pro Asn Pro Gln Leu Asn Ile Lys Ala Leu Phe
                95                  100                 105

Phe Gly Leu Phe Ser Arg Lys Val Ile His Met Phe Ser Thr Ala
                110                 115                 120

Glu Gly Leu Leu Thr Gln Gly Asp Lys Ile Thr Ala Asp Gly Leu
                125                 130                 135

Gln Glu Val Phe Glu Thr Asn Val Phe Gly His Phe Ile Leu Ile
                140                 145                 150

Arg Glu Leu Glu Pro Leu Leu Cys His Ser Asp Asn Pro Ser Gln
                155                 160                 165

Leu Ile Trp Thr Ser Ser Arg Ser Ala Arg Lys Ser Asn Phe Ser
                170                 175                 180

Leu Glu Asp Phe Gln His Ser Lys Gly Lys Glu Pro Tyr Ser Ser
                185                 190                 195

Ser Lys Tyr Ala Thr Asp Leu Leu Ser Val Ala Leu Asn Arg Asn
                200                 205                 210

Phe Asn Gln Gln Gly Leu Tyr Ser Asn Val Ala Cys Pro Gly Thr
                215                 220                 225

Ala Leu Thr Asn Leu Thr Tyr Gly Ile Leu Pro Pro Phe Ile Trp
                230                 235                 240

Thr Leu Leu Met Pro Ala Ile Leu Leu Leu Arg Phe Phe Ala Asn
                245                 250                 255

Ala Phe Thr Leu Thr Pro Tyr Asn Gly Thr Glu Ala Leu Val Trp
                260                 265                 270

Leu Phe His Gln Lys Pro Glu Ser Leu Asn Pro Leu Ile Lys Tyr
                275                 280                 285

Leu Ser Ala Thr Thr Gly Phe Gly Arg Asn Tyr Ile Met Thr Gln
                290                 295                 300

Lys Met Asp Leu Asp Glu Asp Thr Ala Glu Lys Phe Tyr Gln Lys
                305                 310                 315

Leu Leu Glu Leu Glu Lys His Ile Arg Val Thr Ile Gln Lys Thr
                320                 325                 330

Asp Asn Gln Ala Arg Leu Ser Gly Ser Cys Leu
                335                 340

<210> 93
<211> 1591
<212> DNA
<213> Homo sapiens

<400> 93
agcctggggc ggccggccag gaaccacccg ttaaggtgtc ttctctttag 50

ggatggtgag gttggaaaaa ggctcctgta accctcctcc aggatgaacc 100

acctgccaga agacatggag aacgctctca ccgggagcca gagctcccat 150
gcttctctgc gcaatatcca ttccatcaac cccacacaac tcatggccag 200
gattgagtcc tatgaaggaa gggaaaagaa aggcatatct gatgtcagga 250
ggactttctg tttgtttgtc acctttgacc tcttattcgt aacattactg 300
tggataatag agttaaatgt gaatggaggc attgagaaca cattagagaa 350
ggaggtgatg cagtatgact actattcttc atattttgat atatttcttc 400
tggcagtttt tcgatttaaa gtgttaatac ttgcatatgc tgtgtgcaga 450
ctgcgccatt ggtgggcaat agcgttgaca acggcagtga ccagtgcctt 500
tttactagca aaagtgatcc tttcgaagct tttctctcaa ggggcttttg 550
gctatgtgct gcccatcatt tcattcatcc ttgcctggat tgagacgtgg 600
ttcctggatt tcaaagtgtt acctcaagaa gcagaagaag aaaacagact 650
cctgatagtt caggatgctt cagagagggc agcacttata cctggtggtc 700
tttctgatgg tcagttttat tcccctcctg aatccgaagc aggatctgaa 750
gaagctgaag aaaaacagga cagtgagaaa ccacttttag aactatgagt 800
actacttttg ttaaatgtga aaaaccctca cagaaagtca tcgaggcaaa 850
aagaggcagg cagtggagtc tccctgtcga cagtaaagtt gaaatggtga 900
cgtccactgc tggctttatt gaacagctaa taaagattta tttattgtaa 950
tacctcacag acgttgcacc atatccatgc acatttagtt gcctgcctgt 1000
ggctggtaag gtaatgtcat gattcatcct ctcttcagtg agactgagcc 1050
tgatgtgtta acaaataggt gaagaaagtc ttgtgctgta ttcctaatca 1100
aaagacttaa tatattgaag taacactttt ttagtaagca agataccttt 1150
ttatttcaat tcacagaatg gaattttttt gtttcatgtc tcagatttat 1200
tttgtatttc ttttttaaca ctctacattt cccttgtttt ttaactcatg 1250
cacatgtgct ctttgtacag ttttaaaaag tgtaataaaa tctgacatgt 1300
caatgtggct agttttattt ttcttgtttt gcattatgtg tatggcctga 1350
agtgttggac ttgcaaaagg ggaagaaagg aattgcgaat acatgtaaaa 1400
tgtcaccaga catttgtatt atttttatca tgaaatcatg tttttctctg 1450
attgttctga aatgttctaa atactcttat tttgaatgca caaaatgact 1500
taaaccattc atatcatgtt tcctttgcgt tcagccaatt tcaattaaaa 1550
tgaactaaat taaaaaaaaa aaaaaaaaaa aaaaaaaaaa a 1591

<210> 94
<211> 234
<212> PRT
<213> Homo sapiens

<400> 94
Met Asn His Leu Pro Glu Asp Met Glu Asn Ala Leu Thr Gly Ser

1               5                   10                  15

Gln Ser Ser His Ala Ser Leu Arg Asn Ile His Ser Ile Asn Pro
                20                  25                  30

Thr Gln Leu Met Ala Arg Ile Glu Ser Tyr Glu Gly Arg Glu Lys
                35                  40                  45

Lys Gly Ile Ser Asp Val Arg Arg Thr Phe Cys Leu Phe Val Thr
                50                  55                  60

Phe Asp Leu Leu Phe Val Thr Leu Leu Trp Ile Ile Glu Leu Asn
                65                  70                  75

Val Asn Gly Gly Ile Glu Asn Thr Leu Glu Lys Glu Val Met Gln
                80                  85                  90

Tyr Asp Tyr Tyr Ser Ser Tyr Phe Asp Ile Phe Leu Leu Ala Val
                95                  100                 105

Phe Arg Phe Lys Val Leu Ile Leu Ala Tyr Ala Val Cys Arg Leu
                110                 115                 120

Arg His Trp Trp Ala Ile Ala Leu Thr Thr Ala Val Thr Ser Ala
                125                 130                 135

Phe Leu Leu Ala Lys Val Ile Leu Ser Lys Leu Phe Ser Gln Gly
                140                 145                 150

Ala Phe Gly Tyr Val Leu Pro Ile Ile Ser Phe Ile Leu Ala Trp
                155                 160                 165

Ile Glu Thr Trp Phe Leu Asp Phe Lys Val Leu Pro Gln Glu Ala
                170                 175                 180

Glu Glu Glu Asn Arg Leu Leu Ile Val Gln Asp Ala Ser Glu Arg
                185                 190                 195

Ala Ala Leu Ile Pro Gly Gly Leu Ser Asp Gly Gln Phe Tyr Ser
                200                 205                 210

Pro Pro Glu Ser Glu Ala Gly Ser Glu Glu Ala Glu Glu Lys Gln
                215                 220                 225

Asp Ser Glu Lys Pro Leu Leu Glu Leu
                230

<210> 95
<211> 1399
<212> DNA
<213> Homo sapiens

<220>
<221> unsure
<222> 210
<223> unknown base

<400> 95
gtcgtatttc caaggactcc aaagcgaggc cggggactga aggtgtgggt 50

gtcgagccct ctggcagagg gttaacctgg gtcaaatgca cggattctca 100

cctcgtacag ttacgctctc ccgcggcacg tccgaaggat ttggaagtcc 150

tgagcgctca agtttgtccg tagtcgagag aaggccatgg aggtgccgcc 200

accggacgcn gggagctttc tctgtagagc attgtgccta tttccccgag 250

tctttgctgc cgaagctgtg actgccgatt cggaagtcct tgaggagcgt 300
cagaagcggc ttccctacgt cccagagccc tattacccgg aatctggatg 350
ggaccgcctc cgggagctgt ttggcaaaga tgaacagcag agaatttcaa 400
aggaccttgc taatatctgt aagacggcag ctacagcagg catcattggc 450
tgggtgtatg ggggaatacc agcttttatt catgctaaac aacaatacat 500
tgagcagagc caggcagaaa tttatcataa ccggtttgat gctgtgcaat 550
ctgcacatcg tgctgccaca cgaggcttca ttcgttatgg ctggcgctgg 600
ggttggagaa ctgcagtgtt tgtgactata ttcaacacag tgaacactag 650
tctgaatgta taccgaaata aagatgcctt aagccatttt gtaattgcag 700
gagctgtcac gggaagtctt tttaggataa acgtaggcct gcgtggcctg 750
gtggctggtg gcataattgg agccttgctg ggcactcctg taggaggcct 800
gctgatggca tttcagaagt actctggtga gactgttcag gaaagaaaac 850
agaaggatcg aaaggcactc catgagctaa aactggaaga gtggaaaggc 900
agactacaag ttactgagca cctccctgag aaaattgaaa gtagtttaca 950
ggaagatgaa cctgagaatg atgctaagaa aattgaagca ctgctaaacc 1000
ttcctagaaa cccttcagta atagataaac aagacaagga ctgaaagtgc 1050
tctgaacttg aaactcactg gagagctgaa gggagctgcc atgtccgatg 1100
aatgccaaca gacaggccac tctttggtca gcctgctgac aaatttaagt 1150
gctggtacct gtggtggcag tggcttgctc ttgtcttttt cttttctttt 1200
taactaagaa tggggctgtt gtactctcac tttacttatc cttaaattta 1250
aatacatact tatgtttgta ttaatctatc aatatatgca tacatgaata 1300
tatccaccca cctagatttt aagcagtaaa taaaacattt cgcaaaagat 1350
taaagttgaa ttttacagtt aaaaaaaaaa aaaaaaaaaa aaaaaaaaa 1399

<210> 96
<211> 285
<212> PRT
<213> Homo sapiens

<400> 96
Met Glu Val Pro Pro Pro Asp Ala Gly Ser Phe Leu Cys Arg Ala
1               5                   10                  15

Leu Cys Leu Phe Pro Arg Val Phe Ala Ala Glu Ala Val Thr Ala
                20                  25                  30

Asp Ser Glu Val Leu Glu Glu Arg Gln Lys Arg Leu Pro Tyr Val
                35                  40                  45

Pro Glu Pro Tyr Tyr Pro Glu Ser Gly Trp Asp Arg Leu Arg Glu
                50                  55                  60

Leu Phe Gly Lys Asp Glu Gln Gln Arg Ile Ser Lys Asp Leu Ala
                65                  70                  75

Asn Ile Cys Lys Thr Ala Ala Thr Ala Gly Ile Ile Gly Trp Val

80                  85                  90

Tyr Gly Gly Ile Pro Ala Phe Ile His Ala Lys Gln Gln Tyr Ile
                95                  100                 105

Glu Gln Ser Gln Ala Glu Ile Tyr His Asn Arg Phe Asp Ala Val
                110                 115                 120

Gln Ser Ala His Arg Ala Ala Thr Arg Gly Phe Ile Arg Tyr Gly
                125                 130                 135

Trp Arg Trp Gly Trp Arg Thr Ala Val Phe Val Thr Ile Phe Asn
                140                 145                 150

Thr Val Asn Thr Ser Leu Asn Val Tyr Arg Asn Lys Asp Ala Leu
                155                 160                 165

Ser His Phe Val Ile Ala Gly Ala Val Thr Gly Ser Leu Phe Arg
                170                 175                 180

Ile Asn Val Gly Leu Arg Gly Leu Val Ala Gly Gly Ile Ile Gly
                185                 190                 195

Ala Leu Leu Gly Thr Pro Val Gly Gly Leu Leu Met Ala Phe Gln
                200                 205                 210

Lys Tyr Ser Gly Glu Thr Val Gln Glu Arg Lys Gln Lys Asp Arg
                215                 220                 225

Lys Ala Leu His Glu Leu Lys Leu Glu Glu Trp Lys Gly Arg Leu
                230                 235                 240

Gln Val Thr Glu His Leu Pro Glu Lys Ile Glu Ser Ser Leu Gln
                245                 250                 255

Glu Asp Glu Pro Glu Asn Asp Ala Lys Lys Ile Glu Ala Leu Leu
                260                 265                 270

Asn Leu Pro Arg Asn Pro Ser Val Ile Asp Lys Gln Asp Lys Asp
                275                 280                 285

<210> 97
<211> 1816
<212> DNA
<213> Homo sapiens

<400> 97
gcacgagcga tgtcgctcgt gctgctaagc ctggccgcgc tgtgcaggag 50

cgccgtaccc cgagagccga ccgttcaatg tggctctgaa actgggccat 100

ctccagagtg gatgctacaa catgatctaa tccccggaga cttgagggac 150

ctccgagtag aacctgttac aactagtgtt gcaacagggg actattcaat 200

tttgatgaat gtaagctggg tactccgggc agatgccagc atccgcttgt 250

tgaaggccac caagatttgt gtgacgggca aaagcaactt ccagtcctac 300

agctgtgtga ggtgcaatta cacagaggcc ttccagactc agaccagacc 350

ctctggtggt aaatggacat tttcctacat cggcttccct gtagagctga 400

acacagtcta tttcattggg gcccataata ttcctaatgc aaatatgaat 450

gaagatggcc cttccatgtc tgtgaatttc acctcaccag gctgcctaga 500

ccacataatg aaatataaaa aaaagtgtgt caaggccgga agcctgtggg 550

atccgaacat cactgcttgt aagaagaatg aggagacagt agaagtgaac 600
ttcacaacca ctcccctggg aaacagatac atggctctta tccaacacag 650
cactatcatc gggttttctc aggtgtttga gccacaccag aagaaacaaa 700
cgcgagcttc agtggtgatt ccagtgactg gggatagtga aggtgctacg 750
gtgcagctga ctccatattt tcctacttgt ggcagcgact gcatccgaca 800
taaaggaaca gttgtgctct gcccacaaac aggcgtccct ttccctctgg 850
ataacaacaa aagcaagccg ggaggctggc tgcctctcct cctgctgtct 900
ctgctggtgg ccacatgggt gctggtggca gggatctatc taatgtggag 950
gcacgaaagg atcaagaaga cttccttttc taccaccaca ctactgcccc 1000
ccattaaggt tcttgtggtt tacccatctg aaatatgttt ccatcacaca 1050
atttgttact tcactgaatt tcttcaaaac cattgcagaa gtgaggtcat 1100
ccttgaaaag tggcagaaaa agaaaatagc agagatgggt ccagtgcagt 1150
ggcttgccac tcaaaagaag gcagcagaca aagtcgtctt ccttctttcc 1200
aatgacgtca acagtgtgtg cgatggtacc tgtggcaaga gcgagggcag 1250
tcccagtgag aactctcaag actcttcccc ttgcctttaa ccttttctgc 1300
agtgatctaa gaagccagat tcatctgcac aaatacgtgg tggtctactt 1350
tagagagatt gatacaaaag acgattacaa tgctctcagt gtctgcccca 1400
agtaccacct catgaaggat gccactgctt tctgtgcaga acttctccat 1450
gtcaagtagc aggtgtcagc aggaaaaaga tcacaagcct gccacgatgg 1500
ctgctgctcc ttgtagccca cccatgagaa gcaagagacc ttaaaggctt 1550
cctatcccac caattacagg gaaaaaacgt gtgatgatcc tgaagcttac 1600
tatgcagcct acaaacagcc ttagtaatta aaacatttta taccaataaa 1650
attttcaaat attgctaact aatgtagcat taactaacga ttggaaacta 1700
catttacaac ttcaaagctg ttttatacat agaaatcaat tacagtttta 1750
attgaaaact ataaccattt tgataatgca acaataaagc atcttcagcc 1800
aaaaaaaaaa aaaaaa 1816

<210> 98
<211> 426
<212> PRT
<213> Homo sapiens

<400> 98
Met Ser Leu Val Leu Leu Ser Leu Ala Ala Leu Cys Arg Ser Ala
1               5                   10                  15

Val Pro Arg Glu Pro Thr Val Gln Cys Gly Ser Glu Thr Gly Pro
                20                  25                  30

Ser Pro Glu Trp Met Leu Gln His Asp Leu Ile Pro Gly Asp Leu
                35                  40                  45

Arg Asp Leu Arg Val Glu Pro Val Thr Thr Ser Val Ala Thr Gly
                50                  55                  60

Asp Tyr Ser Ile Leu Met Asn Val Ser Trp Val Leu Arg Ala Asp
                65                  70                  75

Ala Ser Ile Arg Leu Leu Lys Ala Thr Lys Ile Cys Val Thr Gly
                80                  85                  90

Lys Ser Asn Phe Gln Ser Tyr Ser Cys Val Arg Cys Asn Tyr Thr
                95                  100                 105

Glu Ala Phe Gln Thr Gln Thr Arg Pro Ser Gly Gly Lys Trp Thr
                110                 115                 120

Phe Ser Tyr Ile Gly Phe Pro Val Glu Leu Asn Thr Val Tyr Phe
                125                 130                 135

Ile Gly Ala His Asn Ile Pro Asn Ala Asn Met Asn Glu Asp Gly
                140                 145                 150

Pro Ser Met Ser Val Asn Phe Thr Ser Pro Gly Cys Leu Asp His
                155                 160                 165

Ile Met Lys Tyr Lys Lys Lys Cys Val Lys Ala Gly Ser Leu Trp
                170                 175                 180

Asp Pro Asn Ile Thr Ala Cys Lys Lys Asn Glu Glu Thr Val Glu
                185                 190                 195

Val Asn Phe Thr Thr Thr Pro Leu Gly Asn Arg Tyr Met Ala Leu
                200                 205                 210

Ile Gln His Ser Thr Ile Ile Gly Phe Ser Gln Val Phe Glu Pro
                215                 220                 225

His Gln Lys Lys Gln Thr Arg Ala Ser Val Val Ile Pro Val Thr
                230                 235                 240

Gly Asp Ser Glu Gly Ala Thr Val Gln Leu Thr Pro Tyr Phe Pro
                245                 250                 255

Thr Cys Gly Ser Asp Cys Ile Arg His Lys Gly Thr Val Val Leu
                260                 265                 270

Cys Pro Gln Thr Gly Val Pro Phe Pro Leu Asp Asn Asn Lys Ser
                275                 280                 285

Lys Pro Gly Gly Trp Leu Pro Leu Leu Leu Leu Ser Leu Leu Val
                290                 295                 300

Ala Thr Trp Val Leu Val Ala Gly Ile Tyr Leu Met Trp Arg His
                305                 310                 315

Glu Arg Ile Lys Lys Thr Ser Phe Ser Thr Thr Thr Leu Leu Pro
                320                 325                 330

Pro Ile Lys Val Leu Val Val Tyr Pro Ser Glu Ile Cys Phe His
                335                 340                 345

His Thr Ile Cys Tyr Phe Thr Glu Phe Leu Gln Asn His Cys Arg
                350                 355                 360

Ser Glu Val Ile Leu Glu Lys Trp Gln Lys Lys Lys Ile Ala Glu
                365                 370                 375

Met Gly Pro Val Gln Trp Leu Ala Thr Gln Lys Lys Ala Ala Asp
                380                 385                 390

Lys Val Val Phe Leu Leu Ser Asn Asp Val Asn Ser Val Cys Asp
                395                 400                 405

Gly Thr Cys Gly Lys Ser Glu Gly Ser Pro Ser Glu Asn Ser Gln
                410                 415                 420

Asp Ser Ser Pro Cys Leu
                425

<210> 99
<211> 2946
<212> DNA
<213> Homo sapiens

<400> 99
   cagcttccca ccctgggctt tccgaggtgc tttcgccgct gtccccacca 50
   ctgcagccat gatctcctta acggacacgc agaaaattgg aatgggatta 100
   acaggatttg gagtgttttt cctgttcttt ggaatgattc tcttttttga 150
   caaagcacta ctggctattg gaaatgtttt atttgtagcc ggcttggctt 200
   ttgtaattgg tttagaaaga acattcagat tcttcttcca aaaacataaa 250
   atgaaagcta caggtttttt tctgggtggt gtatttgtag tccttattgg 300
   ttggcctttg ataggcatga tcttcgaaat ttatggattt tttctcttgt 350
   tcaggggctt ctttcctgtc gttgttggct ttattagaag agtgccagtc 400
   cttggatccc tcctaaattt acctggaatt agatcatttg tagataaagt 450
   tggagaaagc aacaatatgg tataacaaca agtgaatttg aagactcatt 500
   taaaatattg tgttatttat aaagtcattt gaagaatatt cagcacaaaa 550
   ttaaattaca tgaaatagct tgtaatgttc tttacaggag tttaaaacgt 600
   atagcctaca aagtaccagc agcaaattag caaagaagca gtgaaaacag 650
   gcttctactc aagtgaacta agaagaagtc agcaagcaaa ctgagagagg 700
   tgaaatccat gttaatgatg cttaagaaac tcttgaaggc tatttgtgtt 750
   gtttttccac aatgtgcgaa actcagccat ccttagagaa ctgtggtgcc 800
   tgtttctttt ctttttattt tgaaggctca ggagcatcca taggcatttg 850
   ctttttagaa gtgtccactg caatggcaaa aatatttcca gttgcactgt 900
   atctctggaa gtgatgcatg aattcgattg gattgtgtca ttttaaagta 950
   ttaaaaccaa ggaaacccca attttgatgt atggattact tttttttgta 1000
   aacatggtta aaataaaact tttgtggttc ttctgaatct taatatttca 1050
   aagccaggtg aaaatctgaa ctagatattc tttgttggaa tatgcaaagg 1100
   tcattcttta ctaactttta gttactaaat tatagctaag ttttgtcagc 1150
   agcatactcc ggaaagtctc atacttcttg ggagtctgcc ctcctaagta 1200
   tctgtctata tcattcatta cgtgtaagta tttaacaaaa aagcattctt 1250
   gaccatgaat gaagtagttt gtttcatagc ttgtctcatt gaatagtatt 1300
   attgaagata ctaaatgatg caaaccaaat ggattttttc catgtcatga 1350
   tgtaattttt ctttcttctt tctttttttt aaattttagc agtggcttat 1400
   tatttgtttt tcataaatta aaataacttt tgataatgtt tactttaaga 1450
   catgtaacat gttaaaaggt taaacttatg gctgttttta aagggctatt 1500
   catttaatct gagttttccc ttattttcag ctttttccta gcatataata 1550
   gtcattaagc atgacatatc cttcatatga tcactcatct tgagttaatt 1600
   agaaaatacc tgagttcacg tgctaaagtc atttcactgt aataaactga 1650
   ctatggtttc ttaagaacat gacactaaaa aaaaaagtgg tttttttcca 1700
   ccgttgctga ttattagaca gtaggaaata gctgttttct ttagttttac 1750
   aagatgtgac agctttagtg gtagatgtag ggaaacattt caacagccat 1800
   agtactattt gttttaccac tgattgcact attttgtttt tttaacagtt 1850
   gcaaagcttt ttaatgcata aaagtataat tgaaatctgt ggtatttatt 1900
   tacaaacatg tctacaaaaa tagattacag cttattttat ttttagttaa 1950
   atctcttaat acacagagaa ctcccaatct tgctcatcta aataaggaaa 2000
   gacttggtgt atagtgtgat ggtttagtct taaggattaa gacatttttg 2050
   gtacttgcat ttgacttacg atgtatctgt gaaaatggga tgatattgac 2100
   aaatggagac tcctacctca atagttaatg gaataataag aggctactgt 2150
   tgtgtctaat gttcttcaaa aaagtaatat cctcacttgg agagtgtcaa 2200
   atacatactt tgaggattga ctttatataa ggtgccctgt agaactctgt 2250
   tacacatatt tttgacccat attatttaca atgtcttgat aattctacct 2300
   ttttagagca agaatagtat ctgctaatgt aagggacatc tgtatttaac 2350
   tcctttgtag acatgaattt ctatcaaaat gttctttgca ctgtaacaga 2400
   gattcctttt ttcaataatc ttaattcaaa agcattatta gacttgaaag 2450
   ggtttgataa tctcccagtc cttagtaaag attgagagag gctggagcag 2500
   ttttcagttt taaatgagtc tgcagttaat atcaaatgtg agtttgggac 2550
   tgcctggcaa catttatatt tcttattcag aacccttgat gagactattt 2600
   ttaaacatac tagtctgctg atagaaagca ctatacatcc tattgtttct 2650
   ttctttccaa aatcagcctt ctgtctgtaa caaaaatgta ctttatagag 2700
   atggaggaaa aggtctaata ctacatagcc ttaagtgttt ctgtcattgt 2750
   tcaagtgtat tttctgtaac agaaacatat ttggaatgtt tttcttttcc 2800
   ccttataaat tgtaattcct gaaatactgc tgctttaaaa agtcccactg 2850
   tcagattata ttatctaaca attgaatatt gtaaatatac ttgtcttacc 2900
   tctcaataaa agggtacttt tctatcaaaa aaaaaaaaaa aaaaaa 2946

<210> 100
<211> 138

<212> PRT
<213> Homo sapiens

<400> 100
Met Ile Ser Leu Thr Asp Thr Gln Lys Ile Gly Met Gly Leu Thr
1               5                   10                  15

Gly Phe Gly Val Phe Phe Leu Phe Phe Gly Met Ile Leu Phe Phe
                20                  25                  30

Asp Lys Ala Leu Leu Ala Ile Gly Asn Val Leu Phe Val Ala Gly
                35                  40                  45

Leu Ala Phe Val Ile Gly Leu Glu Arg Thr Phe Arg Phe Phe Phe
                50                  55                  60

Gln Lys His Lys Met Lys Ala Thr Gly Phe Phe Leu Gly Gly Val
                65                  70                  75

Phe Val Val Leu Ile Gly Trp Pro Leu Ile Gly Met Ile Phe Glu
                80                  85                  90

Ile Tyr Gly Phe Phe Leu Leu Phe Arg Gly Phe Phe Pro Val Val
                95                  100                 105

Val Gly Phe Ile Arg Arg Val Pro Val Leu Gly Ser Leu Leu Asn
                110                 115                 120

Leu Pro Gly Ile Arg Ser Phe Val Asp Lys Val Gly Glu Ser Asn
                125                 130                 135

Asn Met Val


<210> 101
<211> 2747
<212> DNA
<213> Homo sapiens

<400> 101
attgattaaa aagagattgc cctgcaaggt aaatcagtta aaaccaacct 50

ctcctgccct gagtggatag gtagggttag ggttgccaga tgtcacgaag 100

ttacaggatg ctcagtttta aggtatatcc cttatactat aagggttata 150

gtaaaaaata ttcattatgt gaaattcaaa tataactggg tatcaggtat 200

tctatgtggc aaccctaggt aggggagcac aggttaggca agcgattaga 250

agatttgcag cctccaaagt ttctgcacct cgatgggaca ctagaacagg 300

aaggctcctg ggcctttctg gctctgggaa tgaagcgtgg aaaaccctcc 350

ttaggcgggc gcagtgcttc aagtagccaa gctctgactt ccgagggaag 400

aaaggaggcc atgggcctct gccagagcca tgctctgcac tctggggtca 450

gcagagttca aaacgacctg caacgtctgg cgcttagctc ctaaagaggt 500

ctccagtcca gcgccgacgg ccagcggcta gaggccgtcc gcccgactcc 550

aagatggcgc ccgccacagc tgccaggtgt taagatggcg gcgcggggcc 600

gcgcccgcgc tcccaggctc tcctccccca gccttcctcc ggctggcagc 650

acgactcgcg tagccgtgcg ccgattgcct ctcggcctgg gcaatggtcc 700

cggctgccgg tcgacgaccg ccccgcgtca tgcggctcct cggctggtgg 750
caagtattgc tgtgggtgct gggacttccc gtccgcggcg tggaggttgc 800
agaggaaagt ggtcgcttat ggtcggagga gcagcctgct caccctctcc 850
aggtgggggc tgtgtacctg ggtgaggagg agctcctgca tgacccgatg 900
ggccaggaca gggcagcaga agaggccaat gcggtgctgg ggctgggcac 950
ccaaggcgat cacatggtga tgctgtctgt gattcctggg gaagctgagg 1000
acaaagtgag ttcagagcct agcggcgtca cctgtggtgc tggaggagcg 1050
gaggactcaa ggtgcaacgt ccgagagagc cttttctctc tggatggcgc 1100
tggagcacac ttccctgaca gagaagagga gtattacaca gagccagaag 1150
tggcggaatc tgacgcagcc ccgacagagg actccaataa cactgaaagt 1200
ctgaaatccc caaaggtgaa ctgtgaggag agaaacatta caggattaga 1250
aaatttcact ctgaaaattt taaatatgtc acaggacctt atggattttc 1300
tgaacccaaa cggtagtgac tgtactctag tcctgtttta caccccgtgg 1350
tgccgctttt ctgccagttt ggcccctcac tttaactctc tgccccgggc 1400
atttccagct cttcactttt tggcactgga tgcatctcag cacagcagcc 1450
tttctaccag gtttggcacc gtagctgttc ctaatatttt attatttcaa 1500
ggagctaaac caatggccag atttaatcat acagatcgaa cactggaaac 1550
actgaaaatc ttcattttta atcagacagg tatagaagcc aagaagaatg 1600
tggtggtaac tcaagccgac caaataggcc ctcttcccag cactttgata 1650
aaaagtgtgg actggttgct tgtattttcc ttattctttt taattagttt 1700
tattatgtat gctaccattc gaactgagag tattcggtgg ctaattccag 1750
gacaagagca ggaacatgtg gagtagtgat ggtctgaaag aagttggaaa 1800
gaggaacttc aatccttcgt ttcagaaatt agtgctacag tttcatacat 1850
tttctccagt gacgtgttga cttgaaactt caggcagatt aaaagaatca 1900
tttgttgaac aactgaatgt ataaaaaaaa ttataaactg gtgttttaac 1950
tagtattgca ataagcaaat gcaaaaatat tcaatagatg cactattctt 2000
gtttttactg catgaacgta atccagtatt tggaaagtaa tccagtttga 2050
aatgtgaaga tgtattccgg cagaatagtg agtagaatga catgcttact 2100
atacagaagg caaaaatagg actctcaggt aatagtttaa ggaaaccctt 2150
gattccttat atatgtttaa gaaggttagc tttctgtttc tttgcagttt 2200
ttcttctaga gtccatagca ggaaagtatg taaccagaat tggttagtgt 2250
gaccccctca agtagcaagt gatggaaaat aagagtcaaa taccttgatg 2300
tttgtgatct ctaactcaaa aaatttgaag tgttttaagt tgtttctggg 2350
taagggagat gttaggagaa aggaaatgct gtaactaaag ctcaattatt 2400

atcagttcta tgctaacgta tacattttaa tcatagttac ctaagcagca 2450

tgcattaatt gaaccttaaa atgttcccag caggctggtc tcaaactgct 2500

gacttcaggt gatccacccg cctcggcctc ccaaggtgtt gggattgcag 2550

gtgtgagcca ctgcgcctgg cctaaacaaa ctttttgaaa agctgtttct 2600

aaaagattcc ttaaattcag atatgacagc taattacctc atcataaatt 2650

acttttatac taattgtttc cagggtttta gagtagttga atgtttattt 2700

cacaaggcac cctaaattct atagaaataa aacctcagat gagtctc 2747

<210> 102
<211> 343
<212> PRT
<213> Homo sapiens

<400> 102
Met Val Pro Ala Ala Gly Arg Arg Pro Pro Arg Val Met Arg Leu
1               5                   10                  15

Leu Gly Trp Trp Gln Val Leu Leu Trp Val Leu Gly Leu Pro Val
                20                  25                  30

Arg Gly Val Glu Val Ala Glu Glu Ser Gly Arg Leu Trp Ser Glu
                35                  40                  45

Glu Leu Leu His Asp Pro Met Gly Arg Asp Arg Ala Ala Glu Glu
                50                  55                  60

Ala Asn Ala Val Leu Gly Leu Asp Thr Gln Gly Asp His Met Val
                65                  70                  75

Met Leu Ser Val Ile Pro Gly Glu Ala Glu Asp Lys Val Ser Ser
                80                  85                  90

Glu Pro Ser Gly Val Thr Cys Gly Ala Gly Gly Ala Glu Asp Ser
                95                  100                 105

Arg Cys Asn Val Arg Glu Ser Leu Phe Ser Leu Asp Gly Ala Gly
                110                 115                 120

Ala His Phe Pro Asp Arg Glu Glu Glu Tyr Tyr Thr Glu Pro Glu
                125                 130                 135

Val Ala Glu Ser Asp Ala Ala Pro Thr Glu Asp Ser Asn Asn Thr
                140                 145                 150

Glu Ser Leu Lys Ser Pro Lys Val Asn Cys Glu Glu Arg Asn Ile
                155                 160                 165

Thr Gly Leu Glu Asn Phe Thr Leu Lys Ile Leu Asn Met Ser Gln
                170                 175                 180

Asp Leu Met Asp Phe Leu Asn Pro Asn Gly Ser Asp Cys Thr Leu
                185                 190                 195

Val Leu Phe Tyr Thr Pro Trp Cys Arg Phe Ser Ala Ser Leu Ala
                200                 205                 210

Pro His Phe Asn Ser Leu Pro Arg Ala Phe Pro Ala Leu His Phe
                215                 220                 225

Leu Ala Leu Asp Ala Ser Gln His Ser Ser Leu Ser Thr Arg Phe
                230                 235                 240

Gly Thr Val Ala Val Pro Asn Ile Leu Leu Phe Gln Gly Ala Lys
                245                 250                 255

Pro Met Ala Arg Phe Asn His Thr Asp Arg Thr Leu Glu Thr Leu
                260                 265                 270

Lys Ile Phe Ile Phe Asn Gln Thr Gly Ile Glu Ala Lys Lys Asn
                275                 280                 285

Val Val Val Thr Gln Ala Asp Gln Ile Gly Pro Leu Pro Ser Thr
                290                 295                 300

Leu Ile Lys Ser Val Asp Trp Leu Leu Val Phe Ser Leu Phe Phe
                305                 310                 315

Leu Ile Ser Phe Ile Met Tyr Ala Thr Ile Arg Thr Glu Ser Ile
                320                 325                 330

Arg Trp Leu Ile Pro Gly Gln Glu Gln Glu His Val Glu
                335                 340

<210> 103
<211> 2058
<212> DNA
<213> Homo sapiens

<400> 103
ggcacgaggc ctgggttgcg ctgccggcca cgtccccgcg ccgggcctca 50

ggctccttcc tactgtccga gggccaccag gccgccgggg gcctgctgcg 100

cccggatgcg tctgttacta gagtggagag tctaccttcg tctcacatgt 150

gccacaaagg atggcatggc ccgggagtgc cccaccacgt ggctttcacc 200

ccctgcaaag ccagacttcg cccagcgaca cagtgtcaag cccacagctc 250

tccaaggagg aagatggtcc aggctgggag catcccctta gcagcagcct 300

ctgatccctt ggccaagcag gagggaacca ttagcagcct gaggagctgg 350

ctggctggga gcctcgggga ccgcccagcc ttgctcccag ctcacccaca 400

agatgtggac agctcttgtg ctcatttgga ttttctcctt gtccttatct 450

gaaagccatg cggcatccaa cgatccacgc aactttgtcc ctaacaaaat 500

gtggaaggga ttagtcaaga ggaatgcatc tgtggaaaca gttgataata 550

aaacgtctga ggatgtaacc atggcagcag cttctcctgt cacattgacc 600

aaagggactt cggcagccca cctcaactct atggaagtca caacagagga 650

cacaagcagg acagatgtga gtgaaccagc aacttcagga ggtgcagctg 700

atggtgtgac ctccattgct cccacggctg tggcctccag tacgactgcg 750

gcctccatta cgactgcggc ctccagtatg actgtggcct ccagtgctcc 800

cacgactgca gcctccagta caactgtggc ctccattgct cccacgactg 850

cagcctccag tatgactgcg gcctccagca ctcccatgac acttgcactc 900

cccgcgccca cgtccacttc cacagggcgg accccgtcca ctaccgccac 950

tgggcatcca tctctcagca cagccctcgc acaagtgcca aagagcagcg 1000

cgttgccaag aacagcaacc ctggccacat tggccacacg tgctcagact 1050

gtagcgacca cagcaaacac aagcagcccc atgagcactc gtccaagtcc 1100

ttccaagcac atgcccagtg acaccgcggc aagccctgta ccccctatgc 1150

gtccccaagc acaaggtccc attagccagg tgtcagtgga ccagcctgtg 1200

gttaacacaa caaataaatc cacacccatg ccctcaaaca caaccccaga 1250

gcccgccccc acccccacag tggtgaccac caccaaggca caagccaggg 1300

agccaactgc cagcccagtg ccagtacctc acaccagccc aatccctgag 1350

atggaggcca tgtcccccac gacacagcca agccccatgc catataccca 1400

gagggccgct gggccaggca catcccaggc accggagcag gtagagactg 1450

aagccacacc aggtactgat tccactgggc caacacccag gagctcaggg 1500

ggcactaaga tgccagccac ggactcgtgc cagcccagca cccaaggcca 1550

gtacatggtg gtcaccactg agcccctcac ccaggccgtg gtagacaaaa 1600

ctctccttct ggtggtgctg ttactcgggg tgaccctttt catcacagtc 1650

ttggttttgt ttgccctgca ggcctatgag agctacaaga agaaggacta 1700

cacccaggtg gactacttaa tcaacgggat gtatgcggac tcagaaatgt 1750

gaggggggcg ggggcctggc gggaggcctg gccccttcct cgtcctttcc 1800

ttttgccttt gagaccaaac caagtgcttc caaattcttt tggtgcaatt 1850

gaggagatat gccagatgct taaacacatt taattgctgt cagattaatt 1900

ccatgatcac taaagagttg ctgctttttt catatttatt tttgtaaatg 1950

attctgtgcc caggagcagc tgggggttcc acctcagggt ggggcgggca 2000

ggaccccgtc tccccaggtg tcggagcctg acctgaatta aagtactgac 2050

tgctcgcc 2058

<210> 104
<211> 449
<212> PRT
<213> Homo sapiens

<400> 104
Met Trp Thr Ala Leu Val Leu Ile Trp Ile Phe Ser Leu Ser Leu
1               5                   10                  15

Ser Glu Ser His Ala Ala Ser Asn Asp Pro Arg Asn Phe Val Pro
                20                  25                  30

Asn Lys Met Trp Lys Gly Leu Val Lys Arg Asn Ala Ser Val Glu
                35                  40                  45

Thr Val Asp Asn Lys Thr Ser Glu Asp Val Thr Met Ala Ala Ala
                50                  55                  60

Ser Pro Val Thr Leu Thr Lys Gly Thr Ser Ala Ala His Leu Asn
                65                  70                  75

Ser Met Glu Val Thr Thr Glu Asp Thr Ser Arg Thr Asp Val Ser
                80                  85                  90

Glu Pro Ala Thr Ser Gly Gly Ala Ala Asp Gly Val Thr Ser Ile

                95                  100                 105
Ala Pro Thr Ala Val Ala Ser Ser Thr Thr Ala Ala Ser Ile Thr
                110                 115                 120
Thr Ala Ala Ser Ser Met Thr Val Ala Ser Ser Ala Pro Thr Thr
                125                 130                 135
Ala Ala Ser Ser Thr Thr Val Ala Ser Ile Ala Pro Thr Thr Ala
                140                 145                 150
Ala Ser Ser Met Thr Ala Ala Ser Ser Thr Pro Met Thr Leu Ala
                155                 160                 165
Leu Pro Ala Pro Thr Ser Thr Ser Thr Gly Arg Thr Pro Ser Thr
                170                 175                 180
Thr Ala Thr Gly His Pro Ser Leu Ser Thr Ala Leu Ala Gln Val
                185                 190                 195
Pro Lys Ser Ser Ala Leu Pro Arg Thr Ala Thr Leu Ala Thr Leu
                200                 205                 210
Ala Thr Arg Ala Gln Thr Val Ala Thr Thr Ala Asn Thr Ser Ser
                215                 220                 225
Pro Met Ser Thr Arg Pro Ser Pro Ser Lys His Met Pro Ser Asp
                230                 235                 240
Thr Ala Ala Ser Pro Val Pro Pro Met Arg Pro Gln Ala Gln Gly
                245                 250                 255
Pro Ile Ser Gln Val Ser Val Asp Gln Pro Val Val Asn Thr Thr
                260                 265                 270
Asn Lys Ser Thr Pro Met Pro Ser Asn Thr Thr Pro Glu Pro Ala
                275                 280                 285
Pro Thr Pro Thr Val Val Thr Thr Thr Lys Ala Gln Ala Arg Glu
                290                 295                 300
Pro Thr Ala Ser Pro Val Pro Val Pro His Thr Ser Pro Ile Pro
                305                 310                 315
Glu Met Glu Ala Met Ser Pro Thr Thr Gln Pro Ser Pro Met Pro
                320                 325                 330
Tyr Thr Gln Arg Ala Ala Gly Pro Gly Thr Ser Gln Ala Pro Glu
                335                 340                 345
Gln Val Glu Thr Glu Ala Thr Pro Gly Thr Asp Ser Thr Gly Pro
                350                 355                 360
Thr Pro Arg Ser Ser Gly Gly Thr Lys Met Pro Ala Thr Asp Ser
                365                 370                 375
Cys Gln Pro Ser Thr Gln Gly Gln Tyr Met Val Val Thr Thr Glu
                380                 385                 390
Pro Leu Thr Gln Ala Val Val Asp Lys Thr Leu Leu Leu Val Val
                395                 400                 405
Leu Leu Leu Gly Val Thr Leu Phe Ile Thr Val Leu Val Leu Phe
                410                 415                 420
Ala Leu Gln Ala Tyr Glu Ser Tyr Lys Lys Lys Asp Tyr Thr Gln
                425                 430                 435

Val Asp Tyr Leu Ile Asn Gly Met Tyr Ala Asp Ser Glu Met
                440                 445

130

130